(19) Europäisches Patentamt European Patent Office Office européen des brevets

(11) EP 2 298 778 A1

(12) EUROPEAN PATENT APPLICATION
published in accordance with Art. 153(4) EPC

(43) Date of publication:
**23.03.2011 Bulletin 2011/12**

(21) Application number: **09762499.3**

(22) Date of filing: **10.06.2009**

(51) Int Cl.:
***C07D 513/04*** (2006.01) ***A61K 31/496*** (2006.01)
***A61P 35/00*** (2006.01) ***A61P 43/00*** (2006.01)

(86) International application number:
**PCT/JP2009/060575**

(87) International publication number:
**WO 2009/151069 (17.12.2009 Gazette 2009/51)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **12.06.2008 JP 2008154036**

(71) Applicant: **Daiichi Sankyo Company, Limited**
**Chuo-ku**
**Tokyo 103-8426 (JP)**

(72) Inventors:
- **UOTO, Kouichi**
**Edogawa-ku**
**Tokyo 134-0081 (JP)**
- **KAWATO, Haruko**
**Edogawa-ku**
**Tokyo 134-0081 (JP)**
- **SUGIMOTO, Yuuichi**
**Edogawa-ku**
**Tokyo 134-0081 (JP)**
- **NAITO, Hiroyuki**
**Edogawa-ku**
**Tokyo 134-0081 (JP)**
- **MIYAZAKI, Masaki**
**Edogawa-ku**
**Tokyo 134-0081 (JP)**
- **TANIGUCHI, Toru**
**Edogawa-ku**
**Tokyo 134-0081 (JP)**
- **AONUMA, Masashi**
**Edogawa-ku**
**Tokyo 134-0081 (JP)**

(74) Representative: **Wallace, Sheila Jane**
**Marks & Clerk LLP**
**90 Long Acre**
**London**
**WC2E 9RA (GB)**

(54) **IMIDAZOTHIAZOLE DERIVATIVE HAVING 4,7-DIAZASPIROÝ2.5¨OCTANE RING STRUCTURE**

(57) There is provided a novel compound that inhibits the interaction between murine double minute 2 (Mdm2) protein and p53 protein and exhibits anti-tumor activity. The present invention provides an imidazothiazole derivative represented by the following formula (1) having various substituents that inhibits the interaction between Mdm2 protein and p53 protein and exhibits anti-tumor activity:

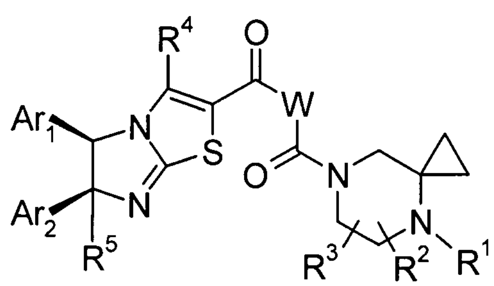

(1)

wherein $R^1$ $R^2$, $R^3$, $R^4$, $R^5$, $Ar_1$, and $Ar_2$ in formula (1) each have the same meanings as defined in the specification.

EP 2 298 778 A1

## Description

### Technical Field

The present invention relates to imidazothiazole derivatives having a 4,7-diazaspiro[2.5]octane ring structure having anti-tumor activity by inhibition of murine double minute 2 (Mdm2).

### Background Art

p53 is known as an important factor for inhibiting canceration of cells. p53 is a transcription factor that induces the expression of genes involved in the cell cycle and cellular apoptosis in response to various stresses. p53 is thought to inhibit canceration of cells by a transcription regulating function thereof. In fact, deletion or mutation of the p53 gene is observed in about half of human cancer cases.

Meanwhile, overexpression of murine double minute 2 (Mdm2), a type of E3 ubiquitin ligase, is known as a factor for canceration of cells that are cancerated in spite of the presence of normal p53. Mdm2 is a protein of which expression is induced by p53. Mdm2 negatively regulates p53 by mediating degradation of p53 by binding to the transcription activity domain of p53 to decrease the transcription activity of p53, exporting p53 out of the nucleus, and further acting as a ubiquitination ligase against p53. Therefore, it is thought that inactivation of functions of and degradation of p53 are promoted in cells in which Mdm2 is overexpressed, resulting in canceration (Non Patent Reference 1).

Paying attention to such functions of Mdm2, many approaches have been attempted to use substances that inhibit the suppression of p53 functions by Mdm2, as candidate anti-tumor agents. As substances that inhibit the suppression of p53 functions by Mdm2, for example, various anti-Mdm2 antisense oligonucleotides (for example, refer to Patent Reference 1), low molecular weight compounds (for example, refer to Non Patent References 1 to 14 and Patent References 2 to 17), and the like have been reported. Recently, Mdm2 inhibitors targeting the Mdm2-p53 binding site have been explored using the results of crystal structure analyses of Mdm2 and p53 (for example, refer to Non Patent References 1, 2, and 4 to 14). Examples of the Mdm2 inhibitors targeting the Mdm2-p53 binding site include imidazoline derivatives having two sites substituted with halogenobenzene (for example, refer to Non Patent References 1 and 2 and Patent References 5 to 11 and 18), benzodiazepine derivatives containing two halogenobenzene moieties in the structure thereof (for example, refer to Non Patent References 4 to 11 and Patent References 12 to 16) or spiro oxindole derivatives containing two halogenobenzene moieties in the structure thereof (for example, refer to Non Patent References 12 to 14 and Patent Reference 19), and so forth. However, no report has demonstrated that these compounds actually showed efficacy in clinical practice.

### Citation list

#### Patent References

Patent Reference 1: WO1999/49065
Patent Reference 2: WO2000/15657
Patent Reference 3: WO2006/24837
Patent Reference 4: WO2004/80460
Patent Reference 5: WO2003/51359
Patent Reference 6: WO2003/51360
Patent Reference 7: WO2005/3097
Patent Reference 8: WO2005/2575
Patent Reference 9: WO2005/11099
Patent Reference 10: WO2005/123691
Patent Reference 11: WO2006/97261
Patent Reference 12: WO2003/41715
Patent Reference 13: WO2003/95625
Patent Reference 14: U.S. Patent Application Publication No. 2004/197893
Patent Reference 15: U.S. Patent Application Publication No. 2004/220179
Patent Reference 16: WO2004/96134
Patent Reference 17: WO2006/91646
Patent Reference 18: WO2007/63013
Patent Reference 19: WO2008/36168

Non Patent References

Non Patent Reference 1: Science, 2004, 303, 844-848
Non Patent Reference 2: Proceedings of the National Academy of Sciences of the United States of America, 2006, 103, 1888-1893
Non Patent Reference 3: Analytical Biochemistry, 2004, 331, 138-146
Non Patent Reference 4: Bioorganic & Medicinal Chemistry Letters, 2005, 15, 765-770
Non Patent Reference 5: Journal of Medicinal Chemistry, 2005, 48, 909-912
Non Patent Reference 6: Chemical Biology & Drug Design, 2006, 67, 201-205
Non Patent Reference 7: Bioorganic & Medicinal Chemistry Letters, 2005, 15, 1857-1861
Non Patent Reference 8: Molecular Cancer Therapeutics, 2006, 5(1), 160-169
Non Patent Reference 9: Bioorganic & Medicinal Chemistry Letters, 2006, 16, 3115-3120
Non Patent Reference 10: Bioorganic & Medicinal Chemistry Letters, 2006, 16, 3310-3314
Non Patent Reference 11: Bioorganic & Medicinal Chemistry Letters, 2006, 16, 3463-3468
Non Patent Reference 12: Journal of the American Chemical Society, 2005, 127, 10130-10131
Non Patent Reference 13: Tetrahedron Letters, 2005, 46, 5949-5951
Non Patent Reference 14: Journal of Medicinal Chemistry, 2006, 49, 3432-3435

Summary of the Invention

Problems to be Solved by the Invention

The present invention provides a novel Mdm2 inhibiting compound. Furthermore, the present invention provides an anti-tumor agent containing the Mdm2 inhibiting compound.

Means for Solving the Problems

As a result of extensive studies to solve the above problems, the present inventors have found that a compound having a structure represented by the following formula (1) had potent Mdm2 inhibiting activity, anti-tumor activity and excellent metabolic stability, and accomplished the present invention.
Specifically, the present invention relates to:

[1] A compound represented by general formula (1) or a salt thereof:

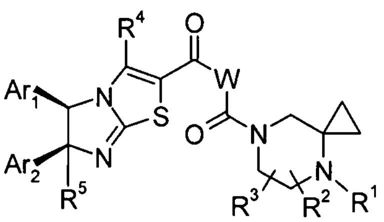

(1)

wherein
$Ar_1$ and $Ar_2$ each independently represent a phenyl group which may have a substituent(s) or a 5- or 6-membered aromatic heterocyclic group which may have a substituent(s);
$R^1$ represents a $C_1$-$C_6$ alkyl group which may have a substituent(s), a $C_2$-$C_6$ alkenyl group which may have a substituent (s), a $C_2$-$C_6$ alkynyl group which may have a substituent(s), an amino group which may have a substituent(s), a $C_1$-$C_6$ alkanoyl group which may have a substituent(s), a $C_2$-$C_6$ alkoxycarbonyl group which may have a substituent(s), a $C_2$-$C_6$ alkylaminocarbonyl group which may have a substituent(s), a $C_1$-$C_6$ alkylsulfonyl group which may have a substituent(s), a phenyl group which may have a substituent(s), an aralkyl group which may have a substituent(s), a 5- or 6-membered aromatic heterocyclic group which may have a substituent(s), a hydrogen atom, or a hydroxy group;
$R^2$ and $R^3$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), a phenyl group which may

have a substituent(s), an aralkyl group which may have a substituent(s), a 5- or 6-membered aromatic heterocyclic group which may have a substituent(s), or a hydrogen atom, or $R^2$ and $R^3$ may together form an oxo group, or $R^2$ and $R^3$ together with the carbon atoms to which $R^2$ and $R^3$ are respectively bonded may form a 3- to 5-membered saturated hydrocarbon ring in a spiro or condensed form;

$R^4$ represents a $C_1$-$C_6$ alkyl group which may have a substituent(s);

$R^5$ represents a $C_1$-$C_6$ alkyl group which may have a substituent(s); and

W represents a group represented by the following general formula (2a) or (2b):

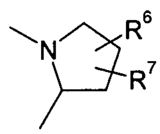

(2a)

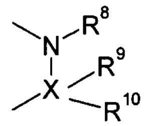

(2b)

wherein

in general formula (2a),

$R^6$ and $R^7$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), or a hydrogen atom, or $R^6$ and $R^7$ may together form an oxo group, or $R^6$ and $R^7$ together with the carbon atoms to which $R^6$ and $R^7$ are respectively bonded may form a 3- to 5-membered saturated hydrocarbon ring in a condensed form; and

in general formula (2b),

$R^8$ represents a $C_1$-$C_6$ alkyl group which may have a substituent(s), a $C_2$-$C_6$ alkenyl group which may have a substituent (s), or a $C_2$-$C_6$ alkynyl group which may have a substituent(s); X represents a $C_1$-$C_3$ alkylene group; and $R^9$ and $R^{10}$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), a phenyl group which may have a substituent(s), an aralkyl group which may have a substituent(s), a 5- or 6-membered aromatic heterocyclic group which may have a substituent(s), a $C_1$-$C_6$ alkoxy group which may have a substituent(s), an amino group which may have a substituent(s), a $C_1$-$C_6$ alkanoyl group which may have a substituent(s), a carbamoyl group which may have a substituent (s), a carbamoyloxy group which may have a substituent(s), a hydrogen atom, a halogen atom, a hydroxy group, or a cyano group, or $R^9$ and $R^{10}$ together with the carbon atoms to which $R^9$ and $R^{10}$ are respectively bonded may form a 3- to 5-membered saturated hydrocarbon ring in a spiro or condensed form.

[2] A compound according to [1] or a salt thereof, wherein

$Ar_1$ and $Ar_2$ are each independently a phenyl group which may have one or more substituents selected from the following Group A, or a 5- or 6-membered aromatic heterocyclic group which may have one or more substituents selected from the following Group B;

$R^1$ is a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, an amino group which may have one or more substituents selected from the following Group D, a $C_1$-$C_6$ alkanoyl group which may have one or more substituents selected from the following Group E, a $C_1$-$C_6$ alkoxycarbonyl group which may have one or more substituents selected from the following Group E, a $C_1$-$C_6$ alkylaminocarbonyl group which may have one or more substituents selected from the following Group E, a $C_1$-$C_6$ alkylsulfonyl group which may have one or more substituents selected from the following Group E, a phenyl group which may have one or more substituents selected from the following Group A, an aralkyl group which may have one or more substituents selected from the following Group F, a 5- or 6-membered aromatic heterocyclic group which may have one or more substituents selected from the following Group B, a hydrogen atom, or a hydroxy group;

$R^2$ and $R^3$ are each independently a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, a phenyl group which may have one or more substituents selected from the following Group A, an aralkyl group which may have one or more substituents selected from the following Group F, a 5- or 6-membered aromatic heterocyclic group which may have one or more substituents selected from the following Group B, or a hydrogen atom, or $R^2$ and $R^3$ together form an oxo group, or $R^2$ and $R^3$ together with the carbon atoms to which $R^2$ and $R^3$ are respectively bonded form a 3- to 5-membered saturated hydrocarbon ring in a spiro or condensed form;

$R^4$ is a $C_1$-$C_6$ alkyl group which may have one or more substituents selected from the following Group C; $R^5$ is a $C_1$-$C_6$ alkyl group which may have one or more substituents selected from the following Group C; and W represents a group represented by the following general formula (2a) or (2b):

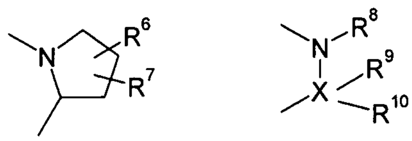

(2a) (2b)

wherein
in general formula (2a),
$R^6$ and $R^7$ are each independently a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, or a hydrogen atom, or $R^6$ and $R^7$ together form an oxo group, or $R^6$ and $R^7$ together with the carbon atoms to which $R^6$ and $R^7$ are respectively bonded form a 3- to 5-membered saturated hydrocarbon ring in a condensed form; and
in general formula (2b),
$R^8$ represents a $C_1$-$C_6$ alkyl group which may have one or more substituents selected from the following Group C, a $C_2$-$C_6$ alkenyl group which may have one or more substituents selected from the following Group C, or a $C_2$-$C_6$ alkynyl group which may have one or more substituents selected from the following Group C; X represents a $C_1$-$C_3$ alkylene group; $R^9$ and $R^{10}$ are each independently a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, a phenyl group which may have one or more substituents selected from the following Group A, an aralkyl group which may have one or more substituents selected from the following Group F, a 5- or 6-membered aromatic heterocyclic group which may have one or more substituents selected from the following Group B, a $C_1$-$C_6$ alkoxy group which may have one or more substituents selected from the following Group G, an amino group which may have one or more substituents selected from the following Group D, a $C_1$-$C_6$ alkanoyl group which may have one or more substituents selected from the following Group E, a carbamoyl group which may have one or more substituents selected from the following Group H, a carbamoyloxy group which may have one or more substituents selected from the following Group H, a hydrogen atom, a halogen atom, a hydroxy group, or a cyano group, or $R^9$ and $R^{10}$ together with the carbon atoms to which $R^9$ and $R^{10}$ are respectively bonded form a 3- to 5-membered saturated hydrocarbon ring in a spiro or condensed form:

Group A: a halogen atom, a hydroxy group, a $C_1$-$C_6$ alkyl group, a $C_1$-$C_6$ alkoxy group, a carbamoyl group, an amino group, a $C_1$-$C_6$ alkanoyl group, and a cyano group;
Group B: a halogen atom, a $C_1$-$C_6$ alkyl group, an amino group, and a $C_1$-$C_6$ alkoxy group;
Group C: a halogen atom, a hydroxy group, a $C_1$-$C_6$ alkoxy group, a carbamoyl group, an amino group, a $C_1$-$C_6$ alkanoyl group, and a cyano group;
Group D: a $C_1$-$C_6$ alkyl group, a $C_1$-$C_6$ alkanoyl group, a $C_1$-$C_6$ alkylsulfonyl group, a $C_2$-$C_6$ alkoxycarbonyl group, a carbamoyl group, a $C_1$-$C_6$ alkylcarbamoyl group, and a di-$C_1$-$C_6$ alkylcarbamoyl group;
Group E: a halogen atom and a hydroxy group;
Group F: a halogen atom, a hydroxy group, a $C_1$-$C_6$ alkoxy group, an amino group, and a $C_1$-$C_6$ alkyl group;
Group G: a halogen atom; and
Group H: a $C_1$-$C_6$ alkyl group.

[3] A compound according to [1] or [2] or a salt thereof, wherein $R^1$ is a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, a hydrogen atom, or a hydroxy group.
[4] A compound according to any one of [1] to [3] or a salt thereof, wherein $R^2$ and $R^3$ are each independently a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, or a hydrogen atom.

[5] A compound according to any one of [1] to [4] or a salt thereof, wherein $R^5$ is a $C_1$-$C_6$ alkyl group.
[6] A compound according to any one of [1] to [5] or a salt thereof, wherein W is represented by the following general formula (2a):
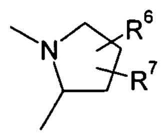

(2a)

wherein $R^6$ and $R^7$ are each independently a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, or a hydrogen atom.
[7] A compound according to any one of [1] to [5] or a salt thereof, wherein W is represented by the following general formula (2b):
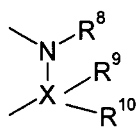

(2b)

wherein $R^8$ is a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group.
[8] A compound according to any one of [1] to [5] and [7] or a salt thereof, wherein W is represented by the following general formula (2b):
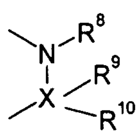

(2b)

wherein $R^9$ and $R^{10}$ are each independently a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, or a hydrogen atom, or $R^9$ and $R^{10}$ together with the carbon atoms to which $R^9$ and $R^{10}$ are respectively bonded form a cyclopropane ring bonded in a spiro or condensed form.
[9] A compound according to any one of [1] to [8] or a salt thereof, wherein $Ar_1$ is a phenyl group which may have one or more substituents selected from a halogen atom, a hydroxy group, a $C_1$-$C_6$ alkyl group, a $C_1$-$C_6$ alkoxy group, a carbamoyl group, an amino group, a $C_1$-$C_6$ alkanoyl group, and a cyano group.
[10] A compound according to any one of [1] to [9] or a salt thereof, wherein $Ar_2$ is a phenyl group which may have one

or more substituents selected from a halogen atom, a hydroxy group, a $C_1$-$C_6$ alkyl group, a $C_1$-$C_6$ alkoxy group, a carbamoyl group, an amino group, a $C_1$-$C_6$ alkanoyl group, and a cyano group, or a 6-membered aromatic heterocyclic group which may have one or more substituents selected from a halogen atom, a $C_1$-$C_6$ alkyl group, an amino group, and a $C_1$-$C_6$ alkoxy group.

[11] A compound according to any one of [1] to [10] or a salt thereof, wherein $R^4$ is a $C_1$-$C_6$ alkyl group.

[12] An inhibitor of Mdm2 comprising a compound according to any one of [1] to [11] or a salt thereof.

[13] An inhibitor of Mdm2 ubiquitin ligase comprising a compound according to any one of [1] to [11] or a salt thereof.

[14] An inhibitor of p53-Mdm2 binding comprising a compound according to any one of [1] to [11] or a salt thereof.

[15] An inhibitor of suppression of p53 transcription activity comprising a compound according to any one of [1] to [11] or a salt thereof.

[16] An inhibitor of p53 degradation comprising a compound according to any one of [1] to [11] or a salt thereof.

[17] A medicament comprising a compound according to any one of [1] to [11] or a salt thereof as an active ingredient.

[18] An anti-tumor agent comprising a compound according to any one of [1] to [11] or a salt thereof as an active ingredient.

[19] A pharmaceutical composition comprising a compound according to any one of [1] to [11] or a salt thereof and a pharmaceutically acceptable carrier.

[20] A method for treating cancer, comprising administering a compound according to any one of [1] to [11] or a salt thereof.

[21] Use of a compound according to any one of [1] to [11] or a salt thereof for the manufacture of a medicament.

[22] Use of a compound according to any one of [1] to [11] or a salt thereof for the manufacture of an anti-tumor agent.

## Advantages of the Invention

The present invention provides a novel imidazothiazole derivative represented by the above formula (1), which has Mdm2 inhibiting activity. Such a novel compound is useful as an anti-tumor agent.

## Description of Embodiments

In the present invention, "Mdm2" means a protein encoded by the murine double minute 2 gene. "Mdm2" includes Mdm2 proteins encoded by a complete length of the Mdm2 gene, Mdm2 proteins encoded by mutated Mdm2 genes (including deletion mutants, substitution mutants, and addition mutants), and so forth. In the present invention, "Mdm2" also includes homologues derived from various animal species such as, for example, human Mdm2 homologue (HDM2).

In the present invention, "p53" means a protein encoded by the p53 gene. "p53" means the p53 protein encoded by a full length p53 gene or a p53 protein that has a mutation (including mutations by deletion, substitution, and addition), but functions normally.

In the present invention, "Mdm2 inhibitor" means a factor that restores p53 functions suppressed by Mdm2 by acting on either Mdm2 or p53, or on both p53 and Mdm2. The p53 functions are not particularly limited so long as they are functions which p53 normally has. Examples thereof include inhibition of canceration of cells by inducing the expression of genes involved in the cell cycle or cellular apoptosis. Examples of Mdm2 inhibitors include factors that inhibit binding of Mdm2 to p53 (hereinafter, referred to as p53-Mdm2 binding inhibitors) or factors that inhibit ubiquitination of p53 by Mdm2 (hereinafter, referred to as Mdm2 ubiquitin ligase inhibitors).

In the present invention, "inhibitor of suppression of p53 transcription activity" means a factor that restores the functions of p53 as a transcription factor suppressed by Mdm2.

In the present invention, "inhibitor of p53 degradation" means a factor that inhibits degradation of p53 in proteasomes by inhibiting ubiquitination of p53 by Mdm2.

In the present invention, the terms "tumor" and "cancer" are used interchangeably. Furthermore, in the present invention, tumor, malignant tumor, cancer, malignant neoplasm, carcinoma, sarcoma, and the like may be collectively referred to as "tumor" or "cancer."

In the present invention, "$C_1$-$C_6$ alkyl group" means a straight, branched, or cyclic alkyl group having 1 to 6 carbon atoms. Examples of a "$C_1$-$C_6$ alkyl group" include a methyl group, an ethyl group, a propyl group, an isopropyl group, a cyclopropyl group, a butyl group, a tert-butyl group, a cyclobutyl group, a cyclopentyl group, and a cyclohexyl group.

"$C_2$-$C_6$ alkenyl group" means a straight or branched alkenyl group having 2 to 6 carbon atoms. Examples of a "$C_2$-$C_6$ alkenyl group" include a vinyl group, an allyl group, and a butenyl group.

"$C_2$-$C_6$ alkynyl group" means a straight or branched alkynyl group having 2 to 6 carbon atoms. Examples of a "$C_2$-$C_6$ alkynyl group" include an ethynyl group, a propynyl group, a butynyl group, a pentynyl group, and a hexynyl group.

"$C_1$-$C_6$ alkoxy group" means an alkoxy group having a straight, branched, or cyclic alkyl group having 1 to 6 carbon atoms. Examples of a "$C_1$-$C_6$ alkoxy group" include a methoxy group, an ethoxy group, a propoxy group, an isopropoxy group, a butoxy group, and a cyclopentyloxy group.

"$C_1$-$C_6$ alkanoyl group" means an alkanoyl group having a straight, branched, or cyclic alkyl group having 1 to 6 carbon atoms. Examples of a "$C_1$-$C_6$ alkanoyl group" include a formyl group, an acetyl group, a propionyl group, and a methylpropionyl group.

"$C_1$-$C_6$ alkoxycarbonyl group" means a carbonyl group (-CO-) bonded to a straight, branched, or cyclic alkoxy group having 1 to 6 carbon atoms. Examples of a "$C_1$-$C_6$ alkoxycarbonyl group" include a methoxycarbonyl group, an ethoxycarbonyl group, and a tert-butoxycarbonyl group.

"$C_1$-$C_6$ alkylaminocarbonyl group" means a carbonyl group (-CO-) bonded to an amino group having one or more straight, branched, or cyclic alkyl groups having 1 to 6 carbon atoms as a substituent. Examples of a "$C_1$-$C_6$ alkylaminocarbonyl group" include a methylaminocarbonyl group, an ethylaminocarbonyl group, a dimethylaminocarbonyl group, and a diethylaminocarbonyl group.

"$C_1$-$C_6$ alkylsulfonyl group" means a sulfonyl group (-$SO_2$-) bonded to a straight, branched, or cyclic alkyl group having 1 to 6 carbon atoms. Examples of a "$C_1$-$C_6$ alkylsulfonyl group" include a methanesulfonyl group and an ethanesulfonyl group.

"$C_1$-$C_6$ alkylcarbamoyl group" means a carbamoyl group bonded to one or more straight, branched, or cyclic alkyl groups having 1 to 6 carbon atoms. Examples of a "$C_1$-$C_6$ alkylcarbamoyl group" include a methylcarbamoyl group, an ethylcarbamoyl group, a dimethylcarbamoyl group, and a diethylcarbamoyl group.

"Aralkyl group" means a substituted aromatic alkyl group. Examples thereof include a benzyl group and a phenethyl group.

"$C_1$-$C_3$ alkylene group" means a straight divalent saturated hydrocarbon chain having 1 to 3 carbon atoms. Examples thereof include a methylene group, a dimethylene group, and a trimethylene group.

Examples of "halogen atom" include a fluorine atom, a chlorine atom, a bromine atom, and an iodine atom.

"Oxo group" means a group represented by "=O" unless otherwise specified.

"5- or 6-membered aromatic heterocyclic group" means a group derived from a 5- or 6-membered aromatic heterocyclic ring that contains one or more oxygen atoms, nitrogen atoms, or sulfur atoms as constituent atoms of the ring structure. These aromatic heterocyclic groups may be bonded at any position. Examples of a 5- or 6-membered aromatic heterocyclic group include groups derived from pyridine, pyrimidine, pyridazine, pyrrole, furan, thiophene, imidazole, pyrazole, oxazole, thiazole, isothiazole, oxadiazole, and triazole.

Hereafter, each substituent in formula (1) will be explained.

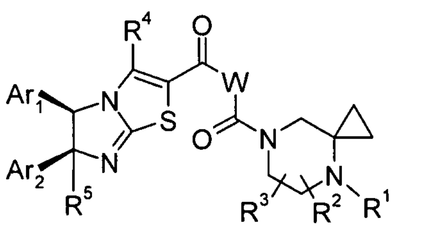

(1)

$R^1$ represents a $C_1$-$C_6$ alkyl group which may have a substituent(s), a $C_2$-$C_6$ alkenyl group which may have a substituent(s), a $C_2$-$C_6$ alkynyl group which may have a substituent(s), an amino group which may have a substituent (s), a $C_1$-$C_6$ alkanoyl group which may have a substituent(s), a $C_1$-$C_6$ alkoxycarbonyl group which may have a substituent (s), a $C_1$-$C_6$ alkylaminocarbonyl group which may have a substituent(s), a $C_1$-$C_6$ alkylsulfonyl group which may have a substituent(s), a phenyl group which may have a substituent(s), an aralkyl group which may have a substituent(s), a 5- or 6-membered aromatic heterocyclic group which may have a substituent(s), a hydrogen atom, or a hydroxy group.

Here, the "$C_1$-$C_6$ alkyl group which may have a substituent(s)" may have one or more substituents, preferably 0 to 3 substituents. The substituent(s) is preferably a halogen atom, a hydroxy group, a $C_1$-$C_6$ alkoxy group, a carbamoyl group, an amino group, a $C_1$-$C_6$ alkanoyl group, or a cyano group. The "$C_1$-$C_6$ alkyl group which may have a substituent (s)" is more preferably an unsubstituted $C_1$-$C_6$ alkyl group or a $C_1$-$C_6$ alkyl group having a halogen atom or a hydroxy group as a substituent, yet more preferably an unsubstituted $C_1$-$C_6$ alkyl group or a $C_1$-$C_6$ alkyl group having a fluorine atom or a hydroxy group as a substituent.

The "$C_2$-$C_6$ alkenyl group which may have a substituent(s)" may have one or more substituents. The number of substituents is preferably 0 to 3. The substituent(s) is preferably a halogen atom, a hydroxy group, a $C_1$-$C_6$ alkoxy group, a carbamoyl group, an amino group, a $C_1$-$C_6$ alkanoyl group, or a cyano group.

The "$C_2$-$C_6$ alkynyl group which may have a substituent(s)" may have one or more substituents. The number of substituents is preferably 0 to 3. The substituent(s) is preferably a halogen atom, a hydroxy group, a $C_1$-$C_6$ alkoxy group, a carbamoyl group, an amino group, a $C_1$-$C_6$ alkanoyl group, or a cyano group.

The "amino group which may have a substituent(s)" may have one or more substituents. The number of substituents is 0 to 2. Examples of the substituent(s) include a $C_1$-$C_6$ alkyl group, a $C_1$-$C_6$ alkanoyl group, a $C_1$-$C_6$ alkylsulfonyl group, a $C_2$-$C_6$ alkoxycarbonyl group, a carbamoyl group, a $C_1$-$C_6$ alkylcarbamoyl group, and a di-$C_1$-$C_6$ alkylcarbamoyl group and more specifically include a methyl group, an ethyl group, an acetyl group, a methanesulfonyl group, an ethanesulfonyl group, a methoxycarbonyl group, an ethoxycarbonyl group, a carbamoyl group, a methylcarbamoyl group, an ethylcarbamoyl group, a dimethylcarbamoyl group, and a diethylcarbamoyl group.

The "$C_1$-$C_6$ alkanoyl group which may have a substituent(s)" may have one or more substituents. The number of substituents is preferably 0 to 3. The substituent(s) is preferably a halogen atom or a hydroxy group.

The "$C_1$-$C_6$ alkoxycarbonyl group which may have a substituent(s)" may have one or more substituents. The number of substituents is preferably 0 to 3. Examples of the substituent(s) include a halogen atom or a hydroxy group. An unsubstituted $C_1$-$C_6$ alkoxycarbonyl group is preferred.

The "$C_1$-$C_6$ alkylaminocarbonyl group which may have a substituent(s)" may have one or more substituents. The number of substituents is preferably 0 to 3. Examples of the substituent(s) include a halogen atom or a hydroxy group. An unsubstituted $C_1$-$C_6$ alkylaminocarbonyl group is preferred.

The "$C_1$-$C_6$ alkylsulfonyl group which may have a substituent(s)" may have one or more substituents. The number of substituents is preferably 0 to 3. Examples of the substituent(s) include a halogen atom or a hydroxy group. An unsubstituted $C_1$-$C_6$ alkylsulfonyl group is preferred.

The "phenyl group which may have a substituent(s)" may have one or more substituents. The number of substituents is preferably 0 to 3, more preferably 1 to 2. The substituent(s) is preferably a halogen atom, a hydroxy group, a $C_1$-$C_6$ alkyl group, a $C_1$-$C_6$ alkoxy group, a carbamoyl group, an amino group, a cyano group, or a $C_1$-$C_6$ alkanoyl group, more preferably a fluorine atom, a chlorine atom, a bromine atom, a hydroxy group, a methyl group, an ethyl group, a propyl group, an isopropyl group, a butyl group, a tert-butyl group, a methoxy group, an ethoxy group, a propoxy group, a carbamoyl group, a cyano group, an amino group, an acetyl group, or an ethylcarbonyl group.

The "aralkyl group which may have a substituent(s)" may have one or more substituents. The number of substituents is preferably 0 to 3, more preferably 1 to 2. The substituent(s) is preferably a halogen atom, a hydroxy group, a $C_1$-$C_6$ alkyl group, a $C_1$-$C_6$ alkoxy group, or an amino group, more preferably a fluorine atom, a chlorine atom, a bromine atom, a hydroxy group, a methyl group, an ethyl group, a propyl group, an isopropyl group, a butyl group, a tert-butyl group, a methoxy group, an ethoxy group, a propoxy group, or an amino group.

The "5- or 6-membered aromatic heterocyclic group which may have a substituent(s)" may have one or more substituents. The number of substituents is preferably 0 to 3, more preferably 1 to 2. The substituent(s) is preferably a halogen atom, a $C_1$-$C_6$ alkyl group, a $C_1$-$C_6$ alkoxy group, or an amino group, more preferably a fluorine atom, a chlorine atom, a bromine atom, a methyl group, an ethyl group, a propyl group, an isopropyl group, a butyl group, a tert-butyl group, a methoxy group, an ethoxy group, a propoxy group, or an amino group.

$R^1$ is preferably a $C_1$-$C_6$ alkanoyl group, a $C_1$-$C_6$ alkyl group which may have a substituent(s), a hydrogen atom, or a hydroxy group. The substituent(s) in the $C_1$-$C_6$ alkyl group is preferably a fluorine atom or a hydroxy group. $R^1$ is more preferably an unsubstituted $C_1$-$C_6$ alkyl group or a hydrogen atom, yet more preferably a hydrogen atom, a methyl group, an ethyl group, or a hydroxyethyl group, even more preferably a hydrogen atom, a methyl group, or ethyl group.

$R^2$ and $R^3$ each independently mean a substituent(s) substituted on the carbon atom in the piperazine ring in the structure represented by the general formula (1) (the substituent(s) may be substituted at any position). $R^2$ and $R^3$ may be substituents on the same carbon atom or may be substituents on different carbon atoms.

$R^2$ and $R^3$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), a phenyl group which may have a substituent(s), an aralkyl group which may have a substituent(s), a 5- or 6-membered aromatic heterocyclic group which may have a substituent(s), or a hydrogen atom, or $R^2$ and $R^3$ may together form an oxo group, or $R^2$ and $R^3$ together with the carbon atoms to which $R^2$ and $R^3$ are respectively bonded may form a 3- to 5-membered saturated hydrocarbon ring in a spiro or condensed form.

Here, the "$C_1$-$C_6$ alkyl group which may have a substituent(s)", "$C_2$-$C_6$ alkenyl group which may have a substituent(s)", "$C_2$-$C_6$ alkynyl group which may have a substituent(s)", "phenyl group which may have a substituent (s)", "aralkyl group which may have a substituent(s)", and "5- or 6-membered aromatic heterocyclic group which may have a substituent(s)" each have the same meanings as defined in $R^1$. The phrase "$R^2$ and $R^3$ together with the carbon atoms to which $R^2$ and $R^3$ are respectively bonded may form a 3- to 5-membered saturated hydrocarbon ring in a spiro or condensed form" means that $R^2$ and $R^3$ may be bonded to form a 3- to 5-membered saturated hydrocarbon ring in a spiro form when $R^2$ and $R^3$ are substituents bonded to the same carbon atom, or $R^2$ and $R^3$ may be bonded to form a 3- to 5-membered saturated hydrocarbon ring in a condensed form when $R^2$ and $R^3$ are bonded to different carbon atoms. Examples of the "3- to 5-membered saturated hydrocarbon ring" include a cyclopropane ring, a cyclobutane ring, and a cyclopentane ring.

Preferably, $R^2$ and $R^3$ are each independently a hydrogen atom or a $C_1$-$C_6$ alkyl group which may have a

substituent(s), or preferably, $R^2$ and $R^3$ together form an oxo group. The substituent(s) substituted on the $C_1$-$C_6$ alkyl group is preferably a fluorine atom or a hydroxy group. More preferably, $R^2$ and $R^3$ are respectively a hydrogen atom, or one of $R^2$ and $R^3$ is a hydrogen atom and the other moiety is a methyl group or an ethyl group. Yet more preferably, one of $R^2$ and $R^3$ is a hydrogen atom and the other moiety is a methyl group substituted at the 5- or 6- position.

$R^4$ represents a $C_1$-$C_6$ alkyl group which may have a substituent(s). Here, the "$C_1$-$C_6$ alkyl group which may have a substituent(s)" has the same meaning as defined above in $R^1$.

$R^4$ is preferably an unsubstituted $C_1$-$C_6$ alkyl group, more preferably an unsubstituted $C_1$-$C_3$ alkyl group, yet more preferably an isopropyl group.

$R^5$ represents a $C_1$-$C_6$ alkyl group which may have a substituent(s). Here, the "$C_1$-$C_6$ alkyl group which may have a substituent(s)" has the same meaning as defined above in $R^1$.

$R^5$ is preferably an unsubstituted $C_1$-$C_6$ alkyl group, more preferably an unsubstituted $C_1$-$C_3$ alkyl group, yet more preferably a methyl group or an ethyl group.

W represents a group represented by the following general formula (2a) or (2b):

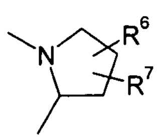

(2a)

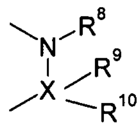

(2b)

In the general formula (2a), $R^6$ and $R^7$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent (s), or a hydrogen atom, or $R^6$ and $R^7$ may together form an oxo group.

In the general formula (2b), $R^8$ represents a $C_1$-$C_6$ alkyl group which may have a substituent(s), a $C_2$-$C_6$ alkenyl group which may have a substituent(s), or a $C_2$-$C_6$ alkynyl group which may have a substituent(s); X represents a $C_1$-$C_3$ alkylene group; and $R^9$ and $R^{10}$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), a phenyl group which may have a substituent(s), an aralkyl group which may have a substituent(s), a 5- or 6-membered aromatic heterocyclic group which may have a substituent(s), a $C_1$-$C_6$ alkoxy group which may have a substituent(s), an amino group which may have a substituent(s), a $C_1$-$C_6$ alkanoyl group which may have a substituent(s), a carbamoyl group which may have a substituent(s), a carbamoyloxy group which may have a substituent(s), a hydrogen atom, a halogen atom, a hydroxy group, or a cyano group, or $R^9$ and $R^{10}$ together with the carbon atoms to which $R^9$ and $R^{10}$ are respectively bonded may form a 3- to 5-membered saturated hydrocarbon ring in a spiro or condensed form.

Here, the "$C_1$-$C_6$ alkyl group which may have a substituent(s)", "$C_2$-$C_6$ alkenyl group which may have a substituent(s)", "$C_2$-$C_6$ alkynyl group which may have a substituent(s)", "$C_1$-$C_6$ alkanoyl group which may have a substituent (s)", "amino group which may have a substituent(s)", "phenyl group which may have a substituent(s)", "aralkyl group which may have a substituent(s)", and "5- or 6-membered aromatic heterocyclic group which may have a substituent (s)" regarding $R^6$, $R^7$, $R^8$, $R^9$, and $R^{10}$ have the same meanings as defined in $R^1$.

$R^6$ and $R^7$ each independently represent a substituent(s) substituted on a carbon atom in the pyrrolidine ring in the structure represented by the formula (2a). $R^6$ and $R^7$ may be substituents on the same carbon atom or may be substituents on different carbon atoms. Moreover, $R^6$ and $R^7$ together with the carbon atoms to which $R^6$ and $R^7$ are respectively bonded may form a 3-to 5-membered saturated hydrocarbon ring in a condensed form. In this case, the formed saturated hydrocarbon ring is preferably a cyclopropane ring.

Both $R^6$ and $R^7$ are preferably substituents substituted on the carbon atom at the 5- position. Preferably, $R^6$ and $R^7$ are each independently a hydrogen atom, or a $C_1$-$C_6$ alkyl group which may have a substituent(s), or $R^6$ and $R^7$ together form an oxo group. More preferably, $R^6$ and $R^7$ are each independently a hydrogen atom, a methyl group, or an ethyl group.

$R^8$ is preferably a $C_1$-$C_6$ alkyl group which may have a substituent(s). The substituent(s) in the $C_1$-$C_6$ alkyl group is preferably a hydroxy group or a halogen atom. $R^8$ is more preferably an unsubstituted $C_1$-$C_6$ alkyl group or a $C_1$-$C_6$ alkyl group substituted by a hydroxy group, yet more preferably a methyl group, an ethyl group, an isopropyl group, a hydroxymethyl group, or a hydroxyethyl group, even more preferably an ethyl group or an isopropyl group.

X represents a $C_1$-$C_3$ alkylene group. "$C_1$-$C_3$ alkylene group" means a straight divalent saturated hydrocarbon chain having 1 to 3 carbon atoms. Examples thereof include a methylene group, an ethylene group, and a propylene

group. X is preferably a methylene group or an ethylene group.

$R^9$ and $R^{10}$ each independently represent a substituent(s) substituted on X above. $R^9$ and $R^{10}$ may be substituents on the same carbon atom or may be substituents on different carbon atoms.

The "$C_1$-$C_6$ alkoxy group which may have a substituent(s)" as $R^9$ and $R^{10}$ may have one or more substituents. The number of substituents is preferably 0 to 3. The substituent(s) is preferably a halogen atom.

The "carbamoyl group which may have a substituent(s)" as $R^9$ and $R^{10}$ may have one or more substituents. The number of substituents is preferably 0 to 2. The substituent(s) is preferably a $C_1$-$C_6$ alkyl group.

The "carbamoyloxy group which may have a substituent(s)" as $R^9$ and $R^{10}$ may have one or more substituents. The number of substituents is preferably 0 to 2. The substituent(s) is preferably a $C_1$-$C_6$ alkyl group.

Furthermore, $R^9$ and $R^{10}$ together with the carbon atoms to which $R^9$ and $R^{10}$ are respectively bonded may form a 3- to 5-membered saturated hydrocarbon ring in a spiro or condensed form. The phrase "$R^9$ and $R^{10}$ together with the carbon atoms to which $R^9$ and $R^{10}$ are respectively bonded may form a 3- to 5-membered saturated hydrocarbon ring in a spiro or condensed form" has the same meaning as defined above in the similar structure which $R^2$ and $R^3$ may have.

Preferably, $R^9$ and $R^{10}$ are each independently a hydrogen atom or a $C_1$-$C_6$ alkyl group which may have a substituent(s), or preferably, $R^9$ and $R^{10}$ together with the carbon atoms to which $R^9$ and $R^{10}$ are respectively bonded form a 3- to 5-membered saturated hydrocarbon ring bonded in a spiro form. Furthermore, the saturated hydrocarbon ring is more preferably a cyclopropane ring.

$Ar_1$ and $Ar_2$ each independently represent a phenyl group which may have a substituent(s) or a 5- or 6-membered aromatic heterocyclic group which may have a substituent(s). Here, the "phenyl group which may have a substituent (s)" and "5- or 6-membered aromatic heterocyclic group which may have a substituent(s)" have the same meanings as defined above in $R^1$.

$Ar_1$ is preferably a phenyl group having 1 to 3 $C_1$-$C_6$ alkyl groups which may have a substituent(s), or halogen atoms as a substituent(s). The substituent(s) may be positioned at any position. $Ar_1$ preferably has 1 to 2 substituents at the 2-, 3-, or 4- position and is more preferably a 4-chlorophenyl group, a 4-bromophenyl group, a 4-trifluoromethylphenyl group, a 3-fluoro-4-chlorophenyl group, or a 2-fluoro-4-chlorophenyl group.

$Ar_2$ is preferably a phenyl group having 1 to 3 $C_1$-$C_6$ alkyl groups which may have a substituent(s), amino groups which may have a substituent(s), halogen atoms, or cyano groups as a substituent(s), or a 6-membered aromatic heterocyclic group having 1 to 3 amino groups which may have a substituent(s) as a substituent(s). The 6-membered aromatic heterocyclic group is preferably a pyridyl group, a pyrimidinyl group, or a pyridazinyl group, particularly preferably a pyridyl group. The substituent(s) may be positioned at any position. $Ar_2$ preferably has 1 to 2 substituents at the 2-, 3-, 4-, or 6- position. $Ar_2$ is more preferably a 4-chlorophenyl group, a 4-cyanophenyl group, or a 6-chloropyridin-3-yl group. Examples of preferable combinations of $Ar_1$ and $Ar_2$ include a combination of a 3-fluoro-4-chlorophenyl group as $Ar_1$ and a 4-chlorophenyl group as $Ar_2$ and a combination of a 3-fluoro-4-chlorophenyl group as $Ar_1$ and a 6-chloropyridin-3-yl group as $Ar_2$.

The absolute configurations of $Ar_1$ and $Ar_2$ in the imidazothiazole skeleton are preferably 5R and 6S, respectively.

The compound represented by formula (1) of the present invention may have stereoisomers or optical isomers due to asymmetric carbon atoms, and all these stereoisomers, optical isomers, and mixtures thereof are included in the present invention.

In one embodiment of the present invention, a compound having an absolute configuration represented by formula (3) is preferred:

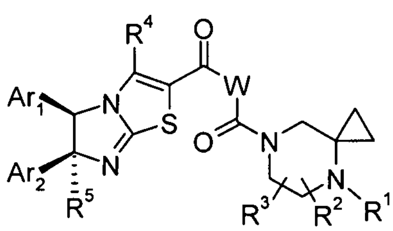

(3)

wherein $Ar_1$, $Ar_2$, $R^1$ to $R^5$ have the same meanings as defined above.

Furthermore, the compound of general formula (1) is preferably a compound or a salt thereof described in any of the Examples and/or tables described later.

The compound represented by general formula (1) of the present invention can form a pharmaceutically acceptable salt, if desired, when having a basic group such as an amino group. Examples of such salts can include:

hydrohalides such as hydrochloride and hydroiodide; inorganic acid salts such as nitrate, perchlorate, sulfate, and phosphate; lower alkanesulfonates such as methanesulfonate, trifluoromethanesulfonate, and ethanesulfonate; arylsulfonates such as benzenesulfonate and p-toluenesulfonate; organic acid salts such as formic acid, acetic acid, malic acid, fumarate, succinate, citrate, tartrate, oxalate, and maleate; and amino acid salts such as ornithine salt, glutamate, and aspartate. Hydrohalides and organic acid salts are preferred.

The compound represented by general formula (1) of the present invention may generally form a base addition salt when having an acidic group such as a carboxy group. Examples of pharmaceutically acceptable salts can include: alkali metal salts such as sodium salt, potassium salt, and lithium salt; alkaline earth metal salts such as calcium salt and magnesium salt; inorganic salts such as ammonium salt; and organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, and tris(hydroxymethyl)aminomethane salt.

The compound represented by general formula (1) of the present invention or the salt thereof may be present in a free or solvate form. The compound represented by general formula (1) of the present invention or the salt thereof may be present in a hydrate form, for example, by absorbing moisture in the air. The solvate is not particularly limited so long as it is pharmaceutically acceptable. Specifically, the solvate is preferably a hydrate, an ethanol solvate, or the like. Moreover, the compound represented by general formula (1) of the present invention may be in an N-oxide form when containing a nitrogen atom. These solvate and N-oxide forms are also included in the present invention.

The compound represented by general formula (1) of the present invention or the salt thereof may have various isomers such as geometrical isomers (e.g., cis and trans forms), tautomers, and optical isomers (e.g., d and 1 forms), depending on the types or combinations of substituents. The compound of the present invention also encompasses all these isomers, stereoisomers, and mixtures of these isomers and stereoisomers in any ratio, unless otherwise specified.

The compound represented by general formula (1) of the present invention may contain an isotope in a non-natural proportion as one or more constituent atoms. Examples of an isotope include heavy hydrogen ($^{2}H$), tritium ($^{3}H$) , iodine-125 ($^{125}I$), and carbon-14 ($^{14}C$).

These compounds are useful as a therapeutic or preventive agent, a research reagent (e.g., an assay reagent), and a diagnostic agent (e.g., an *in vivo* diagnostic imaging agent). All isotopic variants of the compound represented by general formula (1) are included in the scope of the present invention, regardless of the presence or absence of radioactivity.

Moreover, the present invention also encompasses a compound that is converted to the compound represented by general formula (1) as an active ingredient in the pharmaceutical composition of the present invention due to a reaction induced by an enzyme, gastric acid, or the like under physiological conditions *in vivo,* i.e., a compound that is converted to the compound (1) through enzymatic oxidation, reduction, hydrolysis, or the like or a "pharmaceutically acceptable prodrug compound" that is converted to the compound represented by general formula (1) through hydrolysis or the like induced by gastric acid or the like.

Examples of a prodrug can include: compounds in which an amino group in the compound represented by general formula (1) is acylated, alkylated, or phosphorylated (e.g., compounds in which the amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated); compounds in which a hydroxy group in the compound (1) is acylated, alkylated, phosphorylated, or borated (e.g., compounds in which the hydroxy group is acetylated, palmitoylated, propanoylated, pivaloylated, succinylated, fumarylated, alanylated, or dimethylaminomethylcarbonylated); and compounds in which a carboxy group in the compound represented by the general formula (1) is esterified or amidated (e.g., compounds in which the carboxy group is ethyl esterified, phenyl esterified, carboxymethyl esterified, dimethylaminomethyl esterified, pivaloyloxymethyl esterified, ethoxycarbonyloxyethyl esterified, amidated, or methylamidated).

A prodrug of the compound of the present invention can be produced from a compound represented by general formula (1) according to a method known in the art. Moreover, a prodrug of the compound of the present invention also includes those converted to the compound represented by general formula (1) under physiological conditions as described in "Development of Pharmaceutical Products", vol. 7, Molecule Design, p. 163-198, Hirokawa-Shoten Ltd. (1990).

Specific examples of the compound represented by general formula (1) of the present invention can include compounds described in, for example, the following Compound Tables 1 to 4. These compounds can be synthesized according to production methods described later or methods described in the Examples. In the tables, $R^1$ to $R^{10}$, $Ar_1$, and $Ar_2$ have the same meanings as defined above in the compound represented by general formula (1).

[Compounds wherein W is represented by general formula (2a)]

[Table 1]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 1 | H | H | H | $CH(CH_3)_2$ | $CH_3$ | H | H | 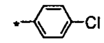 | 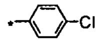 |
| 2 | H | $CH_3$ | H | $CH(CH_3)_2$ | $CH_3$ | H | H | 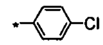 | 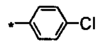 |
| 3 | H | $CH_3$ | $CH_3$ | $CH(CH_3)_2$ | $CH_3$ | H | H | 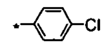 | 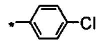 |
| 4 | H | $CH_2CH_3$ | H | $CH(CH_3)_2$ | $CH_3$ | H | H | 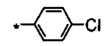 | 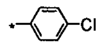 |
| 5 | H | $- CH_2 -$ | | $CH(CH_3)_2$ | $CH_3$ | H | H | 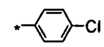 | 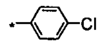 |
| 6 | $CH_3$ | H | H | $CH(CH_3)_2$ | $CH_3$ | H | H | 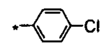 | 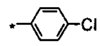 |
| 7 | $CH_3$ | $CH_3$ | H | $CH(CH_3)_2$ | $CH_3$ | H | H | 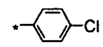 | 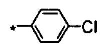 |
| 8 | $CH_2CH_2OH$ | H | H | $CH(CH_3)_2$ | $CH_3$ | H | H | 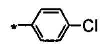 | 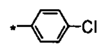 |
| 9 | $CH_2CH_2OH$ | $CH_3$ | H | $CH(CH_3)_2$ | $CH_3$ | H | H | 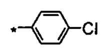 | 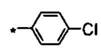 |
| 10 | H | H | H | $CH(CH_3)_2$ | $CH_3$ | $CH_3$ | H | 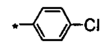 | 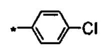 |
| 11 | H | $CH_3$ | H | $CH(CH_3)_2$ | $CH_3$ | $CH_3$ | H | 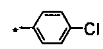 | 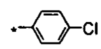 |
| 12 | H | $CH_3$ | $CH_3$ | $CH(CH_3)_2$ | $CH_3$ | $CH_3$ | H | 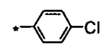 | 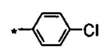 |
| 13 | H | $CH_2CH_3$ | H | $CH(CH_3)_2$ | $CH_3$ | $CH_3$ | H | 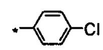 | 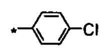 |
| 14 | H | $-CH_2-$ | | $CH(CH_3)_2$ | $CH_3$ | $CH_3$ | H | 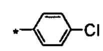 | 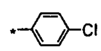 |
| 15 | H | -O- | | $CH(CH_3)_3$ | $CH_4$ | $CH_3$ | H | 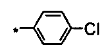 | 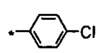 |
| 16 | $CH_3$ | H | H | $CH(CH_3)_2$ | $CH_3$ | $CH_3$ | H | 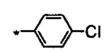 | 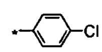 |
| 17 | $CH_3$ | $CH_3$ | H | $CH(CH_3)_2$ | $CH_3$ | $CH_3$ | H | 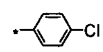 | 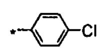 |

[Table 2]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 18 | $CH_2CH_2OH$ | H | H | $CH(CH_3)_2$ | $CH_3$ | $CH_3$ | H | 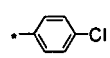 | 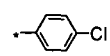 |
| 19 | $CH_2CH_2OH$ | $CH_3$ | H | $CH(CH_3)_2$ | $CH_3$ | $CH_3$ | H | 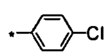 | 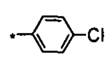 |

(continued)

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 20 | $COCH_3$ | H | H | $CH(CH_3)_2$ | $CH_3$ | $CH_3$ | H | 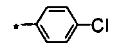 | 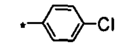 |
| 21 | $COCH_3$ | $CH_3$ | H | $CH(CH_3)_2$ | $CH_3$ | $CH_3$ | H | 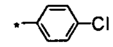 | 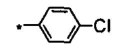 |
| 22 | H | H | H | $CH(CH_3)_2$ | $CH_3$ | $CH_2CH_3$ | H | 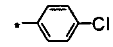 | 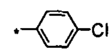 |
| 23 | H | $CH_3$ | H | $CH(CH_3)_2$ | $CH_3$ | $CH_2CH_3$ | H | 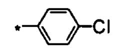 | 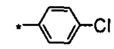 |
| 24 | $CH_3$ | H | H | $CH(CH_3)_2$ | $CH_3$ | $CH_2CH_3$ | H | 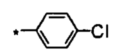 | 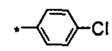 |
| 25 | $CH_3$ | $CH_3$ | H | $CH(CH_3)_2$ | $CH_3$ | $CH_2CH_3$ | H | 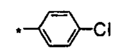 | 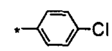 |
| 26 | $CH_2CH_2OH$ | $CH_3$ | H | $CH(CH_3)_2$ | $CH_3$ | $CH_2CH_3$ | H | 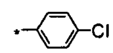 | 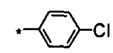 |
| 27 | H | H | H | $CH(CH_3)_2$ | $CH_3$ | $CH_2F$ | H | 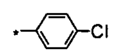 | 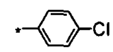 |
| 28 | H | $CH_3$ | H | $CH(CH_3)_2$ | $CH_3$ | $CH_2F$ | H | 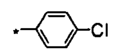 | 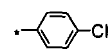 |
| 29 | $CH_3$ | H | H | $CH(CH_3)_2$ | $CH_3$ | $CH_2F$ | H | 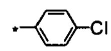 | 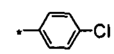 |
| 30 | $CH_3$ | $CH_3$ | H | $CH(CH_3)_2$ | $CH_3$ | $CH_2F$ | H | 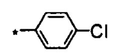 | 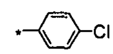 |
| 31 | $CH_2CH_2OH$ | $CH_3$ | H | $CH(CH_3)_2$ | $CH_3$ | $CH_2F$ | H | 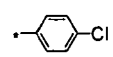 | 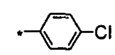 |
| 32 | H | $CH_3$ | H | $CH(CH_3)_2$ | $CH_3$ | $OCH_3$ | H | 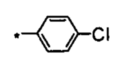 | 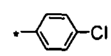 |
| 33 | H | H | H | $CH(CH_3)_2$ | $CH_3$ | -O- | | 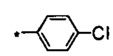 | 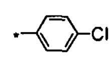 |
| 34 | H | $CH_3$ | H | $CH(CH_3)_2$ | $CH_3$ | -O- | | 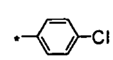 | 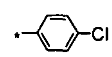 |
| 35 | $CH_3$ | H | H | $CH(CH_3)_2$ | $CH_3$ | -O- | | 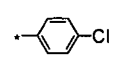 | 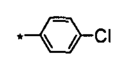 |

[Table 3]

| $R^1$ | $R^2$ | $R^3$ | $R^4$ | $R^5$ | $R^6$ | $R^7$ | $Ar_1$ | $Ar_2$ | $R^1$ |
|---|---|---|---|---|---|---|---|---|---|
| 36 | H | H | H | $CH(CH_3)_2$ | $CH_3$ | H | H | 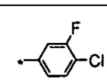 | 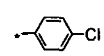 |

(continued)

| $R^1$ | $R^2$ | $R^3$ | $R^4$ | $R^5$ | $R^6$ | $R^7$ | $Ar_1$ | $Ar_2$ | $R^1$ |
|---|---|---|---|---|---|---|---|---|---|
| 37 | H | $CH_3$ | H | $CH(CH_3)_2$ | $CH_3$ | H | H | 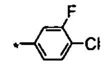 | 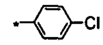 |
| 38 | H | $CH_3$ | $CH_3$ | $CH(CH_3)_2$ | $CH_3$ | H | H | 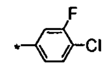 | 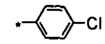 |
| 39 | H | $CH_2CH_3$ | H | $CH(CH_3)_2$ | $CH_3$ | H | H | 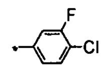 | 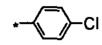 |
| 40 | H | $- CH_2 -$ | | $CH(CH_3)_2$ | $CH_3$ | H | H | 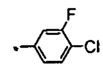 | 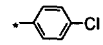 |
| 41 | $CH_3$ | H | H | $CH(CH_3)_2$ | $CH_3$ | H | H | 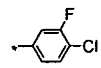 | 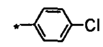 |
| 42 | $CH_3$ | $CH_3$ | H | $CH(CH_3)_2$ | $CH_3$ | H | H | 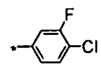 | 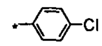 |
| 43 | $CH_2CH_2OH$ | H | H | $CH(CH_3)_2$ | $CH_3$ | H | H | 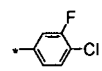 | 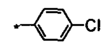 |
| 44 | $CH_2CH_2OH$ | $CH_3$ | H | $CH(CH_3)_2$ | $CH_3$ | H | H | 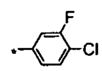 | 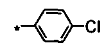 |
| 45 | $COCH_3$ | H | H | $CH(CH_3)_2$ | $CH_3$ | H | H | 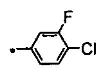 | 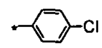 |
| 46 | $COCH_3$ | $CH_3$ | H | $CH(CH_3)_2$ | $CH_3$ | H | H | 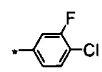 | 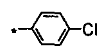 |
| 47 | H | H | H | $CH(CH_3)_2$ | $CH_3$ | $CH_3$ | H | 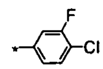 | 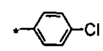 |
| 48 | H | $CH_3$ | H | $CH(CH_3)_2$ | $CH_3$ | $CH_3$ | H | 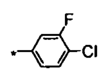 | 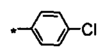 |
| 49 | H | $CH_3$ | $CH_3$ | $CH(CH_3)_2$ | $CH_3$ | $CH_3$ | H | 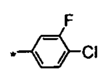 | 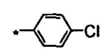 |
| 50 | H | $CH_2CH_3$ | H | $CH(CH_3)_2$ | $CH_3$ | $CH_3$ | H | 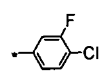 | 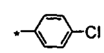 |
| 51 | H | $-CH_2-$ | | $CH(CH_3)_2$ | $CH_3$ | $CH_3$ | H | 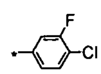 | 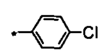 |
| 52 | $CH_3$ | H | H | $CH(CH_3)_2$ | $CH_3$ | $CH_3$ | H | 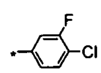 | 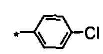 |
| 53 | $CH_3$ | $CH_3$ | H | $CH(CH_3)_2$ | $CH_3$ | $CH_3$ | H | 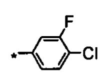 | 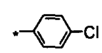 |

[Table 4]

| $R^1$ | $R^2$ | $R^3$ | $R^4$ | $R^5$ | $R^6$ | $R^7$ | $Ar_1$ | $Ar_2$ | $R^1$ |
|---|---|---|---|---|---|---|---|---|---|
| 54 | $CH_2CH_2OH$ | H | H | $CH(CH_3)_2$ | $CH_3$ | $CH_3$ | H | 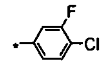 | 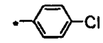 |
| 55 | $CH_2CH_2OH$ | $CH_3$ | H | $CH(CH_3)_2$ | $CH_3$ | $CH_3$ | H | 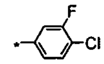 | 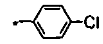 |
| 56 | $COCH_3$ | $CH_3$ | H | $CH(CH_3)_2$ | $CH_3$ | $CH_3$ | H | 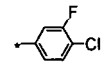 | 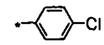 |
| 57 | H | H | H | $CH(CH_3)_2$ | $CH_3$ | $CH_2CH_3$ | H | 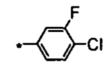 | 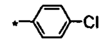 |
| 58 | H | $CH_3$ | H | $CH(CH_3)_2$ | $CH_3$ | $CH_2CH_3$ | H | 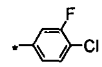 | 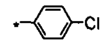 |
| 59 | $CH_3$ | $CH_3$ | H | $CH(CH_3)_2$ | $CH_3$ | $CH_2CH_3$ | H | 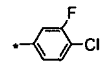 | 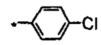 |
| 60 | $CH_2CH_2OH$ | $CH_3$ | H | $CH(CH_3)_2$ | $CH_3$ | $CH_2CH_3$ | H | 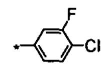 | 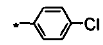 |
| 61 | H | $CH_3$ | H | $CH(CH_3)_2$ | $CH_3$ | $CH_2F$ | H | 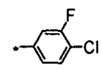 | 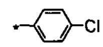 |
| 62 | $CH_3$ | $CH_3$ | H | $CH(CH_3)_2$ | $CH_3$ | $CH_2F$ | H | 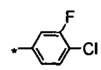 | 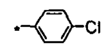 |
| 63 | $CH_2CH_2OH$ | $CH_3$ | H | $CH(CH_3)_2$ | $CH_3$ | $CH_2F$ | H | 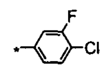 | 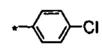 |
| 64 | H | $CH_3$ | H | $CH(CH_3)_2$ | $CH_3$ | $OCH_3$ | H | 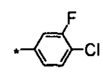 | 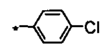 |
| 65 | H | H | H | $CH(CH_3)_2$ | $CH_3$ | H | H | 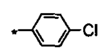 | 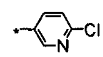 |
| 66 | H | $CH_3$ | H | $CH(CH_3)_2$ | $CH_3$ | H | H | 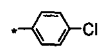 | 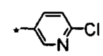 |
| 67 | H | $CH_2CH_3$ | H | $CH(CH_3)_2$ | $CH_3$ | H | H | 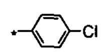 | 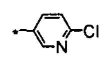 |
| 68 | H | $- CH_2 -$ | | $CH(CH_3)_2$ | $CH_3$ | H | H | 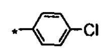 | 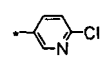 |
| 69 | $CH_3$ | $CH_3$ | H | $CH(CH_3)_2$ | $CH_3$ | H | H | 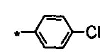 | 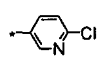 |
| 70 | $CH_2CH_2OH$ | $CH_3$ | H | $CH(CH_3)_2$ | $CH_3$ | H | H | 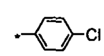 | 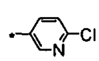 |
| 71 | H | H | H | $CH(CH_3)_2$ | $CH_3$ | $CH_3$ | H | 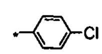 | 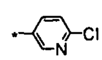 |

[Table 5]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 72 | H | $CH_3$ | H | $CH(CH_3)_2$ | $CH_3$ | $CH_3$ | H | 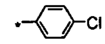 | 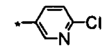 |
| 73 | H | $CH_2CH_3$ | H | $CH(CH_3)_2$ | $CH_3$ | $CH_3$ | H | 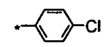 | 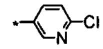 |
| 74 | H | $-CH_2-$ | | $CH(CH_3)_2$ | $CH_3$ | $CH_3$ | H | 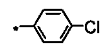 | 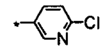 |
| 75 | $CH_3$ | H | H | $CH(CH_3)_2$ | $CH_3$ | $CH_3$ | H | 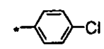 | 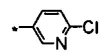 |
| 76 | $CH_3$ | $CH_3$ | H | $CH(CH_3)_2$ | $CH_3$ | $CH_3$ | H | 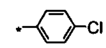 | 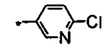 |
| 77 | $CH_2CH_2OH$ | H | H | $CH(CH_3)_2$ | $CH_3$ | $CH_3$ | H | 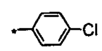 | 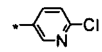 |
| 78 | $CH_2CH_2OH$ | $CH_3$ | H | $CH(CH_3)_2$ | $CH_3$ | $CH_3$ | H | 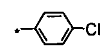 | 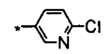 |
| 79 | $COCH_3$ | $CH_3$ | H | $CH(CH_3)_2$ | $CH_3$ | $CH_3$ | H | 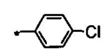 | 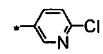 |
| 80 | H | H | H | $CH(CH_3)_2$ | $CH_3$ | $CH_2CH_3$ | H | 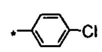 | 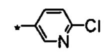 |
| 81 | H | $CH_3$ | H | $CH(CH_3)_2$ | $CH_3$ | $CH_2CH_3$ | H | 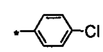 | 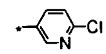 |
| 82 | $CH_3$ | $CH_3$ | H | $CH(CH_3)_2$ | $CH_3$ | $CH_2CH_3$ | H | 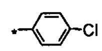 | 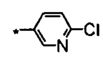 |
| 83 | $CH_2CH_2OH$ | $CH_3$ | H | $CH(CH_3)_2$ | $CH_3$ | $CH_2CH_3$ | H | 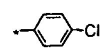 | 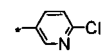 |
| 84 | H | $CH_3$ | H | $CH(CH_3)_2$ | $CH_3$ | $CH_2F$ | H | 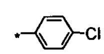 | 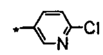 |
| 85 | $CH_3$ | $CH_3$ | H | $CH(CH_3)_2$ | $CH_3$ | $CH_2F$ | H | 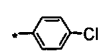 | 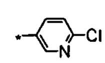 |
| 86 | $CH_2CH_2OH$ | $CH_3$ | H | $CH(CH_3)_2$ | $CH_3$ | $CH_2F$ | H | 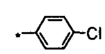 | 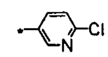 |
| 87 | H | $CH_3$ | H | $CH(CH_3)_2$ | $CH_3$ | $OCH_3$ | H | 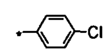 | 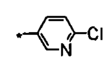 |
| 88 | H | H | H | $CH(CH_3)_2$ | $CH_3$ | H | H | 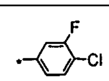 | 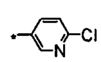 |
| 89 | H | (6R)-$CH_3$ | H | $CH(CH_3)_2$ | $CH_3$ | H | H | 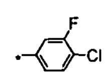 | 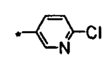 |

[Table 6]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 90 | H | (6S)-$CH_3$ | H | $CH(CH_3)_2$ | $CH_3$ | H | H | 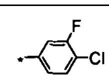 | 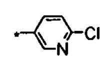 |

(continued)

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 91 | H | $CH_3$ | $CH_3$ | $CH(CH_3)_2$ | $CH_3$ | H | H | 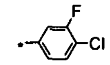 | 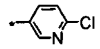 |
| 92 | H | (6R)-$CH_2CH_3$ | H | $CH(CH_3)_2$ | $CH_3$ | H | H | 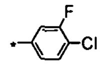 | 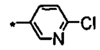 |
| 93 | H | (6s)-$CH_2CH_3$ | H | $CH(CH_3)_2$ | $CH_3$ | H | H | 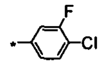 | 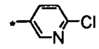 |
| 94 | H | $CH(CH_3)_2$ | H | $CH(CH_3)_2$ | $CH_3$ | H | H | 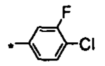 | 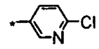 |
| 95 | H | $CH_2OH$ | H | $CH(CH_3)_2$ | $CH_3$ | H | H | 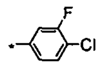 | 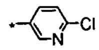 |
| 96 | H | $CH_2F$ | H | $CH(CH_3)_2$ | $CH_3$ | H | H | 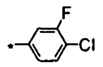 | 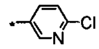 |
| 97 | H | $- CH_2 -$ | | $CH(CH_3)_2$ | $CH_3$ | H | H | 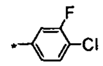 | 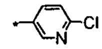 |
| 98 | $CH_3$ | H | H | $CH(CH_3)_2$ | $CH_3$ | H | H | 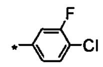 | 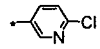 |
| 99 | $CH_3$ | $CH_3$ | H | $CH(CH_3)_2$ | $CH_3$ | H | H | 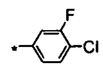 | 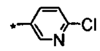 |
| 100 | $CH_3$ | $CH_2CH_3$ | H | $CH(CH_3)_2$ | $CH_3$ | H | H | 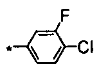 | 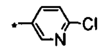 |
| 101 | $CH_2CH_2OH$ | H | H | $CH(CH_3)_2$ | $CH_3$ | H | H | 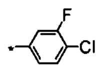 | 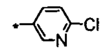 |
| 102 | $CH_2CH_2OH$ | $CH_3$ | H | $CH(CH_3)_2$ | $CH_3$ | H | H | 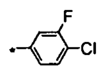 | 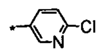 |
| 103 | CHO | $CH_3$ | H | $CH(CH_3)_2$ | $CH_3$ | H | H | 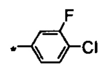 | 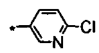 |
| 104 | CHO | $CH_2CH_3$ | H | $CH(CH_3)_2$ | $CH_3$ | H | H | 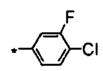 | 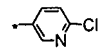 |
| 105 | $COCH_3$ | $CH_3$ | H | $CH(CH_3)_2$ | $CH_3$ | H | H | 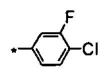 | 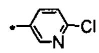 |
| 106 | $COCH_3$ | $CH_2CH_3$ | H | $CH(CH_3)_2$ | $CH_3$ | H | H | 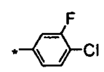 | 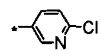 |
| 107 | $COCF_3$ | $CH_3$ | H | $CH(CH_3)_2$ | $CH_3$ | H | H | 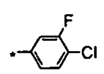 | 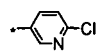 |

[Table 7]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 108 | $COCF_3$ | $CH_2CH_3$ | H | $CH(CH_3)_2$ | $CH_3$ | H | H | 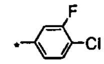 | 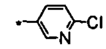 |
| 109 | H | H | H | $CH(CH_3)_2$ | $CH_3$ | $CH_3$ | H | 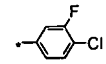 | 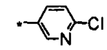 |
| 110 | H | (5R)-$CH_3$ | H | $CH(CH_3)_2$ | $CH_3$ | (5R)-$CH_3$ | H | 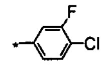 | 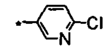 |
| 111 | H | (5S)-$CH_3$ | H | $CH(CH_3)_2$ | $CH_3$ | (5R)-$CH_3$ | H | 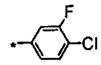 | 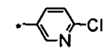 |
| 112 | H | (6R)-$CH_3$ | H | $CH(CH_3)_2$ | $CH_3$ | (5R)-$CH_3$ | H | 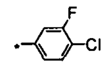 | 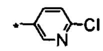 |
| 113 | H | (6S)-$CH_3$ | H | $CH(CH_3)_2$ | $CH_3$ | (5R)-$CH_3$ | H | 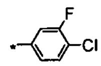 | 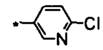 |
| 114 | H | (6S)-$CH_3$ | H | $CH(CH_3)_2$ | $CH_3$ | (5S)-$CH_3$ | H | 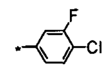 | 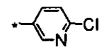 |
| 115 | H | (6R)-$CH_2CH_3$ | H | $CH(CH_3)_2$ | $CH_3$ | $CH_3$ | H | 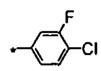 | 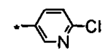 |
| 116 | H | (6S)-$CH_2CH_3$ | H | $CH(CH_3)_2$ | $CH_3$ | $CH_3$ | H | 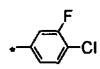 | 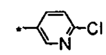 |
| 117 | H | $CH(CH_3)_2$ | H | $CH(CH_3)_2$ | $CH_3$ | $CH_3$ | H | 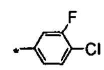 | 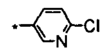 |
| 118 | H | $CH_2OH$ | H | $CH(CH_3)_2$ | $CH_3$ | $CH_3$ | H | 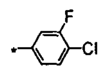 | 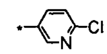 |
| 119 | H | $CH_2F$ | H | $CH(CH_3)_2$ | $CH_3$ | $CH_3$ | H | 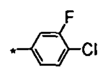 | 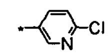 |
| 120 | H | $-CH_2-$ | | $CH(CH_3)_2$ | $CH_3$ | $CH_3$ | H | 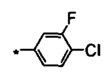 | 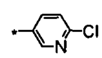 |
| 121 | $CH_3$ | H | H | $CH(CH_3)_2$ | $CH_3$ | $CH_3$ | H | 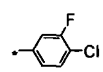 | 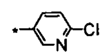 |
| 122 | $CH_3$ | (5R)-$CH_3$ | H | $CH(CH_3)_2$ | $CH_3$ | $CH_3$ | H | 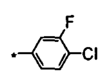 | 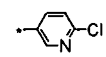 |
| 123 | $CH_3$ | (5S)-$CH_3$ | H | $CH(CH_3)_2$ | $CH_3$ | $CH_3$ | H | 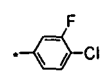 | 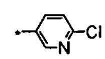 |
| 124 | $CH_3$ | (6R)-$CH_3$ | H | $CH(CH_3)_2$ | $CH_3$ | $CH_3$ | H | 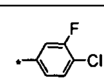 | 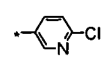 |
| 125 | $CH_3$ | (6S)-$CH_3$ | H | $CH(CH_3)_2$ | $CH_3$ | $CH_3$ | H | 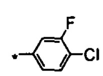 | 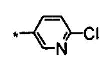 |

[Table 8]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 126 | $CH_3$ | $CH_2CH_3$ | H | $CH(CH_3)_2$ | $CH_3$ | $CH_3$ | H | 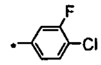 | 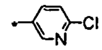 |
| 127 | $CH_2CH_2OH$ | H | H | $CH(CH_3)_2$ | $CH_3$ | $CH_3$ | H | 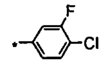 | 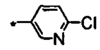 |
| 128 | $CH_2CH_2OH$ | $CH_3$ | H | $CH(CH_3)_2$ | $CH_3$ | $CH_3$ | H | 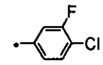 | 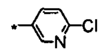 |
| 129 | $CH_2CH_3$ | H | H | $CH(CH_3)_2$ | $CH_3$ | $CH_3$ | H | 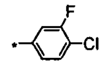 | 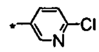 |
| 130 | $CH_2CH_3$ | $CH_3$ | H | $CH(CH_3)_2$ | $CH_3$ | $CH_3$ | H | 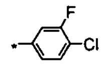 | 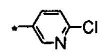 |
| 131 | CHO | $CH_3$ | H | $CH(CH_3)_2$ | $CH_3$ | $CH_3$ | H | 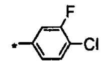 | 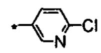 |
| 132 | CHO | $CH_2CH_3$ | H | $CH(CH_3)_2$ | $CH_3$ | $CH_3$ | H | 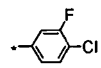 | 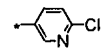 |
| 133 | $COCH_3$ | $CH_3$ | H | $CH(CH_3)_2$ | $CH_3$ | $CH_3$ | H | 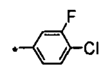 | 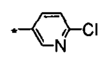 |
| 134 | $COCH_3$ | $CH_2CH_3$ | H | $CH(CH_3)_2$ | $CH_3$ | $CH_3$ | H | 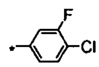 | 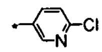 |
| 135 | $COCF_3$ | $CH_3$ | H | $CH(CH_3)_2$ | $CH_3$ | $CH_3$ | H | 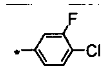 | 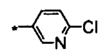 |
| 136 | $COCF_3$ | $CH_2CH_3$ | H | $CH(CH_3)_2$ | $CH_3$ | $CH_3$ | H | 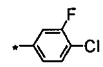 | 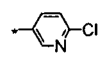 |
| 137 | H | H | H | $CH(CH_3)_2$ | $CH_3$ | $CH_2CH_3$ | H | 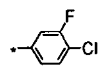 | 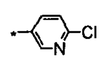 |
| 138 | H | $CH_3$ | H | $CH(CH_3)_2$ | $CH_3$ | $CH_2CH_3$ | H | 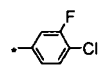 | 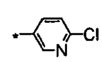 |
| 139 | H | $CH_3$ | H | $CH(CH_3)_2$ | $CH_3$ | $CH_2CH_3$ | H | 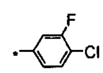 | 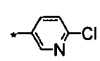 |
| 140 | H | $CH_3$ | $CH_3$ | $CH(CH_3)_2$ | $CH_3$ | $CH_2CH_3$ | H | 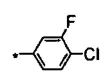 | 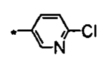 |
| 141 | H | $CH_2CH_3$ | H | $CH(CH_3)_2$ | $CH_3$ | $CH_2CH_3$ | H | 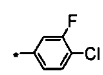 | 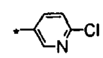 |
| 142 | H | $CH(CH_3)_2$ | H | $CH(CH_3)_2$ | $CH_3$ | $CH_2CH_3$ | H | 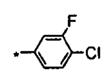 | 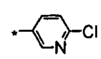 |
| 143 | H | $-CH_2-$ | | $CH(CH_3)_2$ | $CH_3$ | $CH_2CH_3$ | H | 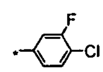 | 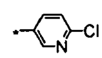 |

[Table 9]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 144 | $CH_3$ | H | H | $CH(CH_3)_2$ | $CH_3$ | $CH_2CH_3$ | H | 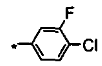 | 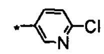 |
| 145 | $CH_3$ | $CH_3$ | H | $CH(CH_3)_2$ | $CH_3$ | $CH_2CH_3$ | H | 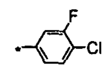 | 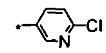 |
| 146 | $CH_3$ | $CH_2CH_3$ | H | $CH(CH_3)_2$ | $CH_3$ | $CH_2CH_3$ | H | 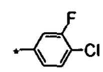 | 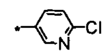 |
| 147 | $CH_2CH_2OH$ | H | H | $CH(CH_3)_2$ | $CH_3$ | $CH_2CH_3$ | H | 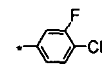 | 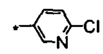 |
| 148 | $CH_2CH_2OH$ | $CH_3$ | H | $CH(CH_3)_2$ | $CH_3$ | $CH_2CH_3$ | H | 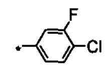 | 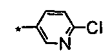 |
| 149 | CHO | $CH_3$ | H | $CH(CH_3)_2$ | $CH_3$ | $CH_2CH_3$ | H | 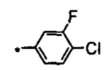 | 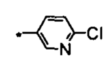 |
| 150 | $COCH_3$ | H | H | $CH(CH_3)_2$ | $CH_3$ | $CH_2CH_3$ | H | 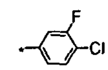 | 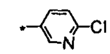 |
| 151 | $COCH_3$ | $CH_3$ | H | $CH(CH_3)_2$ | $CH_3$ | $CH_2CH_3$ | H | 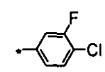 | 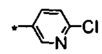 |
| 152 | $COCF_3$ | $CH_3$ | H | $CH(CH_3)_2$ | $CH_3$ | $CH_2CH_3$ | H | 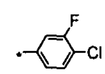 | 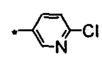 |
| 153 | H | H | H | $CH(CH_3)_2$ | $CH_3$ | $CH_2F$ | H | 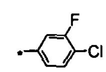 | 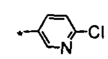 |
| 154 | H | $CH_3$ | H | $CH(CH_3)_2$ | $CH_3$ | $CH_2F$ | H | 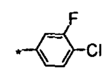 | 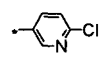 |
| 155 | H | $-CH_2-$ | | $CH(CH_3)_2$ | $CH_3$ | $CH_2F$ | H | 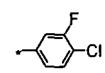 | 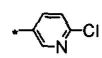 |
| 156 | $CH_3$ | H | H | $CH(CH_3)_2$ | $CH_3$ | $CH_2F$ | H | 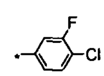 | 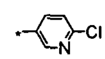 |
| 157 | $CH_3$ | $CH_3$ | H | $CH(CH_3)_2$ | $CH_3$ | $CH_2F$ | H | 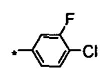 | 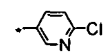 |
| 158 | $CH_2CH_2OH$ | H | H | $CH(CH_3)_2$ | $CH_3$ | $CH_2F$ | H | 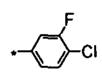 | 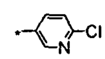 |
| 159 | $CH_2CH_2OH$ | $CH_3$ | H | $CH(CH_3)_2$ | $CH_3$ | $CH_2F$ | H | 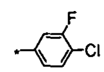 | 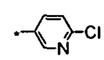 |
| 160 | H | H | H | $CH(CH_3)_2$ | $CH_3$ | $OCH_3$ | H | 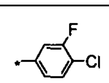 | 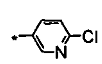 |
| 161 | H | $CH_3$ | H | $CH(CH_3)_2$ | $CH_3$ | $OCH_3$ | H | 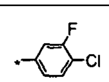 | 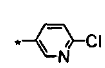 |

[Table 10]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 162 | H | $CH_3$ | $CH_3$ | CH $(CH_3)_2$ | $CH_3$ | $OCH_3$ | H | 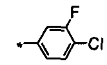 | 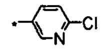 |
| 163 | H | $CH_2CH_3$ | H | CH $(CH_3)_2$ | $CH_3$ | $OCH_3$ | H | 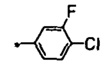 | 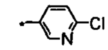 |
| 164 | H | $-CH_2-$ | | CH $(CH_3)_2$ | $CH_3$ | $OCH_3$ | H | 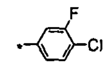 | 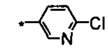 |
| 165 | $CH_3$ | H | H | CH $(CH_3)_2$ | $CH_3$ | $OCH_3$ | H | 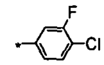 | 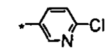 |
| 166 | $CH_3$ | $CH_3$ | H | CH $(CH_3)_2$ | $CH_3$ | $OCH_3$ | H | 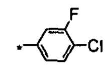 | 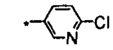 |
| 167 | $CH_3$ | $-CH_2-$ | | CH $(CH_3)_2$ | $CH_3$ | $OCH_3$ | H | 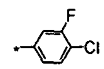 | 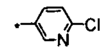 |
| 168 | $CH_2CH_2OH$ | H | H | CH $(CH_3)_2$ | $CH_3$ | $OCH_3$ | H | 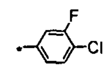 | 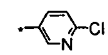 |
| 169 | $CH_2CH_2OH$ | $CH_3$ | H | CH $(CH_3)_2$ | $CH_3$ | $OCH_3$ | H | 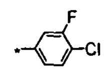 | 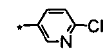 |
| 170 | H | H | H | CH $(CH_3)_2$ | $CH_3$ | $CH_3$ | OH | 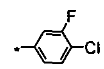 | 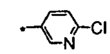 |
| 171 | H | $CH_3$ | H | CH $(CH_3)_2$ | $CH_3$ | $CH_3$ | OH | 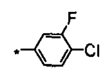 | 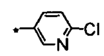 |
| 172 | H | H | H | CH $(CH_3)_2$ | $CH_3$ | $-CH_2-$ | | 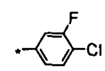 | 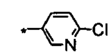 |
| 173 | H | $CH_3$ | H | CH $(CH_3)_2$ | $CH_3$ | (3-azabicyclo[3.1.0] hex-2-yl) | | 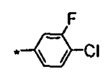 | 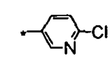 |
| 174 | H | $CH_3$ | H | CH $(CH_3)_2$ | $CH_3$ | (2-azabicyclo[3.1.0] hex-3-yl) | | 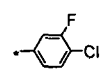 | 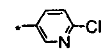 |
| 175 | H | $CH_2CH_3$ | H | CH $(CH_3)_2$ | $CH_3$ | $-CH_2-$ | | 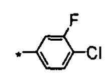 | 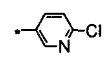 |
| 176 | H | $-CH_2-$ | | CH $(CH_3)_2$ | $CH_3$ | $-CH_2-$ | | 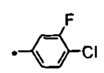 | 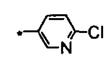 |
| 177 | $CH_3$ | $CH_3$ | H | CH $(CH_3)_2$ | $CH_3$ | $-CH_2-$ | | 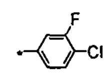 | 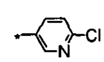 |
| 178 | $CH_3$ | $-CH_2-$ | | CH $(CH_3)_2$ | $CH_3$ | $-CH_2-$ | | 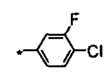 | 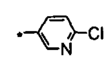 |
| 179 | H | H | H | CH $(CH_3)_2$ | $CH_3$ | -O- | | 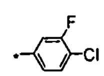 | 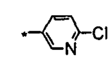 |

[Table 11]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 180 | H | $CH_3$ | H | $CH(CH_3)_2$ | $CH_3$ | -O- | | 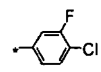 | 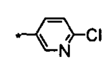 |
| 181 | $COCH_3$ | H | H | $CH(CH_3)_2$ | $CH_3$ | -O- | | 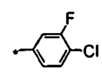 | 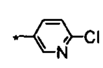 |
| 182 | $COCH_3$ | $CH_3$ | H | $CH(CH_3)_2$ | $CH_3$ | -O- | | 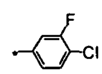 | 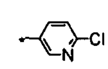 |
| 183 | H | $CH_3$ | H | $CH(CH_3)_2$ | $CH_3$ | H | H | 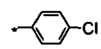 | 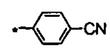 |
| 184 | H | H | H | $CH(CH_3)_2$ | $CH_3$ | $CH_3$ | H | 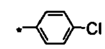 | 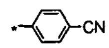 |
| 185 | H | $CH_3$ | H | $CH(CH_3)_2$ | $CH_3$ | $CH_3$ | H | 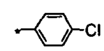 | 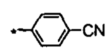 |
| 186 | $CH_3$ | H | H | $CH(CH_3)_2$ | $CH_3$ | $CH_3$ | H | 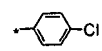 | 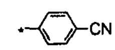 |
| 187 | $CH_3$ | $CH_3$ | H | $CH(CH_3)_2$ | $CH_3$ | $CH_3$ | H | 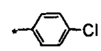 | 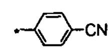 |
| 188 | H | H | H | $CH(CH_3)_2$ | $CH_3$ | H | H | 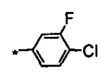 | 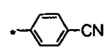 |
| 189 | H | $CH_3$ | H | $CH(CH_3)_2$ | $CH_3$ | H | H | 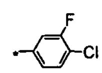 | 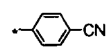 |
| 190 | H | H | H | $CH(CH_3)_2$ | $CH_3$ | $CH_3$ | H | 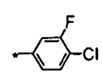 | 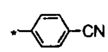 |
| 191 | H | $CH_3$ | H | $CH(CH_3)_2$ | $CH_3$ | $CH_3$ | H | 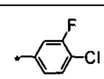 | 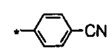 |
| 192 | H | $CH_3$ | H | $CH(CH_3)_2$ | $CH_3$ | H | H | 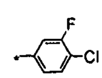 | 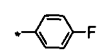 |
| 193 | H | H | H | $CH(CH_3)_2$ | $CH_3$ | $CH_3$ | H | 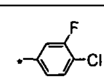 | 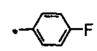 |
| 194 | H | $CH_3$ | H | $CH(CH_3)_2$ | $CH_3$ | $CH_3$ | H | 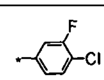 | 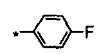 |
| 195 | H | $CH_3$ | H | $CH(CH_3)_2$ | $CH_3$ | H | H | 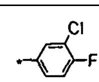 | 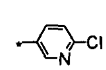 |
| 196 | H | H | H | $CH(CH_3)_2$ | $CH_3$ | $CH_3$ | H | 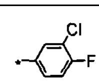 | 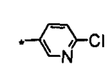 |
| 197 | H | $CH_3$ | H | $CH(CH_3)_2$ | $CH_3$ | $CH_3$ | H | 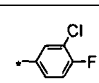 | 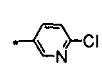 |

[Table 12]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 198 | H | $CH_3$ | H | $CH(CH_3)_2$ | $CH_3$ | H | H | 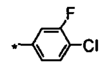 | 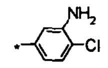 |
| 199 | H | H | H | $CH(CH_3)_2$ | $CH_3$ | $CH_3$ | H | 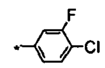 | 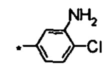 |
| 200 | H | $CH_3$ | H | $CH(CH_3)_2$ | $CH_3$ | $CH_3$ | H | 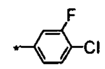 | 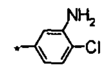 |
| 201 | H | $CH_3$ | H | $CH(CH_3)_2$ | $CH_3$ | H | H | 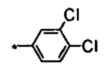 | 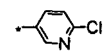 |
| 202 | H | H | H | $CH(CH_3)_2$ | $CH_3$ | $CH_3$ | H | 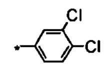 | 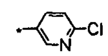 |
| 203 | H | $CH_3$ | H | $CH(CH_3)_2$ | $CH_3$ | $CH_3$ | H | 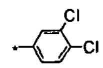 | 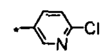 |
| 204 | H | $CH_3$ | H | $CH(CH_3)_2$ | $CH_3$ | H | H | 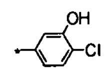 | 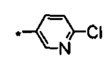 |
| 205 | H | H | H | $CH(CH_3)_2$ | $CH_3$ | $CH_3$ | H | 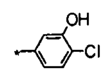 | 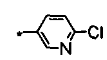 |
| 206 | H | $CH_3$ | H | $CH(CH_3)_2$ | $CH_3$ | $CH_3$ | H | 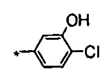 | 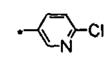 |
| 207 | H | $CH_3$ | H | $CH(CH_3)_2$ | $CH_3$ | H | H | 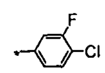 | 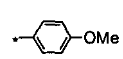 |
| 208 | H | H | H | $CH(CH_3)_2$ | $CH_3$ | $CH_3$ | H | 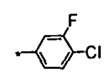 | 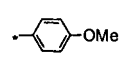 |
| 209 | H | $CH_3$ | H | $CH(CH_3)_2$ | $CH_3$ | $CH_3$ | H | 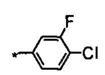 | 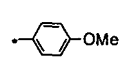 |
| 210 | H | $CH_3$ | H | $CH(CH_3)_2$ | $CH_3$ | H | H | 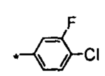 | 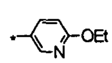 |
| 211 | H | $CH_2CH_3$ | H | $CH(CH_3)_2$ | $CH_3$ | H | H | 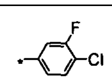 | 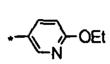 |
| 212 | H | H | H | $CH(CH_3)_2$ | $CH_3$ | $CH_3$ | H | 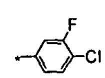 | 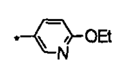 |
| 213 | H | $CH_3$ | H | $CH(CH_3)_2$ | $CH_3$ | $CH_3$ | H | 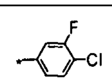 | 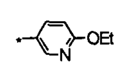 |
| 214 | H | $CH_2CH_3$ | H | $CH(CH_3)_2$ | $CH_3$ | $CH_3$ | H | 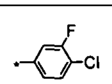 | 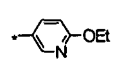 |
| 215 | H | $CH_3$ | H | $CH(CH_3)_2$ | $CH_3$ | H | H | 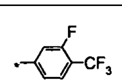 | 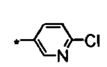 |

[Table 13]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 216 | H | $CH_2CH_3$ | H | $CH(CH_3)_2$ | $CH_3$ | H | H | 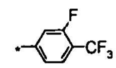 | 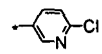 |
| 217 | H | H | H | $CH(CH_3)_2$ | $CH_3$ | $CH_3$ | H | 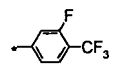 | 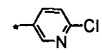 |
| 218 | H | $CH_3$ | H | $CH(CH_3)_2$ | $CH_3$ | $CH_3$ | H | 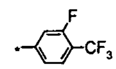 | 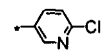 |
| 219 | H | $CH_2CH_3$ | H | $CH(CH_3)_2$ | $CH_3$ | $CH_3$ | H | 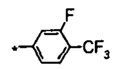 | 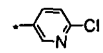 |
| 220 | H | $CH_3$ | H | $CH(CH_3)_2$ | $CH_3$ | H | H | 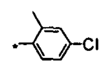 | 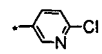 |
| 221 | H | $CH_2CH_3$ | H | $CH(CH_3)_2$ | $CH_3$ | H | H | 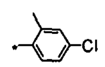 | 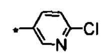 |
| 222 | H | H | H | $CH(CH_3)_2$ | $CH_3$ | $CH_3$ | H | 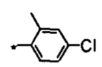 | 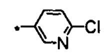 |
| 223 | H | $CH_3$ | H | $CH(CH_3)_2$ | $CH_3$ | $CH_3$ | H | 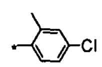 | 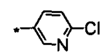 |
| 224 | H | $CH_2CH_3$ | H | $CH(CH_3)_2$ | $CH_3$ | $CH_3$ | H | 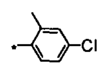 | 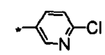 |
| 225 | H | $CH_3$ | H | $CH(CH_3)_2$ | $CH_3$ | H | H | 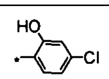 | 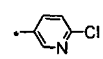 |
| 226 | H | H | H | $CH(CH_3)_2$ | $CH_3$ | $CH_3$ | H | 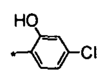 | 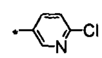 |
| 227 | H | $CH_3$ | H | $CH(CH_3)_2$ | $CH_3$ | $CH_3$ | H | 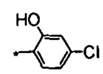 | 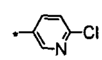 |
| 228 | H | $CH_3$ | H | $CH(CH_3)_2$ | $CH_3$ | H | H | 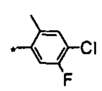 | 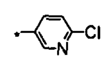 |
| 229 | H | $CH_2CH_3$ | H | $CH(CH_3)_2$ | $CH_3$ | H | H | 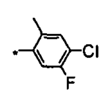 | 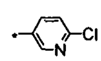 |
| 230 | H | $CH_3$ | H | $CH(CH_3)_2$ | $CH_3$ | $CH_3$ | H | 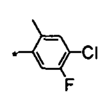 | 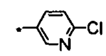 |
| 231 | H | $CH_2CH_3$ | H | $CH(CH_3)_2$ | $CH_3$ | $CH_3$ | H | 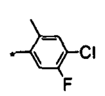 | 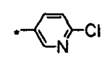 |

[Table 14]

| No. | $R^1$ | $R^2$ | $R^3$ | $R^4$ | $R^5$ | $R^6$ | $R^7$ | $Ar_1$ | $Ar_2$ |
|---|---|---|---|---|---|---|---|---|---|
| 232 | H | $CH_3$ | H | $CH(CH_3)_2$ | $CH_3$ | H | H | 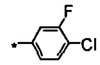 | 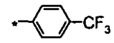 |
| 233 | H | H | H | $CH(CH_3)_2$ | $CH_3$ | $CH_3$ | H | 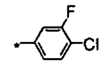 | 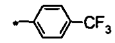 |
| 234 | H | $CH_3$ | H | $CH(CH_3)_2$ | $CH_3$ | $CH_3$ | H | 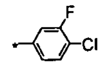 | 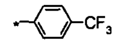 |
| 235 | H | $CH_3$ | H | $CH(CH_3)_2$ | $CH_3$ | H | H | 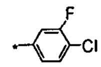 | 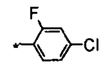 |
| 236 | H | $CH_2CH_3$ | H | $CH(CH_3)_2$ | $CH_3$ | H | H | 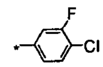 | 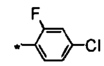 |
| 237 | H | H | H | $CH(CH_3)_2$ | $CH_3$ | $CH_3$ | H | 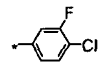 | 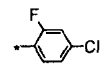 |
| 238 | H | $CH_3$ | H | $CH(CH_3)_2$ | $CH_3$ | $CH_3$ | H | 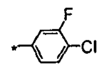 | 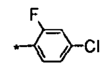 |
| 239 | H | $CH_2CH_3$ | H | $CH(CH_3)_2$ | $CH_3$ | $CH_3$ | H | 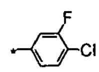 | 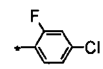 |
| 240 | H | $CH_3$ | H | $CH(CH_3)_2$ | $CH_3$ | H | H | 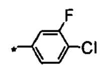 | 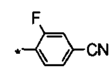 |
| 241 | H | H | H | $CH(CH_3)_2$ | $CH_3$ | $CH_3$ | H | 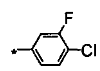 | 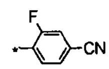 |
| 242 | H | $CH_3$ | H | $CH(CH_3)_2$ | $CH_3$ | $CH_3$ | H | 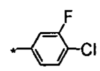 | 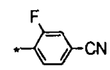 |

[Compounds wherein W is represented by general formula (2b)]

[Table 15]

| No. | $R^1$ | $R^2$ | $R^3$ | $R^4$ | $R^5$ | $R^8$ | $R^9$ | $R^{10}$ | $Ar_1$ | $Ar_2$ |
|---|---|---|---|---|---|---|---|---|---|---|
| 243 | H | H | H | $CH(CH_3)_2$ | $CH_3$ | $CH(CH_3)_2$ | H | H | 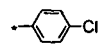 | 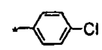 |
| 244 | $CH_3$ | H | H | $CH(CH_3)_2$ | $CH_3$ | $CH(CH_3)_2$ | H | H | 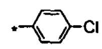 | 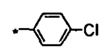 |
| 245 | H | H | H | $CH(CH_3)_2$ | $CH_3$ | $CH_2CH_3$ | $CH_3$ | H | 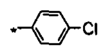 | 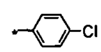 |
| 246 | H | H | H | $CH(CH_3)_2$ | $CH_3$ | $CH_2CH_3$ | $-CH_2-$ | | 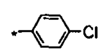 | 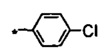 |
| 247 | $CH_3$ | H | H | $CH(CH_3)_2$ | $CH_3$ | $CH_2CH_3$ | $-CH_2-$ | | 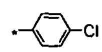 | 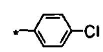 |
| 248 | H | H | H | $CH(CH_3)_2$ | $CH_3$ | $CH_2CH_2OH$ | H | H | 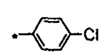 | 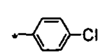 |

(continued)

| No. | $R^1$ | $R^2$ | $R^3$ | $R^4$ | $R^5$ | $R^8$ | $R^9$ | $R^{10}$ | $Ar_1$ | $Ar_2$ |
|---|---|---|---|---|---|---|---|---|---|---|
| 249 | $CH_3$ | H | H | $CH(CH_3)_2$ | $CH_3$ | $CH_2CH_2OH$ | H | H | 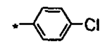 | 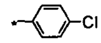 |
| 250 | H | H | H | $CH(CH_3)_2$ | $CH_3$ | $CH_2CH_2OH$ | $CH_3$ | H | 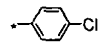 | 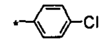 |
| 251 | H | H | H | $CH(CH_3)_2$ | $CH_3$ | $CH_2CH_2OH$ | $-CH_2-$ | | 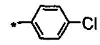 | 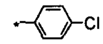 |
| 252 | H | H | H | $CH(CH_3)_2$ | $CH_3$ | $CH_2CH_3$ | H | H | 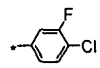 | 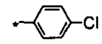 |
| 253 | H | H | H | $CH(CH_3)_2$ | $CH_3$ | $CH_2CH_3$ | $CH_3$ | H | 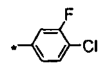 | 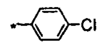 |
| 254 | H | H | H | $CH(CH_3)_2$ | $CH_3$ | $CH_2CH_3$ | $- CH_2-$ | | 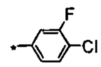 | 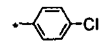 |
| 255 | H | H | H | $CH(CH_3)_2$ | $CH_3$ | $CH_2CH_3$ | H | H | 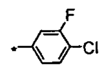 | 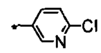 |
| 256 | H | H | H | $CH(CH_3)_2$ | $CH_3$ | $CH_2CH_3$ | $CH_3$ | H | 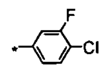 | 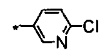 |
| 257 | H | H | H | $CH(CH_3)_2$ | $CH_3$ | $CH_2CH_3$ | $-CH_2-$ | | 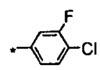 | 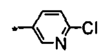 |

Next, a representative method for producing a compound represented by general formula (1) will be explained. The compound of the present invention can be produced by various production methods and the following production methods are illustrative and should not be construed in any limitative way. Reactions shown below can be performed by protecting substituents with appropriate protective groups, if necessary, and the types of protective groups are not particularly limited.

First, a method for producing a compound (1a) represented by general formula (1) wherein W is represented by general formula (2a) will be explained.

[Production Method 1]

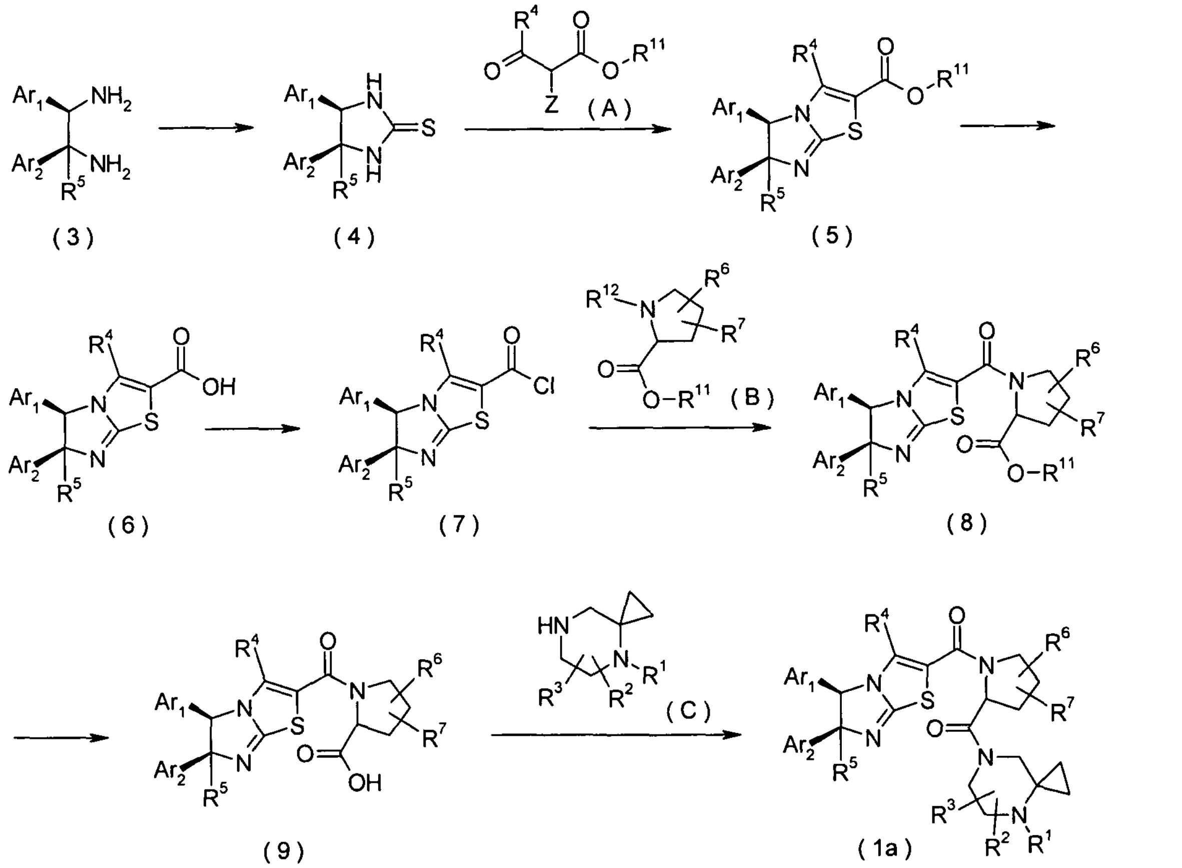


(3) (4) (5) (6) (7) (8) (9) (1a)

wherein Z means a halogen atom such as a chlorine atom or a bromine atom; $R^{11}$ means a protective group for the carboxy group; and $Ar_1$, $Ar_2$, and $R^1$ to $R^7$ have the same meanings as defined above.
Examples of the protective group for the carboxy group include substituted or unsubstituted alkyl groups or aralkyl groups such as a methyl group, an ethyl group, a tert-butyl group, and a benzyl group.

Synthesis of compound (4)

A compound (4) can be obtained by reacting an optically active diamine compound (3) having the positional configuration shown above with carbon disulfide. Here, the solvent used in the reaction is not particularly limited and examples thereof include ethanol, tetrahydrofuran, dioxane, acetonitrile, dimethylformamide, chloroform, toluene, and mixed solvents thereof. The reaction temperature is usually in the range from -78 to 100˚C or the boiling point of the solvent, preferably in the range from around room temperature to 100˚C.

Synthesis of compound (5)

A compound (5) can be obtained by reacting compound (4) with a compound (A). Here, the solvent used in the reaction is not particularly limited and examples thereof include ethanol, tetrahydrofuran, dioxane, acetonitrile, dimethylformamide, chloroform, toluene, and mixed solvents thereof. The reaction temperature is usually in the range from -78 to 100˚C or the boiling point of the solvent, preferably in the range from around room temperature to 100˚C.

Synthesis of compound (6)

This step is performed for the elimination of $R^{11}$.
Although deprotection reaction conditions differ depending on the type of $R^{11}$, $R^{11}$ may be deprotected by hydrolysis. When $R^{11}$ is a methyl group, an ethyl group, a benzyl group, or the like, a compound (6) can be obtained by treating compound (5) with a base (e.g., sodium hydroxide, potassium hydroxide, lithium hydroxide, or potassium tert-butoxide) or hydrochloric acid, p-toluenesulfonic acid, or the like. Here, examples of the solvent used in the reaction

include methanol, ethanol, water, tetrahydrofuran, dioxane, and mixed solvents thereof. However, organic solvents that can be mixed with water in an arbitrary ratio are preferred. The reaction temperature is usually in the range from -78 to 100˚C or the boiling point of the solvent, preferably in the range from around room temperature to 100˚C.

When $R^{11}$ is a tert-butyl group or the like, compound (5) is preferably treated with trifluoroacetic acid or hydrochloric acid or the like. Here, the solvent used in the reaction is not particularly limited and examples thereof include dichloromethane, chloroform, and mixed solvents thereof. The reaction temperature is usually in the range from -78 to 100˚C or the boiling point of the solvent, preferably in the range from -20˚C to around room temperature.

Synthesis of compound (7)

A compound (7) can be obtained by reacting compound (6) with an acid halogenating reagent such as thionyl chloride, oxalyl chloride, or phosphorus oxychloride. Here, the solvent used in the reaction is not particularly limited and examples thereof include dichloromethane, chloroform, toluene, and mixed solvents thereof. Alternatively, the reaction can be performed in the absence of a solvent. The reaction temperature is usually in the range from -78 to 100˚C or the boiling point of the solvent, preferably in the range from 0 to 100˚C.

Synthesis of compound (8)

A compound (8) can be obtained by reacting compound (7) with a compound (B) in the presence of a base. Examples of the base used can include organic bases such as triethylamine, diisopropylethylamine, 4-dimethylaminopyridine, N-methylmorpholine, pyridine, and 2,6-lutidine, and diazabicyclo[5.4.0]undec-7-ene, and inorganic bases such as potassium carbonate, sodium carbonate, and sodium bicarbonate. Here, the solvent used in the reaction is not particularly limited and examples thereof include dichloromethane, chloroform, tetrahydrofuran, ethyl acetate, toluene, and mixed solvents thereof. However, dried solvents are preferred. The reaction temperature is usually in the range from -78 to 100˚C or the boiling point of the solvent, preferably in the range from -10˚C to around room temperature.

Synthesis of compound (9)

A compound (9) can be obtained by performing deprotection under the reaction conditions used in the method for producing compound (6).

Synthesis of compound (1a)

A compound (1a) can be obtained by reacting compound (9) with a compound (C) in the presence of a condensing agent. Examples of the condensing agent used can include N,N'-dicyclohexylcarbodiimide and 1-ethyl-3-(3-dimethylaminopropyl)carbodiimide. The solvent used in the reaction is not particularly limited and examples thereof include dichloromethane, dimethylformamide, tetrahydrofuran, ethyl acetate, and mixed solvents thereof. The reaction temperature is usually in the range from -78 to 100˚C or the boiling point of the solvent, preferably in the range from 0 to 50˚C.

Moreover, a base such as triethylamine, diisopropylethylamine, N-methylmorpholine, or 4-dimethylaminopyridine can be added, if necessary. Furthermore, 1-hydroxybenzotriazole, N-hydroxysuccinimide, or the like may be added as a reaction accelerator.

A compound (1a) of the present invention can also be produced by the following method.

[Production Method 2]

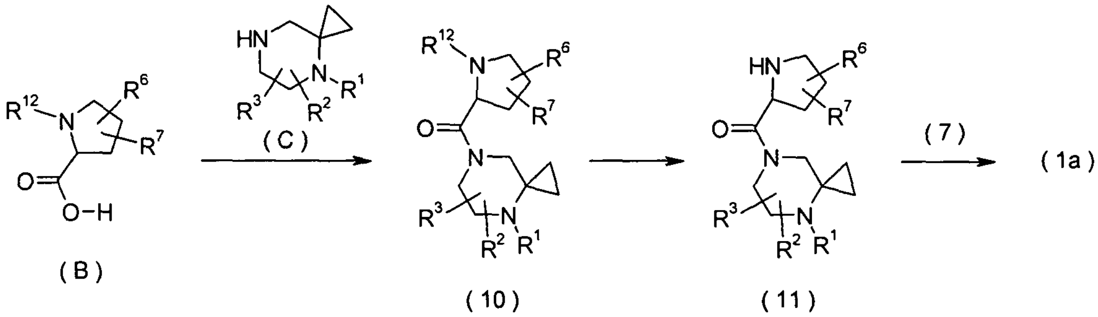


(B) (10) (11) (1a)

wherein $R^{12}$ means a protective group for the amino group; and $R^1$ to $R^3$, $R^6$, and $R^7$ have the same meanings as defined above.
Examples of the protective group for the amino group include a benzyloxycarbonyl group, a tert-butyloxycarbonyl group, and a benzyl group.

Synthesis of compound (10)

A compound (10) can be obtained from a compound (B) and a compound (C) under the reaction conditions used in the method for producing compound (1a) (amidation of compound (9) with a compound (C)) according to Production Method 1 above.

Synthesis of compound (11)

Deprotection reaction conditions differ depending on the type of $R^{12}$. When $R^{12}$ is a benzyloxycarbonyl group or a benzyl group or the like, deprotection can be performed by adding a reduction catalyst such as palladium carbon and reacting compound (10) in a hydrogen atmosphere or in the presence of a hydrogen source such as ammonium formate. Here, the solvent used in the reaction is not particularly limited and examples thereof include alcohols such as methanol and ethanol, tetrahydrofuran, dioxane, ethyl acetate, water, and mixed solvents thereof. The reaction temperature is usually in the range from -78 to 100˚C or the boiling point of the solvent, preferably in the range from 0˚C to around room temperature.

Moreover, when $R^{12}$ is a tert-butyloxycarbonyl group or the like, deprotection can be performed by treating the compound (10) with trifluoroacetic acid or hydrochloric acid or the like. Here, the reaction solvent used is not particularly limited and examples thereof include dichloromethane, chloroform, tetrahydrofuran, dioxane, methanol, ethanol, water, and mixed solvents thereof. The reaction temperature is usually in the range from -78 to 100˚C or the boiling point of the solvent, preferably in the range from 0˚C to around room temperature.

Synthesis of compound (1a)

A compound (1a) can be obtained from compound (11) and compound (7) under the reaction conditions used in the method for producing compound (8) (amidation of compound (7) with a compound (B)) according to Production Method 1 above.

Next, a method for producing a compound (1b) represented by general formula (1) wherein W is represented by general formula (2b) will be explained.

[Production Method 3]

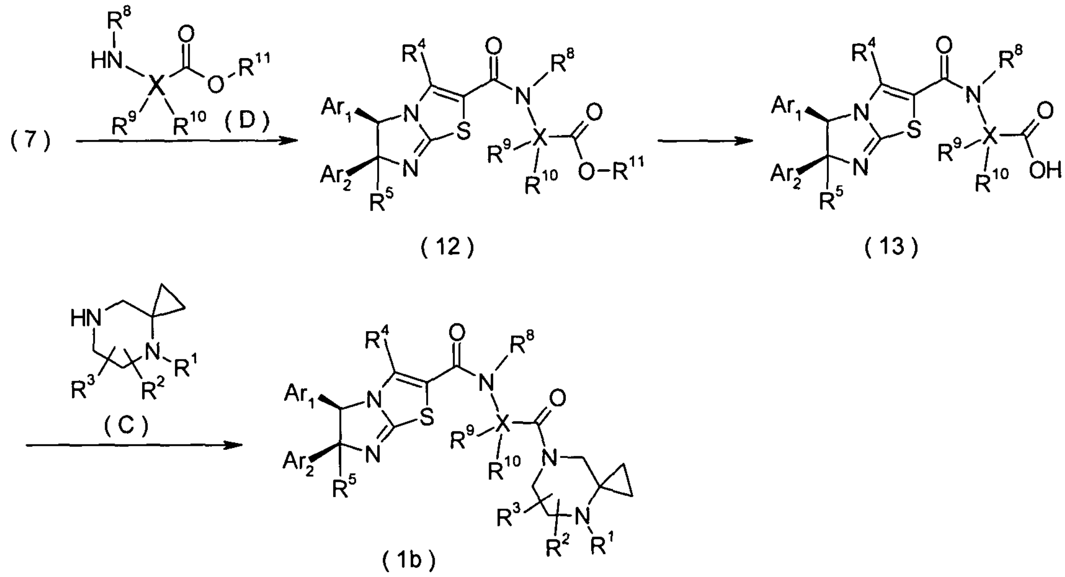

(7) (D) (12) (13) (C) (1b)

wherein $R^{11}$ means a protective group for the carboxy group; and $Ar_1$, $Ar_2$, $R^1$ to $R^5$, $R^8$ to $R^{10}$, and X have the same meanings as defined above.
Examples of the protective group for the carboxy group include a methyl group, an ethyl group, a tert-butyl group, and a benzyl group.

Synthesis of compound (12)

A compound (12) can be obtained from compound (7) and a compound (D) under the reaction conditions used in the method for producing compound (8) (amidation of compound (7) with a compound (B)) according to Production Method 1 above.

Synthesis of compound (13)

A compound (13) can be obtained by deprotecting compound (12) under the reaction conditions used in the method for producing compound (6) according to Production Method 1 above.

Synthesis of compound (1b)

A compound (1b) can be obtained from compound (13) and a compound (C) under the reaction conditions used in the method for producing compound (1a) (amidation of compound (9) with a compound (C)) or the method for producing compound (8) (amidation of compound (7) with a compound (B)) according to Production Method 1 above.
When $R^1$ in compound (1) of the present invention is a tert-butoxycarbonyl group, a benzyloxycarbonyl group, or a trifluoroacetyl group, or the like usually used as a protective group, a compound (1) wherein $R^1$ is a hydrogen atom can be obtained under the reaction conditions used in the method for producing compound (11) (deprotection of $R^{12}$ in compound (10)) according to Production Method 2 above or by treatment with a base such as sodium hydroxide, potassium hydroxide, sodium carbonate, or potassium carbonate in a mixed solvent of methanol or ethanol and water. Furthermore, when $R^1$ is a hydrogen atom, it can be converted to the defined substituent(s) using a conventional organic chemical method. For example, $R^1$ can be converted to an alkyl group, an alkenyl group, an alkynyl group, an aralkyl group, or the like by treatment with a reducing agent such as sodium cyanoborohydride or sodium triacetoxyborohydride in the presence of an aldehyde derivative. Moreover, $R^1$ can be converted to an alkanoyl group or an alkylsulfonyl group by reaction with an acid chloride derivative in the presence of a base such as triethylamine.
The starting material compound (3) can be synthesized according to the method described in the document (Synlett, 1998, 623 or US2005/26916). Moreover, the compound (3) can also be synthesized by the following method.

[Production Method 4]

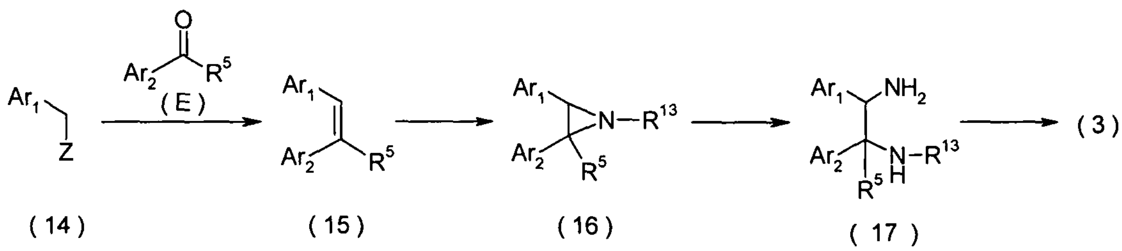

(E)

(14) (15) (16) (17) (3)

wherein Z means a halogen atom such as a chlorine atom or a bromine atom; $R^{13}$ means trichloroethyloxysulfonyl group, a p-toluenesulfonyl group, or the like; and $Ar_1$, $Ar_2$, and $R^5$ have the same meanings as defined above.

Synthesis of compound (15)

A compound (15) can be obtained by treating a phosphonium salt or a phosphonic acid ester obtained from a reaction of a compound (14) and an organic phosphorous compound such as triphenylphosphine or triethyl phosphite with a base such as alkyl lithium, lithium diisopropylamide, lithium bis(trimethylsilyl)amide, sodium hydride, or potassium tert-butoxide and then reacting the resulting product with a compound (E). The solvent used in this reaction is not particularly limited and examples thereof include diethyl ether, tetrahydrofuran, toluene, dimethylformamide, dimethyl sulfoxide, and mixed solvents thereof. However, dried solvents are preferred. The reaction temperature is usually in the range from -78 to 100˚C or the boiling point of the solvent, preferably in the range from -78˚C to room temperature.

Synthesis of compound (16)

A compound (16) can be synthesized according to synthesis methods reported in various documents (e.g., Tetrahedron Lett., 2005, 46 4031; J. Am. Chem. Soc., 2002, 124, 136672; J. Am. Chem. Soc., 2001, 123, 7707; Synlett, 2004, 525; and Japanese Patent Laid-Open No. 2000-72743).

A compound (16) can be obtained by reacting compound (15) with an alkoxysulfonamide derivative or an arylsulfonamide derivative in the presence of a rhodium catalyst after addition of an oxidizing agent such as iodosobenzene acetate and a base such as magnesium oxide. The solvent used in this reaction is not particularly limited and examples thereof include diethyl ether, tetrahydrofuran, toluene, acetonitrile, and mixed solvents thereof. However, dried solvents are preferred. The reaction temperature is usually in the range from -78 to 100˚C or the boiling point of the solvent, preferably in the range from -20 to 80˚C.

Synthesis of compound (17)

A compound (17) can be obtained by treating compound (16) with ammonia water. The solvent used in this reaction is not particularly limited and examples thereof include methanol, ethanol, water, tetrahydrofuran, dioxane, and mixed solvents thereof. However, organic solvents that can be mixed with water in an arbitrary ratio are preferred. The reaction temperature is usually in the range from -78 to 100˚C or the boiling point of the solvent, preferably in the range from room temperature to 80˚C.

Synthesis of compound (3)

A compound (3) can be obtained by treating compound (17) with hydrochloric acid, sulfuric acid, or trifluoroacetic acid, or the like. The solvent used in this reaction is not particularly limited and examples thereof include methanol, ethanol, water, tetrahydrofuran, dioxane, and mixed solvents thereof. However, organic solvents that can be mixed with water in an arbitrary ratio are preferred. The reaction temperature is usually in the range from -78 to 100˚C or the boiling point of the solvent, preferably in the range from room temperature to 80˚C.

The racemic compound (3) obtained by the production method can be optically resolved according to the method described in the document (US2005/26916, Japanese Patent Laid-Open No. 2005-75754, and Tetrahedron Asymmetry, 1995, 6, 3). For example, the racemic compound (3) can be treated with an optical resolving agent such as L-(+)-tartaric acid in a mixed solvent of methanol or ethanol and water to give crystalline tartrate. This tartrate is treated with a base such as sodium hydroxide to give an optically active diamine (3) having the positional configuration shown above.

[Production Method 5]

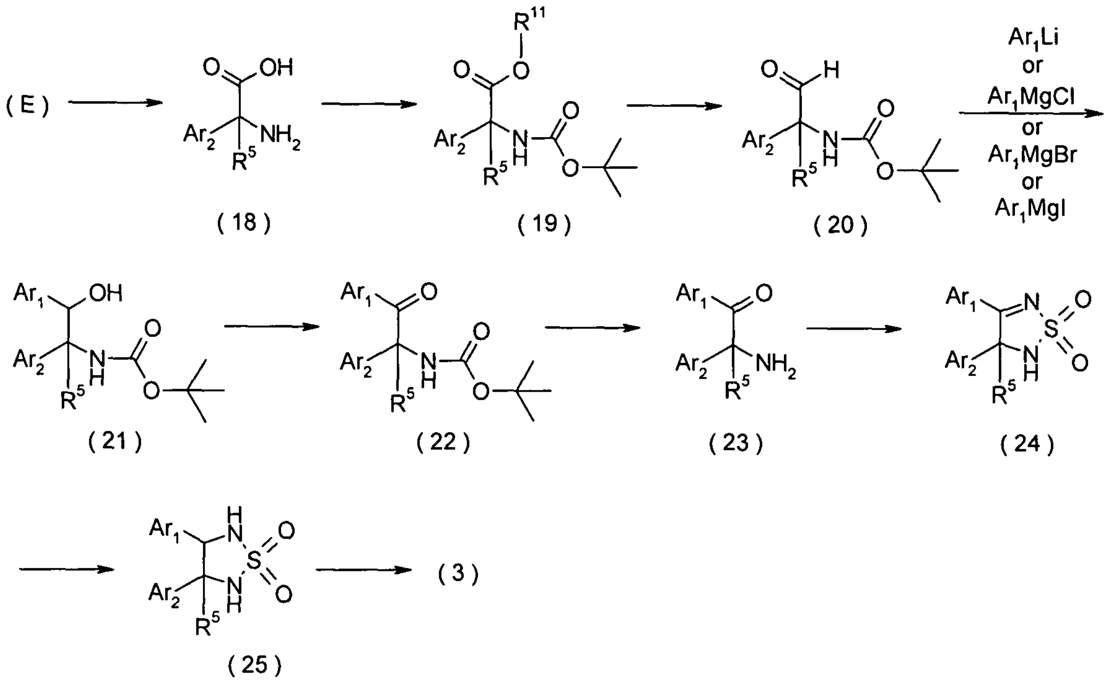


( E ) ( 18 ) ( 19 ) ( 20 ) ( 21 ) ( 22 ) ( 23 ) ( 24 ) ( 25 ) ( 3 )

wherein $R^{11}$ means a protective group for the carboxyl group; and $Ar_1$, $Ar_2$, and $R^5$ have the same meanings as defined above.

Synthesis of compound (18)

A compound (18) can be obtained by treating an aminonitrile form obtained from a reaction (Strecker reaction) of a compound (E) with potassium cyanide or sodium cyanide, ammonium chloride, and ammonia water with a mineral acid (e.g., hydrochloric acid or sulfuric acid) or an organic acid (e.g., p-toluenesulfonic acid or methanesulfonic acid). Here, the solvent used in the reaction is not particularly limited and examples thereof include methanol, ethanol, water, tetrahydrofuran, dioxane, and mixed solvents thereof. The reaction temperature is usually in the range from -20 to 100˚C or the boiling point of the solvent, preferably in the range from around room temperature to 100˚C.

Synthesis of compound (19)

Each of the carboxyl group and the amino group in the compound (18) may be protected according to a standard method. Here, the order in which protective groups are introduced is not particularly limited. Hereinafter, each reaction will be described. Esterification can be performed by treatment with a halogenating reagent such as hydrogen chloride, sulfuric acid, or thionyl chloride in a lower alcohol appropriate for $R^{11}$, such as methanol or ethanol. The reaction temperature is usually in the range from -78 to 100˚C or the boiling point of the solvent, preferably in the range from 0 to 100˚C. Moreover, the tert-butoxycarbonylation of the amino group can be performed by reaction with di-tert-butyl dicarbonate in the presence of a base such as triethylamine, diisopropylethylamine, or 4-dimethylaminopyridine. Here, the solvent used in the reaction is not particularly limited and examples thereof include dichloromethane, chloroform, tetrahydrofuran, ethyl acetate, acetonitrile, toluene, and mixed solvents thereof. However, dried solvents are preferred. The reaction temperature is usually in the range from -78 to 100˚C or the boiling point of the solvent, preferably in the range from 0 to 100˚C.

Synthesis of compound (20)

The ester group in compound (19) can be reduced by reaction using a water-reactive reagent such as lithium aluminum hydride or lithium borohydride at a temperature equal to or lower than room temperature (preferably -40 to 0˚C) in an aprotic solvent such as diethyl ether, tetrahydrofuran, or dioxane to give an alcohol form. Moreover, the reaction using sodium borohydride or the like can be performed at a temperature equal to or lower than room temperature (preferably -20˚C to around room temperature) using a protic solvent such as methanol, ethanol, or water, or a mixed solvent of the protic solvent and the aprotic solvent. The alcohol form thus obtained can be converted to a compound (20) by reaction with an oxidizing agent such as chromic acid [pyridinium chlorochromate (PCC), pyridinium dichromate (PDC), etc.], dimethyl sulfoxide with oxalyl chloride (Swern oxidation), dimethyl sulfoxide with acetic anhydride, dimethyl sulfoxide with a sulfur trioxide-pyridine complex, 1,1,1-triacetoxy-1,1-dihydro-1,2-benziodoxol-3(1H)-one (Dess-Martin reagent), or 2,2,6,6-tetramethyl-1-piperidinyloxy (TEMPO) with hypochlorous acid. Here, the solvent used in the reaction is not particularly limited and examples thereof include dichloromethane, chloroform, tetrahydrofuran, ethyl acetate, dimethyl sulfoxide, water, toluene, and mixed solvents thereof. The reaction temperature is usually in the range from -78 to 100˚C or the boiling point of the solvent, preferably in the range from -78˚C to around room temperature.

Synthesis of compound (21)

A compound (21) can be obtained by reacting compound (20) with an aryl lithium compound ($Ar_1Li$) or a Grignard reagent ($Ar_1MgCl$, $Ar_1MgBr$, or $Ar_1MgI$). The corresponding aryl lithium compound or Grignard reagent is a commercially available product or can be synthesized according to a standard method. The Grignard reagent can be synthesized from the corresponding aryl halide and magnesium metal, and the organic lithium reagent can be synthesized by halogen-metal exchange from the corresponding aryl halide and a commercially available alkyl lithium reagent or the like.

Here, the solvent used in the reaction is not particularly limited and examples thereof include diethyl ether, tetrahydrofuran, dioxane, toluene, and mixed solvents thereof. However, dried solvents are preferred. The reaction temperature is usually in the range from -78 to 100˚C or the boiling point of the solvent, preferably in the range from -78˚C to around room temperature.

Synthesis of compound (22)

A compound (22) can be obtained by appropriately selecting and using the oxidation reaction conditions described in the method for producing compound (20).

Synthesis of compound (23)

Compound (22) can be deprotected by treatment with trifluoroacetic acid or hydrochloric acid or the like. Here, the solvent used in the reaction is not particularly limited and examples thereof include dichloromethane, chloroform, tetrahydrofuran, dioxane, methanol, ethanol, water, and mixed solvents thereof. The reaction temperature is usually in the range from -78 to 100˚C or the boiling point of the solvent, preferably in the range from 0˚C to around room temperature.

Synthesis of compound (24)

A compound (24) can be obtained by heating compound (23) in the presence of sulfamide and a base such as 1,8-diazabicyclo[5.4.0]undec-7-ene. Here, the solvent used in the reaction is not particularly limited and examples thereof include diethyl ether, tetrahydrofuran, dioxane, ethyl acetate, methanol, ethanol, isopropyl alcohol, ethylene glycol, acetonitrile, toluene, and mixed solvents thereof. The reaction temperature is usually in the range from -78 to 180˚C or the boiling point of the solvent, preferably in the range from 70 to 150˚C.

Synthesis of compound (25)

A compound (25) can be obtained from compound (24) by appropriately selecting and using the reduction reaction conditions described in the method for producing compound (20).

Synthesis of compound (3)

A compound (3) can be synthesized by reacting compound (25) according to the method described in the document (Synlett, 1998, 623-624 and US2005/0026916). Moreover, in another method, a compound (3) can be obtained

by hydrolyzing compound (25) in the presence of a base such as pyridine or ethylenediamine. Here, examples of the solvent used in the reaction include methanol, ethanol, water, tetrahydrofuran, dioxane, ethylene glycol, and mixed solvents thereof. However, organic solvents that can be mixed with water in an arbitrary ratio are preferred. The reaction temperature is usually in the range from 0 to 180°C or the boiling point of the solvent, preferably in the range from 60 to 120°C.

[Production Method 6]

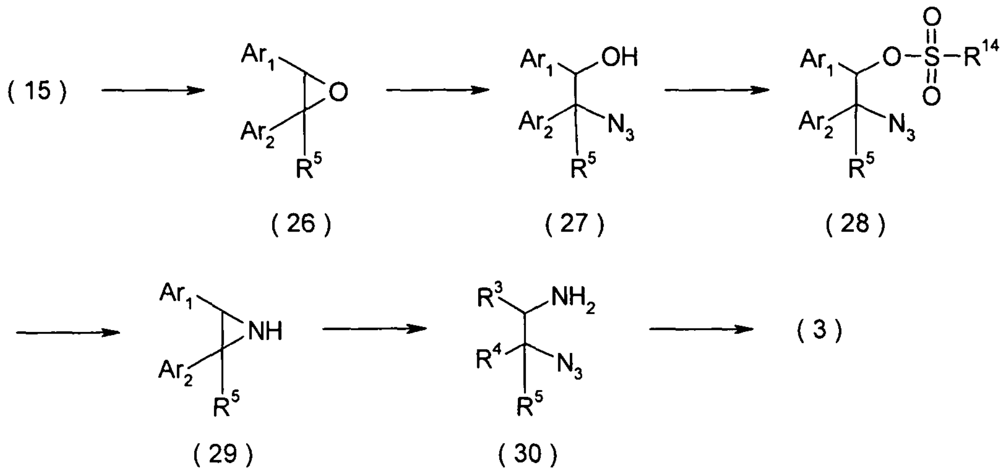

(15) (26) (27) (28) (29) (30) (3)

Synthesis of compound (26)

A compound (26) can be synthesized by treating a compound (15) with a peroxide such as an organic peroxide (m-chloroperbenzoic acid or tert-butyl hydroperoxide), aqueous hydrogen peroxide, or oxone (these peroxides may be used in combination with a catalytic amount of a metal such as vanadium, molybdenum, or tungsten). The solvent used in this reaction is not particularly limited and examples thereof include dichloromethane, chloroform, diethyl ether, toluene, acetone, acetonitrile, and mixed solvents thereof. The reaction temperature is usually in the range from -78 to 100°C or the boiling point of the solvent, preferably in the range from -20 to 60°C.

Synthesis of compound (27)

A compound (27) can be synthesized by reacting compound (26) with sodium azide or lithium azide or the like in the presence of a weak acidic inorganic compound such as ammonium chloride. The solvent used in this reaction is not particularly limited and examples thereof include dimethylformamide, dimethyl sulfoxide, ethanol, methanol, tetrahydrofuran, diethyl ether, and mixed solvents thereof. However, organic solvents that can be mixed with water in an arbitrary ratio are preferred.
The reaction temperature is usually in the range from -20 to 150°C or the boiling point of the solvent, preferably in the range from room temperature to 120°C or the boiling point.

Synthesis of compound (28)

A compound (28) can be obtained by treating compound (27) with a sulfonylating agent such as methanesulfonyl chloride, p-toluenesulfonyl chloride, p-toluenesulfonic anhydride, or trifluoromethanesulfonic anhydride in the presence of a base such as a tertiary amine (e.g., triethylamine) or pyridine. The solvent used in this reaction is not particularly limited and examples thereof include dichloromethane, chloroform, diethyl ether, tetrahydrofuran, toluene, acetonitrile, and mixed solvents thereof. However, dried solvents are preferred. The reaction temperature is usually in the range from -78 to 100°C or the boiling point of the solvent, preferably in the range from -20 to 80°C.

Synthesis of compound (29)

A compound (29) can be obtained in one step by treating compound (28) with a reducing agent capable of converting azide groups to amino groups, for example, lithium aluminum hydride, lithium borohydride, sodium borohydride, diisobutylaluminum hydride, to form an aziridine ring simultaneously with reduction. The solvent used in this reaction is not particularly limited and examples thereof include diethyl ether, tetrahydrofuran, toluene, and mixed solvents thereof. However, dried solvents are preferred. The reaction temperature is usually in the range from -78 to 100˚C or the boiling point of the solvent, preferably in the range from -78 to 80˚C.

Synthesis of compound (30)

A compound (30) can be obtained by using compound (29) under the reaction conditions described in the method for producing compound (27).

Synthesis of compound (3)

A compound (3) can be obtained by using compound (30) under the reaction conditions described in the method for producing compound (29). Moreover, in another method, a compound (3) can also be obtained by a hydrogenation reaction using a catalyst such as palladium on carbon or platinum on carbon. The solvent used in this reaction is not particularly limited and examples thereof include methanol, ethanol, tetrahydrofuran, ethyl acetate, and mixed solvents thereof. The reaction temperature is usually in the range from -20 to 100˚C or the boiling point of the solvent, preferably in the range from room temperature to 50˚C.

The starting material compound (A) is a commercially available product or can be synthesized according to the method described in the document (Tetrahedron Asymmetry, 1995, 6, 2199).

The starting material compound (B), (C), or (D) is a commercially available product or can be synthesized according to methods described in the Reference Examples.

The starting material compound (E) is a commercially available product or can be synthesized according to the method described in various documents (e.g., J. Med. Chem. 2000, 43, 4781).

In one embodiment of the present invention, the compound of the present invention or a salt thereof can be used as a p53-Mdm2 binding inhibitor and/or an Mdm2 ubiquitin ligase inhibitor because it inhibits the binding of p53 with Mdm2 and the ubiquitination of p53 by Mdm2.

The condition of the p53-Mdm2 binding can be examined by a method conventionally used by those skilled in the art to examine binding conditions between proteins (for example, immunological techniques, surface plasmon resonance techniques, etc.). Examples of methods for examining the condition of the Mdm2-p53 binding using an immunological technique include an immuno-sedimentation method and enzyme-linked-immuno-sorbent assay (ELISA). An antibody used in such immunological techniques may be an anti-Mdm2 antibody and/or an anti-p53 antibody that can directly detect Mdm2 and/or p53. When Mdm2 and/or p53 is labeled with a tag (for example, a GST tag or a histidine tag) or the like, an antibody suitable for labeling (for example, an anti-GST antibody or an anti-histidine antibody) can be used. Methods for examining the condition of the Mdm2-p53 binding using an immunological technique are described in, for example, W02003/51359, W02003/51360, U.S. Patent Application Publication No. 2004/259867 or 2004/259884, and W02005/110996. Methods for examining the condition of the Mdm2-p53 binding using a surface plasmon resonance technique are described in, for example, Science, vol.303, p.844-848, 2004.

Ubiquitin ligase activity of Mdm2 against p53 can be examined by an ubiquitin ligase assay conventionally used by those skilled in the art. The ubiquitin ligase activity can be detected by, for example, comparing ubiquitination of p53 by ubiquitin activation enzyme (E1), ubiquitin binding enzyme (E2), and ubiquitin ligase (E3) (Mdm2) in the presence and absence of a test compound (for example, refer to WO2001/75145 and W02003/76608).

In another embodiment, the compound of the present invention or a salt thereof can be used as an inhibitor of suppression of the p53 transcription activity because it restores functions of p53 as a transcription factor that is suppressed by Mdm2 by inhibiting the binding of Mdm2 to the p53 transcription activation domain. The inhibitor of suppression of the p53 transcription activity can be obtained by, for example, measuring the mRNA level or the protein level of a protein whose transcription is regulated by p53 (for example, $p_{21}^{Waf1/Cip1}$) in the presence or absence of a test compound by an mRNA measuring method (for example, Northern blot) or a protein measuring method (for example, Western blot) conventionally used by those skilled in the art and selecting the test compound as an inhibitor of suppression of the p53 transcription activity when the mRNA level or the protein level is increased in the presence of the test compound as compared with that in the absence of the test compound. Furthermore, the inhibitor of suppression of the p53 transcription activity can also be identified by a reporter assay using the reporter activity of a reporter gene including a p53 responsive element as an indicator.

In another embodiment, the compound of the present invention or a salt thereof can be used as a p53 degradation

inhibitor because it inhibits ubiquitination of p53 by Mdm2 and thereby prevents the degradation of p53 in proteasomes. The p53 degradation inhibitor can be obtained by, for example, measuring the mRNA level or the protein level of p53 in the presence or absence of a test compound by an mRNA measuring method (for example, Northern blot) or a protein measuring method (for example, Western blot) conventionally used by those skilled in the art and selecting the test compound as a p53 degradation inhibitor when the mRNA level or the protein level is increased in the presence of the test compound as compared with that in the absence of the test compound.

In another embodiment, the compound of the present invention or a salt thereof can be used as an anti-tumor agent because it normalizes functions of p53 as a cancer-restraining gene by inhibition of the Mdm2-p53 binding and/or ubiquitination of p53 by Mdm2.

Cellular growth inhibiting activity can be examined by methods for testing growth inhibition conventionally used by those skilled in the art. The cell growth inhibition activity can be determined by, for example, comparing the levels of cellular growth (for example, tumor cells) in the presence or absence of a test compound as described in the following Test Example 2. The levels of cellular growth can be examined by using, for example, a test system for measuring living cells. Examples of the method for measuring living cells include the [$^3$H]-thymidine uptake test, the BrdU method, the MTT assay, and so forth.

The compound of the present invention or a salt thereof can be used for the treatment of tumors or cancers such as, for example, lung cancer, digestive system cancer, ovary cancer, uterine cancer, breast cancer, liver cancer, head/neck region cancer, blood cancer, renal cancer, and testicular tumors.

A pharmaceutical composition of the present invention can contain a compound of the present invention and a pharmaceutically acceptable carrier and can be administered as various injections such as intravenous injection, intramuscular injection, and subcutaneous injection or by various methods such as oral administration or percutaneous administration. Pharmaceutically acceptable carrier means a pharmacologically acceptable material that is involved in transport of the compound of the present invention or a composition containing the compound of present invention (for example, an excipient, a diluent, an additive, a solvent, etc.) from a given organ to another organ.

A formulation can be prepared by selecting a suitable formulation form (for example, oral formulation or injection) depending on the administration method and using various conventionally used methods for preparing a formulation. Examples of oral formulations include tablets, powders, granules, capsules, pills, lozenges, solutions, syrups, elixirs, emulsions, oily or aqueous suspensions, and so forth. In oral administration, the free compound or a salt form may be used. An aqueous formulation can be prepared by forming an acid adduct with a pharmacologically acceptable acid or by forming an alkali metal salt such as sodium. As an injection, a stabilizer, a preservative, a dissolving aid, and the like can be used in the formulation. After filling a solution that may contain these aids and the like in a vessel, a formulation for use may be prepared as a solid formulation by lyophilization or the like. Furthermore, one dose may be filled in one vessel, or two or more doses may be filled in a vessel.

Examples of solid formulations include tablets, powders, granules, capsules, pills, and lozenges. These solid formulations may contain pharmaceutically acceptable additives together with the compound of the present invention. Examples of additives include fillers, extenders, binders, disintegrating agents, dissolution promoting agents, skin wetting agents, and lubricants, and these can be selected and mixed as required to prepare a formulation.

Examples of liquid formulations include solutions, syrups, elixirs, emulsions, and suspensions. These liquid formulations may contain pharmaceutically acceptable additives together with the compound of the present invention. Examples of additives include suspending agents and emulsifiers, and these are selected and mixed as required to prepare a formulation.

The compound of the present invention or a salt thereof can be used in cancer treatment of mammals, in particular, humans. The dose and the administration interval can be suitably selected depending on the site of the disease, the patient's height, body weight, sex, or medical history, according to a physician's judgment. When the compound of the present invention is administered to a human, the dose range is approx. 0.01 to 500 mg/kg body weight per day, preferably, approx 0.1 to 100 mg/kg body weight. Preferably, the compound of the present invention is administered to a human once a day, or the dose is divided into two to four times, and administration is repeated at an appropriate interval. Furthermore, the daily dose may exceed the above-mentioned dose at a physician's discretion, if necessary.

The compound of the present invention or the salt thereof may be used in combination with an additional anti-tumor agent. Examples thereof include anti-tumor antibiotics, anti-tumor plant constituents, BRMs (biological response modifiers), hormones, vitamins, anti-tumor antibodies, molecular target drugs, and other anti-tumor agents.

More specifically, examples of alkylating agents include: alkylating agents such as nitrogen mustard, nitrogen mustard N-oxide, and chlorambucil; aziridine alkylating agents such as carboquone and thiotepa; epoxide alkylating agents such as dibromomannitol and dibromodulcitol; nitrosourea alkylating agents such as carmustine, lomustine, semustine, nimustine hydrochloride, streptozocin, chlorozotocin, and ranimustine; and busulfan, improsulfan tosylate, and dacarbazine.

Examples of various metabolic antagonists include: purine metabolic antagonists such as 6-mercaptopurine,

6-thioguanine, and thioinosine; pyrimidine metabolic antagonists such as fluorouracil, tegafur, tegafur-uracil, carmofur, doxifluridine, broxuridine, cytarabine, and enocitabine; and folic acid metabolic antagonists such as methotrexate and trimetrexate.

Examples of anti-tumor antibiotics include: anti-tumor anthracycline antibiotics such as mitomycin C, bleomycin, peplomycin, daunorubicin, aclarubicin, doxorubicin, pirarubicin, THP-adriamycin, 4'-epidoxorubicin, and epirubicin; and chromomycin A3 and actinomycin D.

Examples of anti-tumor plant constituents include: vinca alkaloids such as vindesine, vincristine, and vinblastine; taxanes such as paclitaxel and docetaxel; and epipodophyllotoxins such as etoposide and teniposide.

Examples of BRMs include tumor necrosis factors and indomethacin.

Examples of hormones include hydrocortisone, dexamethasone, methylprednisolone, prednisolone, prasterone, betamethasone, triamcinolone, oxymetholone, nandrolone, metenolone, fosfestrol, ethinylestradiol, chlormadinone, and medroxyprogesterone.

Examples of vitamins include vitamin C and vitamin A.

Examples of anti-tumor antibodies and molecular target drugs include trastuzumab, rituximab, cetuximab, nimotuzumab, denosumab, bevacizumab, infliximab, imatinib mesilate, gefitinib, erlotinib, sunitinib, lapatinib, and sorafenib.

Examples of other anti-tumor agents include cisplatin, carboplatin, oxaliplatin, tamoxifen, camptothecin, ifosfamide, cyclophosphamide, melphalan, L-asparaginase, aceglatone, sizofiran, picibanil, procarbazine, pipobroman, neocarzinostatin, hydroxyurea, ubenimex, and krestin.

The present invention also includes a method for preventing and/or treating cancer, comprising administering a compound of the present invention or a salt thereof.

The present invention further includes use of a compound of the present invention or a salt thereof for the manufacture of the medicament.

Hereinafter, the present invention will be specifically explained with reference to the Examples. However, the present invention is not limited to these examples, and they should not be construed in any limitative way. Furthermore, reagents, solvents, and starting materials in the specification can be readily obtained from commercially available supply sources unless otherwise specified.

Examples

Example 1

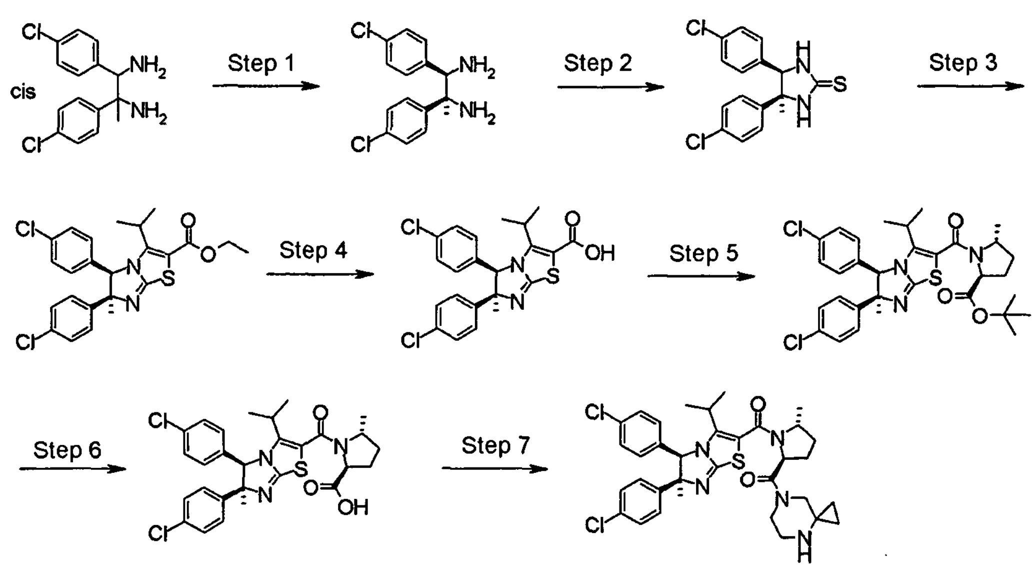

Step 1: (1R,2S)-1,2-bis(4-chlorophenyl)propane-1,2-diamine

L-tartaric acid (5.05 g, 33.9 mmol) was added to an ethanol (100 ml) solution of (1R*,2S*)-1,2-bis(4-chlorophenyl)propane-1,2-diamine (10.0 g, 33.9 mmol) and the resulting mixture was heated to reflux. The reaction mixture was concentrated and then recrystallized from a mixed solvent of ethanol and diethyl ether and the precipitated solid was collected by filtration. The solid obtained was made into an alkaline solution by the addition of 1 N aqueous sodium hydroxide solution, followed by extraction with diethyl ether. The organic layer was dried over potassium carbonate and then the solvent was evaporated under reduced pressure to give the title compound (1R,2S)-1,2-bis(4-chlorophenyl)propane-1,2-diamine (3.05 g, 31%) as a colorless solid.
$^1$H-NMR (400 MHz, $CDCl_3$) δ: 1.49 (3H, s), 4.08 (1H, s), 6.98 (2H, d, J=8.5 Hz), 7.17 (2H, d, J=8.3 Hz), 7.25-7.28 (4H, m).

Step 2: (4S,5R)-4,5-bis(4-chlorophenyl)-4-methylimidazolidine-2-thione

Carbon disulfide (2.04 ml, 33.9 mmol) was added to an ethanol (20 ml) solution of the compound (2.00 g, 6.77 mmol) obtained in Step 1 above and the resulting mixture was heated to reflux for 4 hours. The solvent was evaporated under reduced pressure, isopropanol and diisopropyl ether were added to the residue and the resulting solid was collected by filtration to give the title compound (1.91 g, 84%) as a colorless solid.
$^1$H-NMR (400 MHz, DMSO-$d_6$) δ: 1.71 (3H, s), 4.94 (1H, s), 6.89 (2H, dt, J=8.9, 2.1 Hz), 6.97 (2H, dt, J=8.9, 2.1 Hz), 7.17-7.12 (4H, m), 8.74 (1H, s), 8.92 (1H, s).

Step 3: ethyl (5R,6S)-5,6-bis(4-chlorophenyl)-3-isopropyl-6-methyl-5,6-dihydroimidazo[2,1-b][1,3][1,3]thiazole-2-carboxylate

Ethyl 2-chloro-4-methyl-3-oxopentanoate (1.42 g, 7.36 mmol) was added to an ethanol (20 ml) solution of the compound (1.91 g, 5.66 mmol) obtained in Step 2 above and the resulting mixture was heated to reflux for 18 hours. The solvent was evaporated under reduced pressure, isopropanol and diisopropyl ether were added to the residue and the resulting solid was collected by filtration to give the title compound (2.11 g, 78%) as a colorless solid.
$^1$H-NMR (400 MHz, $CDCl_3$) δ: 1.02 (3H, d, J=7.0 Hz), 1.03 (3H, d, J=7.0 Hz), 1.37 (3H, t, J=7.1 Hz), 2.10 (3H, s), 3.28-3.47 (1H, m), 4.33 (2H, q, J=7.1 Hz), 5.57 (1H, s), 6.45-7.18 (8H, m).

Step 4: (5R,6S)-5,6-bis(4-chlorophenyl)-3-isopropyl-6-methyl-5,6-dihydroimidazo[2,1-b][1,3]thiazole-2-carboxylic acid

1 N aqueous sodium hydroxide solution (20 ml, 20 mmol) was added to an ethanol (20 ml) solution of the compound (2.11 g, 4.44 mmol) obtained in Step 3 above and the resulting mixture was heated to reflux for 4 hours. 1 N aqueous hydrochloric acid solution (22 ml) was added to the reaction mixture, the resulting mixture was diluted with water and stirred and then the precipitated insoluble matter was collected by filtration to give the title compound (1.54 g, 78%) as a colorless solid.
$^1$H-NMR (400 MHz, DMSO-$d_6$) δ: 0.83 (3H, d, J=7.1 Hz), 0.93 (3H, d, J=7.1 Hz), 1.78 (3H, s), 2.99-3.67 (1H, m), 5.79 (1H, s), 6.44-7.43 (8H, m).
MS (ESI) m/z: 447 [$(M+H)^+$].

Step 5: tert-butyl (5R)-1-{[(5R,6S)-5,6-bis(4-chlorophenyl)-3-isopropyl-6-methyl-5,6-dihydroimidazo [2, 1-b] [1,3]thiazol-2-yl]carbonyl}-5-methyl-L-prolinate

Thionyl chloride (10 ml) was added to a toluene (150 ml) suspension of the compound (7.46 g, 17.0 mmol) obtained in Step 4 above and the resulting mixture was stirred under heating at 70˚C for 1.5 hours. After cooling, the reaction mixture was concentrated under reduced pressure and the residue obtained was dissolved in tetrahydrofuran (30 ml) and then added dropwise under ice cooling to a tetrahydrofuran (200 ml) solution of the compound (4.3 g, 20.0 mmol) obtained in Step 2 of Reference Example 1 and triethylamine (7.1 ml, 51.0 mmol). The resulting mixture was stirred at room temperature for 16 hours, then the reaction mixture was diluted with ethyl acetate and the organic layer was washed with saturated aqueous sodium bicarbonate solution and brine and then dried over anhydrous magnesium sulfate. The solvent was evaporated under reduced pressure and the residue obtained was purified by NH-silica gel column chromatography [n-hexane:ethyl acetate = 1:1 (v/v)] to give the title compound (9.08 g, 87%) as a colorless solid.
$^1$H-NMR (400 MHz, $CDCl_3$) δ: 1.00-1.03 (6H, m), 1.20 (3H, d, J=6.1 Hz), 1.45 (9H, s), 1.62 (3H, s), 1.66-1.68 (1H, m), 1.80 (3H, s), 1.96-2.00 (1H, m), 2.23-2.29 (2H, m), 2.62-2.68 (1H, m), 4.47-4.59 (2H, m), 4.95 (1H, s), 6.69 (2H, d, J=8.1 Hz), 7.01 (2H, d, J=8.3 Hz), 7.02 (2H, d, J=8.5 Hz), 7.13 (2H, d, J=8.3 Hz).

Step 6: (5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-proline

Anisole (1.63 ml, 15.0 mmol) and trifluoroacetic acid (40 ml) were added to a chloroform (100 ml) solution of the compound (9.0 g, 15.0 mmol) obtained in Step 5 above and the resulting mixture was stirred at room temperature for 1 hour and then stirred under heating at 40°C for 1 hour. The reaction mixture was evaporated under reduced pressure and then subjected to azeotropic distillation with toluene and the residue obtained was purified by silica gel column chromatography [chloroform:methanol= 9:1 (v/v)] to give the title compound (5.76 g, 69%) as a colorless solid.
$^1$H-NMR (400 MHz, DMSO-$d_6$) δ: 0.92 (3H, d, J=7.3 Hz), 0.95 (3H, d, J=6.8 Hz), 1.16 (3H, d, J=6.1 Hz), 1.63-1.67 (1H, m), 1.94 (3H, s), 1.96-2.01 (1H, m), 2.04-2.10 (1H, m), 2.34-2.40 (1H, m), 2.67-2.75 (1H, m), 4.26-4.35 (1H, m), 4.52-4.59 (1H, m), 5.89 (1H, s), 6.84-6.92 (2H, m), 7.17 (4H, d, J=8.3 Hz), 7.23 (2H, d, J=8.5 Hz).
MS (ESI) m/z: 558 [(M+H)$^+$].

Step 7: 7-[(5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-prolyl]-4,7-diazaspiro[2.5]octane

1-hydroxybenzotriazole (15 mg, 0.11 mmol) and 1-(3-dimethylaminopropyl)-3-ethylcarbodiimide hydrochloride (250 mg, 1.28 mmol) were added to a dimethylformamide (8 ml) solution of the compound (600 mg, 1.07 mmol) obtained in Step 6 above, then 4,7-diazaspiro[2.5]octane hydrochloride (238 mg, 1.28 mmol) and diisopropylethylamine (466 μl, 2.68 mmol) were added and the resulting mixture was stirred at room temperature for 16 hours. The reaction mixture was diluted with ethyl acetate and the organic layer was washed with saturated aqueous sodium bicarbonate solution and brine and dried over anhydrous sodium sulfate. The solvent was evaporated under reduced pressure and the residue obtained was purified by silica gel column chromatography [chloroform:methanol = 20:1 (v/v)] to give the title compound (290 mg, 42%) as a colorless solid.
$^1$H-NMR (400 MHz, $CDCl_3$) δ: 0.56-0.60 (2H, m), 0.68-0.72 (2H, m), 0.94 (3H, d, J=7.1 Hz), 1.03 (3H, d, J=7.1 Hz), 1.23 (3H, d, J=5.9 Hz), 1.79 (3H, s), 1.81-1.88 (2H, m), 2.24-2.30 (1H, m), 2.33-2.38 (1H, m), 2.70-2.76 (1H, m), 2.90-3.04 (2H, m), 3.28-3.33 (1H, m), 3.42-3.57 (3H, m), 4.52-4.57 (1H, m), 4.93 (1H, s), 4.99-5.02 (1H, m), 6.69 (2H, d, J=8.3 Hz), 7.01 (4H, d, J=8.5 Hz), 7.12 (2H, d, J=8.5 Hz).
MS (ESI) m/z: 652 [(M+H)$^+$].

Example 2

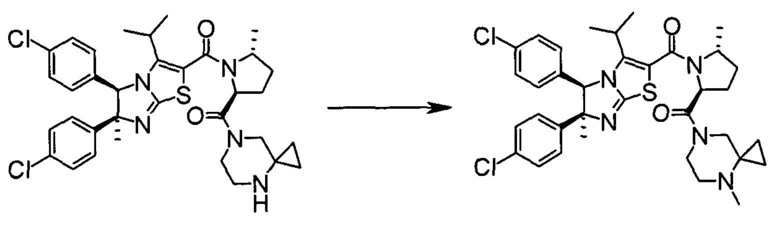

7-[(5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-prolyl]-4-methyl-4,7-diazaspiro[2.5]octane

37% aqueous formaldehyde solution (230 μl, 3.1 mmol) was added to a dioxane (4 ml) solution of the compound (200 mg, 0.31 mmol) obtained in Step 7 of Example 1. The resulting mixture was stirred at room temperature for 15 minutes, then sodium triacetoxyborohydride (131 mg, 0.62 mmol) was added and the mixture was further stirred at room temperature for 1 hour. Saturated aqueous sodium bicarbonate solution (10 ml) was added to the reaction mixture, followed by extraction with ethyl acetate. The organic layer was washed with brine and then dried over anhydrous sodium sulfate. The solvent was evaporated under reduced pressure and the residue obtained was purified by silica gel column chromatography [chloroform:methanol = 20:1 (v/v)] to give the title compound (100 mg, 48%) as a colorless solid.
$^1$H-NMR (400 MHz, $CDCl_3$) δ: 0.48-0.51 (1H, m), 0.62-0.64 (1H, m), 0.66-0.68 (1H, m), 0.77-0.80 (1H, m), 0.94 (3H, d, J=7.1 Hz), 1.03 (3H, d, J=7.1 Hz), 1.23 (3H, d, J=5.9 Hz), 1.50-1.54 (1H, m), 1.61-1.66 (1H, m), 1.79 (3H, s), 1.83-1.88 (1H, m), 2.27-2.33 (1H, m), 2.39 (3H, s), 2.71-2.76 (1H, m), 2.86-2.94 (2H, m), 3.38-3.63 (4H, m), 4.52-4.56 (1H, m), 4.93 (1H, s), 5.00-5.02 (1H, m), 6.69 (2H, d, J=8.5 Hz), 7.01 (4H, d, J=8.5 Hz), 7.12 (2H, d, J=8.5 Hz).
MS (ESI) m/z: 666 [(M+H)$^+$].

Example 3

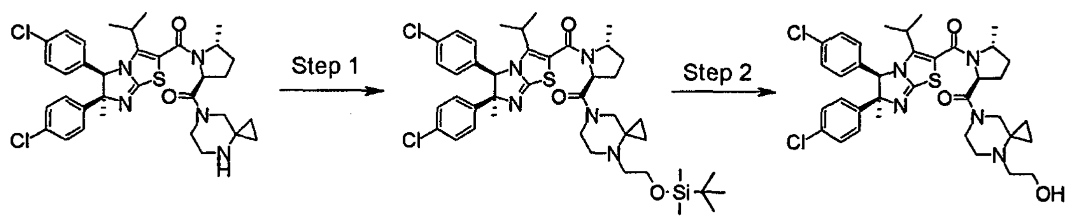


Step 1: 7-[(5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-prolyl]-4-(2-{[tert-butyl(dimethyl)silyl]oxy}ethyl)-4,7-diazaspiro[2.5]octane

The compound obtained in Step 7 of Example 1 was reacted in the same way as in Example 2 using (tert-butyldimethylsilyloxy)acetaldehyde instead of 37% aqueous formaldehyde solution to give the title compound as a colorless solid.
$^1$H-NMR (400 MHz, $CDCl_3$) δ: 0.02 (6H, s), 0.88 and 0.89 (total 9H, each s), 0.90-0.97 (4H, m), 1.02 (6H, d, J=6.3 Hz), 1.20-1.26 (3H, m), 1.64-1.69 (1H, m), 1.79 (3H, s), 1.80-1.82 (1H, m), 1.84-1.89 (1H, m), 2.32-2.44 (1H, m), 2.67-2.71 (1H, m), 2.80-2.84 (1H, m), 2.87-2.90 (1H, m), 2.95-3.02 (2H, m), 3.40-3.48 (2H, m), 3.54-3.65 (4H, m), 4.54-4.57 (1H, m), 4.93 (1H, s), 4.99-5.03 (1H, m), 6.69 (2H, d, J=8.3 Hz), 7.02 (4H, d, J=8.5 Hz), 7.13 (2H, d, J=8.0 Hz).
MS (ESI) m/z: 810 [$(M+H)^+$].

Step 2: 2-{7-[(5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-prolyl]-4,7-diazaspiro[2.5]oct-4-yl}ethanol

Tetra-n-butylammonium fluoride (1 M tetrahydrofuran solution) (341 μl, 0.34 mmol) was added dropwise to a tetrahydrofuran (6 ml) solution of the compound (255 mg, 0.31 mmol) obtained in Step 1 above and the resulting mixture was stirred at room temperature for 6 hours. The reaction mixture was diluted with ethyl acetate and the organic layer was washed with saturated aqueous sodium bicarbonate solution and brine and dried over anhydrous sodium sulfate. The solvent was evaporated under reduced pressure and the residue obtained was purified by silica gel column chromatography [chloroform:methanol = 20:1 (v/v)] to give the title compound (114 mg, 53%) as a colorless solid.
$^1$H-NMR (400 MHz, DMSO-$d_6$) δ: 0.49-0.54 (2H, m), 0.57-0.62 (2H, m), 0.86 (3H, d, J=7.1 Hz), 0.95 (3H, d, J=7.1 Hz), 1.15 (3H, d, J=6.3 Hz), 1.54-1.60 (1H, m), 1.72 (3H, s), 1.73-1.77 (1H, m), 2.04-2.10 (1H, m), 2.27-2.33 (1H, m), 2.65-2.69 (1H, m), 2.72-2.79 (2H, m), 2.86-2.91 (1H, m), 3.25-3.29 (1H, m), 3.35-3.45 (4H, m), 3.49-3.54 (1H, m), 3.97-4.00 (1H, m), 4.29-4.33 (1H, m), 4.88-4.92 (1H, m), 5.39 (1H, s), 6.88 (2H, d, J=8.3 Hz), 7.06 (2H, d, J=8.8 Hz), 7.10 (2H, d, J=8.8 Hz), 7.25 (2H, d, J=8.5 Hz).
MS (ESI) m/z: 696 [$(M+H)^+$].

Example 4

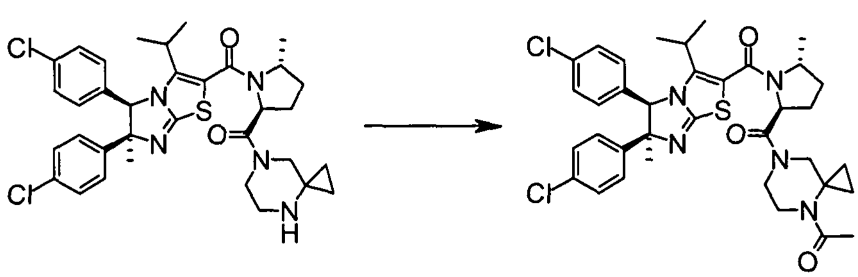

4-acetyl-7-[(5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-prolyl]-4,7-diazaspiro[2.5]octane

The compound (200 mg, 0.3 mmol) obtained in Step 7 of Example 1 was dissolved in acetic anhydride (2 ml), pyridine (500 μl) was added and the resulting mixture was stirred at room temperature for 4 hours. Ice water (10 ml) was added and the resulting mixture was stirred for 1 hour, followed by extraction with ethyl acetate. The organic layer was washed with saturated aqueous sodium bicarbonate solution and brine and then dried over anhydrous magnesium sulfate. The solvent was evaporated under reduced pressure and the residue obtained was purified by silica gel column chromatography [chloroform:methanol = 40:1 (v/v)] to give the title compound (186 mg, 89%) as a colorless solid.
$^1$H-NMR (400 MHz, DMSO-$d_6$) δ: 0.81-0.95 (7H, m), 0.94 (3H, d, J=6.8 Hz), 1.14 (3H, d, J=6.3 Hz), 1.56-1.61 (1H, m), 1.71 (3H, s), 1.72-1.77 (1H, m), 2.01-2.07 (1H, m), 2.08 (3H, s), 2.26-2.32 (1H, m), 2.61-2.69 (1H, m), 3.30-3.43 (2H, m), 3.46-3.73 (4H, m), 4.27-4.36 (1H, m), 4.86-4.99 (1H, m), 5.43 (1H, s), 6.88 (2H, d, J=7.1 Hz), 7.06-7.12 (4H, m), 7.26 (2H, d, J=8.5 Hz).
MS (ESI) m/z: 694 [$(M+H)^+$].

Example 5

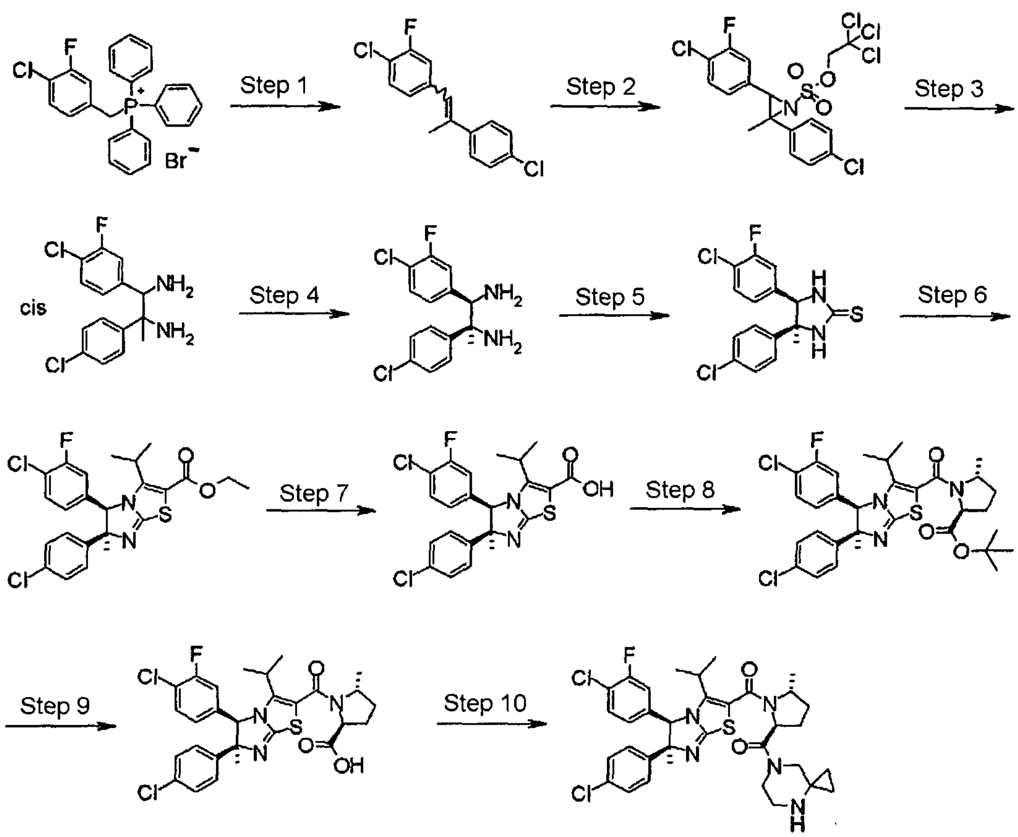


Step 1: 1-chloro-4-[(1E)-2-(4-chlorophenyl)prop-1-en-1-yl]-2-fluorobenzene and 1-chloro-4-[(1Z)-2-(4-chlorophenyl)prop-1-en-1-yl]-2-fluorobenzene

n-butyllithium (2.77 M/n-hexane solution) (100 ml, 277 mmol) was added dropwise to a tetrahydrofuran (400 ml) suspension of (4-chloro-3-fluorobenzyl)(triphenyl)phosphonium bromide (112 g, 231 mmol) at -20˚C in a nitrogen atmosphere. The resulting mixture was stirred at the same temperature for 30 minutes, then a tetrahydrofuran (300 ml) solution of 1-(4-chlorophenyl)ethanone (33 ml, 254 mmol) was added dropwise and the resulting mixture was stirred at room temperature for 17 hours. An aqueous solution of saturated ammonium chloride was added, followed by extraction with ethyl acetate. The organic layer was washed with brine and dried over anhydrous sodium sulfate. The solvent was evaporated under reduced pressure and the residue was purified by silica gel column chromatography to give the title

mixture (E:Z = 5:3) (56 g, 86%) as a colorless solid.

Step 2: 2,2,2-trichloroethyl 3-(4-chloro-3-fluorophenyl)-2-(4-chlorophenyl)-2-methylaziridine-1-sulfonate

Iodosobenzene acetate (52 g, 161 mmol) was added to a dichloromethane (500 ml) suspension of the mixture (56 g, 199 mmol) obtained in Step 1 above, 2,2,2-trichloroethyl sulfamate (34 g, 150 mmol), magnesium oxide (12 g, 300 mmol), and rhodium(II) perfluorobutylamide (1.3 g, 1.24 mmol) in a nitrogen atmosphere and the resulting mixture was stirred at room temperature for 4 days. Insoluble matter was removed by filtration, then the filtrate was concentrated and methanol and n-hexane were added to the residue. The precipitated solid was collected by filtration to give the title compound (36 g, 31%) as a colorless solid. $^1$H-NMR (400 MHz, $CDCl_3$) δ: 1.43 (3H, s), 4.60 (1H, s), 4.73 (2H, dd, J=29.1, 10.9 Hz), 7.21 (1H, d, J=8.0 Hz), 7.26 (1H, dd, J=9.3, 2.0 Hz), 7.42 (2H, dt, J=8.8, 2.0 Hz), 7.48 (1H, t, J=7.8 Hz), 7.52-7.55 (2H, m).

Step 3: 1-(4-chloro-3-fluorophenyl)-2-(4-chlorophenyl)propane-1,2-diamine

7 N ammonia/methanol solution (1000 ml) was added to the compound (88 g, 173 mmol) obtained in Step 2 above and the resulting mixture was stirred at 50°C for 15 hours. The solvent was evaporated under reduced pressure and the residue was suspended in ethanol (100 ml). 1 N hydrochloric acid/ethanol solution (500 ml) was added and the resulting mixture was heated to reflux for 6 hours. The solvent was evaporated under reduced pressure and the residue was diluted with 1 N aqueous hydrochloric acid solution and then washed with ethyl acetate. The aqueous layer was made basic with sodium hydroxide, followed by extraction with diethyl ether. The organic layer was washed with brine and then dried over anhydrous sodium sulfate. The solvent was evaporated under reduced pressure to give the title compound (42 g, 78%) as a pale yellow oil.
$^1$H-NMR ($CDCl_3$) δ: 1.49 (3H, s), 1.53 (4H, brs), 4.07 (1H, s), 6.74 (1H, dd, J=8.2, 1.6 Hz), 6.93 (1H, dd, J=10.5, 2.0 Hz), 7.19 (1H, t, J=7.9 Hz), 7.26 (4H, s).

Step 4: (1R,2S)-1-(4-chloro-3-fluorophenyl)-2-(4-chlorophenyl)propane-1,2-diamine

The compound obtained in Step 3 above was treated in the same way as in Step 1 of Example 1 to give the title compound as a colorless oil.
$^1$H-NMR ($CDCl_3$) δ: 1.49 (3H, s), 1.53 (4H, brs), 4.07 (1H, s), 6.74 (1H, dd, J=8.2, 1.6 Hz), 6.93 (1H, dd, J=10.5, 2.0 Hz), 7.19 (1H, t, J=7.9 Hz), 7.26 (4H, s).

Step 5: (4S,5R)-5-(4-chloro-3-fluorophenyl)-4-(4-chlorophenyl)-4-methylimidazolidine-2-thione

The compound obtained in Step 4 above was reacted in the same way as in Step 2 of Example 1 to give the title compound as a colorless solid.
$^1$H-NMR (400 MHz, $CDCl_3$) δ: 1.61 (3H, s), 1.89 (2H, s), 4.94 (1H, s), 6.49 (1H, s), 6.64 (1H, dd, J=8.2, 1.8 Hz), 6.71 (1H, dd, J=9.6, 2.1 Hz), 6.89-6.91 (2H, m), 7.12-7.17 (2H, m).

Step 6: ethyl (5R,6S)-5-(4-chloro-3-fluorophenyl)-6-(4-chlorophenyl)-3-isopropyl-6-methyl-5,6-dihydroimidazo [2,1-b] [1,3] thiazole-2-carboxylate

The compound obtained in Step 5 above was reacted in the same way as in Step 3 of Example 1 to give the title compound as a pale yellow solid.
$^1$H-NMR (400 MHz, $CDCl_3$) δ: 0.90 (3H, d, J=7.3 Hz), 1.02 (3H, d, J=7.1 Hz), 1.34 (3H, t, J=7.2 Hz), 1.80 (3H, s), 3.35-3.37 (1H, m), 4.25 (2H, q, J=7.1 Hz), 5.03 (1H, s), 6.54 (2H, brs), 7.07-7.12 (5H, m).

Step 7: (5R,6S)-5-(4-chloro-3-fluorophenyl)-6-(4-chlorophenyl)-3-isopropyl-6-methyl-5,6-dihydroimidazo[2,1-b][1,3]thiazole-2-carboxylic acid

The compound obtained in Step 6 above was reacted in the same way as in Step 4 of Example 1 to give the title compound as a colorless solid.
$^1$H-NMR (400 MHz, DMSO-$d_6$) δ: 1.00 (3H, d, J=3.2 Hz), 1.02 (3H, d, J=3.2 Hz), 1.99 (3H, s), 3.29-3.32 (1H, m), 6.10 (1H, s), 6.80 (1H, s), 7.05 (1H, s), 7.18-7.21 (2H, m), 7.23-7.28 (2H, m), 7.33 (1H, t, J=7.9 Hz).

Step 8: tert-butyl (5R)-1-{[(5R,6S)-5-(4-chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-prolinate

The compound obtained in Step 7 above was reacted in the same way as in Step 5 of Example 1 to give the title compound as a pale yellow oil.
$^1$H-NMR (400 MHz, $CDCl_3$) δ: 1.02-1.05 (6H, m), 1.21 (3H, d, J=6.3 Hz), 1.45 (9H, s), 1.68 (1H, brs), 1.80 (3H, s), 1.98 (1H, brs), 2.27 (2H, brs), 2.62 (1H, brs), 4.52 (2H, brs), 4.92 (1H, s), 6.53 (2H, dd, J=15.3, 7.6 Hz), 7.06-7.08 (3H, m), 7.15 (2H, d, J=8.5 Hz).

Step 9: (5R)-1-{[(5R,6S)-5-(4-chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-proline

The compound obtained in Step 8 above was reacted in the same way as in Step 6 of Example 1 to give the title compound as a colorless solid.
$^1$H-NMR (400 MHz, $CDCl_3$) δ: 0.88 (3H, t, J=6.8 Hz), 0.95-0.97 (3H, m), 1.08 (3H, d, J=7.1 Hz), 1.25-1.26 (3H, m), 2.05 (3H, d, J=3.2 Hz), 2.24 (3H, m), 4.47-4.50 (2H, m), 5.33-5.35 (1H, m), 7.07-7.23 (7H, m).

Step 10: 7-[(5R)-1-{[(5R,6S)-5-(4-chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-prolyl]-4,7-diazaspiro[2.5]octane

The compound obtained in Step 9 above was reacted in the same way as in Step 7 of Example 1 to give the title compound as a colorless solid.
$^1$H-NMR (400 MHz, $CDCl_3$) δ: 0.58-0.66 (3H, m), 1.01-1.04 (5H, m), 1.23-1.24 (3H, m), 1.60-1.68 (4H, m), 1.80-1.88 (4H, m), 2.28-2.40 (2H, m), 2.66-2.69 (1H, m), 2.93-3.07 (2H, m), 3.30-3.54 (3H, m), 4.56-4.59 (1H, m), 4.91 (1H, s), 5.02-5.05 (1H, m), 6.51-6.54 (2H, m), 7.05-7.08 (4H, m), 7.15 (2H, d, J=8.5 Hz).
MS (ESI) m/z: 670 [$(M+H)^+$].

Example 6

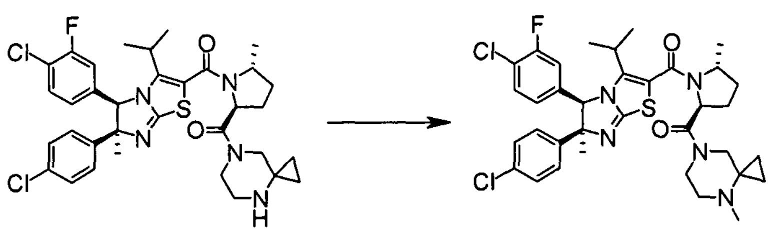

7-[(5R)-1-{[(5R,6S)-5-(4-chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-prolyl]-4-methyl-4,7-diazaspiro[2.5]octane

The compound obtained in Step 10 of Example 5 was reacted in the same way as in Example 2 to give the title compound as a colorless solid.
$^1$H-NMR (400 MHz, $CDCl_3$) δ: 0.58-0.66 (3H, m), 1.01-1.04 (5H, m), 1.23-1.24 (3H, m), 1.60-1.68 (4H, m), 1.80-1.88 (4H, m), 2.28-2.40 (2H, m), 2.66-2.69 (1H, m), 2.93-3.07 (2H, m), 3.30-3.54 (3H, m), 4.56-4.59 (1H, m), 4.91 (1H, s), 5.02-5.05 (1H, m), 6.51-6.54 (2H, m), 7.05-7.08 (4H, m), 7.15 (2H, d, J=8.5 Hz).
MS (ESI) m/z: 684 [$(M+H)^+$].

Example 7

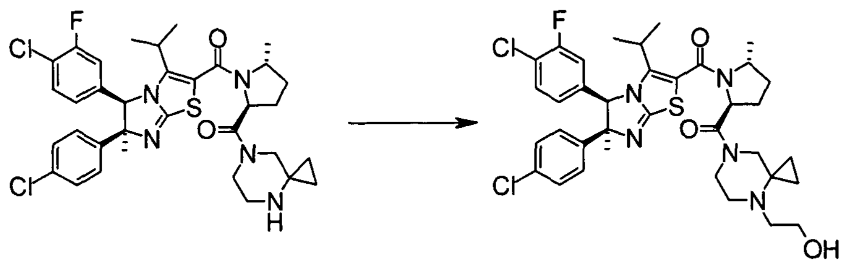

2-{7-[(5R)-1-{[(5R,6S)-5-(4-chloro-3-fluorophenyl)-6-(4-chlorophenyl)-3-isopropyl-6-methyl-5,6-dihydroimidazo[2,1-b][1,3]thiazol-2-yl]carbonyl}-5-methyl-L-prolyl]-4,7-diazaspiro[2.5]oct-4-yl}ethanol

The compound obtained in Step 10 of Example 5 was reacted in the same way as in Step 1 of Example 3 and then reacted in the same way as in Step 2 of Example 3 to give the title compound as a colorless solid.
$^1$H-NMR (400 MHz, $CDCl_3$) δ: 0.61 (2H, s), 0.72 (2H, d, J=31.7 Hz), 0.88-0.89 (2H, m), 0.98-1.03 (6H, m), 1.24-1.25 (4H, m), 1.69 (2H, s), 1.80-1.83 (4H, m), 2.37 (2H, s), 2.66 (1H, s), 2.92-2.94 (3H, m), 3.44-3.54 (4H, m), 4.57 (1H, s), 4.92 (1H, s), 5.01 (1H, s), 6.49-6.52 (2H, m), 7.06-7.08 (3H, m), 7.14-7.16 (2H, m).
MS (ESI) m/z: 714 [$(M+H)^+$].

Example 8

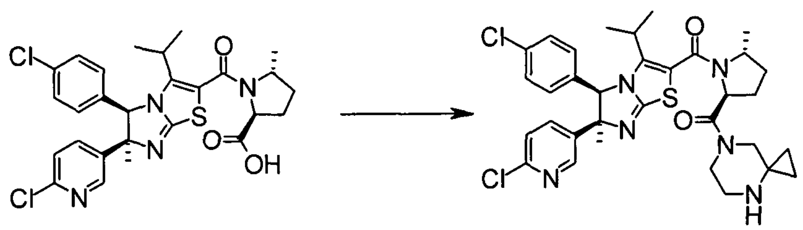

7-[(5R)-1-{[(5R,6S)-5-(4-chlorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-4,7-diazaspiro[2.5]octane

(5R)-1-{[(5R,6S)-5-(4-chlorophenyl)-6-(6-chloropyridin-3-yl)-3-isopropyl-6-methyl-5,6-dihydroimidazo[2,1-b][1,3]thiazol-2-yl]carbonyl}-5-methyl-L-proline obtained from reactions using (4-chlorobenzyl)(triphenyl)phosphonium bromide instead of (4-chloro-3-fluorobenzyl)(triphenyl)phosphonium bromide and 1-(6-chloropyridin-3-yl)-ethanone instead of 1-(4-chlorophenyl)ethanone in Step 1 of Example 5 and then the same reactions as in Step 2 to Step 9 was reacted in the same way as in Step 7 of Example 1 to give the title compound as a colorless solid.
$^1$H-NMR (400 MHz, DMSO-$d_6$) δ: 0.46-0.49 (4H, m), 0.84 (3H, d, J=7.1 Hz), 0.97 (3H, d, J=7.1 Hz), 1.15 (3H, d, J=6.3 Hz), 1.55-1.60 (1H, m), 1.72-1.74 (1H, m), 1.75 (3H, s), 2.03-2.09 (1H, m), 2.28-2.32 (1H, m), 2.68-2.73 (3H, m), 3.24-3.49 (4H, m), 4.29-4.33 (1H, m), 4.87-4.92 (1H, m), 5.45 (1H, s), 6.90 (2H, d, J=7.3 Hz), 7.13 (1H, d, J=9.3 Hz), 7.15 (2H, d, J=8.8 Hz), 7.63 (1H, dd, J=8.3, 2.7 Hz), 8.23 (1H, d, J=2.7 Hz).
MS (ESI) m/z: 653 [$(M+H)^+$].

Example 9

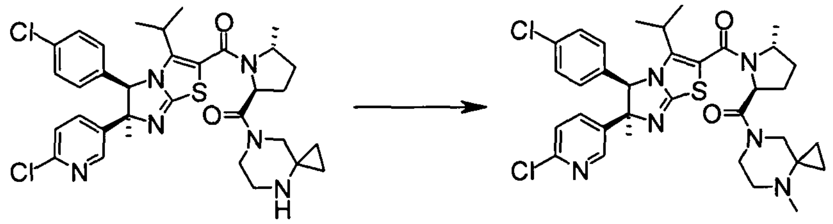

7-[(5R)-1-{[(5R,6S)-5-(4-chlorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-4-methyl-4,7-diazaspiro[2.5]octane

The compound obtained in Example 8 was reacted in the same way as in Example 2 to give the title compound as a colorless solid.
$^1$H-NMR (400 MHz, DMSO-$d_6$) δ: 0.43-0.49 (2H, m), 0.57-0.63 (2H, m), 0.84 (3H, d, J=7.1 Hz), 0.96 (3H, d, J=7.1 Hz), 1.15 (3H, d, J=6.3 Hz), 1.54-1.61 (1H, m), 1.70-1.74 (1H, m), 1.75 (3H, s), 2.04-2.10 (1H, m), 2.29 (3H, s), 2.30-2.32 (1H, m), 2.65-2.70 (1H, m), 2.74-2.78 (2H, m), 3.20-3.21 (2H, m), 3.23-3.29 (2H, m), 4.28-4.32 (1H, m), 4.88-4.92 (1H, m), 5.45 (1H, s), 6.90 (2H, d, J=7.8 Hz), 7.12-7.15 (3H, m), 7.63 (1H, dd, J=8.3, 2.4 Hz), 8.23 (1H, d, J=2.2 Hz).
MS (ESI) m/z: 667 [(M+H)$^+$].

Example 10

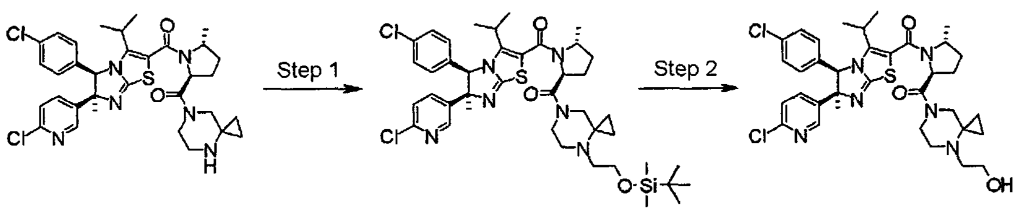


Step 1: 4-(2-{[tert-butyl(dimethyl)silyl]oxy}ethyl)-7-[(5R)-1-{[(5R,6S)-5-(4-chlorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-4,7-diazaspiro[2.5]octane

The compound obtained in Example 8 was reacted in the same way as in Step 1 of Example 3 to give the title compound as a colorless solid.
$^1$H-NMR (400 MHz, $CDCl_3$) δ: 0.01-0.08 (6H, m), 0.87-0.95 (16H, m), 1.02-1.06 (3H, m), 1.21-1.26 (3H, m), 1.63-1.67 (1H, m), 1.82 (3H, s), 1.83-1.87 (1H, m), 2.19-2.37 (2H, m), 2.72-2.77 (1H, m), 2.81-2.86 (1H, m), 2.98-3.02 (1H, m), 3.34-3.72 (8H, m), 4.51-4.54 (1H, m), 4.98 (1H, s), 4.99-5.01 (1H, m), 6.70 (2H, d, J=7.8 Hz), 6.97 (1H, d, J=8.3 Hz), 7.06 (2H, d, J=7.8 Hz), 7.49 (1H, d, J=6.3 Hz), 8.20 (1H, s).
MS (ESI) m/z: 811 [ (M+H)$^+$] .

Step 2: 2-{7-[(5R)-1-{[(5R,6S)-5-(4-chlorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4,7-diazaspiro[2.5]oct-4-yl}ethanol

The compound obtained in Step 1 above was reacted in the same way as in Step 2 of Example 3 to give the title compound as a colorless solid.
$^1$H-NMR (400 MHz, DMSO-$d_6$) δ: 0.49-0.53 (2H, m), 0.56-0.60 (2H, m), 0.84 (3H, d, J=7.1 Hz), 0.96 (3H, d, J=6.8 Hz), 1.15 (3H, d, J=6.3 Hz), 1.54-1.61 (1H, m), 1.70-1.74 (1H, m), 1.75 (3H, s), 2.05-2.11 (1H, m), 2.29-2.34 (1H, m), 2.65-2.70 (1H, m), 2.72-2.78 (1H, m), 2.86-2.90 (1H, m), 3.19-3.22 (2H, m), 3.25-3.28 (2H, m), 3.39-3.43 (2H, m), 3.49-3.52 (1H, m), 3.99-4.03 (1H, m), 4.28-4.33 (1H, m), 4.87-4.92 (1H, m), 5.45 (1H, s), 6.90 (2H, d, J=7.6 Hz), 7.11-7.16 (3H, m), 7.63 (1H, dd, J=8.4, 2.6 Hz), 8.23 (1H, d, J=2.7 Hz).
MS (ESI) m/z: 697 [(M+H)$^+$].

Example 11

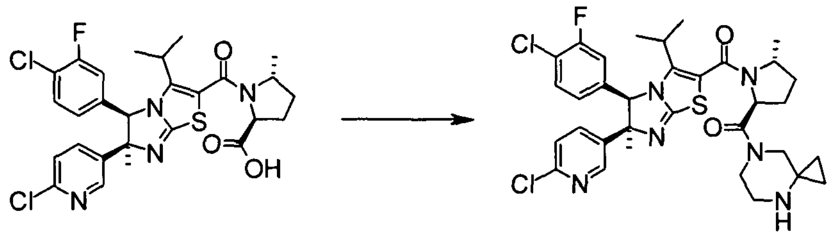

7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b] [1,3]thiazol-2-yl]carbonyl}-5-methyl-L-prolyl]-4,7-diazaspiro[2.5]octane

(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-proline obtained from a reaction using 1-(6-chloropyridin-3-yl)-ethanone instead of 1-(4-chlorophenyl)ethanone in Step 1 of Example 5 and then the same reactions as in Step 2 to Step 9 was reacted in the same way as in Step 7 of Example 1 to give the title compound as a colorless solid.
$^1$H-NMR (400 MHz, DMSO-$d_6$) δ: 0.39-0.54 (4H, m), 0.86 (3H, d, J=6.8 Hz), 0.99 (3H, d, J=7.1 Hz), 1.15 (3H, d, J=6.3 Hz), 1.56-1.59 (1H, m), 1.71-1.74 (1H, m), 1.75 (3H, s), 2.06-2.09 (1H, m), 2.31-2.36 (1H, m), 2.68-2.73 (1H, m), 2.74-2.79 (2H, m), 3.26-3.52 (4H, m), 4.29-4.33 (1H, m), 4.90-4.93 (1H, m), 5.53 (1H, s), 6.72-6.74 (1H, m), 6.90-6.95 (1H, m), 7.19 (1H, d, J=8.3 Hz), 7.35 (1H, t, J=8.1 Hz), 7.67 (1H, dd, J=8.3, 2.4 Hz), 8.27 (1H, d, J=2.4 Hz).
MS (ESI) m/z: 671 [(M+H)$^+$].

Example 12

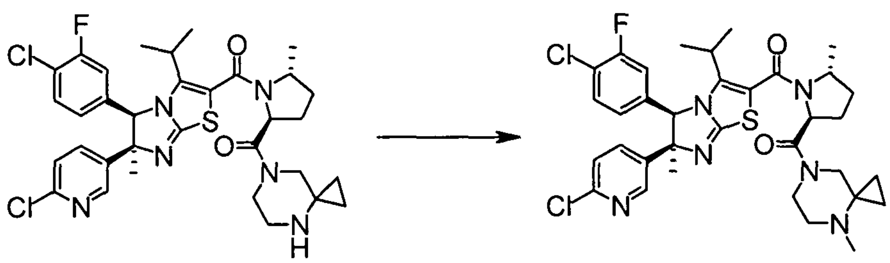

7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4-methyl-4,7-diazaspiro[2.5]octane

The compound obtained in Example 11 was reacted in the same way as in Example 2 to give the title compound as a colorless solid.
$^1$H-NMR (400 MHz, DMSO-$d_6$) δ: 0.38-0.60 (4H, m), 0.85 (3H, d, J=6.6 Hz), 0.97 (3H, d, J=6.8 Hz), 1.14 (3H, d, J=6.1 Hz), 1.56-1.59 (1H, m), 1.70-1.77 (2H, m), 1.74 (3H, s), 2.03-2.08 (1H, m), 2.28 (3H, s), 2.64-2.71 (1H, m), 2.72-2.78 (2H, m), 3.29-3.38 (2H, m), 3.51-3.56 (2H, m), 4.28-4.32 (1H, m), 4.88-4.94 (1H, m), 5.51 (1H, s), 6.70-6.74 (1H, m), 6.86-6.90 (1H, m), 7.18 (1H, d, J=8.3 Hz), 7.32 (1H, t, J=7.9 Hz), 7.66 (1H, dd, J=8.1, 2.7 Hz), 8.26 (1H, d, J=2.2 Hz).
MS (ESI) m/z: 685 [(M+H)$^+$].

Example 13

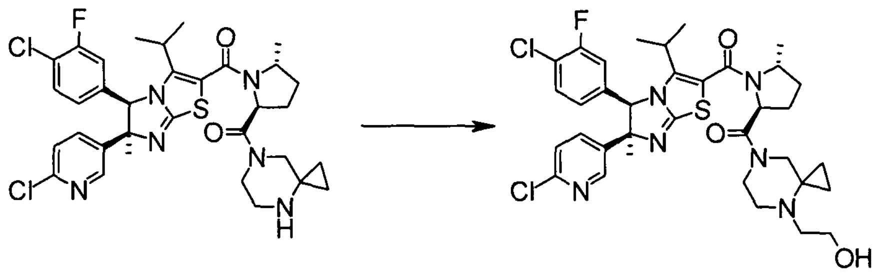

2-{7-[(5R)-1-{[(5R,6S)-5-(4-chlorophenyl)-6-(6-chloropyridin-3-yl)-3-isopropyl-6-methyl-5,6-dihydroimidazo[2,1-b][1,3] thiazol-2-yl]carbonyl}-5-methyl-L-prolyl]-4,7-diazaspiro[2.5]oct-4-yl}ethanol

The compound obtained in Example 11 was reacted in the same way as in Step 1 of Example 3 and then reacted in the same way as in Step 2 of Example 3 to give the title compound as a colorless solid.
$^1$H-NMR (400 MHz, DMSO-$d_6$) δ: 0.44-0.60 (4H, m), 0.85 (3H, d, J=5.9 Hz), 0.97 (3H, d, J=6.6 Hz), 1.14 (3H, d, J=6.3 Hz), 1.55-1.60 (2H, m), 1.74 (3H, s), 2.03-2.10 (1H, m), 2.27-2.34 (1H, m), 2.64-2.70 (1H, m), 2.72-2.78 (2H, m), 2.83-2.90 (2H, m), 3.36-3.42 (4H, m), 3.49-3.53 (1H, m), 4.12-4.16 (1H, m), 4.28-4.34 (1H, m), 4.85-4.95 (1H, m), 5.51 (1H, s), 6.71-6.74 (1H, m), 6.87-6.91 (1H, m), 7.18 (1H, d, J=8.5 Hz), 7.32 (1H, t, J=7.9 Hz), 7.66 (1H, dd, J=8.4, 2.3 Hz), 8.26 (1H, d, J=2.2 Hz).
MS (ESI) m/z: 715 [(M+H)$^+$].

Example 14

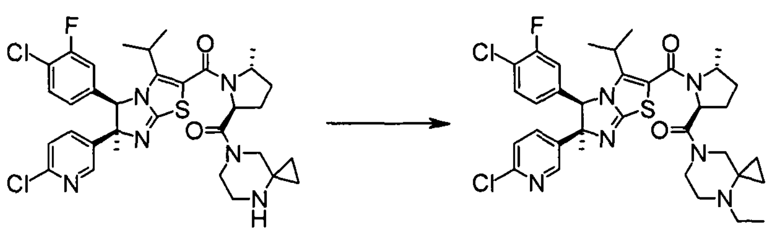

7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4-ethyl-4,7-diazaspiro[2.5]octane

The compound obtained in Example 11 was reacted in the same way as in Example 2 using acetaldehyde instead of 37% aqueous formaldehyde solution to give the title compound as a colorless solid.
$^1$H-NMR (400 MHz, DMSO-$d_6$) δ: 0.03-0.07 (4H, m), 0.38 (3H, d, J=7.1 Hz), 0.47-0.52 (6H, m), 0.67 (3H, d, J=6.3 Hz), 1.06-1.09 (1H, m), 1.27 (4H, s), 1.57-1.65 (1H, m), 1.81 (1H, brs), 2.15-2.26 (3H, m), 2.36 (2H, t, J=5.0 Hz), 2.78-2.81 (2H, m), 2.92-2.94 (1H, m), 2.98-3.04 (1H, m), 3.80-3.83 (1H, m), 4.41 (1H, d, J=8.5 Hz), 5.00 (1H, s), 6.25 (1H, d, J=7.8 Hz), 6.40 (1H, d, J=10.0 Hz), 6.66 (1H, d, J=8.3 Hz), 6.81 (1H, t, J=7.9 Hz), 7.16 (1H, dd, J=8.3, 2.7 Hz), 7.77 (1H, d, J=2.0 Hz).
MS (ESI) m/z: 699 [(M+H)$^+$].

Example 15

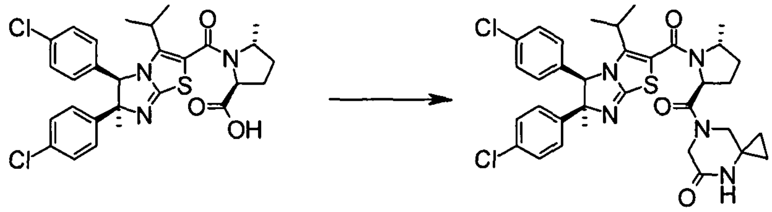

7-{[(5R,6S)-5,6-bis(4-chlorophenyl)-3-isopropyl-6-methyl-5,6-dihydroimidazo[2,1-b][1,3]thiazol-2-yl]carbonyl}-4,7-diazaspiro[2.5]octan-5-one

The compound obtained in Step 6 of Example 1 was reacted in the same way as in Step 7 of Example 1 using the compound obtained in Step 6 of Reference Example 2 instead of 4,7-diazaspiro[2.5]octane hydrochloride to give the title compound as a colorless solid.
$^1$H-NMR (400 MHz, $CDCl_3$) δ: 0.81-0.85 (2H, m), 0.89 (3H, d, J=7.1 Hz), 0.94-1.00 (2H, m), 1.03 (3H, d, J=7.1 Hz), 1.82 (3H, s), 2.43-2.50 (1H, m), 3.70 (2H, AB type d, J=13.4 Hz), 4.33 (2H, AB type d, J=18.0 Hz), 4.96 (1H, s), 6.35 (1H, br), 6.71 (2H, br), 7.02-7.10 (6H, m).
MS (ESI) m/z: 557 [$(M+H)^+$].

Example 16

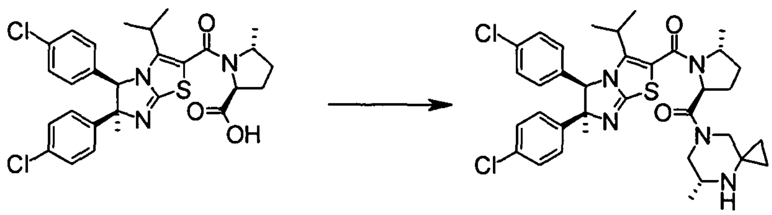

(5R)-7-[(5R)-1-{[(5R,6S)-5,6-bis(4-chlorophenyl)-3-isopropyl-6-methyl-5,6-dihydroimidazo[2,1-b][1,3]thiazol-2-yl]carbonyl}-5-methyl-L-prolyl]-5-methyl-4,7-diazaspiro[2.5]octane

The compound obtained in Step 6 of Example 1 was reacted in the same way as in Step 7 of Example 1 using the compound obtained in Step 7 of Reference Example 3 instead of 4,7-diazaspiro[2.5]octane hydrochloride to give the title compound as a colorless solid.
$^1$H-NMR (400 MHz, DMSO-$d_6$) δ: 0.42 (2H, br), 0.56 (2H, br), 0.86 (3H, d, J=7.0 Hz), 0.98 (6H, d, J=7.0 Hz), 1.16 (3H, d, J=6.3 Hz), 1.53 (2H, br), 1.73 (3H, s), 2.07 (2H, br), 2.27 (2H, br), 2.77 (2H, br), 4.32 (2H, br), 4.91 (2H, br), 5.39 (1H, s), 6.88 (2H, d, J=7.2 Hz), 7.06-7.25 (6H, m) .
MS (ESI) m/z: 668 [$(M+H)^+$].

Example 17

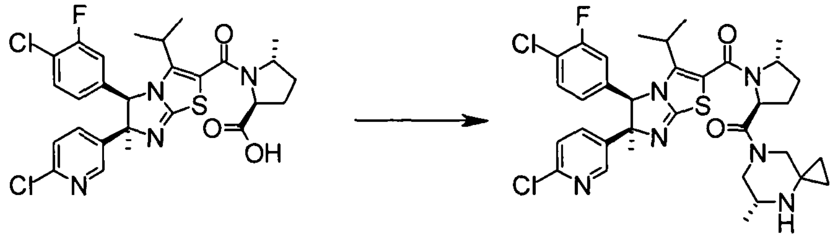

(5R)-7-[(5R)-1-{ [(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-5-methyl-4,7-diazaspiro[2.5]octane

(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-proline was reacted in the same way as in Step 7 of Example 1 using the compound obtained in Step 7 of Reference Example 3 instead of 4,7-diazaspiro[2.5]octane hydrochloride to give the title compound as a colorless solid.
$^1$H-NMR (400 MHz, DMSO-$d_6$) $\delta$: 0.36-0.56 (4H, m), 0.83-0.89 (3H, m), 0.94-1.00 (3H, m), 1.01 (3H, d, J=7.1 Hz), 1.15 (3H, d, J=6.3 Hz), 1.54-1.60 (1H, m), 1.69-1.73 (1H, m), 1.74 (3H, s), 2.06-2.15 (1H, m), 2.28-2.33 (1H, m), 2.64-2.78 (2H, m), 3.29-3.41 (1H, m), 3.49-3.61 (1H, m), 3.75-3.85 (1H, m), 4.04-4.16 (1H, m), 4.27-4.36 (1H, m), 4.84-4.97 (1H, m), 5.54 (1H, s), 6.69-6.75 (1H, m), 6.90-6.96 (1H, m), 7.19 (1H, d, J=8.3 Hz), 7.33-7.39 (1H, m), 7.68 (1H, dd, J=8.3, 2.7 Hz), 8.28 (1H, d, J=2.2 Hz).
MS (ESI) m/z: 685 [(M+H)$^+$].

Example 18

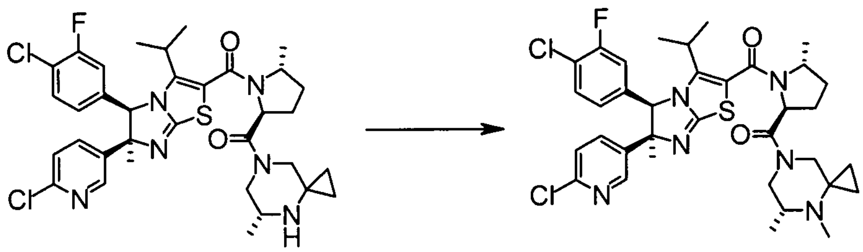

(5R)-7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4,5-dimethyl-4,7-diazaspiro[2.5]octane

The compound obtained in Example 17 was reacted in the same way as in Example 2 to give the title compound as a colorless solid.
$^1$H-NMR (400 MHz, DMSO-$d_6$) $\delta$: 0.45-0.53 (2H, m), 0.58-0.66 (2H, m), 0.87 (3H, d, J=7.3 Hz), 1.00 (3H, d, J=7.1 Hz), 1.02-1.06 (3H, m), 1.15 (3H, d, J=6.1 Hz), 1.56-1.60 (1H, m), 1.72-1.74 (1H, m), 1.75 (3H, s), 2.06-2.11 (1H, m), 2.28 (3H, s), 2.30-2.33 (1H, m), 2.65-2.70 (2H, m), 3.25-3.34 (2H, m), 3.49-3.65 (2H, m), 4.29-4.34 (1H, m), 4.83-4.96 (1H, m), 5.54 (1H, s), 6.73-6.77 (1H, m), 6.88-6.94 (1H, m), 7.19 (1H, d, J=8.3 Hz), 7.34 (1H, t, J=8.2 Hz), 7.67 (1H, dd, J=8.1, 2.4 Hz), 8.28 (1H, d, J=2.2 Hz).
MS (ESI) m/z: 699 [(M+H)$^+$].

Example 19

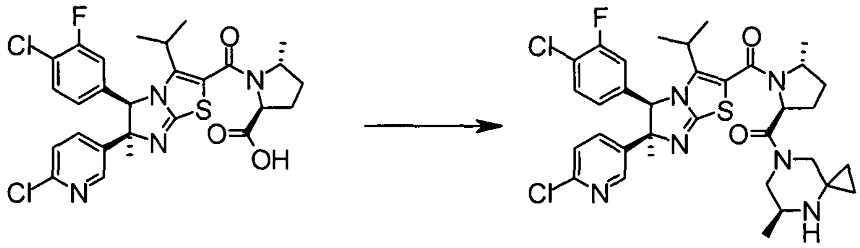

(5S)-7-[(5R)-1-([(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-5-methyl-4,7-diazaspiro[2.5]octane

(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-proline was reacted in the same way as in Step 7 of Example 1 using the compound obtained in Step 3 of Reference Example 4 instead of 4,7-diazaspiro[2.5]octane hydrochloride to give the title compound as a colorless solid.
$^1$H-NMR (400 MHz, DMSO-$d_6$) δ: 0.33-0.45 (1H, m), 0.50-0.60 (2H, m), 0.83-0.90 (5H, m), 0.95-1.02 (4H, m), 0.99 (3H, d, J=6.8 Hz), 1.16 (3H, d, J=6.3 Hz), 1.23-1.32 (1H, m), 1.52-1.61 (1H, m), 1.69-1.78 (2H, m), 1.76 (3H, s), 1.97-2.12 (2H, m), 2.25-2.37 (1H, m), 2.68-2.81 (2H, m), 4.26-4.36 (1H, m), 4.89-4.97 (1H, m), 5.49 (1H, s), 6.68-6.76 (1H, m), 6.85-6.94 (1H, m), 7.16 (1H, d, J=8.3 Hz), 7.30 (1H, t, J=7.9 Hz), 7.65 (1H, dd, J=8.3, 2.4 Hz), 8.26 (1H, d, J=2.2 Hz).
MS (ESI) m/z: 685 [(M+H)$^+$].

Example 20

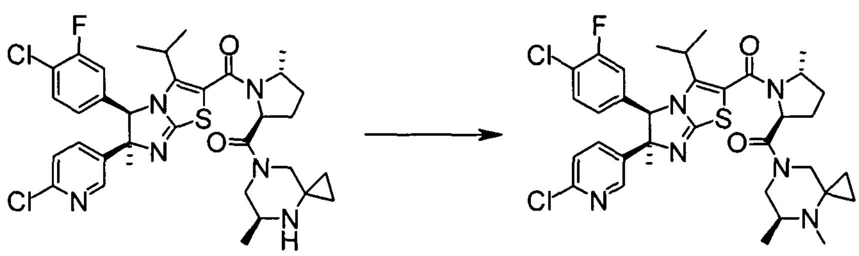

(5S)-7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4,5-dimethyl-4,7-diazaspiro[2.5]octane

The compound obtained in Example 19 was reacted in the same way as in Example 2 to give the title compound as a colorless solid.
$^1$H-NMR (400 MHz, DMSO-$d_6$) δ: 0.40-0. 73 (4H, m), 0.87 (3H, d, J=6.8 Hz), 0.99 (3H, d, J=7.1 Hz), 1.00-1.10 (4H, m), 1.16 (3H, d, J=6.1 Hz), 1.23-1.31 (1H, m), 1.53-1.63 (1H, m), 1.72-1.79 (2H, m), 1.75 (3H, s), 2.00-2.15 (1H, m), 2.29 (3H, s), 2.64-2.76 (1H, m), 3.18-3.33 (2H, m), 3.55-3.70 (1H, m), 4.26-4.37 (1H, m), 4.87-4.95 (1H, m), 5.49 (1H, s), 6.70-6.81 (1H, m), 6.84-6.93 (1H, m), 7.16 (1H, d, J=8.3 Hz), 7.30 (1H, t, J=8.1 Hz), 7.65 (1H, dd, J=8.3, 2.2 Hz), 8.26 (1H, d, J=2.2 Hz).
MS (ESI) m/z: 699 [(M+H)$^+$].

Example 21

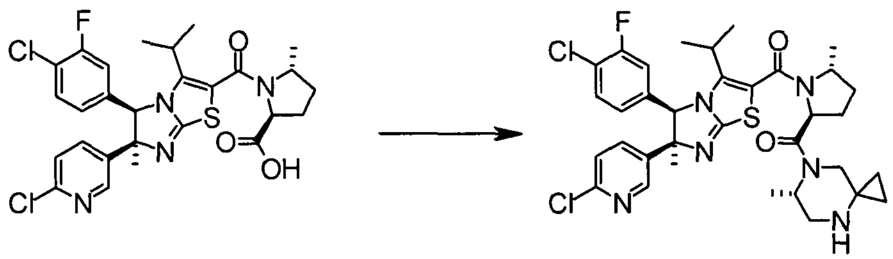

(6S)-7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-6-methyl-4,7-diazaspiro[2.5]octane

(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-proline was reacted in the same way as in Step 7 of Example 1 using the compound obtained in Step 4 of Reference Example 5 instead of 4,7-diazaspiro[2.5]octane hydrochloride to give the title compound as a colorless solid.
$^1$H-NMR (400 MHz, DMSO-$d_6$) δ: 0.35-0.43 (2H, m), 0.50-0.59 (2H, m), 0.88 (3H, d, J=7.1 Hz), 1.00 (3H, d, J=7.1 Hz), 1.15 (3H, d, J=6.3 Hz), 1.16-1.21 (4H, m), 1.53-1.62 (1H, m), 1.68-1.74 (1H, m), 1.75 (3H, s), 2.08-2.19 (1H, m), 2.24-2.35 (1H, m), 2.60-2.74 (2H, m), 2.82-2.94 (2H, m), 4.19-4.34 (2H, m), 4.76-4.88 (2H, m), 5.49 (1H, s), 6.70-6.77 (1H, m), 6.85-6.95 (1H, m), 7.15 (1H, d, J=8.3 Hz), 7.31 (1H, t, J=8.1 Hz), 7.66 (1H, dd, J=8.4, 2.4 Hz), 8.26 (1H, d, J=2.4 Hz).
MS (ESI) m/z: 685 [ $(M+H)^+$] .

Example 22

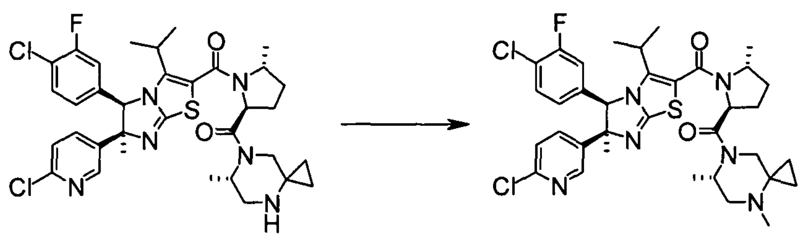

(6S)-7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4,6-dimethyl-4,7-diazaspiro[2.5]octane

The compound obtained in Example 21 was reacted in the same way as in Example 2 to give the title compound as a colorless solid.
$^1$H-NMR (400 MHz, DMSO-$d_6$) δ: 0.21-0.29 (1H, m), 0.40-0.49 (1H, m), 0.62-0.71 (1H, m), 0.83-0.87 (1H, m), 0.88 (3H, d, J=7.1 Hz), 1.00 (3H, d, J=7.1 Hz), 1.15 (3H, d, J=6.3 Hz), 1.23-1.29 (3H, m), 1.53-1.62 (1H, m), 1.67-1.74 (1H, m), 1.75 (3H, s), 2.07 (3H, s), 2.09-2.20 (1H, m), 2.22-2.34 (1H, m), 2.59 (2H, d, J=2.9 Hz), 2.63-2.74 (1H, m), 3.10-3.26 (1H, m), 3.46-3.63 (1H, m), 4.26-4.35 (2H, m), 4.80-4.87 (1H, m), 5.49 (1H, s), 6.71-6.79 (1H, m), 6.85-6.95 (1H, m), 7.15 (1H, d, J=8.3 Hz), 7.30 (1H, t, J=7.9 Hz), 7.65 (1H, dd, J=8.3, 2.4 Hz), 8.26 (1H, d, J=2.0 Hz).
MS (ESI) m/z: 699 [$(M+H)^+$].

Example 23

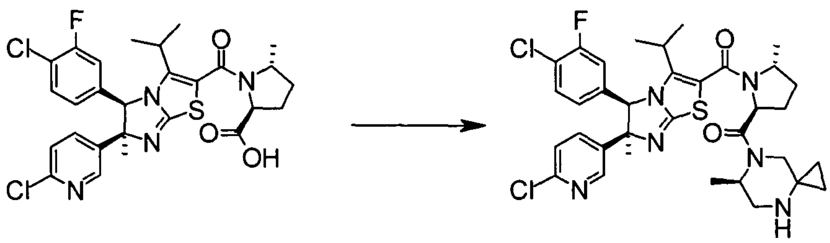

(6R)-7-[(5R)-1-{ [(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-6-methyl-4,7-diazaspiro[2.5]octane

(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-proline was reacted in the same way as in Step 7 of Example 1 using the compound obtained in Step 4 of Reference Example 6 instead of 4,7-diazaspiro[2.5]octane hydrochloride to give the title compound as a colorless solid.
$^1$H-NMR (400 MHz, DMSO-$d_6$) δ: 0.28-0.44 (1H, m), 0.48-0.62 (2H, m), 0.83-0.86 (1H, m), 0.87 (3H, d, J=7.1 Hz), 0.99 (3H, d, J=7.1 Hz), 1.16 (3H, d, J=6.1 Hz), 1.17-1.39 (4H, m), 1.51-1.79 (1H, m), 1.75 (3H, s), 1.96-2.11 (1H, m), 2.26-2.45 (6H, m), 2.61-2.76 (2H, m), 4.26-4.35 (1H, m), 4.84-4.90 (1H, m), 5.48 (1H, s), 6.68-6.76 (1H, m), 6.83-6.94 (1H, m), 7.15 (1H, d, J=8.3 Hz), 7.30 (1H, t, J=8.2 Hz), 7.64 (1H, dd, J=8.3, 2.3 Hz), 8.25 (1H, d, J=2.3 Hz).
MS (ESI) m/z: 685 [$(M+H)^+$].

Example 24

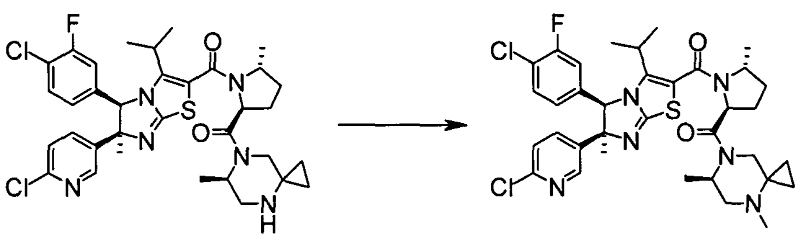

(6R)-7-[(5R)-1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4,6-dimethyl-4,7-diazaspiro[2.5]octane

The compound obtained in Example 23 was reacted in the same way as in Example 2 to give the title compound as a colorless solid.
$^1$H-NMR (400 MHz, DMSO-$d_6$) δ: 0.39-0.52 (1H, m), 0.57-0.68 (1H, m), 0.83-0.86 (2H, m), 0.87 (3H, d, J=6.8 Hz), 0.99 (3H, d, J=6.8 Hz), 1.16 (3H, d, J=6.3 Hz), 1.18-1.42 (4H, m), 1.51-1.74 (2H, m), 1.75 (3H, s), 1.97-2.15 (2H, m), 2.09 (3H, s), 2.25-2.46 (2H, m), 2.58-2.66 (2H, m), 2.67-2.76 (1H, m), 4.26-4.35 (1H, m), 4.85-4.90 (1H, m), 5.48 (1H, s), 6.69-6.78 (1H, m), 6.83-6.91 (1H, m), 7.15 (1H, d, J=8.5 Hz), 7.29 (1H, t, J=8.1 Hz), 7.64 (1H, dd, J=8.3, 2.4 Hz), 8.25 (1H, d, J=2.4 Hz).
MS (ESI) m/z: 699 [$(M+H)^+$].

Example 25

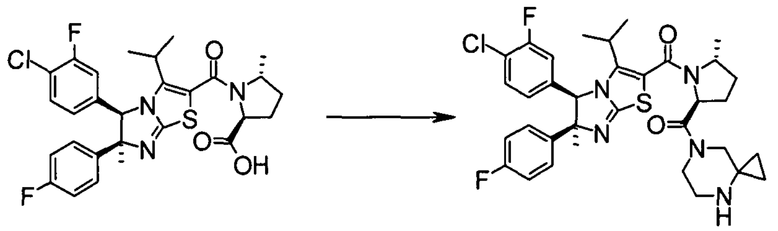

7-[(5R)-1-{[(5R,6S)-5-(4-chloro-3-fluorophenyl)-6-(4-fluorophenyl)-3-isopropyl-6-methyl-5,6-dihydroimidazo[2,1-b][1,3] thiazol-2-yl]carbonyl}-5-methyl-L-prolyl]-4,7-diazaspiro[2.5]octane

(5R)-1-{[(5R,6S)-5-(4-chloro-3-fluorophenyl)-6-(4-fluorophenyl)-3-isopropyl-6-methyl-5,6-dihydroimidazo[2,1-b][1,3]thiazol-2-yl]carbonyl}-5-methyl-L-proline obtained from a reaction using 1-(4-fluorophenyl)ethanone instead of 1-(4-chlorophenyl)ethanone in Step 1 of Example 5 and then the same reactions as in Step 2 to Step 9 was reacted in the same way as in Step 7 of Example 1 to give the title compound as a colorless solid.
$^1$H-NMR (400 MHz, DMSO-$d_6$) δ: 0.44-0. 53 (4H, m), 0.88 (3H, d, J=6.8 Hz), 0.99 (3H, d, J=7.1 Hz), 1.16 (3H, d, J=6.1 Hz), 1.55-1.60 (1H, m), 1.74 (3H, s), 1.75-1.76 (1H, m), 2.06-2.11 (1H, m), 2.28-2.33 (1H, m), 2.68-2.73 (1H, m), 2.76-2.81 (2H, m), 3.30-3.53 (4H, m), 4.30-4.35 (1H, m), 4.90-4.94 (1H, m), 5.43 (1H, s), 6.70 (1H, d, J=8.1 Hz), 6.82-6.87 (3H, m), 7.24-7.30 (3H, m).
MS (ESI) m/z: 654 [$(M+H)^+$].

Example 26

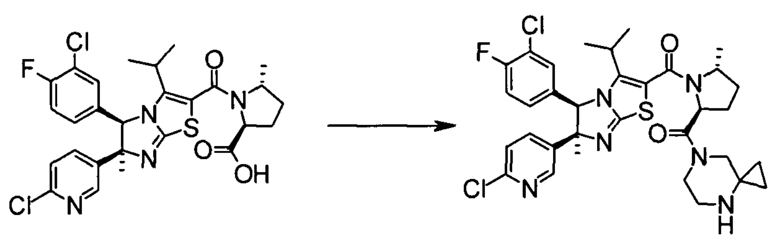

7-[(5R)-1-{[(5R,6S)-5-(3-chloro-4-fluorophenyl)-6-(6-chloropyridin-3-yl)-3-isopropyl-6-methyl-5,6-dihydroimidazo [2,1-b][1,3]thiazol-2-yl]carbonyl}-5-methyl-L-prolyl]-4,7-diazaspiro[2.5]octane

(5R)-1-{[(5R,6S)-5-(3-chloro-4-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-proline obtained from reactions using (3-chloro-4-fluorobenzyl)(triphenyl) phosphonium bromide instead of (4-chloro-3-fluorobenzyl)(triphenyl)phosphonium bromide and 1-(6-chloropyridin-3-yl)-ethanone instead of 1-(4-chlorophenyl)ethanone in Step 1 of Example 5 and then the same reactions as in Step 2 to Step 9 was reacted in the same way as in Step 7 of Example 1 to give the title compound as a colorless solid. $^1$H-NMR (400 MHz, DMSO-$d_6$) δ: 0.46-0.55 (4H, m), 0.88 (3H, d, J=7.1 Hz), 1.00 (3H, d, J=7.1 Hz), 1.17 (3H, d, J=6.3 Hz), 1.56-1.62 (1H, m), 1.71-1.75 (1H, m), 1.76 (3H, s), 2.04-2.13 (1H, m), 2.26-2.35 (1H, m), 2.69-2.75 (1H, m), 2.76-2.80 (2H, m), 3.31-3.35 (2H, m), 3.37-3.43 (1H, m), 3.46-3.52 (1H, m), 4.31-4.35 (1H, m), 4.89-4.93 (1H, m), 5.51 (1H, s), 6.84-6.88 (1H, m), 7.07-7.11 (1H, m), 7.15 (1H, t, J=8.9 Hz), 7.17 (1H, d, J=8.3 Hz), 7.65 (1H, dd, J=8.3, 2.4 Hz), 8.26 (1H, d, J=2.2 Hz).
MS (ESI) m/z: 671 [$(M+H)^+$].

Example 27

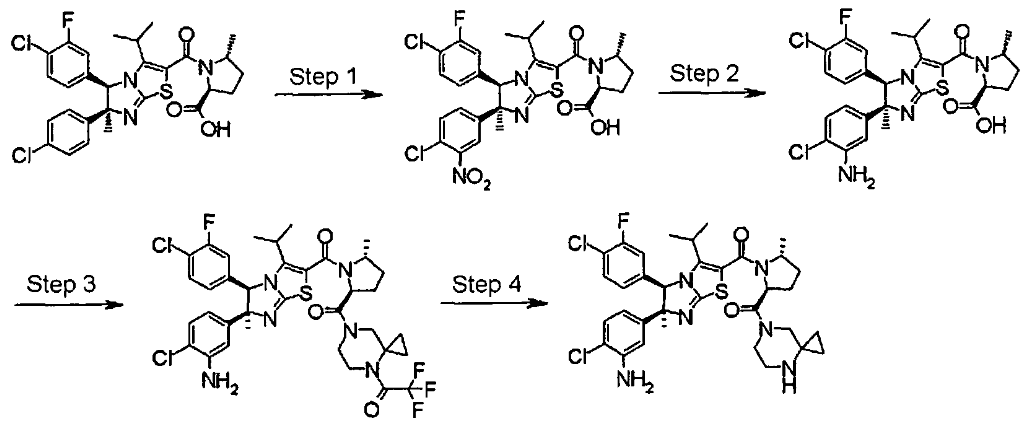


Step 1: (5R)-1-{[(5R,6S)-5-(4-chloro-3-fluorophenyl)-6-(4-chloro-3-nitrophenyl)-3-isopropyl-6-methyl-5,6-dihydroimidazo[2,1-b][1,3]thiazol-2-yl]carbonyl}-5-methyl-L-proline

Potassium nitrate (58 mg, 0.57 mmol) was gradually added to a concentrated sulfuric acid (3 ml) solution of the compound (300 mg, 0.52 mmol) obtained in Step 9 of Example 5 under ice cooling. The resulting mixture was stirred at the same temperature for 30 minutes, then the reaction mixture was poured into ice water and the precipitated solid was collected by filtration and dried to give the title compound (320 mg, 99%) as a colorless solid.
$^1$H-NMR (400 MHz, DMSO-$d_6$) δ: 0.96 (3H, d, J=7.1 Hz), 1.02 (3H, d, J=7.1 Hz), 1.19 (3H, d, J=6.4 Hz), 1.67 (1H, m), 1.98 (1H, m), 2.02 (3H, s), 2.11 (1H, m), 2.38 (1H, m), 2.77 (1H, m), 4.32 (1H, m), 4.57 (1H, d, J=6.8 Hz), 5.97 (1H, s), 6.78 (1H, brs), 7.06 (1H, brs), 7.35 (1H, t, J=8.1 Hz), 7.55-7.58 (2H, m), 7.88 (1H, d, J=1.7 Hz).

Step 2: (5R)-1-{[(5R,6S)-6-(3-amino-4-chlorophenyl)-5-(4-chloro-3-fluorophenyl)-3-isopropyl-6-methyl-5,6-dihydroimidazo[2,1-b][1,3]thiazol-2-yl]carbonyl}-5-methyl-L-proline

Acetic acid (6 ml) and iron powder (144 mg, 2.57 mmol) were added to an ethanol (3 ml) solution of the compound (320 mg, 0.51 mmol) obtained in Step 1 above and the resulting mixture was heated to reflux for 4 hours. After cooling, the reaction mixture was diluted with ethanol, insoluble matter was removed and the filtrate was concentrated under reduced pressure. Water and ethyl acetate were added to the residue for extraction. The organic layer was washed with brine and then dried over anhydrous sodium sulfate and the solvent was evaporated under reduced pressure to give the title compound (300 mg, 99%) as a brown solid.
$^1$H-NMR (400 MHz, DMSO-$d_6$) δ: 0.93 (3H, d, J=7.1 Hz), 0.99 (3H, d, J=7.1 Hz), 1.17 (3H, d, J=6.4 Hz), 1.65 (1H, m), 1.77 (2H, brs), 1.90 (3H, s), 1.91 (1H, m), 2.12 (1H, m), 2.35 (1H, m), 2.72 (1H, m), 4.32 (1H, m), 4.55 (1H, m), 5.54 (1H, s), 6.44 (1H, d, J=8.1 Hz), 6.70 (1H, d, J=8.1 Hz), 6.76 (1H, brs), 6.87-6.90 (2H, m), 7.28 (1H, t, J=8.1 Hz).

Step 3: 2-chloro-5-[(5R,6S)-5-(4-chloro-3-fluorophenyl)-3-isopropyl-6-methyl-2-([(2R,5S)-2-methyl-5-{[4-(trifluoroacetyl)-4,7-diazaspiro[2.5]oct-7-yl]carbonyl}pyrrolidin-1-yl]carbonyl}-5,6-dihydroimidazo[2,1-b][1,3]thiazol-6-yl]aniline

The compound obtained in Step 2 above was reacted in the same way as in Step 7 of Example 1 using the compound obtained in Step 2 of Reference Example 7 instead of 4,7-diazaspiro[2.5]octane hydrochloride to give the title compound as a colorless solid.
$^1$H-NMR (400 MHz, DMSO-$d_6$) δ: 0.91 (3H, d, J=6.8 Hz), 0.96 (3H, d, J=7.1 Hz), 0.98-1.16 (4H, m), 1.17 (3H, d, J=6.8 Hz), 1.61 (1H, m), 1.68 (3H, s), 1.76 (1H, m), 2.13 (1H, m), 2.29 (1H, m), 2.69 (1H, m), 3.44-3.79 (6H, m), 4.34 (1H, m), 4.78 (2H, brs), 4.95 (1H, m), 5.35 (1H, s), 6.44 (1H, dd, J=8.3, 2.2 Hz), 6.75 (1H, d, J=8.3 Hz), 6.80 (1H, d, J=2.0 Hz), 6.81-6.86 (2H, m), 7.25 (1H, t, J=8.1 Hz).

Step 4: 2-chloro-5-[(5R,6S)-5-(4-chloro-3-fluorophenyl)-2-{[(2S,5R)-2-(4,7-diazaspiro[2.5]oct-7-ylcarbonyl)-5-methylpyrrolidin-1-yl]carbonyl}-3-isopropyl-6-methyl-5,6-dihydroimidazo[2,1-b][1,3]thiazol-6-yl]aniline

Potassium carbonate (50 mg, 0.35 mmol) and water (0.4 ml) were added to a methanol (4 ml) solution of the compound (270 mg, 0.35 mmol) obtained in Step 3 above and the resulting mixture was stirred at room temperature for 5 hours. The reaction mixture was concentrated under reduced pressure and water was added to the residue, followed

by extraction with chloroform. The organic layer was washed with brine and then dried over anhydrous sodium sulfate and the solvent was evaporated under reduced pressure. The residue was purified by column chromatography [chloroform:methanol = 8:1 (v/v)] to give the title compound (196 mg, 83%) as a colorless solid. $^1$H-NMR (400 MHz, DMSO-$d_6$) δ: 0.48 (4H, brs), 0.89 (3H, d, J=7.1 Hz), 0.97 (3H, t, J=6.1 Hz), 1.16 (3H, d, J=6.1 Hz), 1.58 (1H, m), 1.68 (3H, s), 1.72 (1H, m), 2.10 (1H, m), 2.25-2.30 (2H, m), 2.69 (1H, m), 2.77 (2H, brs), 3.30 (2H, brs), 3.39 (1H, m), 3.48 (1H, m), 4.31 (1H, m), 4.78 (2H, brs), 4.90 (1H, d, J=7.8 Hz), 5.33 (1H, s), 6.43 (1H, dd, J=8.3, 2.2 Hz), 6.73 (1H, d, J=8.1 Hz), 6.81 (1H, d, J=2.0 Hz), 6.83-6.87 (2H, m), 7.26 (1H, t, J=8.1 Hz).
MS (ESI) m/z: 685 [(M+H)$^+$].

Example 28

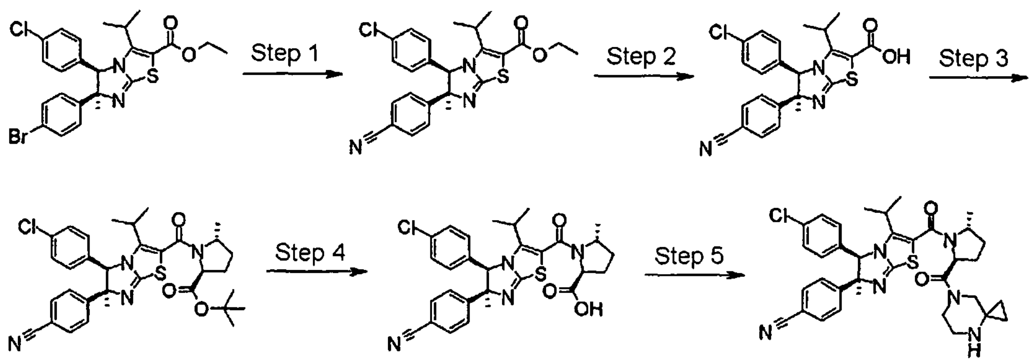


Step 1: ethyl (5R,6S)-5-(4-chlorophenyl)-6-(4-cyanophenyl)-3-isopropyl-6-methyl-5,6-dihydroimidazo[2,1-b][1,3]thiazole-2-carboxylate

Copper cyanide (427 mg, 4.77 mmol) was added to a 1-methyl-2-pyrrolidone (20 ml) solution of ethyl 6-(4-bromophenyl)-5-(4-chlorophenyl)-3-isopropyl-6-methyl-5,6-dihydroimidazo[2,1-b][1,3]thiazole-2-carboxylate (1.24 g, 2.38 mmol) obtained from a reaction using 1-(4-bromophenyl)ethanone instead of 1-(4-chlorophenyl)ethanone in Step 1 of Example 5 and then the same reactions as in Step 2 to Step 6. The resulting mixture was heated at 140˚C for 30 hours in a nitrogen atmosphere. After cooling, ammonia water was added to the reaction mixture. After extraction with ethyl acetate, the organic layer was washed with brine and then dried over anhydrous sodium sulfate. The solvent was evaporated under reduced pressure and the residue was purified by column chromatography [n-hexane:ethyl acetate = 5:1 (v/v)] to give the title compound (756 mg, 68%) as a pale yellow oil.
$^1$H-NMR (400 MHz, $CDCl_3$) δ: 0.87 (3H, d, J=7.1 Hz), 0.97 (3H, d, J=7.1 Hz), 1.32 (3H, t, J=7.2 Hz), 1.78 (3H, s), 3.31 (1H, m), 4.23 (2H, q, J=7.2 Hz), 5.04 (1H, s), 6.70 (2H, brs), 6.99-7.18 (6H, m).
MS (ESI) m/z: 466 [(M+H)$^+$].

Step 2: (5R,6S)-5-(4-chlorophenyl)-6-(4-cyanophenyl)-3-isopropyl-6-methyl-5,6-dihydroimidazo[2,1-b][1,3]thiazole-2-carboxylic acid

The compound obtained in Step 1 above was reacted in the same way as in Step 4 of Example 1 to give the title compound as a colorless solid.
$^1$H-NMR (400 MHz, DMSO-$d_6$) δ: 0.93 (6H, d, J=7.1 Hz), 2.02 (3H, s), 3.27 (1H, m), 6.22 (1H, brs), 7.19-7.22 (2H, m), 7.42 (2H, d, J=8.5 Hz), 7.64 (2H, d, J=8.5 Hz).

Step 3: tert-butyl (5R)-1-{[(5R,6S)-5-(4-chlorophenyl)-6-(4-cyanophenyl)-3-isopropyl-6-methyl-5,6-dihydroimidazo [2,1-b][1,3]thiazol-2-yl]carbonyl}-5-methyl-L-prolinate

The compound obtained in Step 2 above was reacted in the same way as in Step 5 of Example 1 to give the title compound as a pale yellow solid.

$^1$H-NMR (400 MHz, $CDCl_3$) δ: 0.98 (3H, d, J=7.1 Hz), 1.03 (3H, d, J=7.1 Hz), 1.20 (3H, d, J=6.3 Hz), 1.45 (9H, s), 1.58-1.67 (1H, m), 1.82 (3H, s), 1.95-2.02 (1H, m), 2.13-2.33 (2H, m), 2.65-2.76 (1H, m), 4.43-4.51 (1H, m), 4.53-4.58 (1H, m), 4.99 (1H, s), 6.69 (2H, d, J=8.3 Hz), 7.00 (2H, d, J=8.1 Hz), 7.30-7.36 (4H, m).

Step 4:(5R)-1-{[(5R,6S)-5-(4-chlorophenyl)-6-(4-cyanophenyl)-3-isopropyl-6-methyl-5,6-dihydroimidazo[2,1-b][1,3]thiazol-2-yl]carbonyl}-5-methyl-L-proline

The compound obtained in Step 3 above was reacted in the same way as in Step 6 of Example 1 to give the title compound as a colorless solid.
$^1$H-NMR (400 MHz, $CDCl_3$) δ: 0.96 (3H, d, J=7.1 Hz), 1.07 (3H, d, J=7.1 Hz), 1.24 (3H, d, J=6.3 Hz), 1.60-1.66 (1H, m), 2.08 (3H, s), 2.13-2.32 (3H, m), 2.57-2.65 (1H, m), 4.39-4.46 (1H, m), 4.57-4.62 (1H, m), 5.51 (1H, s), 6.67-6.75 (2H, m), 7.11 (2H, d, J=8.5 Hz), 7.24 (2H, d, J=8.5 Hz), 7.40 (2H, d, J=8.5 Hz), 8.31 (1H, brs).

Step 5: 4-[(5R,6S)-5-(4-chlorophenyl)-2-{[(2S,5R)-2-(4,7-diazaspiro[2.5]oct-7-ylcarbonyl)-5-methylpyrrolidin-1-yl]carbonyl}-3-isopropyl-6-methyl-5,6-dihydroimidazo[2,1-b][1,3]thiazol-6-yl]benzonitrile

The compound obtained in Step 4 above was reacted in the same way as in Step 7 of Example 1 to give the title compound as a colorless solid.
$^1$H-NMR (400 MHz, $CDCl_3$) δ: 0.49-0.74 (4H, m), 0.93-1.06 (6H, m), 1.19-2.04 (12H, m), 1.20-1.25 (3H, m), 1.83 (3H, s), 4.99 (1H, s), 6.69 (2H, d, J=7.8 Hz), 7.01 (2H, d, J=8.5 Hz), 7.32-7.39 (4H, m).
ESI-MS: 643 $[(M+H)^+]$.

Example 29

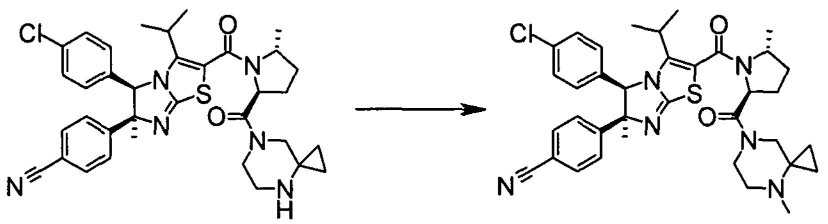

4-[(5R,6S)-5-(4-chlorophenyl)-3-isopropyl-6-methyl-2-({(2R,5S)-2-methyl-5-[(4-methyl-4,7-diazaspiro[2.5]oct-7-yl)carbonyl]pyrrolidin-1-yl}carbonyl)-5,6-dihydroimidazo[2,1-b][1,3]thiazol-6-yl]benzonitrile

The compound obtained in Step 5 of Example 28 was reacted in the same way as in Example 2 to give the title compound as a colorless solid.
$^1$H-NMR (400 MHz, $CDCl_3$) δ: 0.42-0.82 (4H, m), 0.94 (3H, d, J=7.1 Hz), 1.03 (3H, d, J=7.1 Hz), 1.23 (3H, d, J=6.3 Hz), 1.52-2.39 (4H, m), 1.81 (3H, s), 2.38 (3H, s), 2.70-2.80 (1H, m), 2.84-2.94 (2H, m), 3.23-3.69 (4H, m), 4.48-4.56 (1H, m), 4.95-4.99 (1H, m), 4.97 (1H, s), 6.69 (2H, d, J=8.5 Hz), 7.00 (2H, d, J=8.5 Hz), 7.30-7.38 (4H, m).
MS (ESI) m/z: 657 $[(M+H)^+]$.

Example 30

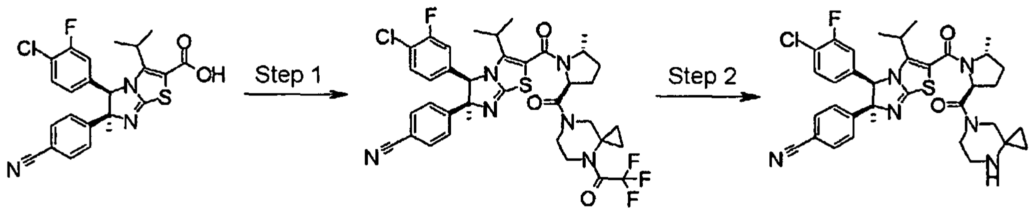


Step 1: 4-[(5R,6S)-5-(4-chloro-3-fluorophenyl)-3-isopropyl-6-methyl-2-{[(2R,5S)-2-methyl-5-{[4-(trifluoroacetyl)-4,7-diazaspiro[2.5]oct-7-yl]carbonyl}pyrrolidin-1-yl]carbonyl}-5,6-dihydroimidazo[2,1-b][1,3]thiazol-6-yl]benzonitrile

(5R,6S)-5-(4-chloro-3-fluorophenyl)-6-(4-cyanophenyl)-3-isopropyl-6-methyl-5,6-dihydroimidazo[2,1-b][1,3] thiazole-2-carboxylic acid obtained from a reaction using 1-(4-bromophenyl)ethanone instead of 1-(4-chlorophenyl) ethanone in Step 1 of Example 5, then the same reactions as in Step 2 to Step 6 and further the same reactions as in Step 1 and Step 2 of Example 28 was reacted in the same way as in Step 5 of Example 1 using the compound obtained in Step 2 of Reference Example 8 instead of the compound obtained in Step 2 of Reference Example 1 to give the title compound as a colorless solid. $^1$H-NMR (400 MHz, DMSO-$d_6$) δ: 0.89 (3H, d, J=7.1 Hz), 0.98 (3H, d, J=7.1 Hz), 1.01-1.04 (4H, m), 1.16 (3H, d, J=6.3 Hz), 1.60 (1H, m), 1.75 (3H, s), 1.78 (1H, m), 2.12 (1H, m), 2.28 (1H, m), 2.70 (1H, m), 3.44-3.80 (6H, m), 4.38 (1H, m), 4.96 (1H, m), 5.51 (1H, s), 6.73 (1H, d, J=7.8 Hz), 6.85 (1H, d, J=9.3 Hz), 7.24 (1H, t, J=8.0 Hz), 7.47 (2H, d, J=8.0 Hz), 7.49 (2H, d, J=8.0 Hz).

Step 2: 4-[(5R,6S)-5-(4-chloro-3-fluorophenyl)-2-{[(2S,5R)-2-(4,7-diazaspiro[2.5]oct-7-ylcarbonyl)-5-methylpyrrolidin-1-yl]carbonyl}-3-isopropyl-6-methyl-5,6-dihydroimidazo[2,1-b][1,3]thiazol-6-yl]benzonitrile

The compound obtained in Step 1 above was reacted in the same way as in Step 4 of Example 27 to give the title compound as a colorless solid.
$^1$H-NMR (400 MHz, DMSO-$d_6$) δ: 0.48 (4H, brs), 0.88 (4H, d, J=7.1 Hz), 0.99 (3H, d, J=7.1 Hz), 1.16 (4H, d, J=6.3 Hz), 1.59 (1H, m), 1.73 (1H, m), 1.76 (3H, s), 2.09 (1H, m), 2.31 (1H, m), 2.68-2.77 (3H, m), 3.33-3.47 (4H, m), 4.32 (1H, m), 4.91 (1H, m), 5.50 (1H, s), 6.72 (1H, d, J=8.1 Hz), 6.88 (1H, d, J=9.8 Hz), 7.26 (1H, t, J=8.1 Hz), 7.49 (4H, brs).
MS (ESI) m/z: 661 [$(M+H)^+$].

Example 31

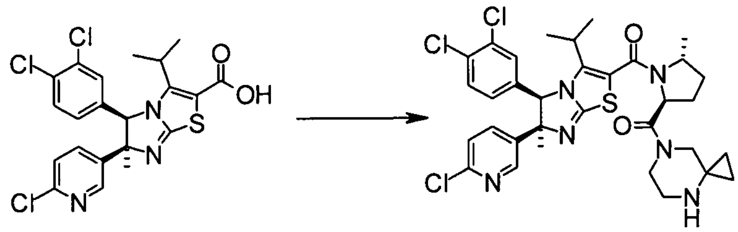

7-[(5R)-1-{[(5R,6S)-6-(6-chloropyridin-3-yl)-5-(3,4-dichlorophenyl)-3-isopropyl-6-methyl-5,6-dihydroimidazo[2,1-b][1,3] thiazol-2-yl]carbonyl}-5-methyl-L-prolyl]-4,7-diazaspiro [2.5] octane

(5R,6S)-6-(6-chloropyridin-3-yl)-5-(3,4-dichlorophenyl)-3-isopropyl-6-methyl-5,6-dihydroimidazo[2,1-b][1,3] thiazole-2-carboxylic acid obtained from reactions using (3,4-dichlorobenzyl)(triphenyl)phosphonium bromide instead of (4-chloro-3-fluorobenzyl)(triphenyl)phosphonium bromide and 1-(6-chloropyridin-3-yl)-ethanone instead of 1-(4-chlorophenyl)ethanone in Step 1 of Example 5 and then the same reactions as in Step 2 to Step 7 was reacted in the same way as in Step 5 of Example 1 and then reacted in the same way as in Step 4 of Example 27 to give the title compound as a colorless solid.

$^1$H-NMR (400 MHz, DMSO-$d_6$) δ: 0.47-0.64 (4H, m), 0.94 (3H, d, J=7.1 Hz), 1.06 (3H, d, J=7.1 Hz), 1.23 (3H, d, J=6.3 Hz), 1.58-1.70 (1H, m), 1.74-1.91 (1H, m), 1.82 (3H, s), 2.08-2.23 (1H, m), 2.29-2.44 (1H, m), 2.71-2.89 (3H, m), 2.93-3.11 (1H, m), 3.34-3.59 (4H, m), 4.33-4.43 (1H, m), 4.96 (1H, d, J=7.8 Hz), 5.56 (1H, s), 6.89 (1H, d, J=8.3 Hz), 7.17-7.26 (2H, m), 7.42 (1H, d, J=8.3 Hz), 7.71 (1H, dd, J=8.3, 2.7 Hz), 8.31 (1H, d, J=2.7 Hz).
MS (ESI) m/z: 687 [(M+H)$^+$].

Example 32

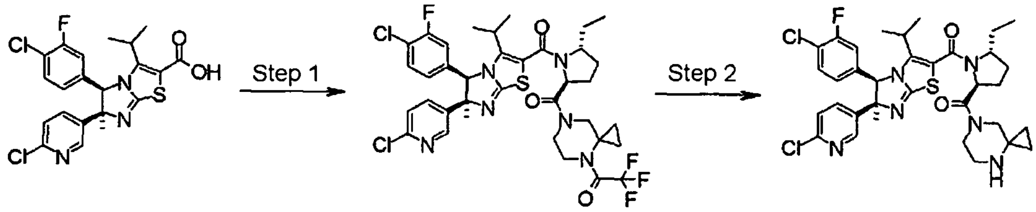


Step 1: 7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolyl]-4-(trifluoroacetyl)-4,7-diazaspiro[2.5]octane

(5R,6S)-5-(4-chloro-3-fluorophenyl)-6-(6-chloropyridin-3-yl)-3-isopropyl-6-methyl-5,6-dihydroimidazo[2,1-b][1,3]thiazole-2-carboxylic acid was reacted in the same way as in Step 5 of Example 1 using the compound obtained in Step 4 of Reference Example 9 instead of the compound obtained in Step 2 of Reference Example 8 to give the title compound as a colorless solid. $^1$H-NMR (400 MHz, DMSO-$d_6$) δ: 0.83-0.87 (6H, m), 0.89-1.05 (3H, m), 1.01 (4H, d, J=6.8 Hz), 1.37-1.42 (1H, m), 1.62-1.68 (1H, m), 1.71-1.79 (2H, m), 1.75 (3H, s), 1.97-2.04 (1H, m), 2.20-2.26 (1H, m), 2.71-2.79 (1H, m), 3.46-3.79 (6H, m), 4.12-4.20 (1H, m), 4.92-4.99 (1H, m), 5.56 (1H, s), 6.74-6.85 (2H, m), 7.19 (1H, d, J=8.3 Hz), 7.32 (1H, t, J=7.9 Hz), 7.68 (1H, d, J=5.9 Hz), 8.28 (2H, d, J=2.0 Hz).
MS (ESI) m/z: 781 [(M+H)$^+$].

Step 2: 7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolyl]-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$) δ: 0.41-0.51 (4H, m), 0.84 (6H, t, J=7.3 Hz), 1.02 (3H, d, J=6.8 Hz), 1.35-1.41 (1H, m), 1.63-1.74 (3H, m), 1.75 (3H, s), 1.94-2.01 (1H, m), 2.22-2.29 (1H, m), 2.72-2.80 (3H, m), 3.27-3.51 (4H, m), 4.12-4.17 (1H, m), 4.89-4.94 (1H, m), 5.55 (1H, s), 6.70-6.76 (1H, m), 6.88-6.95 (1H, m), 7.19 (1H, d, J=8.3 Hz), 7.34 (1H, t, J=7.9 Hz), 7.67 (1H, dd, J=8.2, 2.3 Hz), 8.28 (1H, d, J=2.0 Hz).
MS (ESI) m/z: 685 [(M+H)$^+$].

Example 33

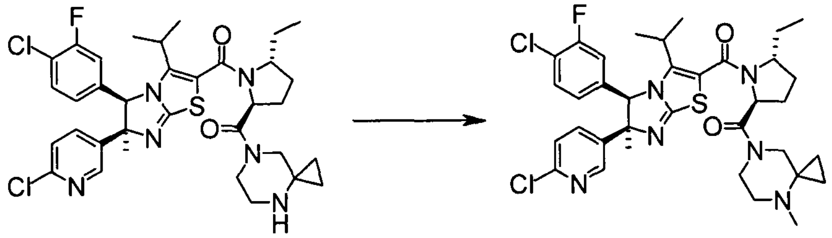

7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolyl]-4-methyl-4,7-diazaspiro[2.5]octane

The compound obtained in Step 2 of Example 32 was reacted in the same way as in Example 2 to give the title compound as a colorless solid.

$^1$H-NMR (400 MHz, DMSO-$d_6$) δ: 0.38-0.62 (4H, m), 0.84 (6H, t, J=7.4 Hz), 1.01 (3H, d, J=6.8 Hz), 1.35-1.41 (1H, m), 1.66-1.74 (3H, m), 1.75 (3H, s), 1.94-2.00 (1H, m), 2.22-2.27 (1H, m), 2.29 (3H, s), 2.72-2.79 (3H, m), 3.26-3.30 (1H, m), 3.42-3.55 (3H, m), 4.12-4.17 (1H, m), 4.89-4.94 (1H, m), 5.55 (1H, s), 6.73-6.78 (1H, m), 6.86-6.91 (1H, m), 7.19 (1H, d, J=8.3 Hz), 7.33 (1H, t, J=7.9 Hz), 7.67 (1H, dd, J=8.4, 2.3 Hz), 8.28 (1H, d, J=2.0 Hz).

MS (ESI) m/z: 699 [(M+H)$^+$].

Example 34

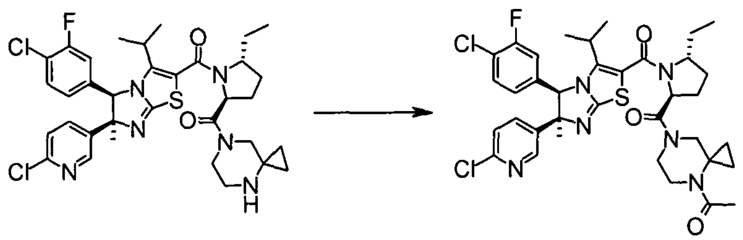

4-acetyl-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolyl]-4,7-diazaspiro[2.5]octane

The compound obtained in Step 2 of Example 32 was reacted in the same way as in Example 4 to give the title compound as a colorless solid.

$^1$H-NMR (400 MHz, DMSO-$d_6$) δ: 0.82-0.95 (10H, m), 1.01 (3H, d, J=7.1 Hz), 1.36-1.40 (1H, m), 1.63-1.71 (3H, m), 1.75 (3H, s), 1.95-2.01 (1H, m), 2.08 (3H, s), 2.21-2.27 (1H, m), 2.72-2.79 (1H, m), 3.05-3.09 (2H, m), 3.36-3.71 (4H, m), 4.13-4.17 (1H, m), 4.88-4.98 (1H, m), 5.56 (1H, s), 6.72-6.76 (1H, m), 6.85-6.90 (1H, m), 7.19 (1H, d, J=8.5 Hz), 7.31 (1H, t, J=8.1 Hz), 7.67 (1H, dd, J=8.2, 2.6 Hz), 8.28 (1H, d, J=2.2 Hz).

MS (ESI) m/z: 727 [(M+H)$^+$].

Example 35

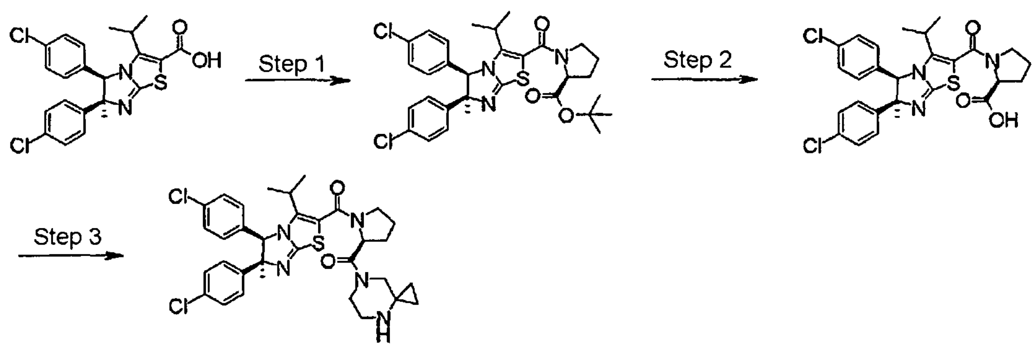


Step 1: tert-butyl-1-{[(5R,6S)-5,6-bis(4-chlorophenyl)-3-isopropyl-6-methyl-5,6-dihydroimidazo[2,1-b][1,3]thiazol-2-yl] carbonyl}-L-prolinate

The compound obtained in Step 4 of Example 1 was reacted in the same way as in Step 7 of Example 1 using tert-butyl L-prolinate hydrochloride instead of the compound obtained in Step 2 of Reference Example 1 in the reaction

in Step 5 of Example 1 to give the title compound as a colorless solid.
$^1$H-NMR (400 MHz, $CDCl_3$) δ: 0.94 (3H, d, J=7.1 Hz), 0.99 (3H, d, J=6.8 Hz), 1.46 (9H, s), 1.81 (3H, s), 1.93-2.04 (3H, m), 2.23-2.28 (1H, m), 2.58-2.63 (1H, m), 3.61-3.66 (1H, m), 3.71-3.76 (1H, m), 4.41-4.45 (1H, m), 4.96 (1H, s), 6.70 (2H, d, J=8.3 Hz), 7.02 (2H, d, J=8.3 Hz), 7.03 (2H, d, J=8.5 Hz), 7.11 (2H, d, J=8.5 Hz).
MS (ESI) m/z: 600 [$(M+H)^+$].

Step 2: 1-{[(5R,6S)-5,6-bis(4-chlorophenyl)-3-isopropyl-6-methyl-5,6-dihydroimidazo[2,1-b][1,3]thiazol-2-yl]carbonyl}-L-proline

The compound obtained in Step 1 above was reacted in the same way as in Step 6 of Example 1 to give the title compound as a colorless solid.
$^1$H-NMR (400 MHz, DMSO-$d_6$) δ: 0.88 (3H, d, J=7.1 Hz), 0.96 (3H, d, J=7.1 Hz), 1.92 (3H, s), 1.93-2.00 (3H, m), 2.23-2.32 (1H, m), 2.63-2.70 (1H, m), 3.59 (2H, t, J=6.5 Hz), 4.42-4.46 (1H, m), 5.79 (1H, s), 6.91 (2H, d, J=7.8 Hz), 7.14 (2H, d, J=8.5 Hz), 7.17 (2H, d, J=8.5 Hz), 7.22 (2H, d, J=8.8 Hz).
MS (ESI) m/z: 544 [$(M+H)^+$].

Step 3: 7-(1-{[(5R,6S)-5,6-bis(4-chlorophenyl)-3-isopropyl-6-methyl-5,6-dihydroimidazo[2,1-b][1,3]thiazol-2-yl]carbonyl}-L-prolyl)-4,7-diazaspiro[2.5]octane

The compound obtained in Step 2 above was reacted in the same way as in Step 7 of Example 1 to give the title compound as a colorless solid.
$^1$H-NMR (400 MHz, DMSO-$d_6$) δ: 0.45-0.51 (4H, m), 0.85-0.91 (6H, m), 1.62-1.67 (1H, m), 1.72 (3H, s), 1.74-1.79 (1H, m), 1.82-1.86 (1H, m), 1.89-1.94 (1H, m), 2.20-2.25 (1H, m), 2.57-2.63 (1H, m), 2.74-2.78 (1H, m), 3.30-3.33 (2H, m), 3.42-3.49 (2H, m), 3.55-3.59 (2H, m), 4.82-4.86 (1H, m), 5.38 (1H, s), 6.88 (2H, d, J=8.3 Hz), 7.06 (2H, d, J=8.8 Hz), 7.12 (2H, d, J=8.8 Hz), 7.24 (2H, d, J=8.5 Hz). MS (ESI) m/z: 638 [$(M+H)^+$].

Example 36

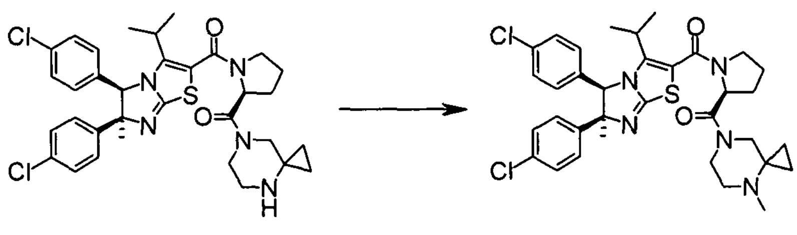

7-(1-{[(5R,6S)-5,6-bis(4-chlorophenyl)-3-isopropyl-6-methyl-5,6-dihydroimidazo[2,1-b][1,3]thiazol-2-yl]carbonyl}-L-prolyl)-4-methyl-4,7-diazaspiro[2.5]octane

The compound obtained in Step 3 of Example 35 was reacted in the same way as in Example 2 to give the title compound as a colorless solid.
$^1$H-NMR (400 MHz, DMSO-$d_6$) δ: 0.42-0.47 (2H, m), 0.57-0.63 (2H, m), 0.82-0.90 (6H, m), 1.72 (3H, s), 1.73-1.80 (1H, m), 1.81-1.86 (1H, m), 1.88-1.94 (1H, m), 2.21-2.26 (1H, m), 2.29 (3H, s), 2.55-2.61 (1H, m), 2.73-2.79 (2H, m), 3.28-3.33 (2H, m), 3.49-3.60 (4H, m), 4.79-4.85 (1H, m), 5.37 (1H, s), 6.87 (2H, d, J=7.8 Hz), 7.05 (2H, d, J=8.8 Hz), 7.10 (2H, d, J=8.8 Hz), 7.23 (2H, d, J=8.5 Hz).
MS (ESI) m/z: 652 [$(M+H)^+$].

Example 37

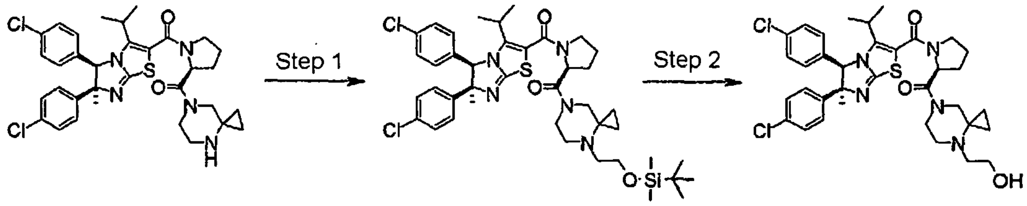


Step 1: 7-(1-{[(5R,6S)-5,6-bis(4-chlorophenyl)-3-isopropyl-6-methyl-5,6-dihydroimidazo[2,1-b][1,3]thiazol-2-yl]carbonyl}-L-prolyl)-4-(2-{[tert-butyl(dimethyl)silyl]oxy}ethyl)-4,7-diazaspiro[2.5]octane

The compound obtained in Example 35 was reacted in the same way as in Step 1 of Example 3 to give the title compound as a colorless solid.
$^1$H-NMR (400 MHz, $CDCl_3$) δ: 0.04 (9H, s), 0.86-0.93 (7H, m), 0.89 (6H, s), 0.96 (3H, d, J=6.6 Hz), 1.80 (3H, s), 1.90-1.96 (2H, m), 2.10-2.18 (2H, m), 2.60-2.66 (1H, m), 2.81-2.86 (1H, m), 2.94-3.00 (1H, m), 3.40-3.78 (10H, m), 4.79-4.84 (1H, m), 4.94 (1H, s), 6.69 (2H, d, J=7.8 Hz), 7.00-7.03 (4H, m), 7.10 (2H, d, J=8.8 Hz).

Step 2: 2-[7-(1-{[(5R,6S)-5,6-bis(4-chlorophenyl)-3-isopropyl-6-methyl-5,6-dihydroimidazo[2,1-b][1,3]thiazol-2-yl]carbonyl}-L-prolyl)-4,7-diazaspiro[2.5]oct-4-yl]ethanol

The compound obtained in Step 1 above was reacted in the same way as in Step 2 of Example 3 to give the title compound as a colorless solid.
$^1$H-NMR (400 MHz, DMSO-$d_6$) δ: 0.48-0.50 (2H, m), 0.56-0.58 (2H, m), 0.86 (3H, d, J=7.1 Hz), 0.87 (3H, d, J=6.6 Hz), 1.72 (3H, s), 1.76-1.80 (1H, m), 1.83-1.86 (1H, m), 1.90-1.94 (1H, m), 2.20-2.26 (1H, m), 2.56-2.63 (1H, m), 2.74-2.78 (1H, m), 2.85-2.90 (1H, m), 3.17-3.20 (2H, m), 3.27-3.31 (2H, m), 3.38-3.42 (2H, m), 3.50-3.52 (1H, m), 3.55-3.59 (2H, m), 4.00-4.04 (1H, m), 4.81-4.84 (1H, m), 5.37 (1H, s), 6.87 (2H, d, J=8.1 Hz), 7.05 (2H, d, J=8.5 Hz), 7.10 (2H, d, J=8.8 Hz), 7.23 (2H, d, J=8.8 Hz).
MS (ESI) m/z: 682 [$(M+H)^+$].

Example 38

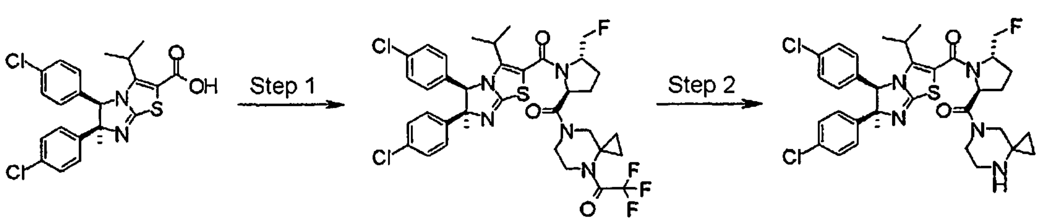


Step 1: 7-[(5S)-1-{[(5R,6S)-5,6-bis(4-chlorophenyl)-3-isopropyl-6-methyl-5,6-dihydroimidazo[2,1-b][1,3]thiazol-2-yl]carbonyl}-5-(fluoromethyl)-L-prolyl]-4-(trifluoroacetyl)-4,7-diazaspiro[2.5]octane

The compound obtained in Step 4 of Example 1 was reacted in the same way as in Step 5 of Example 1 using the compound obtained in Step 4 of Reference Example 10 instead of the compound obtained in Step 2 of Reference Example 1 to give the title compound as a colorless solid. $^1$H-NMR (400 MHz, DMSO-$d_6$) δ: 0.85 (3H, d, J=6.8 Hz), 0.90-1.06 (4H, m), 0.93 (3H, d, J=6.8 Hz), 1.71 (3H, s), 1.78-1.93 (2H, m), 2.01-2.10 (1H, m), 2.24-2.32 (1H, m), 2.62-2.69 (1H, m), 3.42-3.80 (6H, m), 4.37-4.52 (3H, m), 4.97-5.03 (1H, m), 5.44 (1H, s), 6.88 (2H, d, J=6.6 Hz), 7.07 (2H, d, J=8.5 Hz), 7.10 (2H, d, J=8.8 Hz), 7.25 (2H, d, J=8.5 Hz).
MS (ESI) m/z: 766 [$(M+H)^+$].

Step 2: 7-[(5S)-1-{[(5R,6S)-5,6-bis(4-chlorophenyl)-3-isopropyl-6-methyl-5,6-dihydroimidazo[2,1-b][1,3]thiazol-2-yl]carbonyl}-5-(fluoromethyl)-L-prolyl]-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$) δ: 0.42-0.53 (4H, m), 0.82 (3H, d, J=6.1 Hz), 0.95 (3H, d, J=6.8 Hz), 1.71 (3H, s), 1.76-1.80 (1H, m), 1.87-1.92 (1H, m), 1.99-2.03 (1H, m), 2.30-2.33 (1H, m), 2.64-2.79 (3H, m), 3.21-3.54 (4H, m), 4.37-4.54 (3H, m), 4.94-5.00 (1H, m), 5.43 (1H, s), 6.88 (2H, d, J=7.8 Hz), 7.07 (2H, d, J=8.3 Hz), 7.13 (2H, d, J=8.5 Hz), 7.25 (2H, d, J=8.3 Hz).
MS (ESI) m/z: 670 [(M+H)$^+$].

Example 39

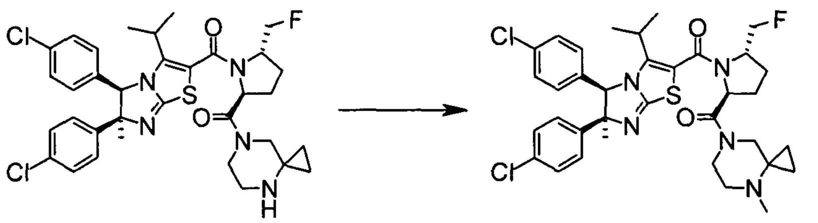

7-[(5S)-1-{[(5R,6S)-5,6-bis(4-chlorophenyl)-3-isopropyl-6-methyl-5,6-dihydroimidazo[2,1-b][1,3]thiazol-2-yl]carbonyl}-5-(fluoromethyl)-L-prolyl]-4-methyl-4,7-diazaspiro[2.5]octane

The compound obtained in Step 2 of Example 38 was reacted in the same way as in Example 2 to give the title compound as a colorless solid.
$^1$H-NMR (400 MHz, DMSO-$d_6$) δ: 0.38-0.62 (4H, m), 0.83 (3H, d, J=6.1 Hz), 0.94 (3H, d, J=5.6 Hz), 1.71 (3H, s), 1.77-1.82 (1H, m), 1.87-1.93 (2H, m), 2.00-2.05 (1H, m), 2.30 (3H, s), 2.64-2.70 (1H, m), 2.74-2.80 (2H, m), 3.22-3.56 (4H, m), 4.36-4.55 (3H, m), 4.93-4.99 (1H, m), 5.44 (1H, s), 6.88 (2H, d, J=7.6 Hz), 7.07 (2H, d, J=8.5 Hz), 7.11 (2H, d, J=8.5 Hz), 7.25 (2H, d, J=8.3 Hz).
MS (ESI) m/z: 684 [(M+H)$^+$].

Example 40

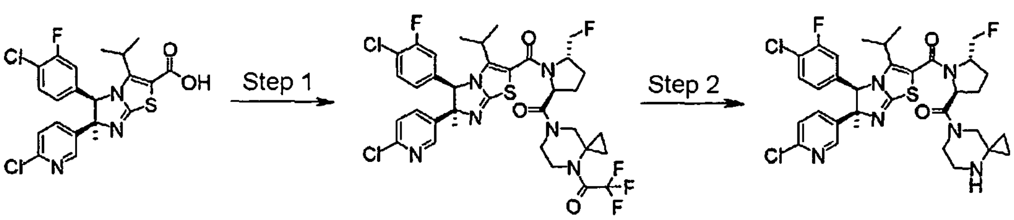


Step 1: 7-[(5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-(fluoromethyl)-L-prolyl]-4-(trifluoroacetyl)-4,7-diazaspiro[2.5]octane

(5R,6S)-5-(4-chloro-3-fluorophenyl)-6-(6-chloropyridin-3-yl)-3-isopropyl-6-methyl-5,6-dihydroimidazo[2,1-b][1,3]thiazole-2-carboxylic acid was reacted in the same way as in Step 5 of Example 1 using the compound obtained in Step 4 of Reference Example 10 instead of the compound obtained in Step 2 of Reference Example 1 to give the title compound as a colorless solid. $^1$H-NMR (400 MHz, DMSO-$d_6$) δ: 0.86 (3H, d, J=5.9 Hz), 0.94-1.08 (4H, m), 0.99 (3H, d, J=7.1 Hz), 1.75 (3H, s), 1.78-1.84 (1H, m), 1.87-1.96 (1H, m), 2.02-2.11 (1H, m), 2.27-2.34 (1H, m), 2.68-2.75 (1H, m), 3.46-3.53 (2H, m), 3.59-3.83 (4H, m), 4.21 (1H, dd, J=47.7, 6.0 Hz), 4.36-4.54 (2H, m), 4.97-5.04 (1H, m), 5.55 (1H,

s), 6.71-6.76 (1H, m), 6.86-6.91 (1H, m), 7.19 (1H, d, J=8.3 Hz), 7.32 (1H, t, J=7.9 Hz), 7.66 (1H, dd, J=8.2, 2.3 Hz), 8.27 (1H, d, J=2.2 Hz).
MS (ESI) m/z: 785 [$(M+H)^+$].

Step 2: 7-[(5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-(fluoromethyl)-L-prolyl]-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$) δ: 0.40-0.58 (4H, m), 0.84 (3H, d, J=7.1 Hz), 0.99 (3H, d, J=6.8 Hz), 1.75 (3H, s), 1.76-1.82 (1H, m), 1.87-1.93 (1H, m), 1.98-2.04 (1H, m), 2.30-2.34 (1H, m), 2.70-2.80 (3H, m), 3.25-3.54 (4H, m), 4.37-4.55 (3H, m), 4.93-5.00 (1H, m), 5.54 (1H, s), 6.68-6.74 (1H, m), 6.91-6.98 (1H, m), 7.19 (1H, d, J=8.3 Hz), 7.35 (1H, t, J=7.8 Hz), 7.66 (1H, dd, J=8.3, 2.4 Hz), 8.27 (1H, d, J=2.4 Hz).
MS (ESI) m/z: 689 [$(M+H)^+$].

Example 41

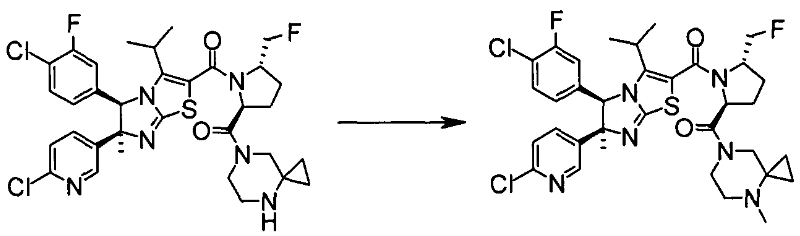

7-[(5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-(fluoromethyl)-L-prolyl]-4-methyl-4,7-diazaspiro[2.5]octane

The compound obtained in Step 2 of Example 40 was reacted in the same way as in Example 2 to give the title compound as a colorless solid.
$^1$H-NMR (400 MHz, DMSO-$d_6$) δ: 0.38-0.61 (4H, m), 0.83-0.87 (3H, m), 0.99 (3H, d, J=6.1 Hz), 1.75 (3H, s), 1.79-1.82 (1H, m), 1.89-1.93 (2H, m), 2.01-2.04 (1H, m), 2.30 (3H, s), 2.67-2.72 (1H, m), 2.74-2.80 (2H, m), 3.23-3.59 (4H, m), 4.38-4.56 (3H, m), 4.94-5.00 (1H, m), 5.54 (1H, s), 6.72-6.76 (1H, m), 6.89-6.95 (1H, m), 7.19 (1H, d, J=8.3 Hz), 7.33 (1H, t, J=7.8 Hz), 7.66 (1H, dd, J=8.2, 2.1 Hz), 8.27 (1H, d, J=2.2 Hz).
MS (ESI) m/z: 703 [$(M+H)^+$].

Example 42

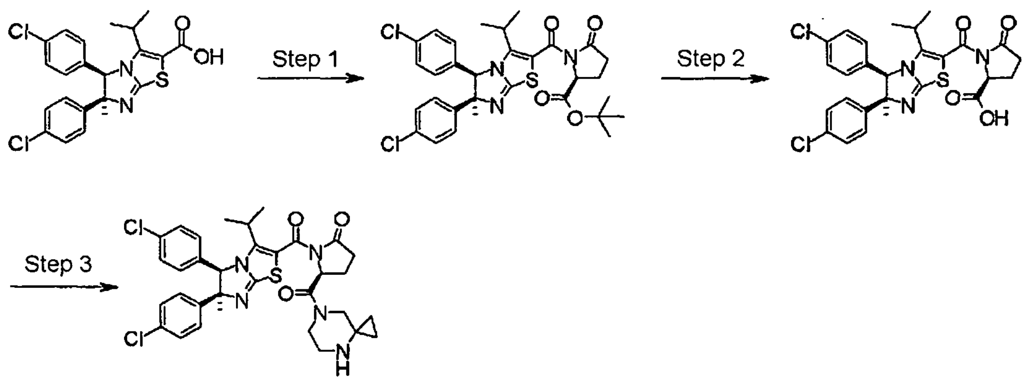


Step 1: tert-butyl 1-{[(5R,6S)-5,6-bis(4-chlorophenyl)-3-isopropyl-6-methyl-5,6-dihydroimidazo[2,1-b][1,3]thiazol-2-yl] carbonyl}-5-oxo-L-prolinate

The compound obtained in Step 4 of Example 1 was reacted in the same way as in Step 5 of Example 1 using tert-butyl 5-oxo-L-prolinate instead of the compound obtained in Step 2 of Reference Example 1 to give the title compound as a colorless solid.
$^1$H-NMR (400 MHz, $CDCl_3$) δ: 0.94 (3H, d, J=6.8 Hz), 0.97 (3H, d, J=7.3 Hz), 1.48 (9H, s), 1.82 (3H, s), 2.02-2.11 (1H, m), 2.34-2.43 (1H, m), 2.49-2.57 (1H, m), 2.63-2.72 (1H, m), 2.82-2.89 (1H, m), 4.65 (1H, dd, J=8.7, 5.2 Hz), 4.99 (1H, s), 6.73 (2H, d, J=8.8 Hz), 7.01 (2H, d, J=8.5 Hz), 7.04 (2H, d, J=8.3 Hz), 7.09 (2H, d, J=8.5 Hz).

Step 2: 1-{[(5R,6S)-5,6-bis(4-chlorophenyl)-3-isopropyl-6-methyl-5,6-dihydroimidazo[2,1-b][1,3]thiazol-2-yl]carbonyl}-5-oxo-L-proline

The compound obtained in Step 1 above was reacted in the same way as in Step 6 of Example 1 to give the title compound as a colorless solid.
$^1$H-NMR (400 MHz, DMSO-$d_6$) δ: 0.87 (3H, d, J=7.1 Hz), 0.92 (3H, d, J=7.1 Hz), 1.91 (3H, s), 1.99-2.05 (1H, m), 2.38-2.44 (1H, m), 2.59-2.64 (2H, m), 2.81-2.88 (1H, m), 4.71 (1H, dd, J=9.0, 4.4 Hz), 5.93 (1H, s), 6.92-6.95 (2H, m), 7.15-7.19 (4H, m), 7.24 (2H, d, J=8.8 Hz).

Step 3: (5S)-1-{[(5R,6S)-5,6-bis(4-chlorophenyl)-3-isopropyl-6-methyl-5,6-dihydroimidazo[2,1-b][1,3]thiazol-2-yl]carbonyl}-5-(4,7-diazaspiro[2.5]oct-7-ylcarbonyl)pyrrolidin-2-one

The compound obtained in Step 2 above was reacted in the same way as in Step 7 of Example 1 to give the title compound as a colorless solid.
$^1$H-NMR (400 MHz, DMSO-$d_6$) δ: 0.43-0.48 (4H, m), 0.84 (3H, d, J=7.1 Hz), 0.90 (3H, d, J=6.8 Hz), 1.73 (3H, s), 1.77-1.82 (1H, m), 2.28-2.41 (1H, m), 2.46-2.56 (2H, m), 2.70-2.77 (1H, m), 2.82-2.91 (2H, m), 3.30-3.53 (4H, m), 5.12-5.22 (1H, m), 5.54 (1H, s), 6.91 (2H, d, J=7.1 Hz), 7.08 (2H, d, J=8.5 Hz), 7.11 (2H, d, J=8.8 Hz), 7.25 (2H, d, J=8.5 Hz).
MS (ESI) m/z: 652 [(M+H)+].

Example 43

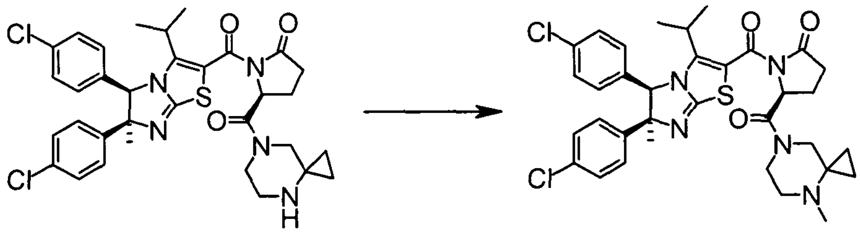

(5S)-1-{[(5R,6S)-5,6-bis(4-chlorophenyl)-3-isopropyl-6-methyl-5,6-dihydroimidazo[2,1-b][1,3]thiazol-2-yl]carbonyl}-5-[(4-methyl-4,7-diazaspiro[2.5]oct-7-yl)carbonyl]pyrrolidin-2-one

The compound obtained in Step 3 of Example 42 was reacted in the same way as in Example 2 to give the title compound as a colorless solid.
$^1$H-NMR (400 MHz, DMSO-$d_6$) δ: 0.40-0.59 (4H, m), 0.84 (3H, d, J=6.6 Hz), 0.90 (3H, d, J=7.1 Hz), 1.73 (3H, s), 1.78-1.84 (1H, m), 2.30 (3H, s), 2.33-2.39 (1H, m), 2.51-2.56 (2H, m), 2.72-2.78 (1H, m), 2.83-2.90 (2H, m), 3.30-3.34 (2H, m), 3.38-3.44 (1H, m), 3.54-3.58 (1H, m), 5.13-5.23 (1H, m), 5.54 (1H, s), 6.92 (2H, d, J=8.3 Hz), 7.08 (2H, d, J=8.5 Hz), 7.11 (2H, d, J=8.8 Hz), 7.25 (2H, d, J=8.5 Hz).
MS (ESI) m/z: 666 [(M+H)$^+$].

Example 44

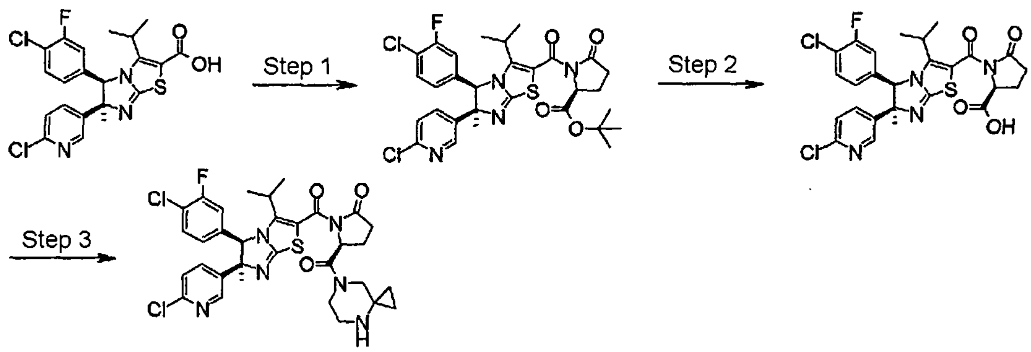


Step 1: tert-butyl 1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-oxo-L-prolinate

(5R, 6S)-5-(4-chloro-3-fluorophenyl)-6-(6-chloropyridin-3-yl)-3-isopropyl-6-methyl-5,6-dihydroimidazo[2,1-b][1,3]thiazole-2-carboxylic acid was reacted in the same way as in Step 5 of Example 1 using tert-butyl 5-oxo-L-prolinate instead of the compound obtained in Step 2 of Reference Example 1 to give the title compound as a colorless solid.
$^1$H-NMR (400 MHz, $CDCl_3$) δ: 0.98 (6H, t, J=6.6 Hz), 1.49 (9H, s), 1.86 (3H, s), 2.07-2.12 (1H, m), 2.39-2.45 (1H, m), 2.57-2.62 (1H, m), 2.67-2.71 (1H, m), 2.82-2.87 (1H, m), 4.66 (1H, dd, J=8.8, 5.4 Hz), 5.04 (1H, s), 6.58 (1H, d, J=8.0 Hz), 6.63 (1H, d, J=9.0 Hz), 7.03 (1H, d, J=8.3 Hz), 7.15-7.19 (1H, m), 7.50-7.53 (1H, m), 8.21 (1H, d, J=2.4 Hz).
MS (ESI) m/z: 633 [(M+H)$^+$].

Step 2: 1-{[(5R,6S)-5-(4-chloro-3-fluorophenyl)-6-(6-chloropyridin-3-yl)-3-isopropyl-6-methyl-5,6-dihydroimidazo[2,1-b][1,3] thiazol-2-yl]carbonyl}-5-oxo-L-proline

The compound obtained in Step 1 above was reacted in the same way as in Step 6 of Example 1 to give the title compound as a colorless solid.
$^1$H-NMR (400 MHz, DMSO-$d_6$) δ: 0.89 (3H, d, J=7.1 Hz), 0.94 (3H, d, J=7.1 Hz), 1.85 (3H, s), 1.98-2.04 (1H, m), 2.38-2.45 (1H, m), 2.58-2.62 (2H, m), 2.85-2.92 (1H, m), 4.70 (1H, dd, J=9.0, 4.9 Hz), 5.80 (1H, s), 6.77-6.82 (1H, m), 6.94-7.01 (1H, m), 7.23 (1H, d, J=8.3 Hz), 7.37 (1H, t, J=8.1 Hz), 7.67 (1H, dd, J=8.4, 2.6 Hz), 8.27 (1H, d, J=2.0 Hz).
MS (ESI) m/z: 577 [(M+H)$^+$].

Step 3: (5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-(4,7-diazaspiro[2.5]oct-7-ylcarbonyl)pyrrolidin-2-one

The compound obtained in Step 2 above was reacted in the same way as in Step 7 of Example 1 to give the title compound as a colorless solid.
$^1$H-NMR (400 MHz, DMSO-$d_6$) δ: 0.43-0.52 (4H, m), 0.87 (3H, d, J=7.1 Hz), 0.94 (3H, d, J=7.1 Hz), 1.77 (3H, s), 1.79-1.83 (1H, m), 2.35-2.41 (1H, m), 2.52-2.58 (2H, m), 2.69-2.77 (2H, m), 2.83-2.90 (1H, m), 3.29-3.36 (2H, m), 3.46-3.53 (2H,

m), 5.17-5.23 (1H, m), 5.63 (1H, s), 6.76-6.80 (1H, m), 6.92-6.96 (1H, m), 7.20 (1H, d, J=8.5 Hz), 7.33 (1H, t, J=8.1 Hz), 7.66 (1H, dd, J=8.3, 2.7 Hz), 8.27 (1H, d, J=2.4 Hz).
MS (ESI) m/z: 671 [$(M+H)^+$].

Example 45

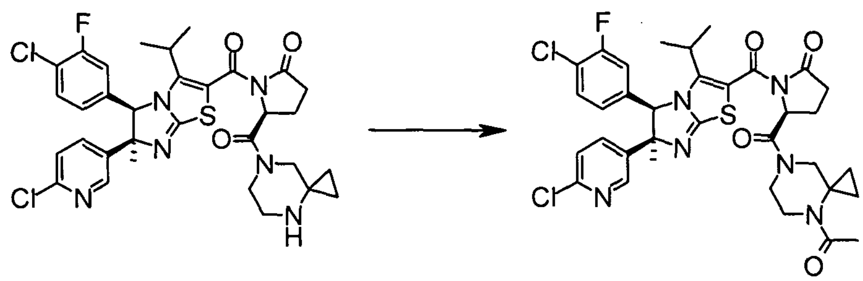

(5S)-5-[(4-acetyl-4,7-diazaspiro[2.5]oct-7-yl)carbonyl]-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}pyrrolidin-2-one

The compound obtained in Step 3 of Example 44 was reacted in the same way as in Example 4 to give the title compound as a colorless solid.
$^1$H-NMR (400 MHz, DMSO-$d_6$) δ: 0.87 (3H, d, J=7.1 Hz), 0.92-1.00 (4H, m), 0.94 (3H, d, J=7.1 Hz), 1.77 (3H, s), 1.81-1.85 (1H, m), 2.09 (3H, s), 2.32-2.37 (1H, m), 2.52-2.57 (2H, m), 2.85-2.89 (1H, m), 3.04-3.08 (2H, m), 3.36-3.44 (2H, m), 3.59-3.75 (2H, m), 5.18-5.25 (1H, m), 5.64 (1H, s), 6.76-6.80 (1H, m), 6.92-6.97 (1H, m), 7.20 (1H, d, J=8.5 Hz), 7.34 (1H, t, J=8.1 Hz), 7.66 (1H, dd, J=8.4, 2.6 Hz), 8.27 (1H, d, J=2.4 Hz).
MS (ESI) m/z: 713 [$(M+H)^+$].

Example 46

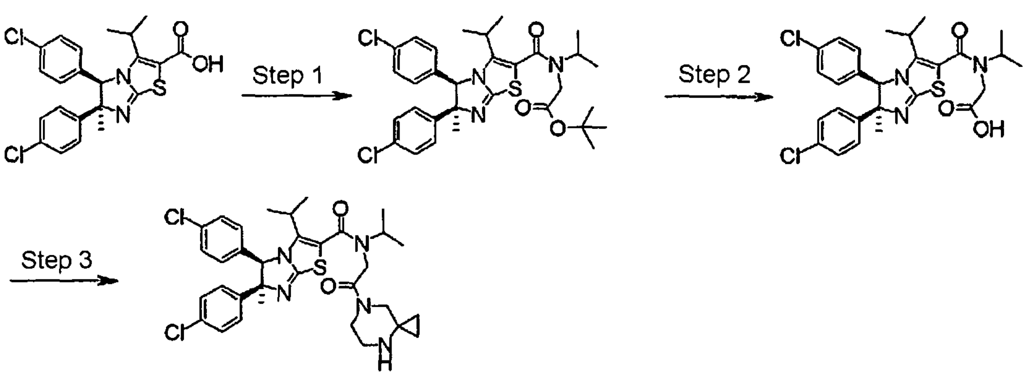


Step 1: tert-butyl N-{[(5R,6S)-5,6-bis(4-chlorophenyl)-3-isopropyl-6-methyl-5,6-dihydroimidazo[2,1-b][1,3]thiazol-2-yl] carbonyl}-N-isopropylglycinate

The compound obtained in Step 4 of Example 1 was reacted in the same way as in Step 5 of Example 1 using tert-butyl N-isopropylglycinate instead of the compound obtained in Step 2 of Reference Example 1 to give the title compound as a colorless solid.

$^1$H-NMR (400 MHz, $CDCl_3$) δ: 0.92 (3H, d, J=7.1 Hz), 1.01 (3H, d, J=7.1 Hz), 1.18 (3H, d, J=6.6 Hz), 1.23 (3H, d, J=6.6 Hz), 1.48 (9H, s), 1.82 (3H, s), 2.40-2.49 (1H, m), 3.88 (2H, s), 4.40-4.49 (1H, m), 4.95 (1H, s), 6.71 (2H, d, J=7.8 Hz), 7.01-7.06 (4H, m), 7.08-7.12 (2H, m).

Step 2: N-{[(5R,6S)-5,6-bis(4-chlorophenyl)-3-isopropyl-6-methyl-5,6-dihydroimidazo[2,1-b][1,3]thiazol-2-yl]carbonyl}-N-isopropylglycine

The compound obtained in Step 1 above was reacted in the same way as in Step 6 of Example 1 to give the title compound as a colorless solid.
$^1$H-NMR (400 MHz, $CDCl_3$) δ: 0.82-0.88 (3H, m), 1.10 (3H, d, J=6.8 Hz), 1.15-1.23 (6H, m), 2.09 (3H, s), 2.47-2.57 (1H, m), 4.00-4.18 (2H, m), 4.61-4.73 (1H, m), 5.50 (1H, s), 6.65-6.89 (2H, m), 7.02-7.16 (6H, m).

Step 3: (5R,6S)-5,6-bis(4-chlorophenyl)-N-[2-(4,7-diazaspiro[2.5]oct-7-yl)-2-oxoethyl]-N,3-diisopropyl-6-methyl-5,6-dihydroimidazo[2,1-b][1,3]thiazole-2-carboxamide

The compound obtained in Step 2 above was reacted in the same way as in Step 7 of Example 1 to give the title compound as a colorless solid.
$^1$H-NMR (400 MHz, $CDCl_3$) δ: 0.54-0.73 (4H, m), 0.93-1.02 (6H, m), 1.16-1.27 (6H, m), 1.82 (3H, s), 2.46-2.57 (1H, m), 2.89-4.14 (8H, m), 4.43-4.51 (1H, m), 4.96 (1H, s), 6.69-6.74 (2H, m), 7.01-7.05 (4H, m), 7.09-7.13 (2H, m). MS (ESI) m/z: 640 [$(M+H)^+$].

Example 47

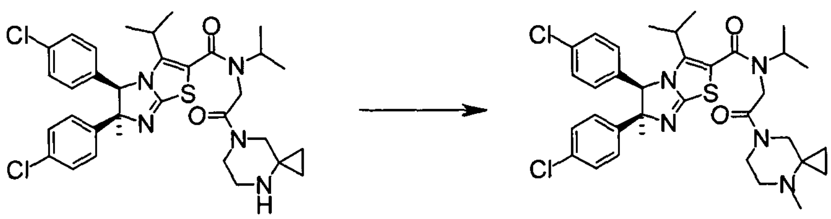

(5R,6S)-5,6-bis(4-chlorophenyl)-N,3-diisopropyl-6-methyl-N-[2-(4-methyl-4,7-diazaspiro[2.5]oct-7-yl)-2-oxoethyl]-5,6-dihydroimidazo[2,1-b][1,3]thiazole-2-carboxamide
The compound obtained in Step 3 of Example 46 was reacted in the same way as in Example 2 to give the title compound as a colorless solid.
$^1$H-NMR (400 MHz, $CDCl_3$) δ: 0.49-0.82 (4H, m), 0.93-1.02 (6H, m), 1.19 (3H, t, J=6.8 Hz), 1.24 (3H, t, J=6.8 Hz), 1.82 (3H, s), 2.41 (3H, s), 2.45-2.58 (1H, m), 2.86-4.07 (8H, m), 4.42-4.51 (1H, m), 4.95 (1H, s), 6.69-6.73 (2H, m), 7.01-7.05 (4H, m), 7.08-7.12 (2H, m).
MS (ESI) m/z: 654 [$(M+H)^+$].

Example 48

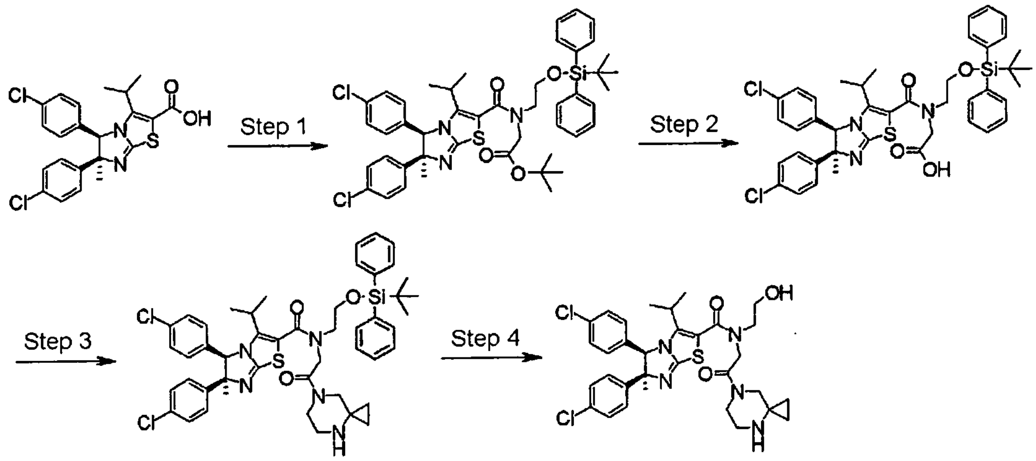


Step 1: tert-butyl N-{[(5R,6S)-5,6-bis(4-chlorophenyl)-3-isopropyl-6-methyl-5,6-dihydroimidazo[2,1-b][1,3]thiazol-2-yl] carbonyl}-N-(2-{[tert-butyl(diphenyl)silyl]oxy}ethyl)glycinate

The compound obtained in Step 4 of Example 1 was reacted in the same way as in Step 5 of Example 1 using tert-butyl N-(2-{[tert-butyl(diphenyl)silyl]oxy}ethyl)glycinate instead of the compound obtained in Step 2 of Reference Example 1 to give the title compound as a colorless solid.
$^1$H-NMR (400 MHz, $CDCl_3$) δ: 0.90 (3H, d, J=6.8 Hz), 0.96 (3H, d, J=7.1 Hz), 1.06 (9H, s), 1.46 (9H, s), 1.80 (3H, s), 2.41-2.51 (1H, m), 3.60-3.67 (2H, m), 3.82-3.87 (2H, m), 4.15-4.40 (2H, m), 4.93 (1H, s), 6.65-6.73 (2H, m), 7.02 (4H, d, J=8.8 Hz), 7.09 (2H, d, J=8.8 Hz), 7.36-7.47 (6H, m), 7.63 (4H, d, J=6.6 Hz).

Step 2: N-{[(5R,6S)-5,6-bis(4-chlorophenyl)-3-isopropyl-6-methyl-5,6-dihydroimidazo[2,1-b][1,3]thiazol-2-yl]carbonyl}-N-(2-{[tert-butyl(diphenyl)silyl]oxy}ethyl)glycine

The compound obtained in Step 1 above was reacted in the same way as in Step 6 of Example 1 to give the title compound as a colorless solid.
$^1$H-NMR (400 MHz, $CDCl_3$) δ: 0.85 (3H, d, J=7.1 Hz), 1.01 (9H, s), 1.06 (3H, d, J=6.8 Hz), 2.09 (3H, s), 2.43-2.53 (1H, m), 3.42-3.53 (1H, m), 3.78-3.92 (3H, m), 4.30 (1H, d, J=18.0 Hz), 4.52 (1H, d, J=18.0 Hz), 5.42 (1H, s), 6.59-6.88 (2H, m), 7.03 (2H, d, J=8.8 Hz), 7.08-7.16 (4H, m), 7.31-7.43 (6H, m), 7.58-7.63 (4H, m).

Step 3: (5R,6S)-N-(2-{[tert-butyl(diphenyl)silyl]oxy}ethyl)-5,6-bis(4-chlorophenyl)-N-[2-(4,7-diazaspiro[2.5]oct-7-yl)-2-oxoethyl]-3-isopropyl-6-methyl-5,6-dihydroimidazo[2,1-b][1,3]thiazole-2-carboxamide

The compound obtained in Step 2 above was reacted in the same way as in Step 7 of Example 1 to give the title compound as a colorless solid.
$^1$H-NMR (400 MHz, $CDCl_3$) δ: 0.44-0.67 (4H, m), 0.90-1.00 (7H, m), 1.05-1.09 (9H, m), 1.80 (3H, s), 2.46-2.61 (1H, m), 2.87-2.93 (2H, m), 3.11-3.14 (1H, m), 3.26-3.32 (1H, m), 3.38-3.44 (1H, m), 3.49-3.60 (1H, m), 3.64-3.78 (2H, m), 3.86-3.93 (2H, m), 4.21-4.51 (2H, m), 4.94 (1H, s), 6.64-6.72 (2H, m), 6.98-7.03 (4H, m), 7.09 (2H, d, J=8.5 Hz), 7.37-7.48 (6H, m), 7.61-7.67 (4H, m).

Step 4: (5R,6S)-5,6-bis(4-chlorophenyl)-N-[2-(4,7-diazaspiro[2.5]oct-7-yl)-2-oxoethyl]-N-(2-hydroxyethyl)-3-isopropyl-6-methyl-5,6-dihydroimidazo[2,1-b][1,3]thiazole-2-carboxamide

The compound obtained in Step 3 above was reacted in the same way as in Step 2 of Example 3 to give the title compound as a colorless solid.
$^1$H-NMR (400 MHz, $CDCl_3$) δ: 0.53-0.60 (4H, m), 0.92-0.98 (6H, m), 1.80 (3H, s), 2.48-2.59 (1H, m), 2.89-3.02 (2H, m), 3.27-3.76 (8H, m), 4.09-4.24 (2H, m), 4.94 (1H, s), 6.69 (2H, d, J=8.3 Hz), 6.97-7.03 (4H, m), 7.09 (2H, d, J=8.3 Hz).
MS (ESI) m/z: 642 [$(M+H)^+$].

Example 49

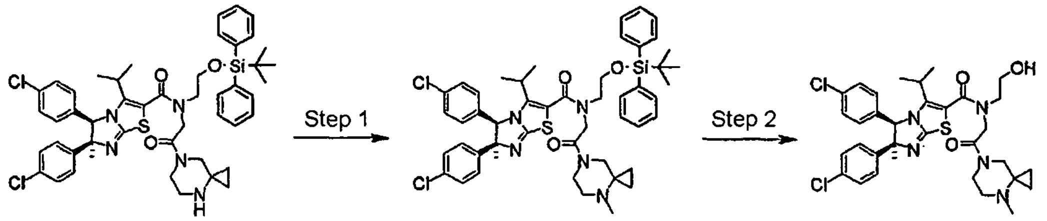


Step 1: (5R,6S)-N-(2-{[tert-butyl(diphenyl)silyl]oxy}ethyl)-5,6-bis(4-chlorophenyl)-3-isopropyl-6-methyl-N-[2-(4-methyl-4,7-diazaspiro[2.5]oct-7-yl)-2-oxoethyl]-5,6-dihydroimidazo[2,1-b][1,3]thiazole-2-carboxamide

The compound obtained in Step 3 of Example 48 was reacted in the same way as in Example 2 to give the title compound as a colorless solid.
$^1$H-NMR (400 MHz, $CDCl_3$) δ: 0.38-0.51 (2H, m), 0.63-0.74 (2H, m), 0.92 (3H, d, J=7.1 Hz), 0.95 (3H, d, J=7.1 Hz), 1.08 (9H, s), 1.79 (3H, s), 2.35 (3H, s), 2.51-2.62 (1H, m), 2.81-2.85 (2H, m), 3.06-3.63 (4H, m), 3.65-3.78 (2H, m), 3.90 (2H, t, J=5.5 Hz), 4.26-4.45 (2H, m), 4.92 (1H, s), 6.67 (2H, d, J=8.5 Hz), 6.97-7.02 (4H, m), 7.09 (2H, d, J=8.5 Hz), 7.33-7.45 (6H, m), 7.61-7.67 (4H, m).

Step 2: (5R,6S)-5,6-bis(4-chlorophenyl)-N-(2-hydroxyethyl)-3-isopropyl-6-methyl-N-[2-(4-methyl-4,7-diazaspiro[2.5] oct-7-yl)-2-oxoethyl]-5,6-dihydroimidazo[2,1-b][1,3]thiazole-2-carboxamide

The compound obtained in Step 1 above was reacted in the same way as in Step 2 of Example 3 to give the title compound as a colorless solid.
$^1$H-NMR (400 MHz, $CDCl_3$) δ: 0.49-0.53 (2H, m), 0.64-0.82 (2H, m), 0.97 (6H, d, J=7.1 Hz), 1.81 (3H, s), 2.38 (3H, s), 2.49-2.60 (1H, m), 2.86-2.97 (2H, m), 3.26-3.76 (8H, m), 4.09-4.41 (3H, m), 4.94 (1H, s), 6.70 (2H, d, J=8.3 Hz), 6.99-7.04 (4H, m), 7.09 (2H, d, J=8.3 Hz).
MS (ESI) m/z: 656 [(M+H)$^+$].

Example 50

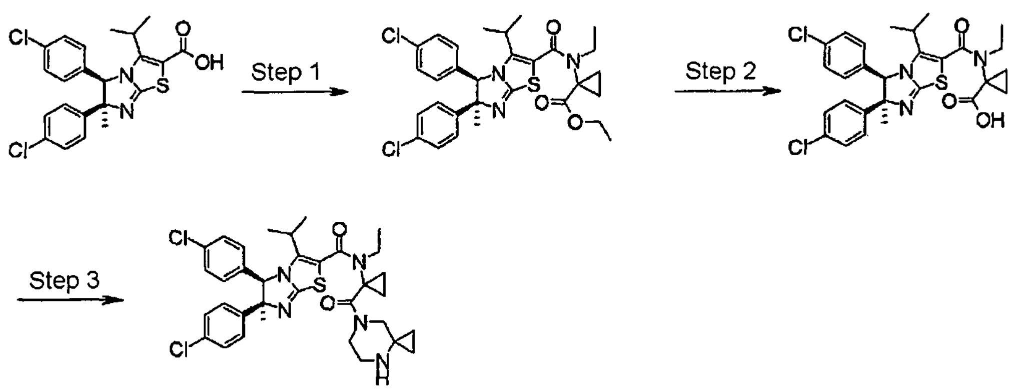

Step 1: ethyl 1-[{[(5R,6S)-5,6-bis(4-chlorophenyl)-3-isopropyl-6-methyl-5,6-dihydroimidazo[2,1-b][1,3]thiazol-2-yl]carbonyl}(ethyl)amino]cyclopropanecarboxylate

The compound obtained in Step 4 of Example 1 was reacted in the same way as in Step 5 of Example 1 using the compound obtained in Step 2 of Reference Example 11 instead of the compound obtained in Step 2 of Reference Example 1 to give the title compound as a colorless solid.
$^1$H-NMR (400 MHz, DMSO-$d_6$) δ: 0.85 (3H, d, J=7.1 Hz), 0.91 (3H, d, J=7.1 Hz), 1.16 (3H, t, J=7.1 Hz), 1.22 (3H, t, J=7.1 Hz), 1.24-1.35 (2H, m), 1.50-1.66 (2H, m), 1.71 (3H, s), 2.50-2.58 (1H, m), 3.45 (2H, q, J=7.1 Hz), 4.13 (2H, q, J=7.1 Hz), 5.45 (1H, s), 6.86-6.92 (2H, m), 7.07 (2H, d, J=8.5 Hz), 7.14 (2H, d, J=8.8 Hz), 7.26 (2H, d, J=8.5 Hz).

Step 2: 1-[{[(5R,6S)-5,6-bis(4-chlorophenyl)-3-isopropyl-6-methyl-5,6-dihydroimidazo[2,1-b][1,3]thiazol-2-yl]carbonyl}(ethyl)amino]cyclopropanecarboxylic acid

The compound obtained in Step 1 above was reacted in the same way as in Step 4 of Example 1 to give the title compound as a colorless solid.
$^1$H-NMR (400 MHz, $CDCl_3$) δ: 0.94 (3H, d, J=6.8 Hz), 1.09-1.15 (3H, m), 1.19-1.32 (2H, m), 1.24 (6H, t, J=7.1 Hz), 1.75-1.83 (2H, m), 2.19 (3H, s), 2.66-2.75 (1H, m), 3.49-3.60 (2H, m), 5.67-5.78 (1H, m), 6.73-6.83 (2H, m), 7.07-7.18 (6H, m).

Step 3: (5R,6S)-5,6-bis(4-chlorophenyl)-N-[1-(4,7-diazaspiro[2.5]oct-7-ylcarbonyl)cyclopropyl]-N-ethyl-3-isopropyl-6-methyl-5,6-dihydroimidazo[2,1-b][1,3] thiazole-2-carboxamide

The compound obtained in Step 2 above was reacted in the same way as in Step 7 of Example 1 to give the title compound as a colorless solid.
$^1$H-NMR (400 MHz, $CDCl_3$) δ: 0.51-0.61 (4H, m), 0.93 (3H, d, J=7.1 Hz), 0.96 (3H, d, J=7.1 Hz), 1.23-1.36 (2H, m), 1.29 (3H, t, J=7.1 Hz), 1.39-1.50 (2H, m), 1.80 (3H, s), 2.43-2.54 (1H, m), 2.90 (2H, t, J=5.1 Hz), 3.43-3.54 (2H, m), 3.62-3.83 (4H, m), 4.92 (1H, s), 6.68 (2H, d, J=8.3 Hz), 6.98-7.03 (4H, m), 7.09 (2H, d, J=8.3 Hz).
MS (ESI) m/z: 652 [$(M+H)^+$].

Example 51

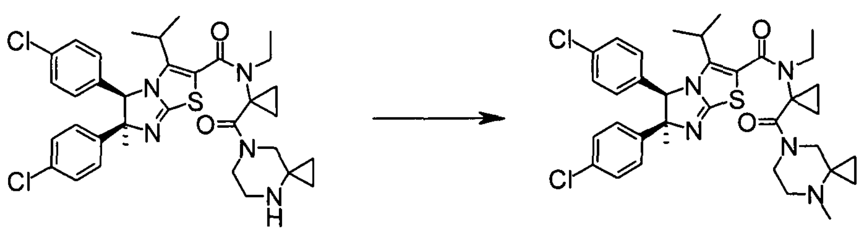

(5R,6S)-5,6-bis(4-chlorophenyl)-N-ethyl-3-isopropyl-6-methyl-N-{1-[(4-methyl-4,7-diazaspiro[2.5]oct-7-yl)carbonyl]cyclopropyl}-5,6-dihydroimidazo[2,1-b][1,3]thiazole-2-carboxamide

The compound obtained in Step 3 of Example 50 was reacted in the same way as in Example 2 to give the title compound as a colorless solid.
$^1$H-NMR (400 MHz, $CDCl_3$) δ: 0.46-0.56 (2H, m), 0.63-0.72 (2H, m), 0.93 (3H, d, J=7.1 Hz), 0.96 (3H, d, J=7.1 Hz), 1.22-1.27 (2H, m), 1.28 (3H, t, J=7.1 Hz), 1.41-1.50 (2H, m), 1.80 (3H, s), 2.35 (3H, s), 2.45-2.54 (1H, m), 2.83 (2H, t, J=5.2 Hz), 3.42-3.53 (2H, m), 3.62-3.91 (4H, m), 4.92 (1H, s), 6.69 (2H, d, J=8.3 Hz), 6.99-7.12 (6H, m).
MS (ESI) m/z: 666 [$(M+H)^+$].

Example 52

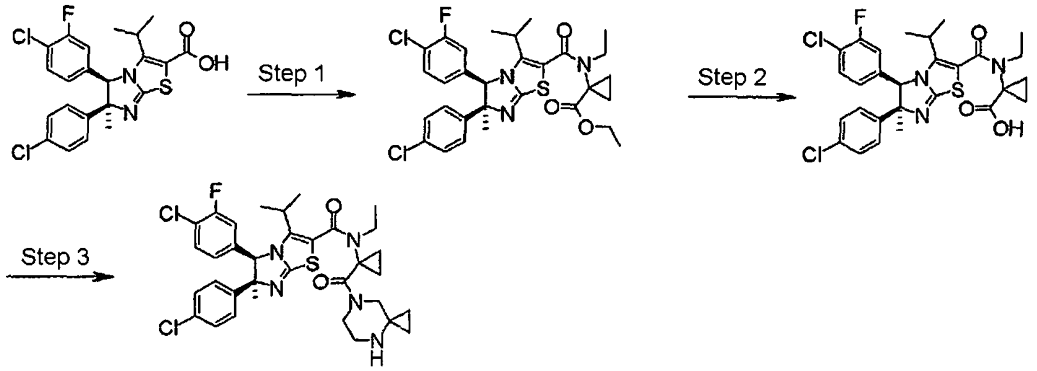


Step 1: ethyl 1-[{[(5R,6S)-5-(4-chloro-3-fluorophenyl)-6-(4-chlorophenyl)-3-isopropyl-6-methyl-5,6-dihydroimidazo [2,1-b][1,3] thiazol-2-yl]carbonyl}(ethyl)amino]cyclopropanecarboxylate

The compound obtained in Step 7 of Example 5 was reacted in the same way as in Step 5 of Example 1 using the compound obtained in Step 2 of Reference Example 11 instead of the compound obtained in Step 2 of Reference Example 1 to give the title compound as a colorless solid. $^1$H-NMR (400 MHz, $CDCl_3$) δ: 0.97 (3H, d, J=7.1 Hz), 1.03 (3H, d, J=6.8 Hz), 1.24-1.32 (2H, m), 1.26 (3H, t, J=7.1 Hz), 1.29 (3H, t, J=7.1 Hz), 1.63-1.77 (2H, m), 1.80 (3H, s), 2.55-2.63 (1H, m), 3.53 (2H, q, J=6.8 Hz), 4.21 (2H, q, J=7.1 Hz), 4.91 (1H, s), 6.51 (1H, dd, J=8.3, 2.0 Hz), 6.56 (1H, dd, J=9.8, 2.0 Hz), 7.02-7.15 (5H, m).

Step 2: 1-[{[(5R,6S)-5-(4-chloro-3-fluorophenyl)-6-(4-chlorophenyl)-3-isopropyl-6-methyl-5,6-dihydroimidazo[2,1-b] [1,3]thiazol-2-yl]carbonyl}(ethyl)amino]cyclopropanecarboxylic acid

The compound obtained in Step 1 above was reacted in the same way as in Step 4 of Example 1 to give the title compound as a colorless solid.
$^1$H-NMR (400 MHz, $CDCl_3$) δ: 0.97 (3H, d, J=6.8 Hz), 1.04-1.13 (3H, m), 1.17-1.24 (2H, m), 1.22 (3H, t, J=7.1 Hz), 1.63-1.83 (2H, m), 2.03 (3H, s), 2.60-2.72 (1H, m), 3.39-3.62 (2H, m), 5.32 (1H, s), 6.53-6.64 (2H, m), 7.08-7.25 (5H, m).

Step 3: (5R,6S)-5-(4-chloro-3-fluorophenyl)-6-(4-chlorophenyl)-N-[1-(4,7-diazaspiro[2.5]oct-7-ylcarbonyl)cyclopropyl]-N-ethyl-3-isopropyl-6-methyl-5,6-dihydroimidazo[2,1-b][1,3]thiazole-2-carboxamide

The compound obtained in Step 2 above was reacted in the same way as in Step 7 of Example 1 to give the title compound as a colorless solid.
$^1$H-NMR (400 MHz, $CDCl_3$) δ: 0.52-0.62 (4H, m), 0.96 (3H, d, J=7.3 Hz), 0.98 (3H, d, J=7.1 Hz), 1.22-1.37 (2H, m), 1.29 (3H, t, J=7.1 Hz), 1.41-1.48 (2H, m), 1.80 (3H, s), 2.44-2.52 (1H, m), 2.90 (2H, t, J=5.0 Hz), 3.43-3.54 (2H, m), 3.64-3.83 (4H, m), 4.89 (1H, s), 6.47-6.56 (2H, m), 7.02-7.13 (5H, m).
MS (ESI) m/z: 670 [$(M+H)^+$].

Example 53

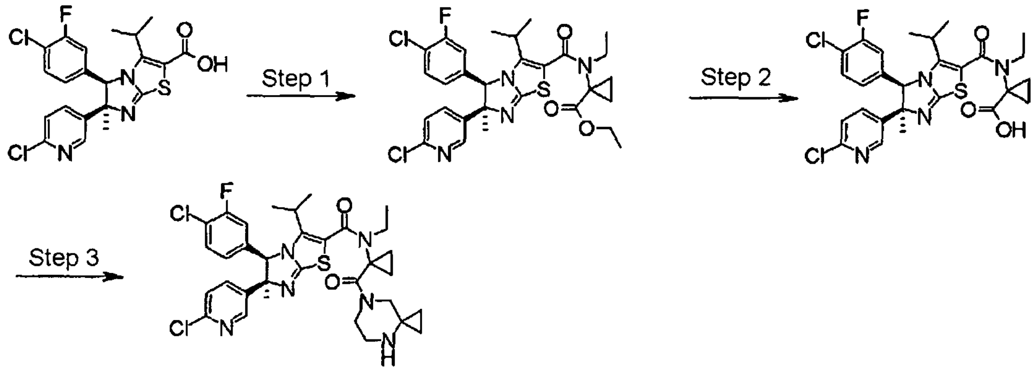


Step 1: ethyl 1-[{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}(ethyl)amino]cyclopropanecarboxylate

(5R,6S)-5-(4-chloro-3-fluorophenyl)-6-(6-chloropyridin-3-yl)-3-isopropyl-6-methyl-5,6-dihydroimidazo[2,1-b][1,3]thiazole-2-carboxylic acid was reacted in the same way as in Step 5 of Example 1 using the compound obtained in Step 2 of Reference Example 11 instead of the compound obtained in Step 2 of Reference Example 1 to give the title compound as a colorless solid. $^1$H-NMR (400 MHz, $CDCl_3$) δ: 0.98 (3H, d, J=6.8 Hz), 1.03 (3H, d, J=7.1 Hz), 1.22-1.32 (8H, m), 1.65-1.78 (2H, m), 1.82 (3H, s), 2.55-2.66 (1H, m), 3.53 (2H, q, J=6.8 Hz), 4.21 (2H, q, J=7.0 Hz), 4.98 (1H, s), 6.52-6.61 (2H, m), 7.01 (1H, d, J=8.3 Hz), 7.14 (1H, t, J=7.7 Hz), 7.52 (1H, dd, J=8.3, 2.2 Hz), 8.21 (1H, d, J=2.2 Hz).

Step 2: 1-[{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}(ethyl)amino]cyclopropanecarboxylic acid

The compound obtained in Step 1 above was reacted in the same way as in Step 4 of Example 1 to give the title compound as a colorless solid.
$^1$H-NMR (400 MHz, $CDCl_3$) δ: 0.98 (3H, d, J=7.1 Hz), 1.00-1.27 (8H, m), 1.62-1.86 (2H, m), 2.00 (3H, s), 2.59-2.73 (1H, m), 3.38-3.64 (2H, m), 5.23 (1H, s), 6.55-6.67 (2H, m), 7.05-7.22 (2H, m), 7.67 (1H, d, J=8.3 Hz), 8.27 (1H, d, J=2.0 Hz).

Step 3: (5R,6S)-5-(4-chloro-3-fluorophenyl)-6-(6-chloropyridin-3-yl)-N-[1-(4,7-diazaspiro[2.5]oct-7-ylcarbonyl)cyclopropyl]-N-ethyl-3-isopropyl-6-methyl-5,6-dihydroimidazo[2,1-b][1,3]thiazole-2-carboxamide

The compound obtained in Step 2 above was reacted in the same way as in Step 7 of Example 1 to give the title compound as a colorless solid.
$^1$H-NMR (400 MHz, $CDCl_3$) δ: 0.53-0.60 (4H, m), 0.97 (3H, d, J=7.1 Hz), 0.99 (3H, d, J=7.1 Hz), 1.23-1.37 (2H, m), 1.29 (3H, t, J=7.2 Hz), 1.42-1.51 (2H, m), 1.83 (3H, s), 2.45-2.54 (1H, m), 2.90 (2H, t, J=5.1 Hz), 3.46-3.53 (2H, m), 3.62-3.83 (4H, m), 4.95 (1H, s), 6.50-6.58 (2H, m), 7.00 (1H, d, J=8.3 Hz), 7.13 (1H, t, J=7.7 Hz), 7.50 (1H, dd, J=8.3, 2.4 Hz), 8.20 (1H, d, J=2.4 Hz).
MS (ESI) m/z: 671 [$(M+H)^+$].

Example 54

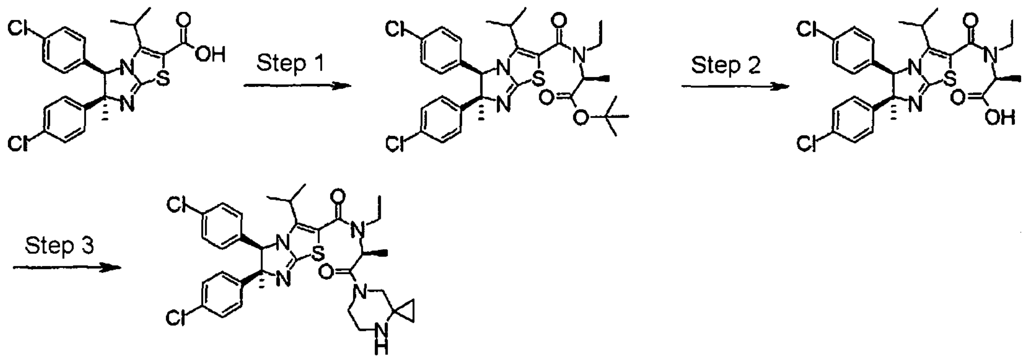


Step 1: tert-butyl N-{[(5R,6S)-5,6-bis(4-chlorophenyl)-3-isopropyl-6-methyl-5,6-dihydroimidazo[2,1-b][1,3]thiazol-2-yl]carbonyl}-N-ethyl-L-alaninate

The compound obtained in Step 4 of Example 1 was reacted in the same way as in Step 5 of Example 1 using the compound obtained in Step 2 of Reference Example 12 instead of the compound obtained in Step 2 of Reference Example 1 to give the title compound as a colorless solid. $^1$H-NMR (400 MHz, $CDCl_3$) δ: 0.94 (3H, d, J=7.1 Hz), 1.00 (3H, d, J=7.1 Hz), 1.23 (3H, t, J=7.1 Hz), 1.39-1.51 (3H, m), 1.44 (9H, s), 1.81 (3H, s), 2.43-2.52 (1H, m), 3.21-3.32 (1H, m), 3.60-3.68 (1H, m), 4.46-4.54 (1H, m), 4.92 (1H, s), 6.70 (2H, d, J=8.6 Hz), 6.99-7.11 (6H, m).

Step 2: N-{[(5R,6S)-5,6-bis(4-chlorophenyl)-3-isopropyl-6-methyl-5,6-dihydroimidazo[2,1-b][1,3]thiazol-2-yl]carbonyl}-N-ethyl-L-alanine

The compound obtained in Step 1 above was reacted in the same way as in Step 6 of Example 1 to give the title compound as a colorless solid.
$^1$H-NMR (400 MHz, DMSO-$d_6$) δ: 0.82 (3H, d, J=7.1 Hz), 0.96 (3H, d, J=7.1 Hz), 1.17 (3H, t, J=7.1 Hz), 1.43 (3H, d, J=7.1 Hz), 1.90 (3H, s), 2.50-2.57 (1H, m), 3.26-3.58 (2H, m), 4.43-4.51 (1H, m), 5.74 (1H, s), 6.90-6.98 (2H, m), 7.09-7.18 (4H, m), 7.21-7.25 (2H, m), 8.24 (1H, s).

Step 3: (5R,6S)-5,6-bis(4-chlorophenyl)-N-[(1S)-2-(4,7-diazaspiro[2.5]oct-7-yl)-1-methyl-2-oxoethyl]-N-ethyl-3-isopropyl-6-methyl-5,6-dihydroimidazo[2,1-b][1,3]thiazole-2-carboxamide

The compound obtained in Step 2 above was reacted in the same way as in Step 7 of Example 1 to give the title compound as a colorless solid.
$^1$H-NMR (400 MHz, $CDCl_3$) δ: 0.53-0.63 (4H, m), 0.86-1.05 (6H, m), 1.23 (3H, t, J=7.1 Hz), 1.34-1.39 (3H, m), 1.81 (3H, s), 2.43-2.58 (1H, m), 2.88-2.95 (2H, m), 3.26-3.66 (6H, m), 4.91-4.95 (1H, m), 5.33-5.43 (1H, m), 6.66-6.72 (2H, m), 6.98-7.12 (6H, m).
MS (ESI) m/z: 640 [$(M+H)^+$].

Example 55

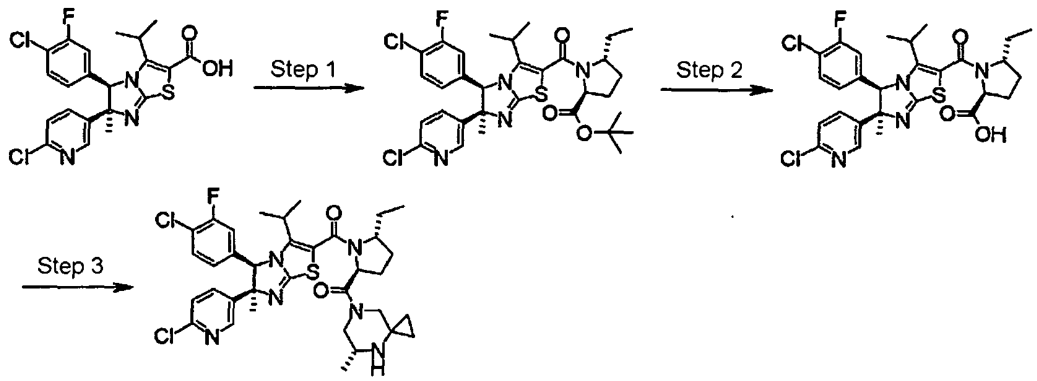


Step 1: tert-butyl (5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolinate

The compound obtained in Step 13 of Reference Example 13 was reacted in the same way as in Step 5 of Example 1 using the compound obtained in Reference Example 30 instead of the compound obtained in Step 2 of Reference Example 1 to give the title compound as a pale yellow solid.
$^1$H-NMR (400 MHz, $CDCl_3$) δ: 0.92 (3H, t, J=7.4 Hz), 0.98 (3H, d, J=7.1 Hz), 1.08 (3H, d, J=7.1 Hz), 1.35-1.42 (1H, m), 1.45 (9H, s), 1.70-1.81 (2H, m), 1.82 (3H, s), 1.94-2.01 (1H, m), 2.07-2.23 (2H, m), 2.72-2.78 (1H, m), 4.27-4.31 (1H, m), 4.53-4.57 (1H, m), 4.98 (1H, s), 6.50-6.56 (2H, m), 7.02 (1H, d, J=8.3 Hz), 7.12 (1H, t, J=7.9 Hz), 7.53 (1H, dd, J=8.3, 2.7 Hz), 8.23 (1H, d, J=2.4 Hz).
MS (ESI) m/z: 647 [$(M+H)^+$].

Step 2: (5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-proline

The compound obtained in Step 1 above was reacted in the same way as in Step 6 of Example 1 to give the title compound as a colorless solid.
$^1$H-NMR (400 MHz, DMSO-$d_6$) δ: 0.83-0.90 (6H, m), 1.03 (3H, d, J=7.1 Hz), 1.38-1.45 (1H, m), 1.61-1.69 (1H, m), 1.75-1.81 (1H, m), 1.88 (3H, s), 1.92-2.06 (2H, m), 2.26-2.34 (1H, m), 2.74-2.81 (1H, m), 4.09-4.16 (1H, m), 4.51-4.56 (1H, m), 5.78 (1H, s), 6.71-6.76 (1H, m), 6.92-6.97 (1H, m), 7.25 (1H, d, J=8.5 Hz), 7.35 (1H, t, J=7.9 Hz), 7.68 (1H, dd, J=8.4, 2.6 Hz), 8.29 (1H, d, J=2.4 Hz).
MS (ESI) m/z: 591 [$(M+H)^+$].

Step 3: (5R)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolyl]-5-methyl-4,7-diazaspiro[2.5]octane

The compound obtained in Step 2 above was reacted in the same way as in Step 7 of Example 1 using the compound obtained in Step 7 of Reference Example 3 instead of 4,7-diazaspiro[2.5]octane hydrochloride to give the title compound as a colorless solid.
$^1$H-NMR (400 MHz, DMSO-$d_6$, 70˚C) δ: 0.47-0.54 (2H, m), 0.62-0.69 (2H, m), 0.84 (6H, t, J=7.4 Hz), 1.03 (6H, d, J=7.1 Hz), 1.37-1.41 (1H, m), 1.64-1.73 (3H, m), 1.74 (3H, s), 1.96-2.03 (1H, m), 2.24-2.29 (1H, m), 2.74-2.85 (2H, m), 3.33-3.46 (2H, m), 3.56-3.65 (1H, m), 3.82-3.91 (1H, m), 4.10-4.18 (1H, m), 4.88-4.95 (1H, m), 5.56 (1H, s), 6.70-6.78 (1H, m), 6.87-6.95 (1H, m), 7.20 (1H, d, J=8.3 Hz), 7.32-7.40 (1H, m), 7.68 (1H, dd, J=8.3, 2.4 Hz), 8.28 (1H, d, J=2.2 Hz).
MS (ESI) m/z: 699 [$(M+H)^+$].

Example 56

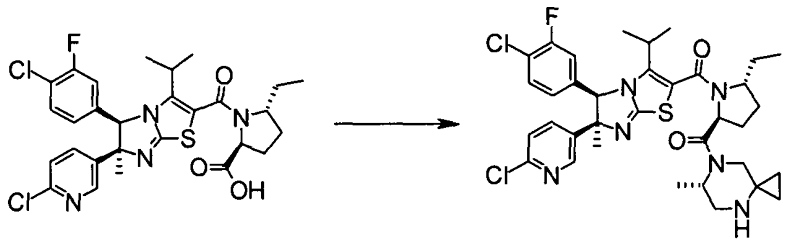

(6S)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-ethyl-L-prolyl]-6-methyl-4,7-diazaspiro[2.5]octane

The compound obtained in Step 2 of Example 55 was reacted in the same way as in Step 7 of Example 1 using the compound obtained in Step 4 of Reference Example 5 instead of 4,7-diazaspiro[2.5]octane hydrochloride to give the title compound as a colorless solid.
$^1$H-NMR (400 MHz, DMSO-$d_6$, 70°C) δ: 0.40-0.46 (2H, m), 0.55-0.65 (2H, m), 0.80-0.89 (6H, m), 1.02 (3H, d, J=7.1 Hz), 1.09-1.17 (3H, m), 1.33-1.41 (1H, m), 1.64-1.73 (3H, m), 1.74 (3H, s), 1.99-2.07 (1H, m), 2.22-2.28 (1H, m), 2.66-2.76 (2H, m), 2.85-2.90 (1H, m), 3.47-3.63 (2H, m), 4.11-4.19 (1H, m), 4.31-4.38 (1H, m), 4.72-4.78 (1H, m), 5.55 (1H, s), 6.71-6.79 (1H, m), 6.87-6.93 (1H, m), 7.19 (1H, d, J=8.3 Hz), 7.33 (1H, t, J=8.1 Hz), 7.68 (1H, dd, J=8.3, 2.4 Hz), 8.28 (1H, d, J=2.4 Hz).
MS (ESI) m/z: 699 [(M+H)$^+$].

Example 57

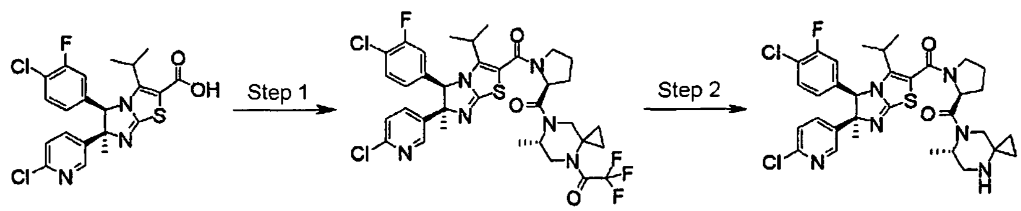


Step 1: (6S)-7-(1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-L-prolyl)-6-methyl-4-(trifluoroacetyl)-4,7-diazaspiro[2.5]octane

1-hydroxybenzotriazole (58.0 mg, 0.43 mmol) was added to a dimethylformamide (3 ml) solution of the compound (200 mg, 0.43 mmol) obtained in Step 13 of Reference Example 13, then the compound (151 mg, 0.48 mmol) obtained in Step 2 of Reference Example 15 and 1-(3-dimethylaminopropyl)-3-ethylcarbodiimide hydrochloride (100 mg, 0.52 mmol) were added and the resulting mixture was stirred at room temperature for 18 hours. The reaction mixture was added to water, followed by extraction with ethyl acetate. The organic layer was washed with brine and then dried over anhydrous sodium sulfate. The solvent was evaporated under reduced pressure and the residue was purified by silica gel column chromatography [chloroform:methanol = 20:1 (v/v)] to give the title compound (270 mg, 82%) as a colorless solid.
$^1$H-NMR (400 MHz, DMSO-$d_6$, 100°C) δ: 0.72-0.74 (2H, m), 0.91 (3H, d, J=7.1 Hz), 0.94 (3H, d, J=7.1 Hz), 1.11 (3H, brd, J=6.1 Hz), 1.29 (1H, m), 1.49 (1H, m), 1.77 (3H, s), 1.80-1.89 (2H, m), 2.05 (1H, m), 2.23 (1H, m), 2.63 (1H, m), 3.58-3.63 (5H, m), 3.92 (1H, brs), 4.68 (1H, m), 4.81 (1H, brs), 5.49 (1H, s), 6.75 (1H, brd, J=7.6 Hz), 6.93 (1H, brd, J=7.6 Hz), 7.16 (1H, dd, J=8.3, 0.6 Hz), 7.33 (1H, t, J=7.9 Hz), 7.65 (1H, dd, J=8.3, 2.4 Hz), 8.26 (1H, dd, J=2.4, 0.7 Hz).
MS (ESI) m/z: 767 [(M+H)$^+$].

Step 2: (6S)-7-(1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-L-prolyl)-6-methyl-4,7-diazaspiro[2.5]octane

The compound obtained in Step 1 above was reacted in the same way as in Step 4 of Example 27 to give the

title compound as a colorless solid.
$^1$H-NMR (400 MHz, DMSO-$d_6$, 100˚C) δ: 0.38-0.39 (2H, m), 0.55-0.57 (2H, m), 0.92 (3H, d, J=7.1 Hz), 0.94 (3H, d, J=7.1 Hz), 1.22 (3H, brd, J=6.1 Hz), 1.28 (1H, m), 1.77 (3H, s), 1.82-1.87 (2H, m), 1.98 (1H, m), 2.22 (1H, m), 2.63 (1H, m), 2.67 (1H, m), 3.23 (1H, brs), 3.42 (1H, brs), 3.56-3.59 (2H, m), 4.30 (1H, m), 4.78 (1H, m), 5.49 (1H, s), 6.74 (1H, d, J=8.3 Hz), 6.93 (1H, d, J=8.3 Hz), 7.16 (1H, dd, J=8.3, 0.7 Hz), 7.33 (1H, t, J=8.3 Hz), 7.65 (1H, dd, J=8.3, 2.6 Hz), 8.26 (1H, dd, J=2.6, 0.7 Hz).
MS (ESI) m/z: 671 [$(M+H)^+$].

Example 58

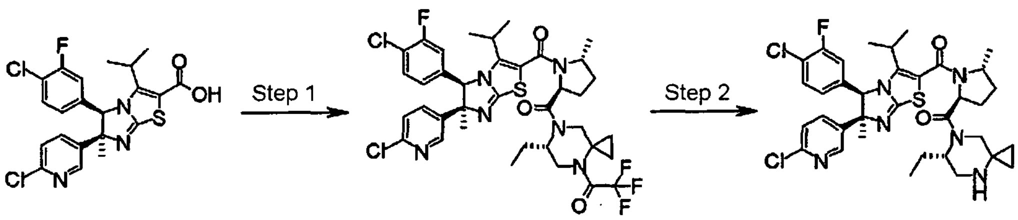


Step 1: (6S)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-ethyl-4-(trifluoroacetyl)-4,7-diazaspiro[2.5]octane

The compound obtained in Step 13 of Reference Example 13 was reacted in the same way as in Step 5 of Example 1 using the compound obtained in Step 2 of Reference Example 17 instead of the compound obtained in Step 2 of Reference Example 1 to give the title compound as a colorless solid.
$^1$H-NMR (400 MHz, DMSO-$d_6$) δ: 0.72-0.77 (2H, m), 0.80-0.87 (3H, m), 0.90 (3H, d, J=7.1 Hz), 1.01 (3H, d, J=7.1 Hz), 1.18 (3H, d, J=6.3 Hz), 1.23-1.30 (1H, m), 1.43-1.53 (2H, m), 1.60-1.66 (1H, m), 1.71-1.76 (2H, m), 1.77 (3H, s), 2.22-2.31 (2H, m), 2.66-2.74 (1H, m), 3.41-4.04 (4H, m), 4.31-4.46 (2H, m), 4.83-5.02 (1H, m), 5.54 (1H, s), 6.74-6.80 (1H, m), 6.88-6.95 (1H, m), 7.18 (1H, d, J=8.3 Hz), 7.32 (1H, t, J=8.1 Hz), 7.68 (1H, dd, J=8.3, 2.4 Hz), 8.28 (1H, d, J=2.4 Hz).
MS (ESI) m/z: 795 [$(M+H)^+$].

Step 2: (6S)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-ethyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 90˚C) δ: 0.43-0.50 (2H, m), 0.56-0.63 (2H, m), 0.77-0.85 (3H, m), 0.88 (3H, d, J=7.1 Hz), 1.01 (3H, d, J=7.1 Hz), 1.16 (3H, d, J=6.3 Hz), 1.58-1.64 (2H, m), 1.71-1.75 (2H, m), 1.76 (3H, s), 2.18-2.32 (2H, m), 2.66-2.75 (1H, m), 2.81-2.87 (2H, m), 3.51-3.60 (2H, m), 4.09-4.20 (1H, m), 4.29-4.38 (1H, m), 4.80-4.88 (1H, m), 5.52 (1H, s), 6.73-6.80 (1H, m), 6.88-6.95 (1H, m), 7.18 (1H, d, J=8.5 Hz), 7.33 (1H, t, J=8.1 Hz), 7.67 (1H, dd, J=8.4, 2.6 Hz), 8.28 (1H, d, J=2.0 Hz).
MS (ESI) m/z: 699 [$(M+H)^+$].

Example 59

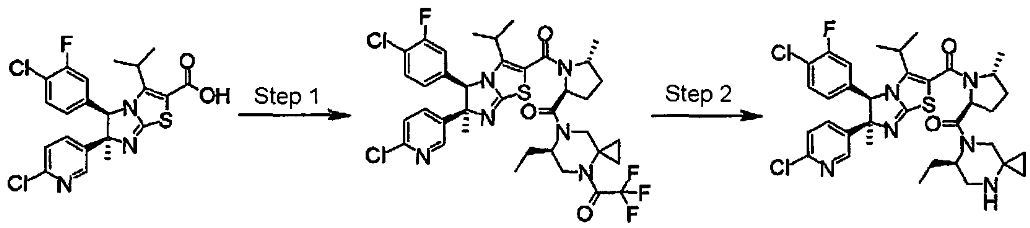


Step 1: (6R)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-ethyl-4-(trifluoroacetyl)-4,7-diazaspiro[2.5]octane

The compound obtained in Step 13 of Reference Example 13 was reacted in the same way as in Step 5 of Example 1 using the compound obtained in Step 2 of Reference Example 19 instead of the compound obtained in Step 2 of Reference Example 1 to give the title compound as a colorless solid.
$^1$H-NMR (400 MHz, DMSO-$d_6$) δ: 0.60-0.83 (2H, m), 0.85-0.92 (3H, m), 0.97-1.03 (3H, m), 0.98 (3H, d, J=7.1 Hz), 1.13-1.22 (3H, m), 1.29-1.39 (1H, m), 1.43-1.80 (5H, m), 1.76 (3H, s), 2.00-2.10 (1H, m), 2.19-2.29 (1H, m), 2.64-2.74 (1H, m), 3.19-3.43 (2H, m), 3.63-3.73 (1H, m), 4.06-4.20 (1H, m), 4.30-4.38 (1H, m), 4.55-4.64 (1H, m), 4.91-5.01 (1H, m), 5.51 (1H, s), 6.72-6.78 (1H, m), 6.85-6.92 (1H, m), 7.17 (1H, d, J=8.3 Hz), 7.32 (1H, t, J=7.9 Hz), 7.62-7.69 (1H, m), 8.23-8.28 (1H, m).
MS (ESI) m/z: 795 [(M+H)$^+$].

Step 2: (6R)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-ethyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 90°C) δ. 0.26-0.44 (2H, m), 0.48-0.62 (2H, m), 0.76-0.82 (3H, m), 0.88 (3H, d, J=7.1 Hz), 0.98 (3H, d, J=6.8 Hz), 1.13-1.17 (3H, m), 1.23-1.31 (1H, m), 1.51-1.67 (3H, m), 1.75 (3H, s), 1.97-2.08 (1H, m), 2.22-2.30 (1H, m), 2.65-2.88 (3H, m), 3.46-3.64 (2H, m), 4.20-4.34 (2H, m), 4.85-4.92 (1H, m), 5.49 (1H, s), 6.69-6.76 (1H, m), 6.86-6.95 (1H, m), 7.17 (1H, d, J=8.3 Hz), 7.26-7.35 (1H, m), 7.65 (1H, dd, J=8.4, 2.6 Hz), 8.25 (1H, d, J=2.2 Hz).
MS (ESI) m/z: 699 [(M+H)$^+$].

Example 60

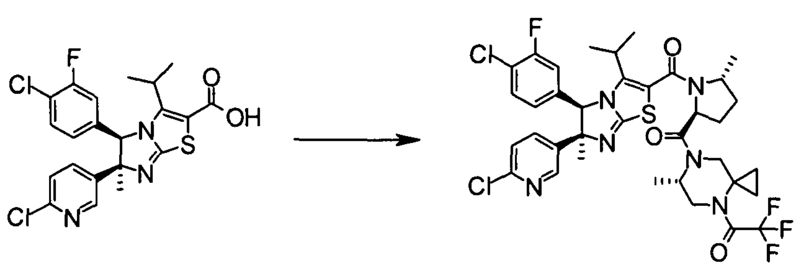

(6S)-7-[(5R)-1-{[(5R, 6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-methyl-4-(trifluoroacetyl)-4,7-diazaspiro[2.5]octane

The compound obtained in Step 13 of Reference Example 13 was reacted in the same way as in Step 5 of Example 1 using the compound obtained in Step 2 of Reference Example 21 instead of the compound obtained in Step 2 of Reference Example 1 to give the title compound as a colorless solid.

$^1$H-NMR (400 MHz, DMSO-$d_6$, 100°C) δ: 0.74 (2H, s), 0.91 (3H, d, J=7.1 Hz), 1.01 (3H, d, J=7.1 Hz), 1.09 (2H, s), 1.18 (3H, d, J=6.3 Hz), 1.28 (1H, q, J=7.6 Hz), 1.49 (1H, t, J=8.1 Hz), 1.60-1.64 (1H, m), 1.72-1.77 (4H, m), 2.24 (2H, ddt, J=33.0, 15.7, 5.7 Hz), 2.67-2.74 (1H, m), 2.96 (2H, m), 3.56 (2H, s), 3.91 (1H, s), 4.34 (1H, dd, J=10.1, 3.8 Hz), 4.63 (1H, s), 4.86 (1H, s), 5.52 (1H, s), 6.77 (1H, d, J=7.1 Hz), 6.91 (1H, d, J=10.0 Hz), 7.17 (1H, d, J=8.3 Hz), 7.31 (1H, t, J=8.1 Hz), 7.67 (1H, dd, J=8.3, 2.4 Hz), 8.27 (1H, d, J=2.4 Hz).
MS (ESI) m/z: 781 [$(M+H)^+$].

Example 61

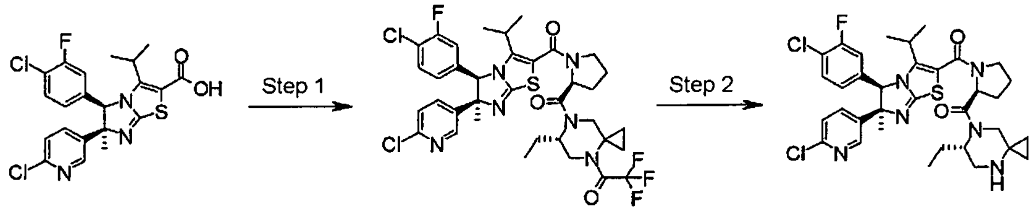


Step 1: (6S)-7-(1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-L-prolyl)-6-ethyl-4-(trifluoroacetyl)-4,7-diazaspiro[2.5]octane

The compound obtained in Step 13 of Reference Example 13 was reacted in the same way as in Step 1 of Example 57 using the compound obtained in Step 2 of Reference Example 22 instead of the compound obtained in Step 2 of Reference Example 15 to give the title compound as a colorless solid.
$^1$H-NMR (400 MHz, DMSO-$d_6$) δ: 0.70-0.74 (2H, m), 0.81-0.87 (3H, m), 0.91 (3H, d, J=7.1 Hz), 0.93 (3H, d, J=7.3 Hz), 1.24-1.31 (1H, m), 1.44-1.55 (3H, m), 1.77 (3H, s), 1.79-1.91 (2H, m), 2.03-2.11 (1H, m), 2.15-2.24 (1H, m), 2.57-2.66 (1H, m), 3.29-3.71 (7H, m), 4.44-4.50 (1H, m), 5.51 (1H, s), 6.72-6.77 (1H, m), 6.90-6.98 (1H, m), 7.17 (1H, d, J=8.3 Hz), 7.34 (1H, t, J=8.1 Hz), 7.65 (1H, dd, J=8.3, 2.4 Hz), 8.26 (1H, d, J=2.4 Hz).
MS (ESI) m/z: 781 [$(M+H)^+$].

Step 2: (6S)-7-(1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-L-prolyl)-6-ethyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 90°C) δ: 0.35-0.40 (2H, m), 0.51-0.57 (2H, m), 0.80-0.86 (3H, m), 0.92 (6H, d, J=6.8 Hz), 1.65-1.72 (1H, m), 1.77 (3H, s), 1.78-1.87 (2H, m), 1.98-2.06 (1H, m), 2.16-2.23 (1H, m), 2.30-2.37 (1H, m), 2.59-2.66 (1H, m), 2.72-2.88 (2H, m), 3.39-3.73 (4H, m), 4.10-4.20 (1H, m), 4.71-4.78 (1H, m), 5.50 (1H, s), 6.71-6.77 (1H, m), 6.89-6.98 (1H, m), 7.17 (1H, d, J=8.5 Hz), 7.34 (1H, t, J=8.1 Hz), 7.66 (1H, dd, J=8.3, 2.4 Hz), 8.26 (1H, d, J=2.4 Hz).
MS (ESI) m/z: 685 [$(M+H)^+$].

Example 62

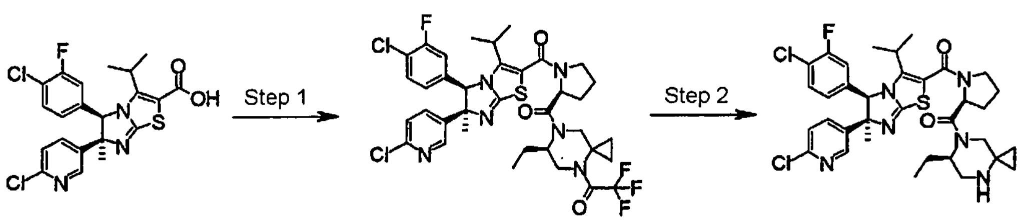

Step 1: (6R)-7-(1-{[(5R,6S)-5-(4-chloro-3-fluorophenyl)-6-(6-chloropyridin-3-yl)-3-isopropyl-6-methyl-5,6-dihydroimidazo[2,l-b][l,3]thiazol-2-yl]carbonyl}-L-prolyl)-6-ethyl-4-(trifluoroacetyl)-4,7-diazaspiro[2.5]octane

The compound obtained in Step 13 of Reference Example 13 was reacted in the same way as in Step 1 of Example 57 using the compound obtained in Step 2 of Reference Example 23 instead of the compound obtained in Step 2 of Reference Example 15 to give the title compound as a colorless solid.
$^1$H-NMR (400 MHz, DMSO-$d_6$) δ: 0.68-0.74 (1H, m), 0.76-0.83 (1H, m), 0.88-0.98 (9H, m), 1.27-1.35 (1H, m), 1.46-1.56 (3H, m), 1.65-1.71 (1H, m), 1.77 (3H, s), 1.81-1.93 (2H, m), 2.14-2.26 (1H, m), 2.61-2.70 (1H, m), 3.19-3.73 (5H, m), 4.01-4.16 (1H, m), 4.59-4.65 (1H, m), 4.86-4.91 (1H, m), 5.51 (1H, s), 6.72-6.76 (1H, m), 6.90-6.95 (1H, m), 7.17 (1H, dd, J=8.4, 0.6 Hz), 7.34 (1H, t, J=8.1 Hz), 7.65 (1H, dd, J=8.3, 2.4 Hz), 8.26 (1H, d, J=2.2 Hz).
MS (ESI) m/z: 781 [(M+H)$^+$].

Step 2: (6R)-7-(1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-L-prolyl)-6-ethyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 90°C) δ: 0.30-0.40 (2H, m), 0.51-0.60 (3H, m), 0.78-0.84 (1H, m), 0.90 (3H, d, J=7.1 Hz), 0.93 (1H, m), 0.94 (3H, d, J=7.1 Hz), 1.63-1.70 (1H, m), 1.77 (3H, s), 1.79-1.90 (3H, m), 2.15-2.25 (1H, m), 2.29-2.40 (1H, m), 2.61-2.68 (1H, m), 2.73-2.86 (2H, m), 3.48-3.69 (4H, m), 4.23-4.29 (1H, m), 4.81-4.87 (1H, m), 5.49 (1H, s), 6.70-6.77 (1H, m), 6.89-6.96 (1H, m), 7.17 (1H, d, J=8.3 Hz), 7.33 (1H, t, J=7.9 Hz), 7.65 (1H, dd, J=8.4, 2.6 Hz), 8.25 (1H, d, J=2.4 Hz).
MS (ESI) m/z: 685 [(M+H)$^+$].

Example 63

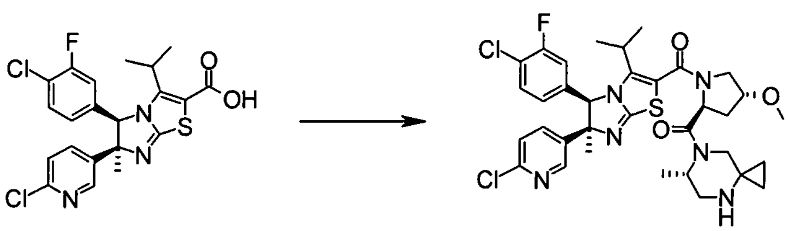

(6S)-7-[(4R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-4-methoxy-L-prolyl]-6-methyl-4,7-diazaspiro[2.5]octane

The compound obtained in Step 13 of Reference Example 13 was reacted in the same way as in Step 1 of Example 57 using the compound obtained in Step 3 of Reference Example 24 instead of the compound obtained in Step 2 of Reference Example 15 and then reacted in the same way as in Step 4 of Example 27 to give the title compound as a colorless solid.
$^1$H-NMR (400 MHz, DMSO-$d_6$, 100°C) δ: 0.49 (1H, s), 0.58 (1H, s), 0.73 (2H, t, J=7.6 Hz), 0.91 (3H, d, J=7.1 Hz), 0.95 (3H, d, J=7.1 Hz), 1.25 (3H, d, J=6.8 Hz), 1.78 (3H, s), 2.00 (1H, ddd, J=17.3, 7.8, 4.2 Hz), 2.28 (1H, t, J=11.2 Hz), 2.56-2.63 (1H, m), 2.83 (2H, d, J=12.0 Hz), 2.98 (2H, s), 3.20 (3H, s), 3.45 (1H, d, J=56.9 Hz), 3.64 (1H, dd, J=11.8, 4.3 Hz), 3.74 (1H, d, J=11.7 Hz), 4.06 (1H, s), 4.45 (1H, s), 4.86 (1H, s), 5.50 (1H, s), 6.76 (1H, d, J=8.5 Hz), 6.94 (1H, d, J=9.5 Hz), 7.17 (1H, d, J=8.3 Hz), 7.33 (1H, t, J=8.1 Hz), 7.66 (1H, dd, J=8.3, 2.4 Hz), 8.27 (1H, d, J=2.2 Hz).
MS (ESI) m/z: 701 [(M+H)$^+$].

Example 64

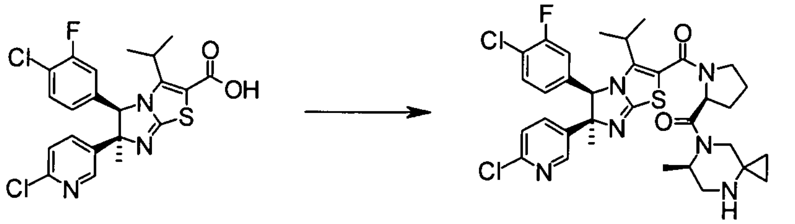

(6R)-7-(1-{[(5R,6S)-5-(4-chloro-3-fluorophenyl)-6-(6-chloropyridin-3-yl)-3-isopropyl-6-methyl-5,6-dihydroimidazo [2,1-b][1,3]thiazol-2-yl]carbonyl}-L-prolyl)-6-methyl-4,7-diazaspiro[2.5]octane

The compound obtained in Step 13 of Reference Example 13 was reacted in the same way as in Step 1 of Example 57 using the compound obtained in Step 2 of Reference Example 25 instead of the compound obtained in Step 2 of Reference Example 15 and then reacted in the same way as in Step 4 of Example 27 to give the title compound as a colorless solid.
$^1$H-NMR (400 MHz, DMSO-d$_6$, 100°C) δ: 0.47 (1H, m), 0.64 (2H, m), 0.90 (1H, m), 0.91 (3H, d, J=7.1 Hz), 0.94 (3H, d, J=7.1 Hz), 1.20-1.38 (4H, m), 1.77 (3H, s), 1.78-1.98 (3H, m), 2.27 (1H, m), 2.62-2.69 (1H, m), 2.77 (1H, d, J=13.2 Hz), 3.20-3.50 (2H, m), 3.58 (2H, t, J=6.7 Hz), 4.20 (1H, m), 4.82 (1H, dd, J=8.4, 4.5 Hz), 5.49 (1H, s), 6.73 (1H, d, J=7.3 Hz), 6.92 (1H, d, J=9.3 Hz), 7.16 (1H, d, J=8.3 Hz), 7.32 (1H, t, J=7.9 Hz), 7.64 (1H, dd, J=8.3, 2.4 Hz), 8.25 (1H, d, J=2.4 Hz).
MS (ESI) m/z: 671 [(M+H)$^+$].

Example 65

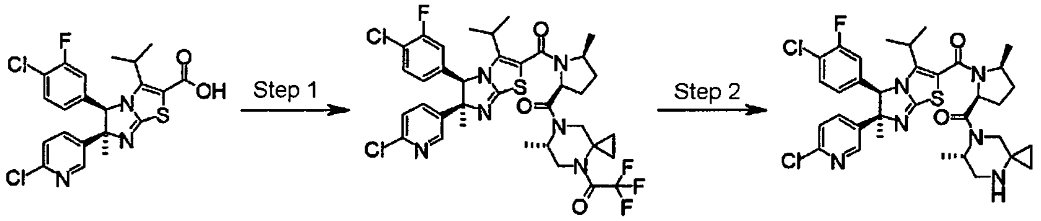


Step 1: (6S)-7-[(5S)-1-([(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl)-5-methyl-L-prolyl]-6-methyl-4-(trifluoroacetyl)-4,7-diazaspiro[2.5]octane

The compound obtained in Step 13 of Reference Example 13 was reacted in the same way as in Step 5 of Example 1 using the compound obtained in Step 2 of Reference Example 26 instead of the compound obtained in Step 2 of Reference Example 1 to give the title compound as a colorless solid.
$^1$H-NMR (400 MHz, DMSO-d$_6$) δ: 0.72-0.79 (2H, m), 0.89 (3H, d, J=7.1 Hz), 0.90 (3H, d, J=6.8 Hz), 1.09-1.15 (3H, m), 1.25-1.32 (1H, m), 1.34 (3H, d, J=6.3 Hz), 1.46-1.52 (1H, m), 1.65-1.71 (1H, m), 1.76 (3H, s), 1.82-1.89 (1H, m), 1.98-2.04 (1H, m), 2.12-2.20 (1H, m), 2.56-2.63 (1H, m), 3.19-3.66 (4H, m), 4.10-4.17 (1H, m), 4.62-4.69 (1H, m), 4.74-4.83 (1H, m), 5.51 (1H, s), 6.73-6.76 (1H, m), 6.92-6.95 (1H, m), 7.17 (1H, d, J=8.3 Hz), 7.34 (1H, t, J=8.1 Hz), 7.65 (1H, dd, J=8.4, 2.6 Hz), 8.25 (1H, d, J=2.4 Hz). MS (ESI) m/z: 781 [(M+H)$^+$].

Step 2: (6S)-7-[(5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-methyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-d$_6$, 90°C) δ: 0.39-0.44 (2H, m), 0.54-0.58 (2H, m), 0.88 (3H, d, J=7.1 Hz), 0.89 (3H, d, J=7.1 Hz), 1.20-1.28 (3H, m), 1.33 (3H, d, J=6.3 Hz), 1.59-1.68 (1H, m), 1.76 (3H, s), 1.79-1.87 (1H, m), 1.95-2.04 (1H, m), 2.11-2.19 (1H, m), 2.56-2.62 (1H, m), 2.64 (1H, d, J=12.7 Hz), 2.89-2.94 (1H, m), 3.31-3.45 (2H, m), 4.08-4.14 (1H, m), 4.26-4.31 (1H, m), 4.72-4.77 (1H, m), 5.50 (1H, s), 6.71-6.77 (1H, m), 6.91-6.96 (1H, m), 7.17 (1H, d, J=8.3 Hz), 7.34

(1H, t, J=8.1 Hz), 7.65 (1H, dd, J=8.3, 2.7 Hz), 8.25 (1H, d, J=2.7 Hz).
MS (ESI) m/z: 685 [(M+H)$^+$].

Example 66

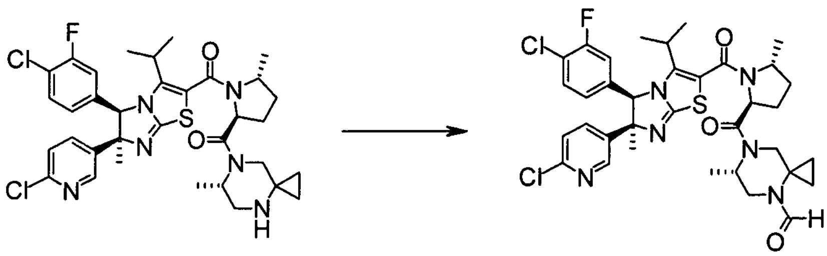

(6S)-7-[(5R)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-6-methyl-4,7-diazaspiro[2.5]octane-4-carbaldehyde

1H-benzotriazole-1-carbaldehyde (0.19 g, 1.31 mmol) was added to a tetrahydrofuran (4 ml) solution of the compound (0.60 g, 0.88 mmol) obtained in Example 21 and the resulting mixture was stirred at room temperature for 3 days. The reaction mixture was diluted with ethyl acetate, washed with saturated aqueous sodium bicarbonate solution and brine, and then dried over anhydrous magnesium sulfate. The solvent was evaporated under reduced pressure and the residue obtained was purified by NH-silica gel column chromatography (chloroform) to give the title compound (0.37 g, 59%) as a colorless solid. $^1$H-NMR (400 MHz, DMSO-$d_6$, 100˚C) δ: 0.50-0.56 (1H, m), 0.63-0.68 (1H, m), 0.91 (3H, d, J=7.1 Hz), 1.01 (3H, d, J=7.1 Hz), 1.04-1.14 (3H, m), 1.18 (3H, d, J=6.3 Hz), 1.48-1.54 (1H, m), 1.58-1.64 (1H, m), 1.75 (1H, m), 1.76 (3H, s), 2.15-2.21 (1H, m), 2.26-2.34 (1H, m), 2.64-2.77 (1H, m), 2.85-3.02 (2H, m), 3.37-3.52 (2H, m), 4.10 (1H, d, J=12.9 Hz), 4.31-4.37 (1H, m), 4.52-4.59 (1H, m), 4.85-4.91 (1H, m), 5.51 (1H, s), 6.77 (1H, d, J=7.8 Hz), 6.91 (1H, d, J=7.8 Hz), 7.17 (1H, d, J=8.3 Hz), 7.31 (1H, t, J=7.8 Hz), 7.67 (1H, dd, J=8.3, 2.4 Hz), 8.27 (1H, d, J=2.4 Hz), 8.31 (1H, s).
MS (ESI) m/z: 713 [(M+H)$^+$].

Example 67

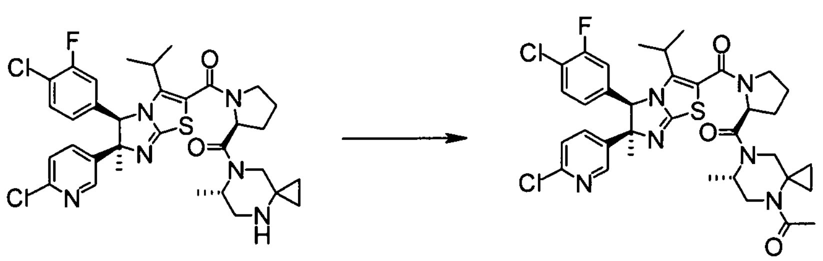

(6S)-4-acetyl-7-(1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-L-prolyl)-6-methyl-4,7-diazaspiro[2.5]octane

The compound obtained in Step 2 of Example 57 was reacted in the same way as in Example 4 to give the title compound as a colorless solid.
$^1$H-NMR (400 MHz, DMSO-$d_6$, 100˚C) δ: 0.51-0.66 (2H, m), 0.92 (3H, d, J=7.1 Hz), 0.94 (3H, d, J=7.1 Hz), 1.08 (3H, brs), 1.19-1.22 (1H, m), 1.44-1.46 (1H, m), 1.77 (3H, s), 1.79-1.88 (2H, m), 1.99-2.02 (1H, m), 2.09 (3H, brs), 2.21-2.24 (1H, m), 2.62-2.63 (1H, m), 3.09-3.66 (5H, m), 4.09-4.11 (1H, m), 4.48-4.51 (1H, m), 4.78-4.81 (1H, m), 5.49 (1H, s), 6.75 (1H, d, J=8.0 Hz), 6.93 (1H, d, J=8.0 Hz), 7.16 (1H, d, J=9.0 Hz), 7.33 (1H, t, J=8.0 Hz), 7.65 (1H, dd, J=8.3, 2.4

Hz), 8.26 (1H, d, J=2.4 Hz).
MS (ESI) m/z: 713 [$(M+H)^+$].

Example 68

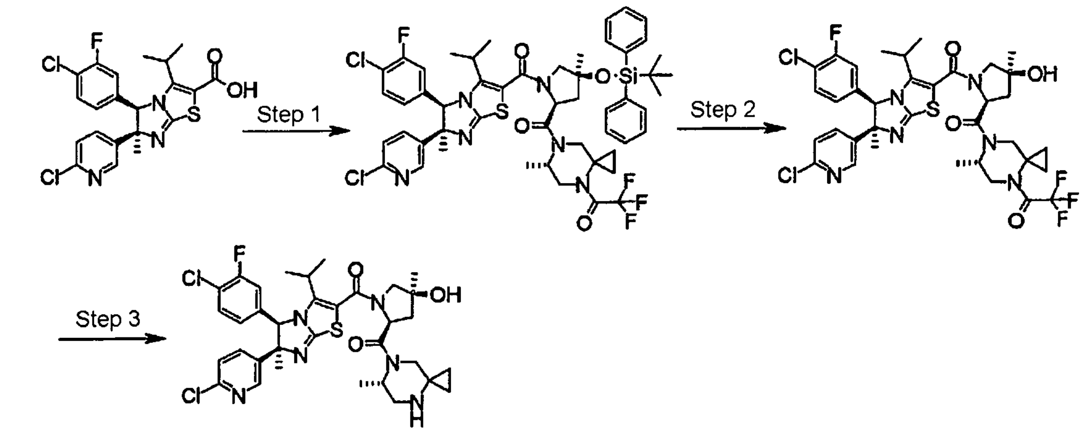


Step 1: (6S)-7-[(4S)-4-{[tert-butyl(diphenyl)silyl]oxy}-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-4-methyl-L-prolyl]-6-methyl-4-(trifluoroacetyl)-4,7-diazaspiro[2.5]octane

The compound obtained in Step 13 of Reference Example 13 was reacted in the same way as in Step 1 of Example 57 using the compound obtained in Step 5 of Reference Example 27 instead of the compound obtained in Step 2 of Reference Example 15 to give the title compound as a colorless solid.
$^1$H-NMR (400 MHz, DMSO-$d_6$, 100˚C) δ: 0.57-0.60 (1H, m), 0.67-0.70 (1H, m), 0.85 (3H, d, J=7.1 Hz), 0.88 (3H, d, J=7.1 Hz), 1.03 (12H, brs), 1.20 (3H, s), 1.24-1.28 (1H, m), 1.46-1.49 (1H, m), 1.80 (3H, s), 2.02 (1H, dd, J=12.0, 8.8 Hz), 2.19 (1H, dd, J=12.0, 8.5 Hz), 2.53-2.60 (1H, m), 3.39 (1H, d, J=10.0 Hz), 3.53 (3H, brs), 3.62 (1H, d, J=10.0 Hz), 3.87 (1H, brs), 4.61 (1H, brs), 4.71 (1H, brs), 5.49 (1H, s), 6.72 (1H, d, J=8.3 Hz), 6.89 (1H, d, J=8.3 Hz), 7.20 (1H, dd, J=8.3, 0.7 Hz), 7.29 (1H, t, J=7.9 Hz), 7.42-7.50 (6H, m), 7.66-7.71 (5H, m), 8.29 (1H, dd, J=2.4, 1.2 Hz).
MS (ESI) m/z: 1035 [$(M+H)^+$].

Step 2: (3S,5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-3-methyl-5-{[(6S)-6-methyl-4-(trifluoroacetyl)-4,7-diazaspiro[2.5]oct-7-yl]carbonyl} pyrrolidin-3-ol

The compound obtained in Step 1 above was reacted in the same way as in Step 2 of Example 3 to give the title compound as a colorless solid.
$^1$H-NMR (400 MHz, DMSO-$d_6$, 100˚C) δ: 0.66-0.77 (2H, m), 0.91 (3H, d, J=7.1 Hz), 0.96 (3H, d, J=7.1 Hz), 1.12 (3H, d, J=5.4 Hz), 1.26 (3H, s), 1.30-1.32 (1H, m), 1.48-1.52 (1H, m), 1.78 (3H, s), 1.86 (1H, dd, J=12.8, 6.0 Hz), 2.24 (1H, dd, J=12.8, 9.2 Hz), 2.56-2.63 (1H, m), 3.51 (1H, d, J=10.3 Hz), 3.59 (1H, d, J=10.3 Hz), 3.61 (3H, brs), 3.92 (1H, brs), 4.70-4.72 (1H, m), 4.85 (1H, brs), 5.30 (1H, s), 5.50 (1H, s), 6.76 (1H, d, J=7.8 Hz), 6.93 (1H, d, J=9.5 Hz), 7.17 (1H, d, J=8.5 Hz), 7.34 (1H, t, J=7.8 Hz), 7.66 (1H, dd, J=8.5, 2.7 Hz), 8.26 (1H, d, J=2.7 Hz).
MS (ESI) m/z: 797 [$(M+H)^+$].

Step 3: (3S,5S)-1-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-3-methyl-5-{[(6S)-6-methyl-4,7-diazaspiro[2.5]oct-7-yl]carbonyl}pyrrolidin-3-ol

The compound obtained in Step 2 above was reacted in the same way as in Step 4 of Example 27 to give the title compound as a colorless solid.
$^1$H-NMR (400 MHz, DMSO-$d_6$, 100˚C) δ: 0.36-0.37 (2H, m), 0.56-0.58 (2H, m), 0.91 (3H, d, J=7.1 Hz), 0.95 (3H, d, J=7.1 Hz), 1.22 (3H, brd, J=6.6 Hz), 1.25 (3H, s), 1.78 (3H, s), 1.82 (1H, dd, J=12.8, 5.5 Hz), 2.23 (1H, dd, J=12.8, 9.2 Hz), 2.34 (1H, dd, J=10.0, 3.4 Hz), 2.59-2.64 (2H, m), 3.20 (1H, brs), 3.45 (1H, brs), 3.49 (1H, d, J=10.5 Hz), 3.59 (1H, d,

J=10.5 Hz), 4.35 (1H, brs), 4.81 (1H, brs), 5.50 (1H, s), 6.75 (1H, d, J=8.1 Hz), 6.93 (1H, d, J=8.1 Hz), 7.17 (1H, d, J=8.3 Hz), 7.34 (1H, t, J=7.9 Hz), 7.66 (1H, dd, J=8.3, 2.4 Hz), 8.26 (1H, d, J=2.4 Hz).
MS (ESI) m/z: 701 [$(M+H)^+$].

Example 69

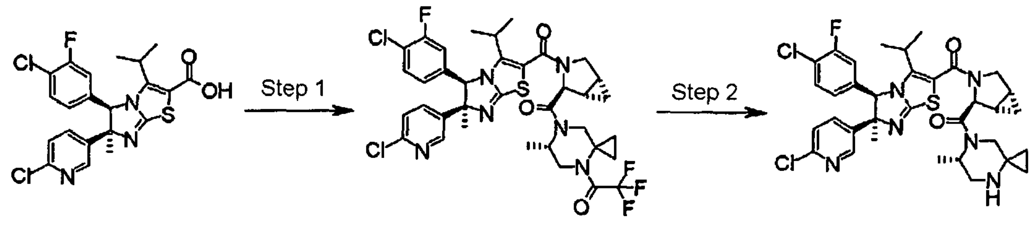


Step 1: (6S)-7-{[(1S,2S,5R)-3-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-3-azabicyclo[3.1.0]hex-2-yl]carbonyl}-6-methyl-4-(trifluoroacetyl)-4,7-diazaspiro[2.5]octane

The compound obtained in Step 13 of Reference Example 13 was reacted in the same way as in Step 1 of Example 57 using the compound obtained in Step 2 of Reference Example 28 instead of the compound obtained in Step 2 of Reference Example 15 to give the title compound as a colorless solid.
MS (ESI) m/z: 779 [$(M+H)^+$].

Step 2: (65)-7-{[(1S,25,5R)-3-{[(5R,65)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-3-azabicyclo[3.1.0]hex-2-yl]carbonyl}-6-methyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 100°C) δ: 0.13-0.14 (1H, m), 0.40 (2H, brs), 0.57 (2H, brs), 0.75-0.80 (1H, m), 0.88-0.92 (6H, m), 1.25 (3H, s), 1.48-1.51 (1H, m), 1.64 (1H, brs), 1.75 (3H, s), 2.36 (1H, brs), 2.52-2.59 (1H, m), 2.65 (1H, d, J=12.3 Hz), 2.92 (1H, s), 3.30 (1H, br), 3.45 (1H, br), 3.67 (1H, d, J=10.5 Hz), 3.75 (1H, brs), 4.31 (1H, brs), 4.82 (1H, brs), 5.47 (1H, s), 6.73 (1H, d, J=6.5 Hz), 6.94 (1H, d, J=9.3 Hz), 7.16 (1H, dd, J=8.2, 0.5 Hz), 7.33 (1H, t, J=8.0 Hz), 7.63 (1H, dd, J=8.5, 2.0 Hz), 8.24 (1H, d, J=2.0 Hz).
MS (ESI) m/z: 683 [$(M+H)^+$].

Example 70

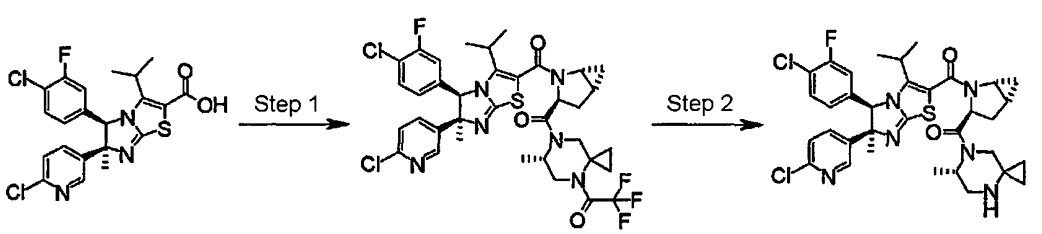


Step 1: (6S)-7-{[(1R,3S,5R)-2-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-2-azabicyclo[3.1.0]hex-3-yl]carbonyl}-6-methyl-4-(trifluoroacetyl)-4,7-diazaspiro[2.5]octane

The compound obtained in Step 13 of Reference Example 13 was reacted in the same way as in Step 1 of

Example 57 using the compound obtained in Step 2 of Reference Example 29 instead of the compound obtained in Step 2 of Reference Example 15 to give the title compound as a colorless solid.
$^1$H-NMR (400 MHz, DMSO-$d_6$, 100˚C) δ: 0.57-0.61 (1H, m), 0.67-0.74 (2H, m), 0.92 (3H, d, J=7.1 Hz), 0.93 (3H, d, J=7.1 Hz), 0.95-0.96 (1H, m), 1.10 (3H, brd, J=6.3 Hz), 1.23-1.27 (1H, m), 1.47-1.50 (1H, m), 1.78 (3H, s), 1.84-1.87 (1H, m), 2.05-2.12 (1H, m), 2.25 (1H, dd, J=12.5, 9.5 Hz), 2.67-2.74 (1H, m), 3.58 (3H, brs), 3.61 (1H, dq, J=7.4, 2.3 Hz), 3.90 (1H, brs), 4.59-4.71 (2H, m), 5.53 (1H, s), 6.75 (1H, d, J=8.5 Hz), 6.92 (1H, d, J=7.1 Hz), 7.17 (1H, dd, J=8.3, 0.5 Hz), 7.33 (1H, t, J=7.9 Hz), 7.66 (1H, dd, J=8.3, 2.7 Hz), 8.27 (1H, d, J=2.2 Hz).

Step 2: (6S)-7-{[(1R,3S,5R)-2-{[(5R,6S)-5-(4-chloro-3-fluorophenyl)-6-(6-chloropyridin-3-yl)-3-isopropyl-6-methyl-5,6-dihydroimidazo[2,1-b][1,3]thiazol-2-yl]carbonyl}-2-azabicyclo[3.1.0]hex-3-yl]carbonyl}-6-methyl-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 100˚C) δ: 0.32-0.40 (2H, m), 0.50-0.59 (3H, m), 0.92 (1H, m), 0.93 (3H, d, J=7.1 Hz), 0.94 (3H, d, J=7.1 Hz), 1.19 (3H, d, J=6.6 Hz), 1.78 (3H, s), 1.80-1.85 (1H, m), 2.03-2.09 (1H, m), 2.23 (1H, ddd, J=13.1, 8.9, 1.3 Hz), 2.27-2.33 (1H, m), 2.62 (1H, dd, J=12.8, 1.8 Hz), 2.67-2.74 (1H, m), 2.91-2.92 (1H, m), 3.10 (1H, brs), 3.41 (1H, brs), 3.59 (1H, dq, J=7.5, 2.3 Hz), 4.27 (1H, brs), 4.64 (1H, brs), 5.52 (1H, s), 6.74 (1H, d, J=8.3 Hz), 6.92 (1H, d, J=8.3 Hz), 7.17 (1H, d, J=8.3 Hz), 7.33 (1H, t, J=8.3 Hz), 7.66 (1H, dd, J=8.3, 2.6 Hz), 8.27 (1H, t, J=1.2 Hz).
MS (ESI) m/z: 683 [$(M+H)^+$].

Example 71

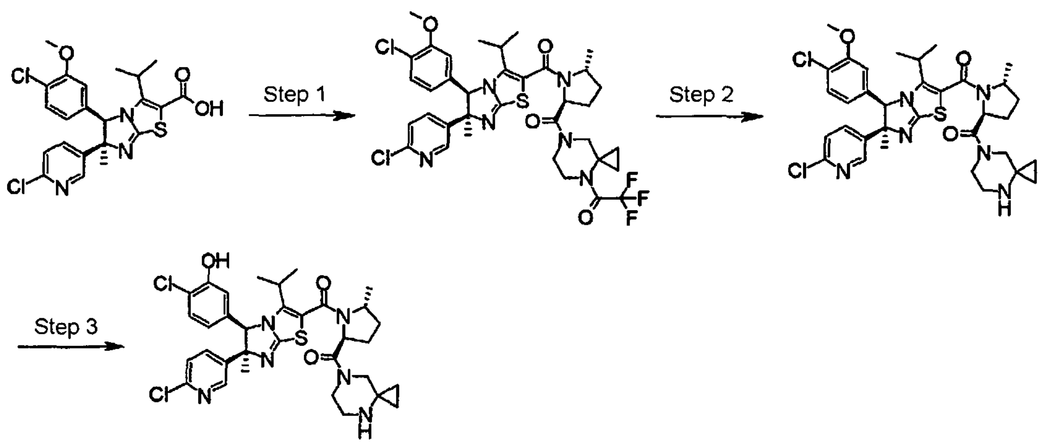


Step 1: 7-[(5R)-1-{[(5R,6S)-5-(4-chloro-3-methoxyphenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-4-(trifluoroacetyl)-4,7-diazaspiro[2.5]octane

The compound obtained in Step 9 of Reference Example 31 was reacted in the same way as in Step 5 of Example 1 using the compound obtained in Step 2 of Reference Example 8 instead of the compound obtained in Step 2 of Reference Example 1 to give the title compound as a pale yellow solid.
$^1$H-NMR (400 MHz, $CDCl_3$, 70˚C) δ: 0.90-1.31 (4H, m), 0.99 (3H, d, J=7.1 Hz), 1.05 (3H, d, J=6.8 Hz), 1.24 (3H, d, J=6.1 Hz), 1.48-1.72 (2H, m), 1.78-1.85 (1H, m), 1.82 (3H, s), 2.12-2.30 (1H, m), 2.34-2.48 (1H, m), 2.69-2.82 (1H, m), 3.38-3.75 (3H, m), 3.70 (3H, s), 3.75-3.97 (2H, m), 4.44-4.55 (1H, m), 4.85-4.97 (1H, m), 4.98 (1H, t, J=8.5 Hz), 6.29 (1H, s), 6.35 (1H, d, J=7.6 Hz), 6.96 (1H, d, J=8.3 Hz), 7.07 (1H, d, J=8.0 Hz), 7.48 (1H, dd, J=8.3, 2.3 Hz), 8.24 (1H, d, J=2.3 Hz).
MS (ESI) m/z: 779 [$(M+H)^+$].

Step 2: 7-[(5R)-1-{[(5R,6S)-5-(4-chloro-3-methoxyphenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-4,7-diazaspiro[2.5]octane

The compound obtained in Step 1 above was reacted in the same way as in Step 4 of Example 27 to give the

title compound as a colorless solid.
$^1$H-NMR (400 MHz, DMSO-$d_6$, 100˚C) δ: 0.39-0.57 (4H, m), 0.81-0.94 (1H, m), 0.88 (3H, d, J=7.1 Hz), 1.00 (3H, d, J=6.8 Hz), 1.16 (3H, d, J=6.3 Hz), 1.49-1.87 (1H, m), 1.76 (3H, s), 1.99-2.16 (1H, m), 2.23-2.37 (1H, m), 2.41-2.52 (2H, m), 2.64-2.83 (2H, m), 2.87-2.99 (1H, m), 3.21-3.55 (3H, m), 3.70 (3H, s), 4.25-4.36 (1H, m), 4.85-4.95 (1H, m), 5.41 (1H, s), 6.33-6.46 (1H, m), 6.64-6.79 (1H, m), 7.05-7.18 (1H, m), 7.13 (1H, d, J=8.3 Hz), 7.65 (1H, dd, J=8.3, 2.2 Hz), 8.27 (1H, d, J=2.2 Hz).
MS (ESI) m/z: 683 [(M+H)$^+$].

Step 3: 2-chloro-5-[(5R,6S)-6-(6-chloropyridin-3-yl)-2-{[(2S,5R)-2-(4,7-diazaspiro[2.5]oct-7-ylcarbonyl)-5-methylpyrrolidin-1-yl]carbonyl}-3-isopropyl-6-methyl-5,6-dihydroimidazo[2,1-b][1,3]thiazol-5-yl]phenol

Boron tribromide (1 M dichloromethane solution; 0.46 ml, 0.46 mmol) was added dropwise to a dichloromethane (2 ml) solution of the compound (62.3 mg, 0.09 mmol) obtained in Step 2 above at -10˚C in a nitrogen atmosphere and the resulting mixture was stirred at the same temperature for 1 hour and then further stirred at room temperature for 5 hours. Water was added to the reaction mixture under ice cooling, then the pH of the resulting mixture was adjusted to 12 with 1 N aqueous sodium hydroxide solution and the reaction mixture was washed with chloroform and chloroform: methanol [10:1 (v/v)]. The pH of the aqueous layer was adjusted to 8 with 1 N aqueous hydrochloric acid solution, followed by extraction with chloroform:methanol [10:1 (v/v)]. The organic layer was dried over anhydrous sodium sulfate and the solvent was evaporated under reduced pressure. The residue was purified by silica gel thin layer chromatography [$CHCl_3$:methanol = 10:1 (v/v)] to give the title compound (25.3 mg, 42%) as a colorless solid. $^1$H-NMR (400 MHz, DMSO-$d_6$, 100˚C) δ: 0.47-0.57 (4H, m), 0.86-0.98 (6H, m), 1.15 and 1.24 (total 3H, each d, J=6.8 Hz), 1.52-1.67 (1H, m), 1.72 (3H, brs), 1.78-1.86 (1H, m), 1,96-2.36 (1H, m), 2.52-2.85 (4H, m), 3.27-3.55 (4H, m), 4.14-4.39 (1H, m), 4.76-4.93 (1H, m), 5.31 (1H, s), 6.32-6.44 (1H, m), 6.48-6.54 (1H, m), 7.02 (1H, dd, J=8.1, 1.7 Hz), 7.13 (1H, d, J=8.3 Hz), 7.59-7.67 (1H, m), 8.25 (1H, dd, J=5.9, 2.4 Hz).
MS (ESI) m/z: 669 [(M+H)$^+$].

Example 72

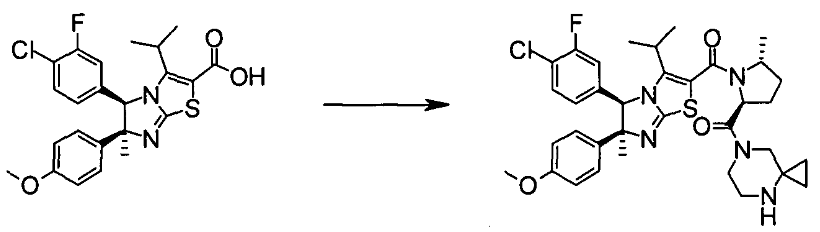

7-[(5R)-1-{[(5R,6S)-5-(4-chloro-3-fluorophenyl)-3-isopropyl-6-(4-methoxyphenyl)-6-methyl-5,6-dihydroimidazo[2,1-b][1,3]thiazol-2-yl]carbonyl}-5-methyl-L-prolyl]-4,7-diazaspiro[2.5]octane

The compound obtained in Step 8 of Reference Example 32 was reacted in the same way as in Step 5 of Example 1 using the compound obtained in Step 2 of Reference Example 8 instead of the compound obtained in Step 2 of Reference Example 1 and then reacted in the same way as in Step 4 of Example 27 to give the title compound as a pale yellow solid.
$^1$H-NMR (400 MHz, DMSO-$d_6$, 100˚C) δ: 0.41-0.59 (4H, m), 0.89 (3H, d, J=7.1 Hz), 1.00 (3H, d, J=7.1 Hz), 1.18 (3H, d, J=6.3 Hz), 1.54-1.64 (1H, m), 1.70-1.80 (1H, m), 1.73 (3H, s), 2.04-2.17 (1H, m), 2.25-2.39 (1H, m), 2.64-2.85 (3H, m), 3.25-3.56 (5H, m), 3.64 (3H, s), 4.27-4.39 (1H, m), 4.93 (1H, d, J=7.3 Hz), 5.36 (1H, s), 6.62 (2H, d, J=8.8 Hz), 6.70 (1H, d, J=7.9 Hz), 6.82 (1H, d, J=7.9 Hz), 7.15 (2H, d, J=8.8 Hz), 7.24 (1H, t, J=7.9 Hz).
MS (ESI) m/z: 666 [(M+H)$^+$].

Example 73

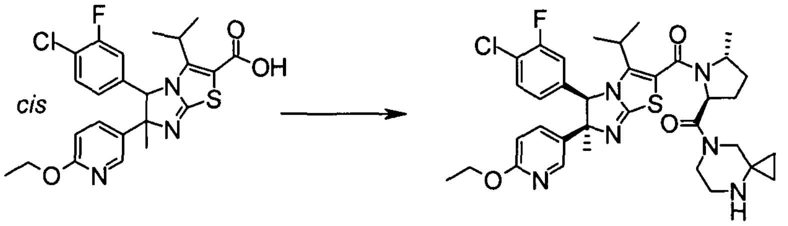

7-[(5R)-1-{[(5R,6S)-5-(4-chloro-3-fluorophenyl)-6-(6-ethoxypyridin-3-yl)-3-isopropyl-6-methyl-5,6-dihydroimidazo [2,1-b] [1,3]-thiazol-2-yl]-carbonyl}-5-methyl-L-prolyl]-4,7-diazaspiro[2.5]octane

The compound obtained in Step 6 of Reference Example 33 was reacted in the same way as in Step 5 of Example 1 using the compound obtained in Step 2 of Reference Example 8 instead of the compound obtained in Step 2 of Reference Example 1 and then reacted in the same way as in Step 4 of Example 27 and the obtained mixture of diastereomers was resolved and purified on an optically active column [CHIRALCEL OD-H (Daicel Chemical Industries, Ltd.)] to give the title compound as a colorless solid. $^1$H-NMR (400 MHz, DMSO-$d_6$, 100°C) δ: 0.42-0.60 (4H, m), 0.89 (3H, d, J=7.1 Hz), 1.01 (3H, d, J=7.1 Hz), 1.18 (3H, d, J=6.1 Hz), 1.22 (3H, t, J=7.0 Hz), 1.53-1.64 (1H, m), 1.71-1.81 (1H, m), 1.75 (3H, s), 2.03-2.18 (1H, m), 2.24-2.38 (1H, m), 2.65-2.85 (4H, m), 3.23-3.60 (4H, m), 4.20 (2H, q, J=7.0 Hz), 4.28-4.38 (1H, m), 4.93 (1H, d, J=7.6 Hz), 5.41 (1H, s), 6.43 (1H, d, J=8.5 Hz), 6.72 (1H, d, J=8.1 Hz), 6.87 (1H, d, J=8.1 Hz), 7.30 (1H, t, J=8.1 Hz), 7.47 (1H, dd, J=8.5, 2.4 Hz), 7.99 (1H, d, J=2.4 Hz).
MS (ESI) m/z: 681 [(M+H)$^+$].

Example 74

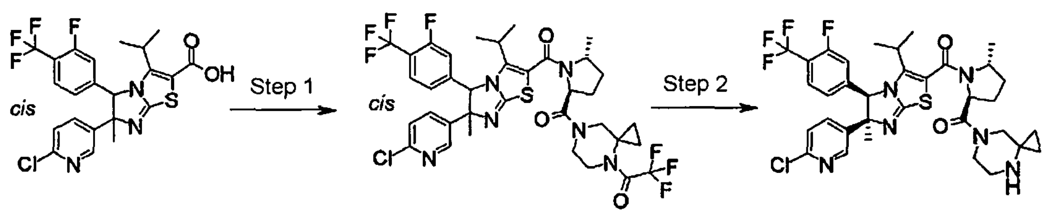


Step 1: 7-[(5R)-1-({(5R*,6S*)-6-(6-chloropyridin-3-yl)-5-[3-fluoro-4-(trifluoromethyl)phenyl]-3-isopropyl-6-methyl-5,6-dihydroimidazo[2,1-b][1,3]thiazol-2-yl}carbonyl)-5-methyl-L-prolyl]-4-(trifluoroacetyl)-4,7-diazaspiro [2.5]octane

The compound obtained in Step 5 of Reference Example 34 was reacted in the same way as in Step 5 of Example 1 using the compound obtained in Step 2 of Reference Example 8 instead of the compound obtained in Step 2 of Reference Example 1 to give the title compound as a pale yellow solid.
MS (ESI) m/z: 802 [(M+H)$^+$].

Step 2: 7-[(5R)-1-({(5R,6S)-6-(6-chloropyridin-3-yl)-5-[3-fluoro-4-(trifluoromethyl)phenyl]-3-isopropyl-6-methyl-5,6-dihydroimidazo[2,1-b][1,3]thiazol-2-yl}carbonyl)-5-methyl-L-prolyl]-4,7-diazaspiro[2.5]octane

The compound obtained in Step 1 above was reacted in the same way as in Step 4 of Example 27 and the obtained mixture of diastereomers was resolved and purified on an optically active column [CHIRALCEL OD-H (Daicel Chemical Industries, Ltd.)] to give the title compound as a colorless solid.
$^1$H-NMR (400 MHz, DMSO-$d_6$) δ: 0.81-0.87 (2H, m), 0.90 (3H, d, J=7.1 Hz), 0.93-0.94 (2H, m), 1.00 (3H, d, J=7.1 Hz), 1.18 (3H, d, J=6.3 Hz), 1.61-1.64 (1H, m), 1.80-1.82 (4H, m), 2.12-2.14 (1H, m), 2.29-2.31 (1H, m), 2.68-2.75 (1H, m), 3.13-3.20 (2H, m), 3.58-3.84 (4H, m), 4.35 (1H, t, J=5.7 Hz), 4.92-4.95 (1H, m), 5.62 (1H, s), 6.92 (1H, d, J=7.3 Hz), 7.00 (1H, d, J=10.7 Hz), 7.16 (1H, d, J=8.3 Hz), 7.26-7.34 (1H, m), 7.53 (1H, t, J=7.8 Hz), 7.66 (1H, dd, J=8.4, 2.6 Hz), 8.27 (1H, d, J=2.0 Hz).

MS (ESI) m/z: 705 [(M+H)+].

Example 75

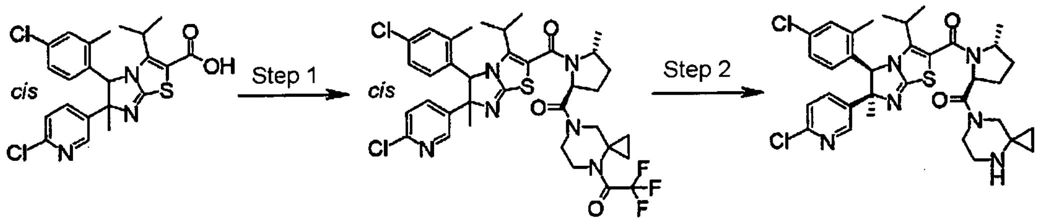


Step 1: 7-[(5R)-1-{[(5R*,6S*)-5-(4-chloro-2-methylphenyl)-6-(6-chloropyridin-3-yl)-3-isopropyl-6-methyl-5,6-dihydroimidazo[2,1-b][1,3]thiazol-2-yl]carbonyl}-5-methyl-L-prolyl]-4-(trifluoroacetyl)-4,7-diazaspiro[2.5]octane

The compound obtained in Step 8 of Reference Example 35 was reacted in the same way as in Step 5 of Example 1 using the compound obtained in Step 2 of Reference Example 8 instead of the compound obtained in Step 2 of Reference Example 1 to give the title compound as a pale yellow solid.
MS (ESI) m/z: 763 [(M+H)+].

Step 2: 7-[(5R)-1-{[(5R,6S)-5-(4-chloro-2-methylphenyl) - 6-(6-chloropyridin-3-yl)-3-isopropyl-6-methyl-5,6-dihydroimidazo[2,1-b][1,3]thiazol-2-yl]carbonyl}-5-methyl-L-prolyl]-4,7-diazaspiro[2.5] octane

The compound obtained in Step 1 above was reacted in the same way as in Step 4 of Example 27 and the obtained mixture of diastereomers was resolved and purified on an optically active column [CHIRALCEL OD-H (Daicel Chemical Industries, Ltd.)] to give the title compound as a colorless solid.
$^1$H-NMR (400 MHz, DMSO-$d_6$, 100˚C) δ: 0.42-0.54 (4H, m), 0.81-0.96 (1H, m), 0.89 (3H, d, J=7.1 Hz), 0.92 (3H, d, J=7.3 Hz), 1.16 (3H, d, J=6.3 Hz), 1.23-1.32 (1H, m), 1.51-1.61 (1H, m), 1.70-1.78 (1H, m), 1.82 (3H, s), 2.00-2.14 (1H, m), 2.22-2.35 (1H, m), 2.26 (3H, s), 2.48-2.58 (1H, m), 2.73-2.82 (2H, m), 3.26-3.55 (3H, m), 4.31 (1H, s), 4.87-4.95 (1H, m), 5.40 (1H, s), 6.58 (1H, d, J=9.0 Hz), 7.00-7.06 (2H, m), 7.11 (1H, d, J=8.4 Hz), 7.60 (1H, dd, J=8.4, 2.4 Hz), 8.21 (1H, d, J=2.4 Hz).
MS (ESI) m/z: 667 [(M+H)+].

Example 76

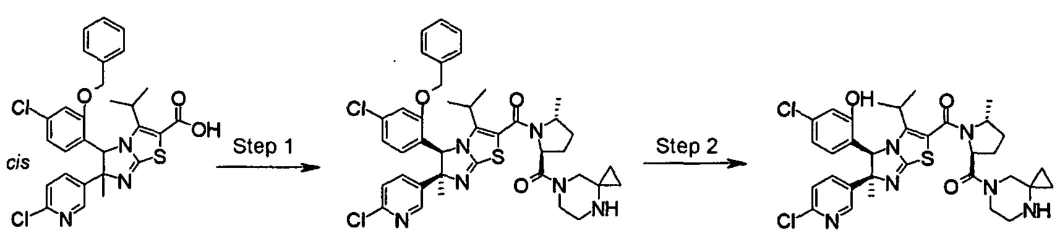


Step 1: 7-[(5R)-1-{[(5R,6S)-5-[2-(benzyloxy)-4-chlorophenyl]-6-(6-chloropyridin-3-yl)-3-isopropyl-6-methyl-5,6-dihydroimidazo[2,1-b][1,3] thiazol-2-yl]carbonyl}-5-methyl-L-prolyl]-4,7-diazaspiro[2.5]octane

The compound obtained in Step 6 of Reference Example 36 was reacted in the same way as in Step 5 of Example 1 using the compound obtained in Step 2 of Reference Example 8 instead of the compound obtained in Step

2 of Reference Example 1 and then reacted in the same way as in Step 4 of Example 27 and the obtained mixture of diastereomers was resolved and purified on an optically active column [CHIRALCEL OD-H (Daicel Chemical Industries, Ltd.)] to give the title compound as a colorless solid. $^1$H-NMR (400 MHz, $CDCl_3$, 70˚C) δ: 0.47-0.70 (4H, m), 0.90 (3H, d, J=7.1 Hz), 1.07 (3H, d, J=7.1 Hz), 1.24 (3H, d, J=6.6 Hz), 1.59-1.68 (1H, m), 1.70 (3H, s), 1.82-1.91 (1H, m), 2.17-2.44 (2H, m), 2.78-2.85 (1H, m), 2.88-3.10 (2H, m), 3.30 (1H, d, J=13.2 Hz), 3.38-3.64 (3H, m), 4.48-4.57 (1H, m), 4.86-5.03 (1H, m), 4.92 (2H, dd, J=25.1, 11.2 Hz), 5.57 (1H, s), 6.56 (1H, d, J=8.3 Hz), 6.67 (1H, d, J=1.8 Hz), 6.70 (1H, dd, J=8.3, 1.8 Hz), 6.90 (1H, d, J=8.3 Hz), 7.37-7.48 (6H, m), 8.20 (1H, d, J=2.4 Hz).
MS (ESI) m/z: 759 [$(M+H)^+$].

Step 2: 5-chloro-2-[(5R,6S)-6-(6-chloropyridin-3-yl)-2-{[(2S,5R)-2-(4,7-diazaspiro[2.5]oct-7-ylcarbonyl)-5-methylpyrrolidin-1-yl]carbonyl}-3-isopropyl-6-methyl-5,6-dihydroimidazo[2,1-b] [1,3] thiazol-5-yl] phenol

Anhydrous aluminum chloride (233 mg, 1.71 mmol) was added to a dichloromethane (20 ml) solution of the compound (260 mg, 0.34 mmol) obtained in Step 1 above under ice cooling and the resulting mixture was gradually returned to room temperature while stirred for 18 hours. Water was added to the reaction mixture and then the mixture was made basic by the addition of potassium carbonate, followed by extraction with chloroform:methanol [9:1 (v/v)]. The organic layer was dried over anhydrous magnesium sulfate. The solvent was evaporated under reduced pressure and the residue obtained was purified by silica gel column chromatography [chloroform:methanol = 4:1 (v/v)] to give the title compound (93.0 mg, 41%) as a pale yellow solid.
$^1$H-NMR (400 MHz, DMSO-$d_6$, 70˚C) δ: 0.36-0. 58 (4H, m), 0.85 (3H, d, J=7.1 Hz), 1.01 (3H, d, J=7.1 Hz), 1.17 (3H, d, J=6.3 Hz), 1.50-1.63 (1H, m), 1.66-1.82 (1H, m), 1.75 (3H, s), 2.00-2.16 (1H, m), 2.24-2.44 (1H, m), 2.64-2.85 (3H, m), 2.93-3.21 (1H, m), 3.22-3.56 (4H, m), 4.23-4.38 (1H, m), 4.88-4.99 (1H, m), 5.59 (1H, s), 6.45 (1H, d, J=8.5 Hz), 6.59-6.68 (2H, m), 7.15 (1H, d, J=8.3 Hz), 7.64 (1H, dd, J=8.3, 2.2 Hz), 8.26 (1H, d, J=2.2 Hz), 10.19 (1H, brs).
MS (ESI) m/z: 669 [$(M+H)^+$].

Example 77

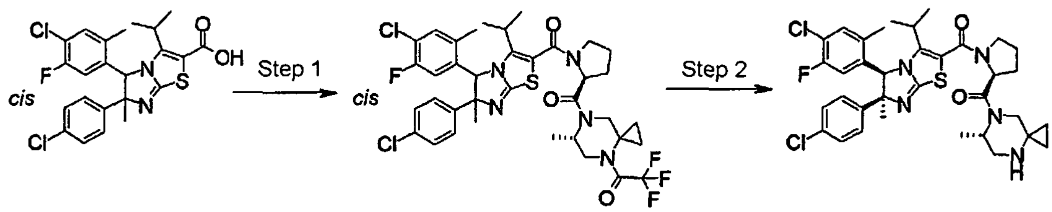


Step 1: (6S)-7-(1-{[(5R*,6S*)-5-(4-chloro-5-fluoro-2-methylphenyl)-6-(6-chloropyridin-3-yl)-3-isopropyl-6-methyl-5,6-dihydroimidazo[2,1-b][1,3]thiazol-2-yl]carbonyl}-L-prolyl)-6-methyl-4-(trifluoroacetyl)-4,7-diazaspiro[2.5]octane

The compound obtained in Step 6 of Reference Example 37 was reacted in the same way as in Step 1 of Example 57 to give the title compound as a colorless solid.
MS (ESI) m/z: 781 [$(M+H)^+$].

Step 2: (6S)-7-(1-{[(5R,6S)-5-(4-chloro-5-fluoro-2-methylphenyl)-6-(6-chloropyridin-3-yl)-3-isopropyl-6-methyl-5,6-dihydroimidazo[2,1-b][1,3]thiazol-2-yl]carbonyl}-L-prolyl)-6-methyl-4,7-diazaspiro[2.5]octane

The compound obtained in Step 1 above was reacted in the same way as in Step 4 of Example 27 and the obtained mixture of diastereomers was resolved and purified on an optically active column [CHIRALCEL IC-H (Daicel Chemical Industries, Ltd.)] to give the title compound as a colorless solid.
$^1$H-NMR (400 MHz, DMSO-$d_6$, 100˚C) δ: 0.37 (2H, t, J=7.6 Hz), 0.54-0.59 (2H, m), 0.86 (3H, t, J=7.1 Hz), 0.95 (3H, t, J=7.1 Hz), 1.20 (3H, d, J=6.1 Hz), 1.75-1.81 (1H, m), 1.82 (3H, s), 1.88-1.99 (1H, m), 2.17-2.24 (1H, m), 2.25 (3H, s), 2.35 (1H, brs), 2.64 (1H, d, J=12.9 Hz), 2.92 (2H, brs), 3.20 (1H, brs), 3.41 (1H, brs), 3.49-3.61 (2H, m), 4.29 (1H, brs), 4.76 (1H, brs), 5.42 (1H, s), 6.42 (1H, d, J=10.5 Hz), 7.14-7.20 (2H, m), 7.60-7.64 (1H, m), 8.18-8.22 (1H, m).
MS (ESI) m/z: 685 [$(M+H)^+$].

Example 78

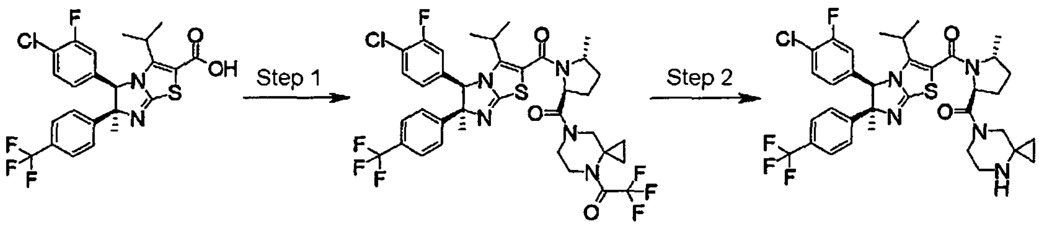

Step 1: 7-[(5R)-1-({(5R,6S)-5-(4-chloro-3-fluorophenyl)-3-isopropyl-6-methyl-6-[4-(trifluoromethyl)phenyl]-5,6-dihydroimidazo[2,l-b] [l,3]thiazol-2-yl}carbonyl)-5-methyl-L-prolyl]-4-(trifluoroacetyl)-4,7-diazaspiro [2.5] octane

The compound obtained in Step 10 of Reference Example 38 was reacted in the same way as in Step 5 of Example 1 using the compound obtained in Step 2 of Reference Example 8 instead of the compound obtained in Step 2 of Reference Example 1 to give the title compound as a colorless solid.
$^1$H-NMR (400 MHz, DMSO-$d_6$, 100°C) δ: 0.91 (3H, d, J=7.1 Hz), 1.00 (3H, d, J=7.1 Hz), 1.05-1.12 (4H, m), 1.18 (3H, d, J=6.3 Hz), 1.60-1.63 (1H, m), 1.78 (3H, s), 2.10-2.14 (1H, m), 2.31-2.32 (1H, m), 2.66-2.74 (1H, m), 2.91-2.92 (1H, m), 3.45-3.81 (6H, m), 4.34-4.36 (1H, m), 4.96 (1H, d, J=6.8 Hz), 5.51 (1H, s), 6.72 (1H, d, J=8.0 Hz), 6.83 (1H, d, J=8.0 Hz), 7.22 (1H, t, J=8.0 Hz), 7.39 (2H, d, J=8.0 Hz), 7.48 (2H, d, J=8.0 Hz).
MS (ESI) m/z: 800 [$(M+H)^+$].

Step 2: 7-[(5R)-1-({(5R,6S)-5-(4-chloro-3-fluorophenyl)-3-isopropyl-6-methyl-6-[4-(trifluoromethyl)phenyl]-5,6-dihydroimidazo[2,1-b][1,3]thiazol-2-yl}carbonyl)-5-methyl-L-prolyl]-4,7-diazaspiro[2.5]octane

The compound obtained in Step 1 above was reacted in the same way as in Step 4 of Example 27 to give the title compound as a colorless solid.
$^1$H-NMR (400 MHz, DMSO-$d_6$, 100°C) δ: 0.47 (4H, brs), 0.87 (3H, d, J=7.1 Hz), 0.98 (3H, d, J=7.1 Hz), 1.15 (3H, d, J=6.3 Hz), 1.26 (1H, m), 1.57 (1H, m), 1.73 (1H, m), 1.77 (3H, s), 2.08 (1H, m), 2.30 (1H, m), 2.71 (1H, m), 2.76 (1H, m), 3.29-3.32 (2H, m), 3.39 (1H, m), 3.47 (1H, m), 4.31 (1H, m), 4.90 (1H, d, J=7.6 Hz), 5.47 (1H, s), 6.69 (1H, d, J=7.8 Hz), 6.83 (1H, d, J=7.8 Hz), 7.21 (1H, t, J=8.3 Hz), 7.37 (2H, d, J=8.3 Hz), 7.46 (2H, d, J=8.3 Hz). MS (ESI) m/z: 704 [$(M+H)^+$].

Example 79

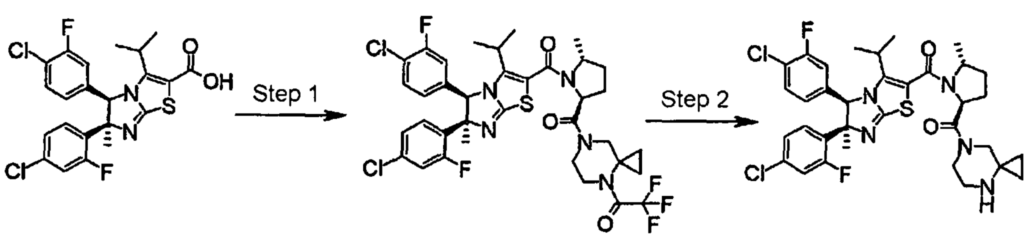


Step 1: 7-[(5R)-1-([(5R,6S)-6-(4-chloro-2-fluorophenyl)-5-(4-chloro-3-fluorophenyl)-3-isopropyl-6-methyl-5,6-dihydroimidazo [2,1-b] [1,3] thiazol-2-yl] carbonyl)-5-methyl-L-prolyl]-4-(trifluoroacetyl)-4,7-diazaspiro[2.5]octane

The compound obtained in Step 7 of Reference Example 39 was reacted in the same way as in Step 5 of Example 1 using the compound obtained in Step 2 of Reference Example 8 instead of the compound obtained in Step

2 of Reference Example 1 to give the title compound as a colorless solid.
$^1$H-NMR (400 MHz, DMSO-$d_6$, 100˚C) δ: 0.81 (3H, d, J=7.1 Hz), 0.94 (3H, d, J=7.1 Hz), 0.98-1.10 (4H, m), 1.20 (3H, d, J=6.3 Hz), 1.57 (1H, m), 1.71 (3H, d, J=1.2 Hz), 1.77 (1H, m), 2.07 (1H, m), 2.33 (1H, m), 2.87 (1H, m), 3.46-3.79 (6H, m), 4.22 (1H, m), 4.95 (1H, d, J=6.8 Hz), 5.49 (1H, d, J=2.9 Hz), 6.83 (1H, d, J=8.1 Hz), 6.96 (1H, d, J=8.1 Hz), 7.01-7.04 (2H, m), 7.32 (1H, t, J=8.1 Hz), 7.61 (1H, t, J=8.1 Hz).
MS (ESI) m/z: 784 [(M+H)$^+$].

Step 2: 7-[(5R)-1-{[(5R,6S)-6-(4-chloro-2-fluorophenyl)-5-(4-chloro-3-fluorophenyl)-3-isopropyl-6-methyl-5,6-dihydroimidazo [2,1-b] [1,3] thiazol-2-yl] carbonyl}-5-methyl-L-prolyl]-4,7-diazaspiro [2.5] octane

The compound obtained in Step 1 above was reacted in the same way as in Step 4 of Example 27 to give the title compound as a colorless solid.
$^1$H-NMR (400 MHz, DMSO-$d_6$, 100˚C) δ: 0.49-0.51 (4H, m), 0.79 (3H, d, J=7.1 Hz), 0.94 (3H, d, J=7.1 Hz), 1.19 (3H, d, J=6.3 Hz), 1.55 (1H, m), 1.72 (3H, d, J=1.2 Hz), 1.77 (1H, m), 2.04 (1H, m), 2.20-2.38 (2H, m), 2.80-2.85 (3H, m), 3.25-3.52 (4H, m), 4.21 (1H, m), 4.91 (1H, d, J=6.8 Hz), 5.47 (1H, d, J=2.9 Hz), 6.83 (1H, d, J=7.6 Hz), 6.97 (1H, d, J=7.6 Hz), 7.01-7.05 (2H, m), 7.32 (1H, t, J=8.4 Hz), 7.62 (1H, t, J=8.4 Hz).
MS (ESI) m/z: 688 [(M+H)$^+$].

Example 80

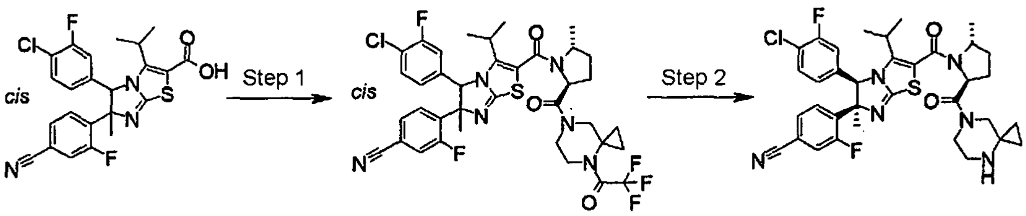


Step 1: 4-[(5R*,6S*)-5-(4-chloro-3-fluorophenyl)-3-isopropyl-6-methyl-2-{[(2R,5S)-2-methyl-5-{[4-(trifluoroacetyl)-4,7-diazaspiro[2.5]oct-7-yl]carbonyl}pyrrolidin-1-yl]carbonyl}-5,6-dihydroimidazo [2,1-b] [1,3] thiazol-6-yl] -3-fluorobenzonitrile

The compound obtained in Step 11 of Reference Example 40 was reacted in the same way as in Step 5 of Example 1 using the compound obtained in Step 2 of Reference Example 8 instead of the compound obtained in Step 2 of Reference Example 1 to give the title compound as a colorless solid.
$^1$H-NMR (400 MHz, DMSO-$d_6$, 100˚C) δ: 0.92-0.93 (3H, m), 0.97 (3H, d, J=7.1 Hz), 1.01-1.06 (4H, m), 1.13-1.20 (3H, m), 1.60-1.62 (1H, m), 1.73 (3H, d, J=1.0 Hz), 1.78 (1H, brs), 2.08-2.19 (1H, m), 2.27-2.30 (1H, m), 2.68-2.73 (1H, m), 3.56-3.77 (4H, m), 4.20-4.34 (1H, m), 4.94 (1H, d, J=8.3 Hz), 5.52-5.55 (1H, m), 6.86 (1H, d, J=9.0 Hz), 6.97 (1H, d, J=9.5 Hz), 7.30-7.32 (1H, m), 7.42-7.46 (2H, m), 7.78-7.83 (1H, m).
MS (ESI) m/z: 775 [(M+H)$^+$].

Step 2: 4-[(5R,6S)-5-(4-chloro-3-fluorophenyl)-2-{[(2S,5R)-2-(4,7-diazaspiro[2.5]oct-7-ylcarbonyl)-5-methylpyrrolidin-1-yl]carbonyl}-3-isopropyl-6-methyl-5,6-dihydroimidazo [2,1-b] [1,3] thiazol-6-yl]-3-fluorobenzonitrile

The compound obtained in Step 1 above was reacted in the same way as in Step 4 of Example 27 and the obtained mixture of diastereomers was resolved and purified on an optically active column [CHIRALCEL IC-H (Daicel Chemical Industries, Ltd.)] to give the title compound as a colorless solid.
$^1$H-NMR (400 MHz, DMSO-$d_6$, 100˚C) δ: 0.47 (4H, brs), 0.93 (3H, d, J=7.1 Hz), 0.95 (3H, d, J=7.1 Hz), 1.14 (3H, d, J=6.3 Hz), 1.27-1.32 (1H, m), 1.55-1.60 (1H, m), 1.73 (3H, d, J=1.2 Hz), 1.76-1.78 (1H, m), 2.06-2.11 (1H, m), 2.29-2.31 (1H, m), 2.69-2.72 (1H, m), 2.75-2.76 (2H, m), 3.29 (2H, brs), 3.38-3.40 (1H, m), 3.45-3.48 (1H, m), 4.29-4.31 (1H, m), 4.89 (1H, d, J=8.3 Hz), 5.50 (1H, d, J=3.2 Hz), 6.84 (1H, d, J=8.3 Hz), 6.99 (1H, d, J=8.3 Hz), 7.31 (1H, t, J=8.3 Hz), 7.42-7.45 (2H, m), 7.84 (1H, t, J=7.9 Hz).
MS (ESI) m/z: 679 [(M+H)$^+$].

Reference Example 1

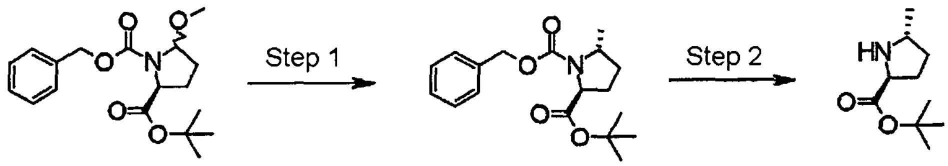


Step 1: 1-benzyl 2-tert-butyl(2S,5R)-5-methylpyrrolidine-1,2-dicarboxylate

Methyllithium (0.98 M diethyl ether solution) (59 ml, 58.0 mmol) was added dropwise to a mixture of a copper bromide-dimethyl sulfide complex (11.7 g, 58.0 mmol) in diethyl ether (100 ml) at -78°C in a nitrogen atmosphere. The resulting mixture was stirred at the same temperature for 30 minutes, then a boron trifluoride-diethyl ether complex (7.35 ml, 58.0 mmol) was added dropwise and then a diethyl ether (30 ml) solution of 1-benzyl 2-tert-butyl(2S)-5-methoxypyrrolidine-1,2-dicarboxylate (Synlett, 1996, 441) (9.6 g, 29.0 mmol) was added dropwise. The resulting solution was gradually warmed to room temperature and stirred for 16 hours. Then the reaction mixture was filtered, 1 N aqueous hydrochloric acid solution (100 ml) was added to the filtrate and the resulting mixture was stirred for 30 minutes. After extraction with ethyl acetate, the organic layer was washed with brine and then dried over anhydrous magnesium sulfate. The solvent was evaporated under reduced pressure and the residue obtained was purified by silica gel column chromatography [n-hexane:ethyl acetate = 4:1 (v/v)] to give the title compound (8.68 g, 94%) as a colorless oil.
$^1$H-NMR (400 MHz, $CDCl_3$) δ: 1.15 and 1.23 (total 3H, each d, J=6.3 Hz), 1.33 and 1.44 (total 9H, each s), 1.51-1.57 (1H, m), 1.89-1.95 (1H, m), 2.09-2.29 (2H, m), 4.13-4.28 (2H, m), 5.02-5.22 (2H, m), 7.22-7.38 (5H, m).
MS (ESI) m/z: 342 [(M+Na$^+$)].

Step 2: tert-butyl(5R)-5-methyl-L-prolinate

10% palladium carbon (400 mg) was added to a methanol (50 ml) solution of the compound obtained in Step 1 above (4.0 g, 13.0 mmol) and the resulting mixture was stirred at room temperature for 16 hours in a hydrogen atmosphere. The catalyst was removed by filtration, the filtrate was concentrated under reduced pressure and then the residue obtained was purified by silica gel column chromatography [chloroform:methanol = 30:1 (v/v)] to give the title compound (2.17 g, 90%) as a colorless solid.
$^1$H-NMR (400 MHz, $CDCl_3$) δ: 1.22 (3H, d, J=6.1 Hz), 1.38-1.43 (1H, m), 1.47 (9H, s), 1.80-1.88 (1H, m), 1.89-1.95 (1H, m), 2.23-2.31 (1H, m), 3.38-3.46 (1H, m), 3.87 (1H, dd, J=8.7, 6.2 Hz).
MS (ESI) m/z: 186 [(M+H)$^+$].

Reference Example 2

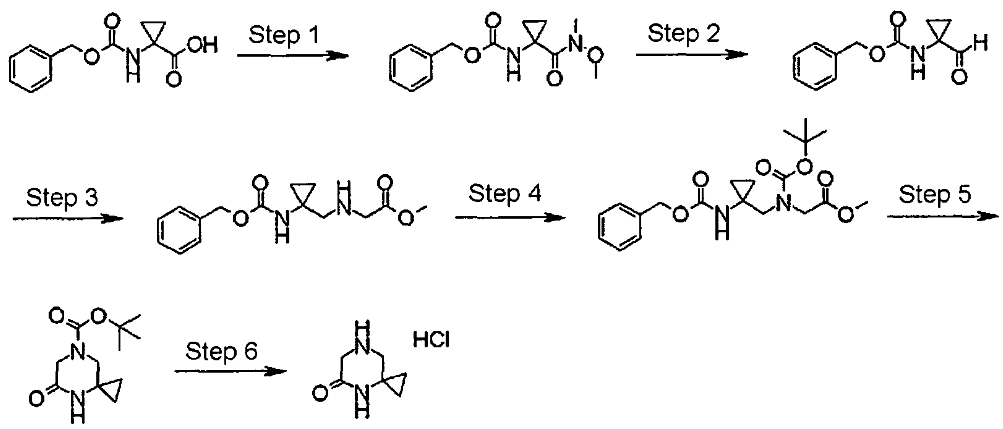


Step 1: benzyl {1-[methoxy(methyl)carbamoyl]cyclopropyl}carbamate

1-{[(benzyloxy)carbonyl]amino}cyclopropanecarboxylic acid was reacted in the same way as in Step 7 of Example 1 using N,O-dimethylhydroxyamine hydrochloride instead of 4,7-diazaspiro[2.5]octane hydrochloride to give the title compound as a colorless solid.
$^1$H-NMR (400 MHz, $CDCl_3$) δ: 1.08 (2H, brs), 1.47-1.50 (2H, m), 3.15 (3H, s), 3.65 (3H, s), 5.12 (2H, s), 5.48 (1H, br), 7.30-7.39 (5H, m).

Step 2: benzyl(1-formylcyclopropyl)carbamate

A tetrahydrofuran (160 ml) solution of the compound (8.00 g, 28.7 mmol) obtained in Step 1 above was cooled on ice. Subsequently, lithium aluminum hydride (1.65 g, 43.5 mmol) was added in small moieties and the resulting mixture was stirred at the same temperature for 30 minutes. Water (1.65 ml), 15% aqueous sodium hydroxide solution (1.65 ml), and water (4.95 ml) were added and the resulting mixture was returned to room temperature and stirred for 1 hour. Insoluble matter was removed by filtration and the residue was concentrated to give the title compound (5.60 g, 89%) as a colorless solid.
$^1$H-NMR (400 MHz, $CDCl_3$) δ: 1.29-1.55 (4H, m), 5.16 (2H, s), 5.45 (1H, brs), 7.27-7.38 (5H, m), 9.13 (1H, s).

Step 3: methyl N-[(1-{[(benzyloxy)carbonyl]amino}cyclopropyl)methyl]glycinate

Methyl glycinate hydrochloride was reacted in the same way as in Example 2 using the compound obtained in Step 2 above instead of 37% aqueous formaldehyde solution to give the title compound as a pale yellow oil.
$^1$H-NMR (400 MHz, $CDCl_3$) δ: 0.68-0.71 (2H, m), 0.81-0.84 (2H, m), 2.72 (2H, s), 3.43 (2H, s), 3.71 (3H, s), 5.08 (2H, s), 5.39 (1H, br), 7.30-7.36 (5H, m).
MS (ESI) m/z: 293 [$(M+H)^+$].

Step 4: methyl N-[(1-{[(benzyloxy) carbonyl]amino}cyclopropyl)methyl]-N-(tert-butyloxycarbonyl)glycinate

The compound (3.50 g, 12 mmol) obtained in Step 3 above was dissolved in tetrahydrofuran (70 ml), di-tert-butyl dicarbonate (3.0 ml, 13 mmol) was added and the resulting mixture was stirred at room temperature for 18 hours. The reaction mixture was concentrated and then the residue obtained was purified by silica gel column chromatography [ethyl acetate:n-hexane = 1:4 (v/v)] to give the title compound (4.26 g, 90%) as a colorless oil. $^1$H-NMR (400 MHz, $CDCl_3$) δ: 0.64-0.81 (4H, m), 1.43, 1.56 (total 9H, each s), 3.41, 3.45 (total 2H, each s), 3.72, 3.74 (total 3H, each s), 3.90, 3.96 (total 2H, each s), 5.76, 5.97 (total 1H, each br), 7.30-7.40 (5H, m).

Step 5: tert-butyl 5-oxo-4,7-diazaspiro[2.5]octane-7-carboxylate

5% palladium carbon (1.0 g) was added to an ethanol (100 ml) solution of the compound (4.25 g, 10.8 mmol) obtained in Step 4 above and the resulting mixture was stirred at room temperature for 4 hours in a hydrogen atmosphere. Insoluble matter was removed by filtration through celite and then the residue obtained was dissolved in toluene (100 ml). 1,8-diazabicyclo[5.4.0]undec-7-ene (0.80 ml, 5.36 mmol) was added and the resulting mixture was stirred at 100°C

for 4 hours. The reaction mixture was left standing to cool and then an aqueous solution of saturated ammonium chloride was added, followed by extraction with ethyl acetate. The organic layer was washed with water and brine and then dried over anhydrous sodium sulfate, the drying agent was removed by filtration and the solvent was concentrated under reduced pressure. Diisopropyl ether was added to the obtained residue and the precipitated solid was collected by filtration to give the title compound (1.58 g, 65%) as a colorless solid. $^1$H-NMR (400 MHz, $CDCl_3$) δ: 0.79-0.99 (4H, m), 1.52 (9H, s), 3.47 (2H, s), 4.14 (2H, s), 6.96 (1H, br).
MS (ESI) m/z: 249 [$(M+Na)^+$].

Step 6: 4,7-diazaspiro[2.5]octan-5-one hydrochloride

The compound (1.50 g, 6.63 mmol) obtained in Step 5 above was dissolved in dichloromethane (10 ml), trifluoroacetic acid (5 ml) was added dropwise at room temperature and the resulting mixture was stirred for 1 hour. The reaction mixture was concentrated and then the residue obtained was subjected to azeotropic distillation with 1 N hydrochloric acid/ethanol and then dried under reduced pressure to give the title compound (1.05 g, quantitative) as a colorless solid.
$^1$H-NMR (400 MHz, DMSO-$d_6$) δ: 0.80-0.88 (4H, m), 3.22 (2H, s), 3.66 (2H, s), 8.47 (1H, s), 10.04 (2H, br).
MS (ESI) m/z: 127 [$(M+H)^+$].

Reference Example 3

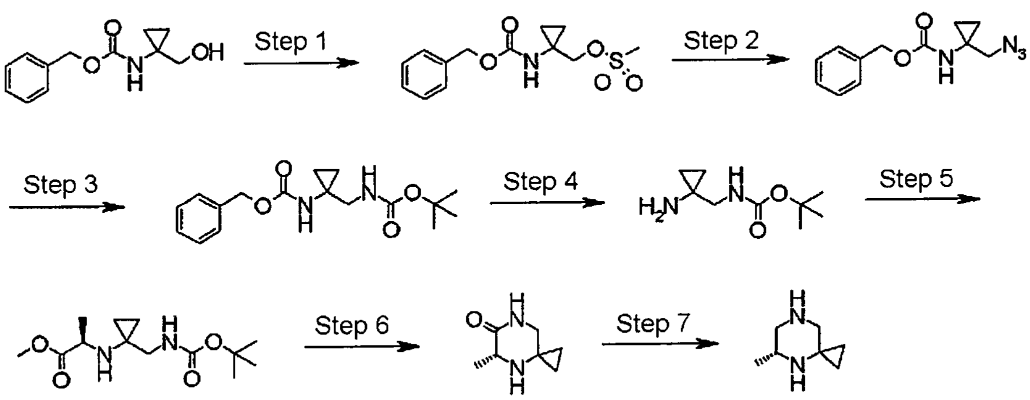


Step 1: (1-{[(benzyloxy)carbonyl]amino}cyclopropyl)methylmethanesulf onate

Methanesulfonyl chloride (2.3 ml, 30.0 mmol) was added dropwise to a 1,2-dichloromethane (80 ml) solution of benzyl [1-(hydroxymethyl)cyclopropyl]carbamate (US2001/827292) (5.4 g, 25.0 mmol) and triethylamine (5.2 ml, 38.0 mmol) under ice cooling. The resulting mixture was stirred at room temperature for 1 hour and then diluted with chloroform and the organic layer was washed with saturated aqueous sodium bicarbonate solution and brine and dried over anhydrous sodium sulfate. The solvent was evaporated under reduced pressure to give the title compound as a pale orange oil. This compound was used in the next reaction without being purified.

Step 2: benzyl [1-(azidomethyl)cyclopropyl]carbamate

Sodium azide (3.26 g, 50.0 mmol) was added to a dimethylformamide (100 ml) solution of the compound obtained in Step 1 above and the resulting mixture was stirred under heating at 70˚C for 2 days. The reaction mixture was left standing to cool and then water was added to the reaction mixture, followed by extraction with ethyl acetate. The organic layer was washed with water, saturated aqueous sodium bicarbonate solution, and brine and dried over anhydrous sodium sulfate. The solvent was evaporated under reduced pressure and the residue obtained was purified by silica gel column chromatography [n-hexane:ethyl acetate = 4:1 (v/v)] to give the title compound (4.26 g, 69%) as a colorless oil. $^1$H-NMR (400 MHz, $CDCl_3$) δ: 0.81-0.85 (2H, m), 0.87-0.92 (2H, m), 3.40 (2H, s), 5.10 (2H, brs), 5.26 (1H, brs), 7.31-7.39 (5H, m).

MS (ESI) m/z: 269 [$(M+H)^+$].

Step 3: benzyl (1-{[(tert-butyloxycarbonyl)amino]methyl}cyclopropyl)carbamate

Triphenylphosphine (8.9 g, 34.0 mmol) was added to a mixture of the compound (4.2 g, 17.0 mmol) obtained in Step 2 above in tetrahydrofuran (60 ml) and water (30 ml) and the resulting mixture was stirred at room temperature for 3 hours. Next, triethylamine (4.7 ml, 34.0 mmol) and di-tert-butyl dicarbonate (5.9 ml, 26.0 mmol) were added at room temperature and the resulting mixture was stirred for 2 hours. The reaction mixture was diluted with ethyl acetate and the organic layer was washed with brine and dried over anhydrous magnesium sulfate. The solvent was evaporated under reduced pressure and the residue obtained was purified by silica gel column chromatography [n-hexane:ethyl acetate = 2:1 (v/v)] to give the title compound (4.63 g, 85%) as a colorless solid.
$^1$H-NMR (400 MHz, $CDCl_3$) δ: 0.82 (4H, s), 1.43 (9H, s), 3.24 (2H, brs), 5.08 (2H, brs), 5.19 (1H, brs), 5.31 (1H, brs), 7.32-7.38 (5H, m).
MS (ESI) m/z: 221 [$(M-99)^+$].

Step 4: tert-butyl [(1-aminocyclopropyl)methyl]carbamate

The compound obtained in Step 3 above was reacted in the same way as in Step 2 of Reference Example 1 to give the title compound as a colorless oil.
$^1$H-NMR (400 MHz, $CDCl_3$) δ: 0.49-0.51 (2H, m), 0.59-0.62 (2H, m), 1.46 (9H, s), 3.12 (2H, d, J=6.1 Hz), 4.80 (1H, brs).
MS (ESI) m/z: 187 [$(M+H)^+$].

Step 5: methyl N-(1-{[(tert-butyloxycarbonyl)amino]methyl}cyclopropyl)-D-alaninate

Triflate anhydride (3.1 ml, 18.0 mmol) and 2,6-lutidine (3.56 ml, 20.0 mmol) were added dropwise to a dichloromethane (50 ml) solution of methyl L-(-)-lactate (1.6 ml, 17.0 mmol) under ice cooling and the resulting mixture was stirred for 10 minutes. Next, a dichloromethane (30 ml) solution of tert-butyl [(1-aminocyclopropyl)methyl]carbamate and triethylamine (2.86 ml, 21.0 mmol) was added dropwise at the same temperature and the resulting mixture was stirred for 1 hour and then further stirred at room temperature for 6 hours. The reaction mixture was diluted with chloroform, washed with saturated aqueous sodium bicarbonate solution and brine, and dried over anhydrous sodium sulfate. The solvent was evaporated under reduced pressure and the residue obtained was purified by silica gel column chromatography [n-hexane:ethyl acetate = 4:1 (v/v)] to give the title compound as a crude product.

Step 6: (5R)-5-methyl-4,7-diazaspiro[2.5]octan-6-one

Trifluoroacetic acid (15 ml) was added to a chloroform (10 ml) solution of the compound (4.6 g) obtained in Step 5 above and the resulting mixture was stirred at room temperature for 16 hours. The solvent was evaporated under reduced pressure and then the residue was subjected to azeotropic distillation by the addition of toluene. The residue obtained was dissolved in 1,2-dichloroethane (50 ml), 5 N aqueous sodium hydroxide solution (5 ml) was added and the resulting mixture was stirred at room temperature for 30 minutes. Potassium carbonate (20 g) was added to this mixture and the resulting mixture was further stirred at room temperature for 30 minutes. Insoluble matter was removed by filtration, then the filtrate was evaporated under reduced pressure and the residue obtained was solidified with diethyl ether to give the title compound (1.03 g, 52%) as a colorless solid.
$^1$H-NMR (400 MHz, $CDCl_3$) δ: 0.58-0.65 (2H, m), 0.74-0.78 (1H, m), 0.83-0.88 (1H, m), 1.39 (3H, d, J=7.1 Hz), 1.74 (1H, brs), 2.81-2.85 (1H, m), 3.57 (1H, q, J=7.1 Hz), 3.77 (1H, d, J=12.0 Hz), 6.39 (1H, brs).
MS (ESI) m/z: 141 [$(M+H)^+$].

Step 7: (5R)-5-methyl-4,7-diazaspiro[2.5] octane dihydrochloride

A borane-tetrahydrofuran complex (0.93 M tetrahydrofuran solution) (39 ml) was added dropwise to a tetrahydrofuran (20 ml) suspension of the compound (1.0 g, 7.13 mmol) obtained in Step 6 above under ice cooling in a nitrogen atmosphere and the resulting mixture was stirred at room temperature for 20 hours. The reaction mixture was cooled on ice, then methanol (20 ml) was gradually added and the resulting mixture was stirred at the same temperature for 20 minutes. The reaction solvent was evaporated under reduced pressure and the residue obtained was dissolved in ethanol (30 ml). Water (5 ml) and triethylamine (10 ml) were added and the resulting mixture was stirred under heating at 90°C for 3 hours. The reaction mixture was left standing to cool, then the reaction solvent was evaporated under reduced pressure and the residue obtained was purified by silica gel column chromatography [chloroform:methanol = 20:1 (v/v)]. The purified product obtained was subjected to azeotropic distillation with 1 N hydrochloric acid/ethanol and

then dried under reduced pressure to give the title compound (660 mg, 46%) as a colorless solid.
$^1$H-NMR (400 MHz, DMSO-$d_6$) δ: 0.93-1.06 (2H, m), 1.16-1.22 (1H, m), 1.25-1.30 (1H, m), 1.32 (3H, d, J=6.6 Hz), 2.98 (1H, d, J=13.4 Hz), 3.09 (1H, t, J=12.5 Hz), 3.47 (1H, dd, J=13.7, 2.9 Hz), 3.59 (2H, d, J=13.7 Hz).
MS (ESI) m/z: 126 [(M+Na)$^+$].

Reference Example 4

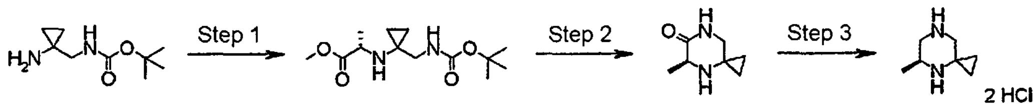


Step 1: methyl N-(1-{[(tert-butyloxycarbonyl)amino]methyl}cyclopropyl)-L-alaninate

The compound obtained in Step 4 of Reference Example 3 was reacted in the same way as in Step 5 of Reference Example 3 using methyl D-(+)-lactate instead of methyl L-(-)-lactate to give the title compound as a colorless oil.
$^1$H-NMR ($CDCl_3$) δ: 0.45-0.66 (4H, m), 1.25 (3H, d, J=7.7 Hz), 1.45 (9H, s), 2.04 (1H, brs), 2.93-3.09 (2H, m), 3.49-3.58 (1H, m), 3.73 (3H, s), 5.07 (1H, brs)

Step 2: (5S)-5-methyl-4,7-diazaspiro[2.5]octan-6-one

The compound obtained in Step 1 above was reacted in the same way as in Step 6 of Reference Example 3 to give the title compound as a colorless solid.
$^1$H-NMR (400 MHz, $CDCl_3$) δ: 0.57-0.66 (2H, m), 0.72-0.79 (1H, m), 0.82-0.88 (1H, m), 1.39 (3H, d, J=7.1 Hz), 1.84 (1H, brs), 2.84 (1H, dd, J=12.0, 3.4 Hz), 3.56 (1H, q, J=7.1 Hz), 3.76 (1H, d, J=12.0 Hz), 7.04 (1H, brs).

Step 3: (5S)-5-methyl-4,7-diazaspiro[2.5]octane dihydrochloride

The compound obtained in Step 2 above was reacted in the same way as in Step 7 of Reference Example 3 to give the title compound as a colorless oil.
$^1$H-NMR (400 MHz, DMSO-$d_6$) δ: 0.91-1.06 (2H, m), 1.16-1.30 (2H, m), 1.32 (3H, d, J=6.3 Hz), 2.99 (1H, d, J=13.4 Hz), 3.09 (1H, t, J=12.6 Hz), 3.45-3.52 (1H, m), 3.53-3.63 (2H, m), 9.53-10.76 (4H, m).

Reference Example 5

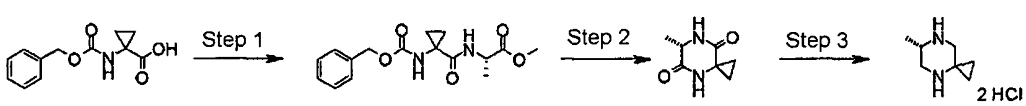


Step 1: methyl N-[(1-{[(benzyloxy)carbonyl]amino}cyclopropyl)carbonyl]-L-alaninate

1-{[(benzyloxy)carbonyl]amino}cyclopropanecarboxylic acid was reacted in the same way as in Step 7 of Example 1 using L-alanine methyl ester hydrochloride instead of 4,7-diazaspiro[2.5]octane hydrochloride to give the title compound as a colorless oil.
$^1$H-NMR (400 MHz, $CDCl_3$) δ: 1.00-1.12 (2H, m), 1.29-1.41 (2H, m), 1.53-1.66 (3H, m), 3.73 (3H, s), 4.51-4.60 (1H, m), 5.15 (2H, s), 5.31 (1H, brs), 6.90 (1H, d, J=6.3 Hz), 7.31-7.38 (5H, m).

Step 2: methyl N-[(1-aminocyclopropyl)carbonyl]-L-alaninate

The compound obtained in Step 1 above was reacted in the same way as in Step 5 of Reference Example 2 to give the title compound as a colorless solid.

$^1$H-NMR [400 MHz, $CDCl_3$-$CD_3OD$ (1: 1, v/v)] δ: 1.01-1.11 (2H, m), 1.39-1.49 (2H, m), 1.51 (3H, d, J=7.0 Hz), 4.11 (1H, q, J=7.0 Hz).

Step 3: (6S)-6-methyl-4,7-diazaspiro[2.5]octane dihydrochloride

The compound obtained in Step 2 above was reacted in the same way as in Step 7 of Reference Example 3, then subjected to azeotropic distillation with 4 N hydrochloric acid/dioxane, and then dried under reduced pressure to give the title compound as a colorless solid. $^1$H-NMR (400 MHz, DMSO-$d_6$) δ: 0.93-1.05 (2H, m), 1.13-1.25 (2H, m), 1.34 (3H, d, J=6.6 Hz), 2.97-3.06 (2H, m), 3.51 (1H, dd, J=13.2, 2.9 Hz), 3.55-3.66 (2H, m), 9.58 (1H, brs), 10.12 (3H, brs).

Reference Example 6

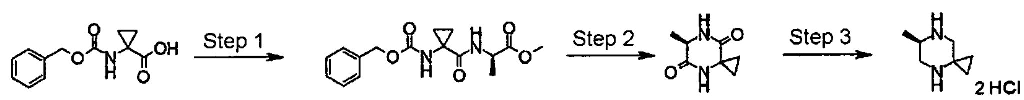


Step 1: methyl N-[(1-{[(benzyloxy)carbonyl]amino}cyclopropyl)carbonyl]-D-alaninate

1-{[(benzyloxy)carbonyl]amino}cyclopropanecarboxylic acid was reacted in the same way as in Step 7 of Example 1 using D-alanine methyl ester hydrochloride instead of 4,7-diazaspiro[2.5]octane hydrochloride to give the title compound as a colorless oil.
$^1$H-NMR (400 MHz, $CDCl_3$) δ: 1.00-1.12 (2H, m), 1.30-1.42 (3H, m), 1.53-1.66 (2H, m), 3.73 (3H, s), 4.51-4.60 (1H, m), 5.15 (2H, s), 5.30 (1H, brs), 6.85-6.94 (1H, m), 7.32-7.40 (5H, m).

Step 2: methyl N-[(1-aminocyclopropyl)carbonyl]-D-alaninate

The compound obtained in Step 1 above was reacted in the same way as in Step 5 of Reference Example 2 to give the title compound as a colorless solid.
$^1$H-NMR (400 MHz, $CDCl_3$-$CD_3OD$ (10: 1, v/v)) δ: 1.00-1.10 (2H, m), 1.42-1.52 (2H, m), 1.52 (3H, d, J=7.0 Hz), 4.12 (1H, q, J=7.0 Hz).

Step 3: (6R)-6-methyl-4,7-diazaspiro[2.5]octane dihydrochloride

The compound obtained in Step 2 above was reacted in the same way as in Step 3 of Reference Example 5 to give the title compound as a colorless solid.
$^1$H-NMR (400 MHz, DMSO-$d_6$) δ: 0.95-1.05 (2H, m), 1.12-1.28 (2H, m), 1.35 (3H, d, J=6.3 Hz), 2.98-3.07 (2H, m), 3.50 (1H, dd, J=13.3, 3.0 Hz), 3.56-3.68 (2H, m), 9.71 (1H, brs), 10.33 (3H, brs).

Reference Example 7

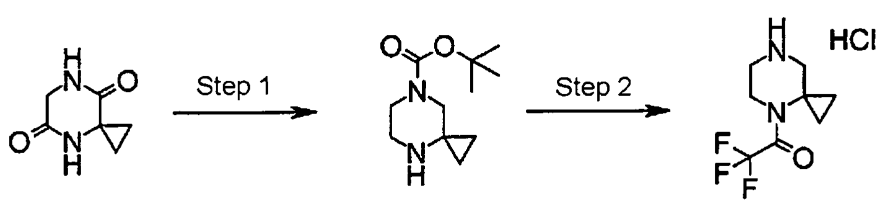


Step 1: tert-butyl 4,7-diazaspiro[2.5]octane-7-carboxylate

4,7-diazaspiro[2.5]octane-5,8-dione (WO2004/094407) was reacted in the same way as in Step 7 of Reference

Example 3 and then reacted in the same way as in Step 4 of Reference Example 2 to give the title compound as a colorless oil.
$^1$H-NMR (400 MHz, $CDCl_3$) δ: 0.55-0.59 (4H, m), 1.46 (9H, s), 2.88-2.92 (2H, m), 3.25 (2H, s), 3.37-3.41 (2H, m).

Step 2: 4-(trifluoroacetyl)-4,7-diazaspiro[2.5]octane hydrochloride

Pyridine (500 μl) and trifluoroacetic anhydride (786 μl, 5.64 mmol) were added to a dichloromethane (10 ml) solution of the compound (1.0 g, 4.70 mmol) obtained in Step 1 above under ice cooling and the resulting mixture was stirred for 15 minutes. After completion of the reaction, trifluoroacetic acid (6 ml) was added and the resulting mixture was stirred at room temperature for 1 hour. The reaction solvent was evaporated under reduced pressure and then the residue was subjected to azeotropic distillation with toluene. The residue was diluted with chloroform and the organic layer was washed with saturated aqueous sodium bicarbonate solution and brine and dried over anhydrous sodium sulfate. The solvent was evaporated under reduced pressure and the residue obtained was purified by silica gel column chromatography [chloroform:methanol = 40:1 (v/v)]. The residue obtained was subjected to azeotropic distillation with 4 N hydrochloric acid/dioxane and then dried under reduced pressure to give the title compound (751 mg, 65%) as a colorless solid.
$^1$H-NMR (400 MHz, DMSO-$d_6$) δ: 1.15-1.21 (4H, m), 3.14-3.19 (2H, m), 3.24 (2H, t, J=5.4 Hz), 3.84-3.91 (2H, m).
MS (ESI) m/z: 209 [(M+H)+].

Reference Example 8

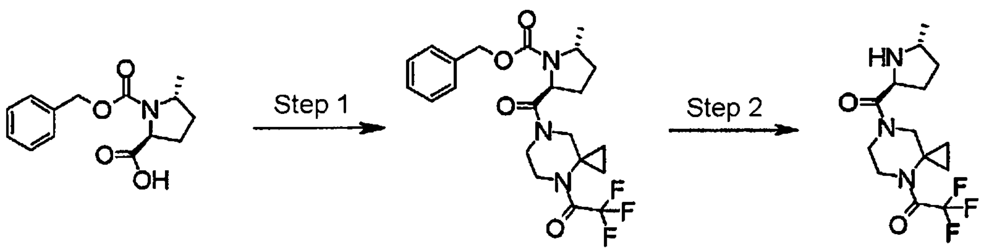


Step 1: benzyl (2R,5S)-2-methyl-5-{[4-(trifluoroacetyl)-4,7-diazaspiro[2.5]oct-7-yl]carbonyl}pyrrolidine-1-carboxylate

(5R)-1-[(benzyloxy)carbonyl]-5-methyl-L-proline was reacted in the same way as in Step 7 of Example 1 using the compound obtained in Step 2 of Reference Example 7 instead of 4,7-diazaspiro[2.5]octane hydrochloride to give the title compound as a colorless solid.
$^1$H-NMR (400 MHz, $CDCl_3$) δ: 0.88-1.28 (7H, m), 1.53-1.57 (1H, m), 1.70-1.77 (1H, m), 2.18-2.35 (2H, m), 3.35-3.80 (6H, m), 4.21-4.29 (1H, m), 4.53-4.73 (1H, m), 5.08 (1H, d, J=12.5 Hz), 5.20 (1H, d, J=12.5 Hz), 7.27-7.35 (5H, m). MS (ESI) m/z: 454 [$(M+H)^+$].

Step 2: 7-[(5R)-5-methyl-L-prolyl]-4-(trifluoroacetyl)-4,7-diazaspiro[2.5] octane

The compound obtained in Step 1 above was reacted in the same way as in Step 2 of Reference Example 1 to give the title compound as a colorless solid.
$^1$H-NMR (400 MHz, DMSO-$d_6$) δ: 0.99-1.17 (4H, m), 1.26 (3H, d, J=6.6 Hz), 1.47-1.55 (1H, m), 1.72-1.80 (1H, m), 1.97-2.04 (1H, m), 2.30-2.36 (1H, m), 3.50-3.58 (2H, m), 3.60-3.69 (2H, m), 3.70-3.81 (3H, m), 4.39-4.47 (1H, m).
MS (ESI) m/z: 320 [(M+H)+].

Reference Example 9

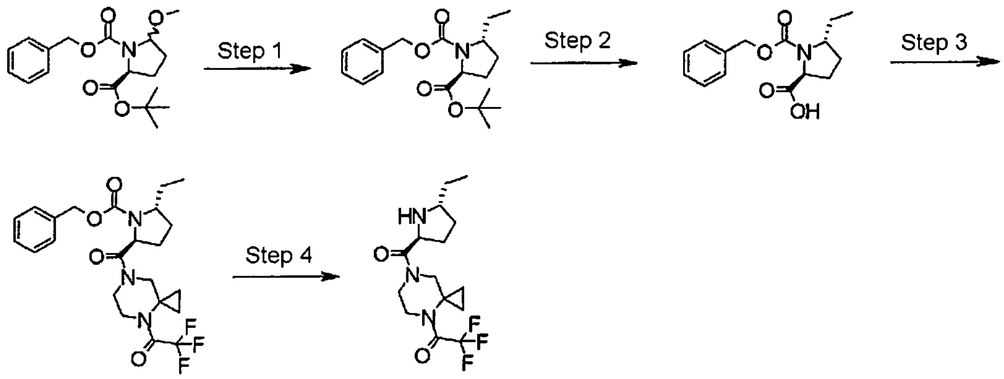


Step 1: 1-benzyl 2-tert-butyl(2S,5R)-5-ethylpyrrolidine-1,2-dicarboxylate

1-benzyl 2-tert-butyl(2S)-5-methoxypyrrolidine-1,2-dicarboxylate was reacted in the same way as in Step 1 of Reference Example 1 using ethyllithium instead of methyllithium to give the title compound as a colorless oil.
$^1$H-NMR (400 MHz, $CDCl_3$) δ: 0.83 and 0.89 (total 3H, each t, J=7.4 Hz), 1.33 and 1.44 (total 9H, each s), 1.66-1.73 (2H, m), 1.83-1.94 (2H, m), 1.99-2.07 (1H, m), 2.15-2.23 (1H, m), 3.92-3.99 (1H, m), 4.24 (1H, t, J=7.8 Hz), 5.06-5.20 (2H, m), 7.27-7.36 (5H, m).
MS (ESI) m/z: 356 [(M+Na)$^+$)].

Step 2: (5R)-1-[(benzyloxy)carbonyl]-5-ethyl-L-proline

The compound obtained in Step 1 above was reacted in the same way as in Step 6 of Example 1 to give the title compound as a pale yellow oil.
$^1$H-NMR (400 MHz, $CDCl_3$) δ: 0.85 and 0.89 (total 3H, each t, J=7.6 Hz), 1.31-1.48 (2H, m), 1.76 and 1.86 (total 1H, each m), 2.02-2.32 (3H, m), 3.91 and 3.99 (total 1H, each m), 4.41 (1H, dd, J=13.5, 8.9 Hz), 5.06-5.23 (2H, m), 7.20-7.38 (5H, m).
MS (ESI) m/z: 278 [(M+Na$^+$)].

Step 3: benzyl (2R,5S)-2-ethyl-5-{[4-(trifluoroacetyl)-4,7-diazaspiro[2.5]oct-7-yl]carbonyl}pyrrolidine-1-carboxylate

The compound obtained in Step 2 above was reacted in the same way as in Step 7 of Example 1 using the compound obtained in Step 2 of Reference Example 7 instead of 4,7-diazaspiro[2.5]octane hydrochloride to give the title compound as a pale yellow solid.
$^1$H-NMR (400 MHz, $CDCl_3$) δ: 0.83-0.93 (4H, m), 1.01-1.21 (3H, m), 1.36-1.41 (1H, m), 1.67-1.75 (3H, m), 2.09-2.26 (2H, m), 3.35-3.89 (6H, m), 3.97-4.11 (1H, m), 4.48-4.75 (1H, m), 5.03-5.21 (2H, m), 7.28-7.34 (5H, m).
MS (ESI) m/z: 468 [(M+H)$^+$].

Step 4: 7-[(5R)-5-ethyl-L-prolyl]-4-(trifluoroacetyl)-4,7-diazaspiro [2.5] octane

The compound obtained in Step 3 above was reacted in the same way as in Step 2 of Reference Example 1 to give the title compound as a pale yellow solid.
$^1$H-NMR (400 MHz, DMSO-$d_6$) δ: 0.94-1.12 (7H, m), 1.70-1.78 (3H, m), 1.85-1.92 (1H, m), 2.06-2.12 (1H, m), 2.31-2.47 (1H, m), 3.37-4.06 (7H, m), 4.46-4.64 (1H, m).
MS (ESI) m/z: 334 [(M+H)$^+$].

Reference Example 10

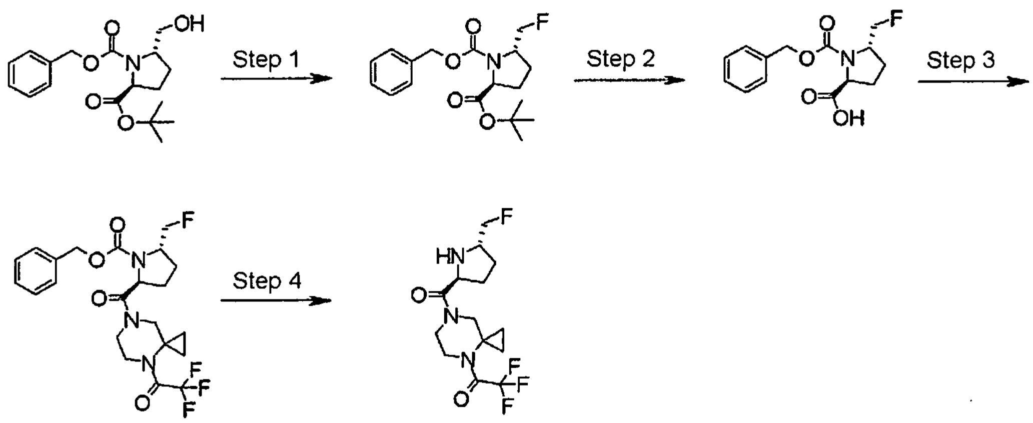


Step 1: 1-benzyl 2-tert-butyl(2S,5S)-5-(fluoromethyl)pyrrolidine-1,2-dicarboxylate

Triethylamine (1.2 ml, 8.58 mmol) and methanesulfonyl chloride (500 μl, 6.44 mmol) were added to a dichloromethane (20 ml) solution of 1-benzyl 2-tert-butyl(2S,5S)-5-(hydroxymethyl)pyrrolidine-1,2-dicarboxylate (WO9748706) (1.44 g, 4.29 mmol) at 0˚C under ice cooling and the resulting mixture was stirred for 30 minutes. The reaction mixture was diluted with chloroform and the organic layer was washed with 10% aqueous citric acid solution, saturated aqueous sodium bicarbonate solution, and brine and dried over anhydrous magnesium sulfate. The solvent was evaporated under reduced pressure, the residue obtained was dissolved in tetra-n-butylammonium fluoride (1 M tetrahydrofuran solution) (50 ml, 50.0 mmol) and the resulting mixture was heated to reflux at 70˚C for 16 hours. The reaction mixture was left standing to cool and then the reaction solvent was evaporated under reduced pressure, followed by extraction with ethyl acetate. The organic layer was washed with brine and then dried over anhydrous magnesium sulfate. The solvent was evaporated under reduced pressure and the residue obtained was purified by silica gel column chromatography [n-hexane:ethyl acetate = 4:1 (v/v)] to give the title compound (1.14 g, 79%) as a colorless oil.
$^1$H-NMR (400 MHz, $CDCl_3$) δ: 1.34 and 1.44 (total 9H, each s), 1.90-1.99 (2H, m), 2.11-2.32 (2H, m), 4.22-4.44 (3H, m), 4.54 and 4.67 (total 1H, each m), 5.07-5.20 (2H, m), 7.27-7.35 (5H, m).
MS (ESI) m/z: 360 ($[M+Na]^+$)

Step 2: (5S)-1-[(benzyloxy)carbonyl]-5-(fluoromethyl)-L-proline

The compound obtained in Step 1 above was reacted in the same way as in Step 6 of Example 1 to give the title compound as a colorless oil.
$^1$H-NMR (400 MHz, DMSO-$d_6$) δ: 1.85-1.94 (2H, m), 1.99-2.09 (1H, m), 2.19-2.32 (1H, m), 4.07-4.55 (4H, m), 5.04-5.13 (2H, m), 7.29-7.37 (5H, m).
MS (ESI) m/z: 304 ($[M+Na]^+$)

Step 3: benzyl (2S,5S)-2-(fluoromethyl)-5-{[4-(trifluoroacetyl)-4,7-diazaspiro[2.5]oct-7-yl]carbonyl}pyrrolidine-1-carboxylate

The compound obtained in Step 2 above was reacted in the same way as in Step 7 of Example 1 using the compound obtained in Step 2 of Reference Example 7 instead of 4,7-diazaspiro[2.5]octane hydrochloride to give the title compound as a pale yellow solid.
$^1$H-NMR (400 MHz, $CDCl_3$) δ: 0.68-1.23 (4H, m), 1.69-1.79 (1H, m), 1.94-2.01 (1H, m), 2.18-2.38 (2H, m), 3.29-3.91 (4H, m), 4.23-4.47 (2H, m), 4.51-4.88 (2H, m), 5.02-5.24 (2H, m), 7.27-7.36 (5H, m).
MS (ESI) m/z: 472 [$(M+H)^+$].

Step 4: 7-[(5S)-5-(fluoromethyl)-L-prolyl]-4-(trifluoroacetyl)-4,7-diazaspiro[2.5]octane

The compound obtained in Step 3 above was reacted in the same way as in Step 2 of Reference Example 1 to give the title compound as a colorless solid.
$^1$H-NMR (400 MHz, DMSO-$d_6$) δ: 0.95-1.16 (4H, m), 1.64-1.76 (2H, m), 1.96-2.05 (1H, m), 2.23-2.37 (1H, m), 3.47-3.56 (2H, m), 3.59-3.94 (5H, m), 4.36-4.43 (1H, m), 4.53 (2H, dd, J=47.2, 5.0 Hz).
MS (ESI) m/z: 338 [$(M+H)^+$].

Reference Example 11

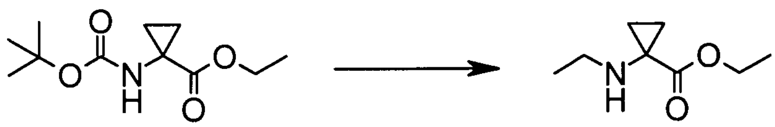

ethyl 1-(ethylamino)cyclopropanecarboxylate

Sodium hydride (55% oil) (1.90 g) was added to a dimethylformamide (50 ml) solution of ethyl 1-[(tert-butyloxycarbonyl)amino]cyclopropanecarboxylate (J. Med. Chem., 1988, 31, 2004) (1.0 g, 21.8 mmol) under ice cooling and the resulting mixture was stirred at room temperature for 30 minutes. Subsequently, p-toluenesulfonic acid ethyl ester (18.6 ml, 109 mmol) was added dropwise and the resulting mixture was stirred at 0˚C for 30 minutes and at room temperature for 2 hours. The reaction mixture was diluted with ethyl acetate, washed with 10% aqueous citric acid solution, saturated aqueous sodium bicarbonate solution, and brine, and then dried over anhydrous magnesium sulfate. The solvent was evaporated under reduced pressure and the residue obtained was purified by silica gel column chromatography [n-hexane:ethyl acetate = 4:1 (v/v)]. The ethyl form obtained (9.0 g) was dissolved in dichloromethane (90 ml). Trifluoroacetic acid (13.5 ml, 175 mmol) was added under ice cooling and the resulting mixture was stirred at room temperature for 16 hours. The solvent was evaporated under reduced pressure and the residue obtained was purified by silica gel column chromatography (ethyl acetate) to give the title compound (3.15 g, quantitative) as a yellow oil.
$^1$H-NMR (400 MHz, $CDCl_3$) δ: 1.29 (3H, t, J=7.1 Hz), 1.34 (3H, t, J=7.3 Hz), 1.48-1.53 (2H, m), 1.67-1.71 (2H, m), 3.27 (2H, q, J=7.3 Hz), 4.26 (2H, q, J=7.1 Hz).

Reference Example 12

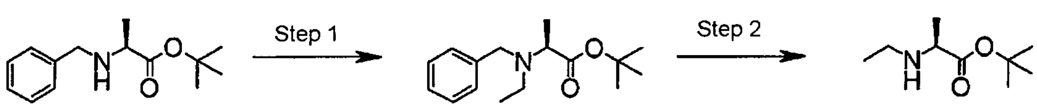


Step 1: tert-butyl N-benzyl-N-ethyl-L-alaninate

Tert-butyl N-benzyl-L-alaninate (WO2007/26920) was reacted in the same way as in Example 2 using acetaldehyde instead of 37% aqueous formaldehyde solution to give the title compound as a colorless oil.
$^1$H-NMR (400 MHz, $CDCl_3$) δ: 1.03 (3H, t, J=7.2 Hz), 1.24 (3H, d, J=7.1 Hz), 1.49 (10H, d, J=0.7 Hz), 2.52-2.61 (1H, m), 2.64-2.74 (1H, m), 3.42 (1H, q, J=7.1 Hz), 3.64 (1H, d, J=14.4 Hz), 3.88 (1H, d, J=14.4 Hz), 7.19-7.39 (5H, m).

Step 2: tert-butyl N-ethyl-L-alaninate

5% palladium carbon (1.20 g) was added to an ethanol (48 ml) solution of the compound (2.40 g, 9.11 mmol) obtained in Step 1 above and the resulting mixture was stirred at room temperature for 16 hours in a hydrogen atmosphere. The catalyst was removed by filtration and then the solvent was concentrated under reduced pressure to give the title compound (681 mg, 43%) as a colorless oil.
$^1$H-NMR (400 MHz, $CDCl_3$) δ: 1.29 (3H, t, J=7.2 Hz), 1.47 (3H, d, J=7.1 Hz), 1.49 (9H, s), 2.74-2.83 (1H, m), 2.83-2.92 (1H, m), 3.47 (1H, q, J=7.1 Hz), 5.72 (1H, brs).

Reference Example 13

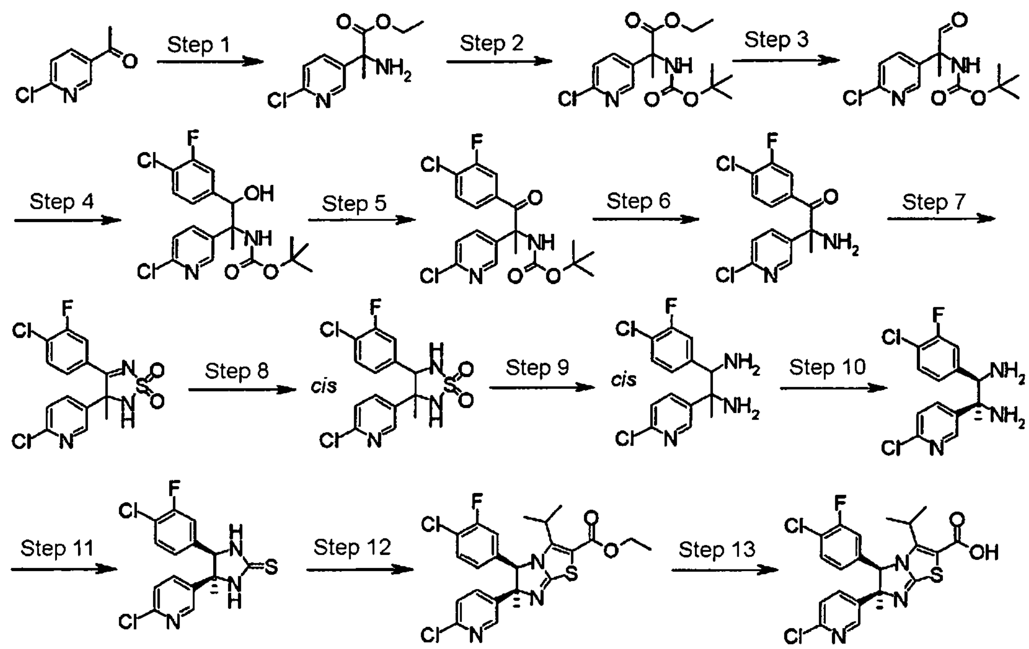


Step 1: ethyl 2-(6-chloropyridin-3-yl)propanoate

A methanol (200 ml) solution of ammonium chloride (6.88 g, 129 mmol) and 1-(6-chloropyridin-3-yl)ethanone (10.0 g, 64.3 mmol) was added to an aqueous concentrated ammonia solution (100 ml) of potassium cyanide (10.7 g, 161 mmol) under ice cooling and the resulting mixture was gradually returned to room temperature while being stirred for 3 days. The reaction mixture was concentrated under reduced pressure to approximately half volume and the residue was subjected to extraction with dichloromethane. The organic layer was dried over anhydrous magnesium sulfate and then the solvent was evaporated under reduced pressure. Concentrated hydrochloric acid (100 ml) was added to the residue obtained under ice cooling and then the resulting mixture was heated to reflux for 2 hours. The reaction mixture was concentrated under reduced pressure and then subjected to azeotropic distillation with toluene and thionyl chloride (10 ml) was added dropwise to an ethanol (100 ml) solution of the residue obtained under ice cooling. The resulting mixture was heated to reflux for 3 hours, then the reaction mixture was concentrated under reduced pressure and the residue obtained was diluted with dichloromethane and then washed with saturated aqueous sodium bicarbonate solution and brine. The organic layer was dried over anhydrous magnesium sulfate and the solvent was evaporated under reduced pressure. The residue obtained was purified by silica gel column chromatography (ethyl acetate) to give the title compound (7.24 g, 49%) as a pale yellow oil.
$^1$H-NMR (400 MHz, $CDCl_3$) δ: 1.25 (3H, t, J=7.1 Hz), 1.70 (3H, s), 1.98 (2H, brs), 4.19 (2H, q, J=7.1 Hz), 7.30 (1H, d, J=8.3 Hz), 7.85 (1H, dd, J=8.3, 2.4 Hz), 8.56 (1H, d, J=2.4 Hz).

Step 2: ethyl 2-[(tert-butoxycarbonyl)amino]-2-(6-chloropyridin-3-yl)propanoate

Triethylamine (1.22 ml, 8.75 mmol) and di-tert-butyl dicarbonate (1.12 ml, 4.81 mmol) were added to a tetrahydrofuran (20 ml) solution of the compound (1.00 g, 4.37 mmol) obtained in Step 1 above and the resulting mixture was heated to reflux for 18 hours. Di-tert-butyl dicarbonate (0.51 ml, 2.19 mmol) was further added and the resulting mixture was heated to reflux for 18 hours. The reaction mixture was concentrated under reduced pressure and then the residue obtained was diluted with ethyl acetate, washed with 10% aqueous citric acid solution and brine, and then dried over anhydrous magnesium sulfate. The solvent was evaporated under reduced pressure and the residue obtained was purified by silica gel column chromatography [n-hexane:ethyl acetate = 2:18 (v/v)] to give the title compound (1.20 g, 84%) as a pale yellow oil.
$^1$H-NMR (400 MHz, $CDCl_3$) δ: 1.19 (3H, t, J=7.2 Hz), 1.38 (9H, brs), 1.99 (3H, s), 4.08-4.26 (2H, m), 7.30 (1H, d, J=8.5 Hz), 7.74 (1H, dd, J=8.5, 2.7 Hz), 8.47 (1H, d, J=2.7 Hz)

Step 3: tert-butyl [1-(6-chloropyridin-3-yl)-1-methyl-2-oxoethyl]carbamate

A tetrahydrofuran (100 ml) solution of the compound (5.70 g, 17.3 mmol) obtained in Step 2 above was added dropwise to a tetrahydrofuran (20 ml) suspension of lithium aluminum hydride (1.43 g, 34.7 mmol) under ice cooling and the resulting mixture was stirred at the same temperature for 1 hour. 1 N aqueous sodium hydroxide solution (6 ml) was added to the reaction mixture under ice cooling and the precipitated insoluble matter was removed by filtration. The filtrate was concentrated under reduced pressure and the residue obtained was dissolved in dimethyl sulfoxide (100 ml). Triethylamine (60 ml) and a sulfur trioxide-pyridine complex (5.52 g, 34.7 mmol) were added at room temperature and the resulting mixture was stirred for 2 hours. The reaction mixture was poured into water, followed by extraction with ethyl acetate. The organic layer was washed with brine. The organic layer was dried over anhydrous magnesium sulfate, then the solvent was evaporated under reduced pressure and the residue obtained was purified by silica gel column chromatography [n-hexane:ethyl acetate = 2:1 (v/v)] to give the title compound (2.77 g, 56%) as a pale yellow oil.
$^1$H-NMR (400 MHz, $CDCl_3$) δ: 1.43 (9H, s), 1.79 (3H, s), 5.73 (1H, brs), 7.36 (1H, d, J=8.5 Hz), 7.69 (1H, dd, J=8.5, 2.7 Hz), 8.42 (1H, s), 9.34 (1H, s).

Step 4: tert-butyl [2-(4-chloro-3-fluorophenyl)-1-(6-chloropyridin-3-yl)-2-hydroxy-1-methylethyl]carbamate

A tetrahydrofuran (20 ml) solution of the compound (6.47 g, 22.7 mmol) obtained in Step 3 above was added dropwise to 4-chloro-3-fluorophenyl magnesium bromide (0.5 M tetrahydrofuran solution) (100 ml, 50.0 mmol) under ice cooling and the resulting mixture was stirred at the same temperature for 1 hour. An aqueous solution of saturated ammonium chloride was added to the reaction mixture, followed by extraction with ethyl acetate. The organic layer was dried over anhydrous magnesium sulfate and then the solvent was evaporated under reduced pressure. An n-hexane/ethyl acetate mixed solvent was added to the residue obtained and the resulting solid was collected by filtration and dried to give the title compound (7.92 g, 84%) as a white solid.
$^1$H-NMR (400 MHz, $CDCl_3$) δ: 1.45 (9H, brs), 1.56 (3H, brs), 5.08 (2H, brs), 6.86 (1H, brs), 7.09 (1H, d, J=8.8 Hz), 7.31-7.35 (2H, m), 7.68 (1H, d, J=7.3 Hz), 8.44 (1H, s). MS (API) m/z: 415 [$(M+H)^+$].

Step 5: tert-butyl [2-(4-chloro-3-fluorophenyl)-1-(6-chloropyridin-3-yl)-1-methyl-2-oxoethyl]carbamate

Acetic anhydride (2.3 ml, 24.7 mmol) was added to a dimethyl sulfoxide (8 ml) solution of the compound (1.14 g, 2.75 mmol) obtained in Step 4 above under ice cooling and the resulting mixture was gradually returned to room temperature while being stirred for 18 hours. The reaction mixture was diluted with ethyl acetate, washed with 6% aqueous sodium perchlorate solution, 10% aqueous sodium thiosulfate solution, and brine, and then dried over anhydrous magnesium sulfate. The solvent was evaporated under reduced pressure and the residue obtained was purified by silica gel column chromatography [n-hexane:ethyl acetate = 4:1 (v/v)] to give the title compound (1.10 g, 97%) as a pale yellow solid.
$^1$H-NMR (400 MHz, $CDCl_3$) δ: 1.31 (9H, s), 2.03 (3H, s), 6.28 (1H, brs), 7.26-7.37 (3H, m), 7.49 (1H, d, J=9.5 Hz), 7.71 (1H, d, J=6.3 Hz), 8.45 (1H, s).

Step 6: 2-amino-1-(4-chloro-3-fluorophenyl)-2-(6-chloropyridin-3-yl)propan-l-one

A dioxane (1 ml) solution of the compound (1.23 g, 2.55 mmol) obtained in Step 5 above was added to a 4 N hydrochloric acid/dioxane solution (5 ml) under ice cooling and the resulting mixture was gradually warmed and stirred at room temperature for 2 hours. The reaction mixture was diluted with water and washed with an n-hexane/ethyl acetate [1:1 (v/v)] mixed solvent and then the aqueous layer was made alkaline by the addition of 15% aqueous sodium hydroxide solution, followed by extraction with dichloromethane. The organic layer was dried over anhydrous magnesium sulfate, then the solvent was evaporated under reduced pressure and the residue obtained was purified by silica gel column chromatography [n-hexane:ethyl acetate = 1:1 (v/v)] to give the title compound (0.75 g, 95%) as a pale yellow solid.
MS (API) m/z: 313 [$(M+H)^+$].

Step 7: 2-chloro-5-[4-(4-chloro-3-fluorophenyl)-3-methyl-1,1-dioxido-2,3-dihydro-1,2,5-thiadiazol-3-yl]pyridine

A molecular sieve (4A, 4.5 g), sulfamide (2.75 g, 28.6 mmol), and 1,8-diazabicyclo[5.4.0]undec-7-ene (1.45 g, 9.52 mmol) were added to a dioxane (60 ml) solution of the compound (2.99 g, 9.55 mmol) obtained in Step 6 above and the resulting mixture was stirred under heating at 95°C for 18 hours. Sulfamide (2.75 g, 28.6 mmol) was further added and the resulting mixture was stirred under heating for 24 hours. The reaction mixture was concentrated under reduced pressure and then the residue obtained was diluted with ethyl acetate, washed with 10% aqueous citric acid solution and brine, and then dried over anhydrous magnesium sulfate. A diisopropyl ether/ethyl acetate mixed solvent

was added to the residue obtained and the resulting solid was collected by filtration and dried to give the title compound (2.86 g, 80%) as a colorless solid.
$^1$H-NMR (400 MHz, $CDCl_3$) δ: 2.25 (3H, s), 7.29-7.47 (3H, m), 7.59 (1H, dd, J=9.5, 2.0 Hz), 7.68 (1H, dd, J=8.5, 2.9 Hz), 8.53 (1H, d, J=2.9 Hz).

Step 8: 2-chloro-5-[(3R*,4S*)-4-(4-chloro-3-fluorophenyl)-3-methyl-1,1-dioxido-1,2,5-thiadiazolidin-3-yl] pyridine

Sodium borohydride (1.00 g, 26.4 mmol) was gradually added to an ethanol (63 ml) solution of the compound (7.13 g, 19.1 mmol) obtained in Step 7 above under ice cooling and the resulting mixture was stirred at the same temperature for 2 hours. The reaction mixture was concentrated under reduced pressure and then the residue obtained was diluted with ethyl acetate, washed with 1 N aqueous hydrochloric acid solution and brine, and then dried over anhydrous magnesium sulfate. The solvent was evaporated under reduced pressure and the residue obtained was purified by silica gel column chromatography [n-hexane:ethyl acetate = 1:2 (v/v)] to give the title compound (4.36 g, 59%) as a pale yellow solid.
$^1$H-NMR (400 MHz, $CDCl_3$) δ: 1.85 (3H, s), 3.23-3.32 (4H, m), 4.92 (1H, d, J=5.6 Hz), 6.74-6.76 (1H, m), 6.80-6.83 (1H, m), 7.23-7.26 (2H, m), 7.55-7.60 (1H, m), 7.94-7.95 (1H, m).

Step 9: (1R*,2S*)-1-(4-chloro-3-fluorophenyl)-2-(6-chloropyridin-3-yl)propane-1,2-diamine

Ethylenediamine (7.40 ml, 111 mmol) was added to a dioxane (80 ml) solution of the compound (4.15 g, 11.0 mmol) obtained in Step 8 above and the resulting mixture was stirred under heating at 100˚C for 18 hours. The reaction mixture was concentrated under reduced pressure and the residue obtained was diluted with chloroform, washed with 1 N aqueous sodium hydroxide solution and brine, and then dried over anhydrous magnesium sulfate. The solvent was evaporated under reduced pressure and the residue obtained was purified by silica gel column chromatography [chloroform:methanol = 93:7 (v/v)] to give the title compound (3.35 g, 97%) as a pale yellow oil. $^1$H-NMR (400 MHz, $CDCl_3$) δ: 1.54 (3H, s), 1.58 (4H, brs), 4.08 (1H, s), 6.72 (1H, dd, J=8.3, 2.0 Hz), 6.96 (1H, dd, J=10.3, 2.0 Hz), 7.21 (1H, t, J=7.9 Hz), 7.23 (1H, dd, J=8.3, 0.7 Hz), 7.59 (1H, dd, J=8.5, 2.7 Hz), 8.36 (1H, dd, J=2.7, 0.7 Hz).
MS (ESI) m/z: 314 [$(M+H)^+$].

Step 10: (1R,2S)-1-(4-chloro-3-fluorophenyl)-2-(6-chloropyridin-3-yl)propane-1,2-diamine

The same procedures as in Step 1 of Example 1 were performed using the compound obtained in Step 9 above to give the title compound as a colorless solid.
MS (ESI) m/z: 314 [$(M+H)^+$].

Step 11: (4S,5R)-5-(4-chloro-3-fluorophenyl)-4-(6-chloropyridin-3-yl)-4-methylimidazolidine-2-thione

The compound obtained in Step 10 above was reacted in the same way as in Step 2 of Example 1 to give the title compound as a colorless solid.
$^1$H-NMR (400 MHz, $CDCl_3$) δ: 1.94 (3H, s), 5.00 (1H, s), 6.67 (1H, d, J=7.8 Hz), 6.68 (1H, brs), 6.75 (1H, dd, J=9.5, 2.0 Hz), 7.10 (1H, brs), 7.15 (1H, d, J=8.3 Hz), 7.21 (1H, t, J=7.8 Hz), 7.31 (1H, dd, J=8.5, 2.7 Hz), 8.01 (1H, d, J=2.2 Hz).
MS (ESI) m/z: 356 [$(M+H)^+$].

Step 12: ethyl (5R,6S)-5-(4-chloro-3-fluorophenyl)-6-(6-chloropyridin-3-yl)-3-isopropyl-6-methyl-5,6-dihydroimidazo[2,1-b][1,3]thiazole-2-carboxylate

The compound obtained in Step 11 above was reacted in the same way as in Step 3 of Example 1 to give the title compound as a colorless solid.
$^1$H-NMR (400 MHz, $CDCl_3$) δ: 0.90 (3H, d, J=7.3 Hz), 1.04 (3H, d, J=7.1 Hz), 1.34 (3H, t, J=7.2 Hz), 1.83 (3H, s), 4.26 (2H, q, J=7.2 Hz), 5.10 (1H, s), 6.51-6.62 (2H, m), 7.04 (1H, d, J=8.3 Hz), 7.16 (1H, t, J=7.7 Hz), 7.51-7.55 (1H, m), 8.20-8.22 (1H, m).
MS (ESI) m/z: 494 [$(M+H)^+$].

Step 13: (5R,6S)-5-(4-chloro-3-fluorophenyl)-6-(6-chloropyridin-3-yl)-3-isopropyl-6-methyl-5,6-dihydroimidazo[2,1-b][1,3]thiazole-2-carboxylic acid

The compound obtained in Step 12 above was reacted in the same way as in Step 4 of Example 1 to give the title compound as a colorless solid.

$^1$H-NMR (400 MHz, DMSO-$d_6$) δ: 0.79 (3H, d, J=7.1 Hz), 0.98 (3H, d, J=7.1 Hz), 1.73 (3H, s), 3.32-3.40 (1H, m), 5.71 (1H, s), 6.38-6.48 (1H, m), 7.23 (1H, d, J=8.3 Hz), 7.34-7.46 (2H, m), 7.66 (1H, dd, J=8.3, 2.4 Hz), 8.25 (1H, d, J=2.4 Hz). MS (ESI) m/z: 466 [$(M+H)^+$].

Reference Example 14

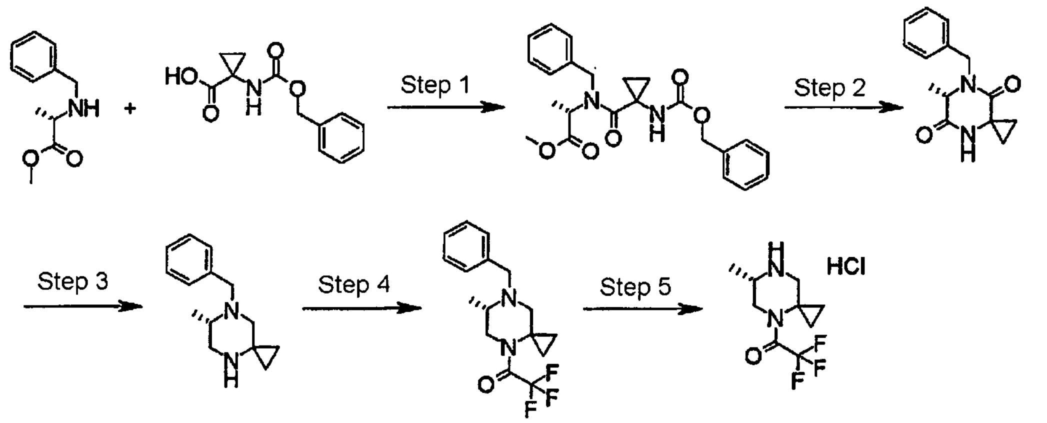


Step 1: methyl N-benzyl-N-[(1-{[(benzyloxy)carbonyl]amino}cyclopropyl)carbonyl]-L-alaninate

N,N$^1$-dicyclohexylcarbodiimide (6.0 g, 29.0 mmol) was added to a dichloromethane (150 ml) solution of methyl N-benzyl-L-alaninate (5.20 g, 26.9 mmol) and 1-{[(benzyloxy)carbonyl]amino}cyclopropanecarboxylic acid (6.30 g, 26.7 mmol) under ice cooling and the resulting mixture was stirred overnight at room temperature. The reaction mixture was concentrated under reduced pressure and then diluted with ethyl acetate, insoluble matter was removed by filtration and then the filtrate was washed with 1 N aqueous hydrochloric acid solution and brine and dried over anhydrous sodium sulfate. The solvent was evaporated under reduced pressure and the residue obtained was purified by silica gel column chromatography [n-hexane:ethyl acetate = 1:3 (v/v)] to give the title compound (7.80 g, 71%) as a colorless oil.
$^1$H-NMR (400 MHz, $CDCl_3$, 70°C) δ: 1.03-1.11 (2H, m), 1.37 (3H, d, J=7.1 Hz), 1.43-1.47 (2H, m), 3.72 (3H, s), 4.48 (1H, brs), 4.61 (1H, d, J=16.6 Hz), 4.91 (1H, d, J=16.6 Hz), 4.99 (2H, s), 5.20 (1H, brs), 7.20-7.34 (10H, m).

Step 2: (6S)-7-benzyl-6-methyl-4,7-diazaspiro[2.5]octane-5,8-dione

5% palladium carbon (5.0 g) was added to an ethanol (500 ml) solution of the compound (33.5 g, 78.9 mmol) obtained in Step 1 above and the resulting mixture was subjected to catalytic reduction for 8 hours in a hydrogen atmosphere. The catalyst was removed by filtration through celite and then the filtrate was concentrated under reduced pressure to give the title compound (18.6 g, 96%) as a colorless oil.
$^1$H-NMR (400 MHz, $CDCl_3$) δ: 0.98-1.07 (2H, m), 1.36 (1H, m), 1.47 (3H, d, J=7.1 Hz), 1.85 (1H, m), 3.91 (1H, q, J=7.1 Hz), 4.06 (1H, d, J=14.9 Hz), 5.21 (1H, d, J=14.9 Hz), 7.26-7.37 (5H, m).

Step 3: (6S)-7-benzyl-6-methyl-4,7-diazaspiro[2.5]octane

A borane-tetrahydrofuran complex (0.93 M tetrahydrofuran solution) (102 ml, 95.0 mmol) was added to a tetrahydrofuran (100 ml) solution of the compound (5.80 g, 23.7 mmol) obtained in Step 2 above under ice cooling and the resulting mixture was heated to reflux for 6 hours. The reaction mixture was left standing to cool, then methanol (35 ml) was added under ice cooling and the resulting mixture was stirred for 30 minutes and then concentrated under reduced pressure. Ethanol (180 ml), water (60 ml), and triethylamine (60 ml) were added to the residue obtained and the resulting mixture was heated to reflux for 2 hours. The reaction mixture was concentrated under reduced pressure and then the residue obtained was diluted with ethyl acetate, washed with saturated aqueous sodium bicarbonate solution and brine, and then dried over anhydrous sodium sulfate. The solvent was evaporated under reduced pressure and then the residue obtained was purified by silica gel column chromatography [chloroform:methanol = 50:1 (v/v)] to give the title compound (2.50 g, 49%) as a colorless solid. $^1$H-NMR (400 MHz, $CDCl_3$) δ: 0.30-0.34 (1H, m), 0.36-0.40 (1H, m), 0.48-0.53 (1H, m), 0.55-0.61 (1H, m), 1.16 (3H, d, J=6.3 Hz), 2.12 (1H, d, J=11.7 Hz), 2.26-2.33 (2H, m), 2.74

(1H, dd, J=13.2, 9.3 Hz), 2.90 (1H, dd, J=13.2, 3.4 Hz), 3.15 (1H, d, J=13.4 Hz), 4.07 (1H, d, J=13.4 Hz), 7.20-7.34 (6H, m).

Step 4: (6S)-7-benzyl-6-methyl-4-(trifluoroacetyl)-4,7-diazaspiro[2.5]octane

Trifluoroacetic anhydride (2.00 ml, 14.3 mmol) was added dropwise to a dichloromethane (50 ml) solution of the compound (2.50 g, 11.5 mmol) obtained in Step 3 above and triethylamine (4.20 ml, 30 mmol) under ice cooling and the resulting mixture was stirred at the same temperature for 1 hour. Saturated aqueous sodium bicarbonate solution was added to the reaction mixture, followed by extraction with chloroform. The organic layer was washed with brine and then dried over anhydrous sodium sulfate. The solvent was evaporated under reduced pressure and the residue obtained was purified by silica gel column chromatography [n-hexane:ethyl acetate = 1:9 (v/v)] to give the title compound (3.30 g, 92%) as a colorless oil.
$^1$H-NMR (400 MHz, $CDCl_3$) δ: 0.68 (1H, m), 0.79 (1H, m), 0.93 (1H, m), 1.21 (3H, d, J=6.1 Hz), 1.33 (1H, m), 2.17 (1H, d, J=12.0 Hz), 2.49 (1H, d, J=12.0 Hz), 2.59 (1H, m), 3.16 (1H, d, J=13.4 Hz), 3.33 (1H, dd, J=13.4, 9.3 Hz), 3.77 (1H, d, J=13.4 Hz), 4.04 (1H, d, J=13.4 Hz), 7.23-7.34 (5H, m).

Step 5: (6S)-6-methyl-4-(trifluoroacetyl)-4,7-diazaspiro[2.5]octane hydrochloride

1 N hydrochloric acid/ethanol (21 ml, 21 mmol) and 5% palladium carbon (0.65 g) were added to an ethanol (100 ml) solution of the compound (3.25 g, 10.4 mmol) obtained in Step 4 above and the resulting mixture was stirred for 15 hours in a hydrogen atmosphere. The catalyst was removed by filtration and then the filtrate was concentrated under reduced pressure. Ethanol/ether was added to the residue obtained and the deposited solid was collected by filtration and dried to give the title compound (2.57 g, 96%) as a colorless solid.
$^1$H-NMR (400 MHz, DMSO-$d_6$) δ: 0.95 (1H, brs), 1.12 (1H, brs), 1.27 (1H, m), 1.33 (3H, d, J=6.3 Hz), 1.41 (1H, brs), 2.90 (1H, m), 3.38-3.59 (3H, m), 4.03 (1H, brs).
MS (ESI) m/z: 223 [$(M+H)^+$].

Reference Example 15

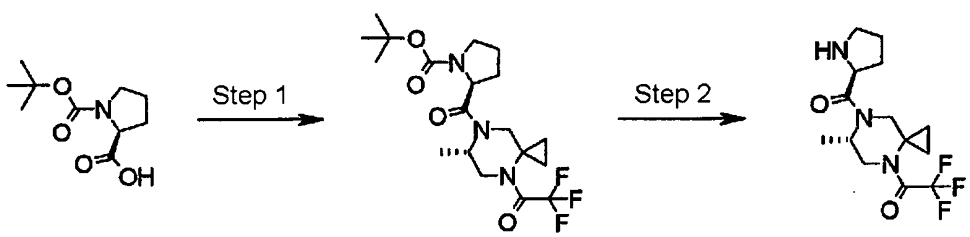


Step 1: tert-butyl (2S)-2-{[(6S)-6-methyl-4-(trifluoroacetyl)-4,7-diazaspiro[2.5]oct-7-yl]carbonyl}pyrrolidine-1-carboxylate

1-(tert-butoxycarbonyl)-L-proline was reacted in the same way as in Step 7 of Example 1 using the compound obtained in Step 5 of Reference Example 14 instead of 4,7-diazaspiro[2.5]octane hydrochloride to give the title compound as a pale yellow oil.
$^1$H-NMR (DMSO-$d_6$) δ: 0.67-0.73 (2H, m), 1.11 (3H, d, J=6.8 Hz), 1.25-1.31 (2H, m), 1.36 (9H, s), 1.46-1.52 (1H, m), 1.67-1.82 (2H, m), 1.85-1.88 (1H, m), 2.15-2.18 (1H, m), 2.90-2.93 (1H, m), 3.31-3.40 (3H, m), 3.52-3.54 (3H, m), 3.89 (1H, brs), 4.55 (1H, brs), 4.64 (1H, brs).
MS (ESI) m/z: 442 [$(M+Na)^+$].

Step 2: (6S)-6-methyl-7-L-prolyl-4-(trifluoroacetyl)-4,7-diazaspiro[2.5]octane

Trifluoroacetic acid (5 ml) was added to a chloroform (10 ml) solution of the compound (1.00 g, 2.38 mmol) obtained in Step 1 above under ice cooling and the resulting mixture was returned to room temperature and stirred for 1 hour. The reaction mixture was concentrated under reduced pressure and then neutralized with saturated aqueous sodium bicarbonate solution, followed by extraction with ethyl acetate. The organic layer was washed with brine and

then dried over anhydrous sodium sulfate. The solvent was evaporated under reduced pressure to give the title compound (0.71 g, 93%) as a colorless oil.
$^1$H-NMR (400 MHz, DMSO-$d_6$, 100°C) δ: 0.67-0.75 (2H, m), 1.11 (3H, d, J=6.8 Hz), 1.25-1.29 (1H, m), 1.47-1.50 (1H, m), 1.61-1.71 (3H, m), 1.87-1.95 (1H, m), 2.66-2.72 (1H, m), 2.91-2.98 (1H, m), 3.42-3.55 (3H, m), 3.75-3.78 (1H, m), 3.81-3.92 (1H, m), 4.63-4.68 (1H, m).
MS (ESI) m/z: 320 [$(M+H)^+$].

Reference Example 16

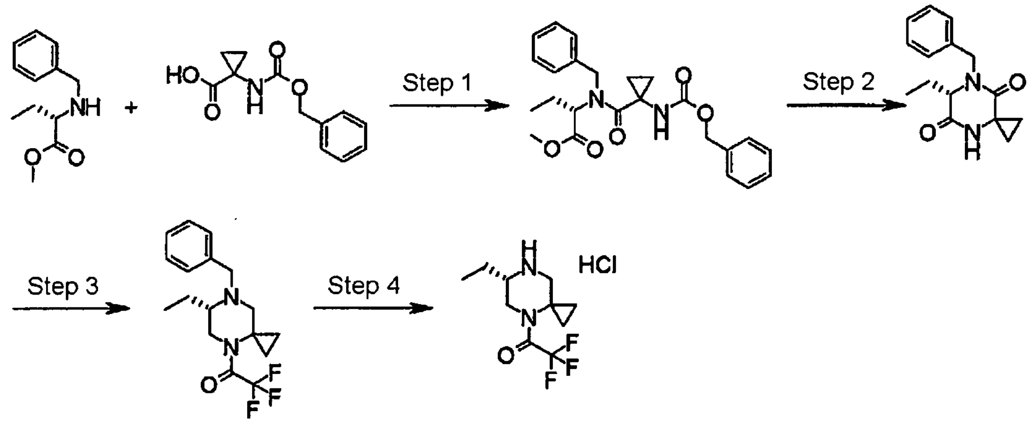


Step 1: methyl (2S)-2-{benzyl[(1-{[(benzyloxy)carbonyl]amino}cyclopropyl)carbonyl]amino}bu tanoate

Methyl (2S)-2-(benzylamino)butanoate (J. Org. Chem., 1995, 60, 6776) instead of methyl N-benzyl-L-alaninate was reacted in the same way as in Step 1 of Reference Example 14 to give the title compound as a colorless solid.
$^1$H-NMR (400 MHz, DMSO-$d_6$) δ: 0.70 (3H, t, J=7.4 Hz), 0.83-0.89 (1H, m), 1.00-1.06 (1H, m), 1.09-1.16 (1H, m), 1.30-1.37 (1H, m), 1.58-1.68 (1H, m), 1.83-1.92 (1H, m), 2.87-2.92 (1H, m), 3.48 (3H, s), 4.50-4.56 (1H, m), 4.64 (1H, d, J=15.9 Hz), 5.00 (2H, q, J=12.1 Hz), 7.17-7.33 (10H, m), 7.76 (1H, brs).
MS (ESI) m/z: 425 [$(M+H)^+$].

Step 2: (6S)-7-benzyl-6-ethyl-4,7-diazaspiro[2.5]octane-5,8-dione

The compound obtained in Step 1 above was reacted in the same way as in Step 2 of Reference Example 14 to give the title compound as a colorless solid.
$^1$H-NMR (400 MHz, $CDCl_3$) δ: 0.93-0.98 (2H, m), 0.99 (3H, t, J=7.4 Hz), 1.35-1.40 (1H, m), 1.79-1.86 (1H, m), 1.91-1.97 (2H, m), 3.90 (1H, t, J=5.4 Hz), 3.94 (1H, d, J=14.9 Hz), 5.35 (1H, d, J=14.9 Hz), 7.25-7.36 (5H, m), 7.39 (1H, brs).
MS (ESI) m/z: 259 [$(M+H)^+$].

Step 3: (6S)-7-benzyl-6-ethyl-4-(trifluoroacetyl)-4,7-diazaspiro[2.5]octane

The compound obtained in Step 2 above was reacted in the same way as in Step 3 of Reference Example 14 and then reacted in the same way as in Step 4 of Reference Example 14 to give the title compound as a colorless oil. $^1$H-NMR (400 MHz, DMSO-$d_6$) δ: 0.65-0.70 (1H, m), 0.85-0.90 (2H, m), 0.91 (3H, t, J=7.4 Hz), 1.18-1.23 (1H, m), 1.46-1.53 (1H, m), 1.66-1.75 (1H, m), 2.31-2.36 (1H, m), 2.38-2.45 (2H, m), 3.32 (1H, d, J=13.9 Hz), 3.40-3.47 (1H, m), 3.84 (1H, d, J=11.7 Hz), 3.97 (1H, d, J=13.9 Hz), 7.18-7.23 (1H, m), 7.27-7.31 (4H, m).
MS (ESI) m/z: 327 [$(M+H)^+$].

Step 4: (6S)-6-ethyl-4-(trifluoroacetyl)-4,7-diazaspiro[2.5]octane hydrochloride

The compound obtained in Step 3 above was reacted in the same way as in Step 5 of Reference Example 14 to give the title compound as a colorless solid.
$^1$H-NMR (400 MHz, DMSO-$d_6$) δ: 0.89-0.94 (1H, m), 0.99 (3H, t, J=7.6 Hz), 1.16-1.21 (1H, m), 1.25-1.31 (1H, m),

1.41-1.48 (1H, m), 1.66-1.74 (1H, m), 1.77-1.85 (1H, m), 2.86 (1H, d, J=12.9 Hz), 3.24-3.32 (1H, m), 3.37-3.44 (1H, m), 3.45 (1H, d, J=12.9 Hz), 4.06-4.14 (1H, m).
MS (ESI) m/z: 237 [$(M+H)^+$].

Reference Example 17

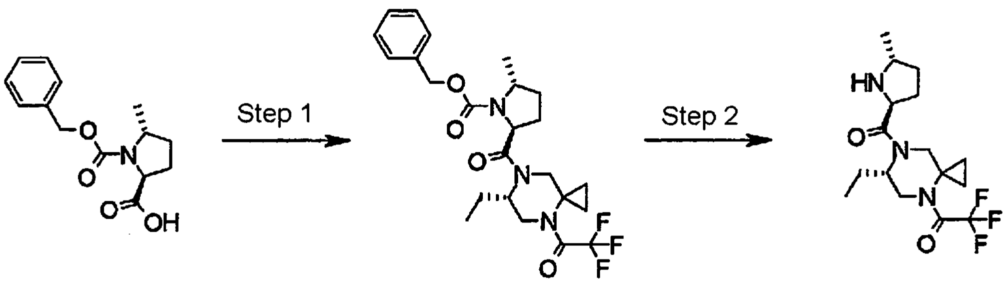


Step 1: benzyl (2S,5R)-2-{[(6S)-6-ethyl-4-(trifluoroacetyl)-4,7-diazaspiro[2.5]oct-7-yl]carbonyl}-5-methylpyrrolidine-1-carboxylate

1-chloro-N,N,2-trimethyl-1-propenylamine (0.32 ml, 2.40 mmol) was added to a dichloromethane (7 ml) solution of (5R)-1-[(benzyloxy)carbonyl]-5-methyl-L-proline (520 mg, 1.98 mmol) under ice cooling. The resulting mixture was stirred at the same temperature for 1 hour, then the compound (648 mg, 2.38 mmol) obtained in Step 4 of Reference Example 16 and triethylamine (0.78 ml, 5.58 mmol) were added and the mixture was further stirred at room temperature for 3 hours. The reaction mixture was diluted with chloroform and the organic layer was washed with saturated aqueous sodium bicarbonate solution and brine and dried over anhydrous sodium sulfate. The solvent was evaporated under reduced pressure and the residue obtained was purified by silica gel column chromatography [n-hexane:ethyl acetate = 2:1 (v/v)] to give the title compound (690 mg, 77%) as a colorless solid.
$^1$H-NMR (400 MHz, DMSO-$d_6$) δ: 0.64-0.87 (5H, m), 1.18 (3H, d, J=6.3 Hz), 1.19-1.27 (2H, m), 1.40-1.55 (3H, m), 1.66-1.74 (1H, m), 2.17-2.31 (2H, m), 3.26-3.64 (3H, m), 3.92-4.00 (1H, m), 4.01-4.11 (1H, m), 4.30-4.41 (1H, m), 4.68-4.81 (1H, m), 4.91-5.01 (1H, m), 5.04-5.14 (1H, m), 7.25-7.40 (5H, m).
MS (ESI) m/z: 482 [$(M+H)^+$].

Step 2: (6S)-6-ethyl-7-[(5R)-5-methyl-L-prolyl]-4-(trifluoroacetyl)-4,7-diazaspiro[2.5]octane

The compound obtained in Step 1 above was reacted in the same way as in Step 2 of Reference Example 1 to give the title compound as a colorless solid.
$^1$H-NMR (400 MHz, DMSO-$d_6$) δ: 0.67-0.77 (2H, m), 0.81-0.89 (3H, m), 1.19 (3H, d, J=6.6 Hz), 1.28-1.36 (1H, m), 1.39-1.53 (4H, m), 1.73-1.84 (1H, m), 1.93-2.03 (1H, m), 2.15-2.25 (1H, m), 2.93-3.78 (5H, m), 4.15-4.55 (2H, m).
MS (ESI) m/z: 348 [$(M+H)^+$].

Reference Example 18

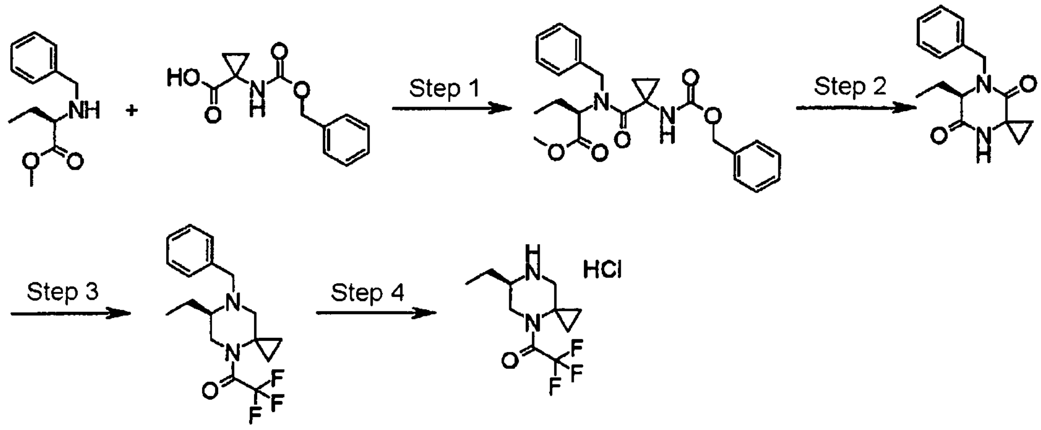


Step 1: methyl (2R)-2-{benzyl[(1-{[(benzyloxy)carbonyl]amino}cyclopropyl)carbonyl]amino}bu tanoate

Methyl (2R)-2-(benzylamino)butanoate instead of methyl N-benzyl-L-alaninate was reacted in the same way as in Step 1 of Reference Example 14 to give the title compound as a pale yellow solid.
$^1$H-NMR (400 MHz, DMSO-$d_6$) δ: 0.71 (3H, t, J=7.4 Hz), 0.85-0.90 (1H, m), 1.02-1.08 (1H, m), 1.11-1.16 (1H, m), 1.32-1.38 (1H, m), 1.59-1.68 (1H, m), 1.84-1.93 (1H, m), 3.49 (3H, s), 4.30-4.38 (1H, m), 4.49-4.57 (1H, m), 4.65 (1H, d, J=16.1 Hz), 5.01 (2H, q, J=12.2 Hz), 7.18-7.34 (10H, m), 7.77 (1H, brs).
MS (ESI) m/z: 425 [$(M+H)^+$].

Step 2: (6R)-7-benzyl-6-ethyl-4,7-diazaspiro[2.5]octane-5,8-dione

The compound obtained in Step 1 above was reacted in the same way as in Step 2 of Reference Example 14 to give the title compound as a colorless solid.
$^1$H-NMR (400 MHz, $CDCl_3$) δ: 0.93-0.98 (2H, m), 0.99 (3H, t, J=7.6 Hz), 1.35-1.40 (1H, m), 1.80-1.86 (1H, m), 1.91-1.98 (2H, m), 3.89 (1H, t, J=5.2 Hz), 3.94 (1H, d, J=14.9 Hz), 5.35 (1H, d, J=14.9 Hz), 7.25-7.35 (5H, m), 7.51 (1H, brs).
MS (ESI) m/z: 259 [$(M+1)^+$].

Step 3: (6R)-7-benzyl-6-ethyl-4-(trifluoroacetyl)-4,7-diazaspiro[2.5]octane

The compound obtained in Step 2 above was reacted in the same way as in Step 3 of Reference Example 14 and then reacted in the same way as in Step 4 of Reference Example 14 to give the title compound as a colorless oil. $^1$H-NMR (400 MHz, DMSO-$d_6$) δ: 0.65-0.70 (1H, m), 0.85-0.90 (2H, m), 0.91 (3H, t, J=7.4 Hz), 1.18-1.23 (1H, m), 1.46-1.53 (1H, m), 1.66-1.75 (1H, m), 2.31-2.36 (1H, m), 2.38-2.45 (2H, m), 3.32 (1H, d, J=13.9 Hz), 3.40-3.47 (1H, m), 3.84 (1H, d, J=11.7 Hz), 3.97 (1H, d, J=13.9 Hz), 7.18-7.23 (1H, m), 7.27-7.31 (4H, m).
MS (ESI) m/z: 327 [$(M+H)^+$].

Step 4: (6S)-6-ethyl-4-(trifluoroacetyl)-4,7-diazaspiro[2.5]octane hydrochloride

The compound obtained in Step 3 above was reacted in the same way as in Step 5 of Reference Example 14 to give the title compound as a colorless solid.
$^1$H-NMR (400 MHz, DMSO-$d_6$) δ: 0.89-0.94 (1H, m), 0.99 (3H, t, J=7.6 Hz), 1.16-1.21 (1H, m), 1.25-1.31 (1H, m), 1.41-1.48 (1H, m), 1.66-1.74 (1H, m), 1.77-1.85 (1H, m), 2.86 (1H, d, J=12.9 Hz), 3.24-3.32 (1H, m), 3.37-3.44 (1H, m), 3.45 (1H, d, J=12.9 Hz), 4.06-4.14 (1H, m).
MS (ESI) m/z: 237 [$(M+H)^+$].

Reference Example 19

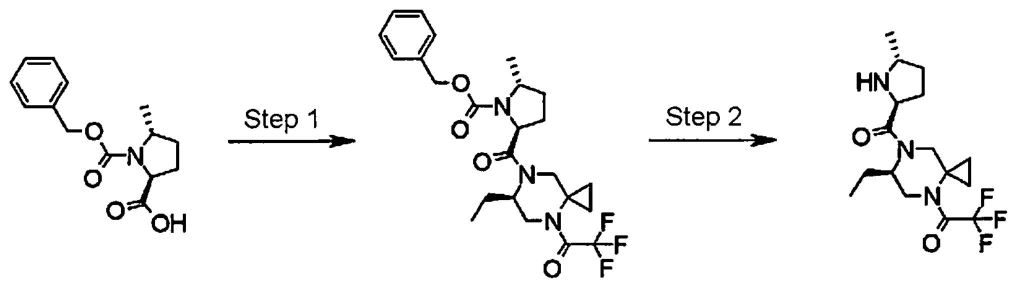


Step 1: benzyl (2S,5R)-2-{[(6R)-6-ethyl-4-(trifluoroacetyl)-4,7-diazaspiro[2.5]oct-7-yl]carbonyl}-5-methylpyrrolidine-1-carboxylate

(5R)-1-[(benzyloxy)carbonyl]-5-methyl-L-proline was reacted in the same way as in Step 1 of Example 17 using the compound obtained in Step 4 of Reference Example 18 instead of the compound obtained in Step 4 of Reference Example 16 to give the title compound as a colorless solid.
$^1$H-NMR (400 MHz, DMSO-$d_6$) δ: 0.64-0.84 (5H, m), 1.17 (3H, d, J=5.4 Hz), 1.18-1.30 (2H, m), 1.37-1.61 (4H, m), 1.97-2.06 (1H, m), 2.15-2.25 (1H, m), 3.18-3.67 (3H, m), 3.93-4.09 (2H, m), 4.52-4.61 (1H, m), 4.68-4.76 (1H, m), 4.89-4.99 (1H, m), 5.02-5.12 (1H, m), 7.24-7.39 (5H, m).
MS (ESI) m/z: 482 [(M+H)$^+$].

Step 2: (6R)-6-ethyl-7-[(5R)-5-methyl-L-prolyl]-4-(trifluoroacetyl)-4,7-diazaspiro[2.5]octane

The compound obtained in Step 1 above was reacted in the same way as in Step 2 of Reference Example 1 to give the title compound as a colorless solid.
$^1$H-NMR (400 MHz, DMSO-$d_6$) δ: 0.57-0.62 (1H, m), 0.70-0.77 (2H, m), 0.80 (3H, t, J=7.3 Hz), 0.92-0.96 (1H, m), 1.37 (3H, d, J=6.6 Hz), 1.45-1.53 (2H, m), 1.58-1.65 (2H, m), 2.04-2.10 (1H, m), 2.40-2.46 (1H, m), 2.86-3.03 (2H, m), 3.34 (1H, d, J=13.9 Hz), 3.63-3.76 (2H, m), 4.60-4.67 (1H, m), 4.73 (1H, t, J=8.2 Hz).
MS (ESI) m/z: 348 [(M+H)$^+$].

Reference Example 20

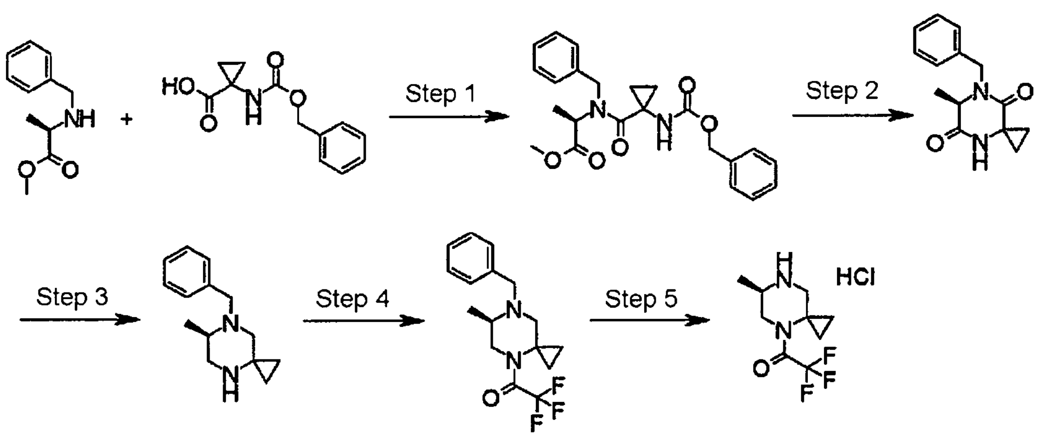


Step 1: methyl N-benzyl-N-[(1-{[(benzyloxy)carbonyl]amino}cyclopropyl)carbonyl]-D-alaninate

Methyl N-benzyl-D-alaninate instead of methyl N-benzyl-L-alaninate was reacted in the same way as in Step 1 of Reference Example 14 to give the title compound as a colorless oil.

$^1H$-NMR (400 MHz, $CDCl_3$) δ: 1.07 (2H, brs), 1.36 (3H, d, J=7.1 Hz), 1.43 (2H, brs), 3.61 (3H, s), 4.48-4.52 (1H, m), 4.61 (1H, d, J=17.4 Hz), 4.87 (1H, d, J=17.4 Hz), 4.98 (2H, s), 5.12 (1H, brs), 7.20-7.32 (10H, m).
MS (ESI) m/z: 411 [$(M+H)^+$].

Step 2: (6R)-7-benzyl-6-methyl-4,7-diazaspiro[2.5]octane-5,8-dione

The compound obtained in Step 1 above was reacted in the same way as in Step 2 of Reference Example 14 to give the title compound as a colorless oil.
$^1H$-NMR (400 MHz, $CDCl_3$) δ: 0.97-1.03 (2H, m), 1.34-1.41 (2H, m), 1.47 (3H, d, J=7.1 Hz), 1.81-1.88 (1H, m), 4.06 (1H, d, J=14.9 Hz), 5.19 (1H, d, J=14.9 Hz), 6.74 (1H, brs), 7.25-7.37 (5H, m).

Step 3: (6R)-7-benzyl-6-methyl-4,7-diazaspiro[2.5]octane

The compound obtained in Step 2 above was reacted in the same way as in Step 3 of Reference Example 14 to give the title compound as a colorless solid.
$^1H$-NMR (400 MHz, $CDCl_3$) δ: 0.30-0.40 (2H, m), 0.48-0.61 (2H, m), 1.16 (3H, d, J=6.3 Hz), 1.58 (1H, brs), 2.12 (1H, d, J=11.5 Hz), 2.25-2.33 (2H, m), 2.73 (1H, dd, J=13.1, 9.3 Hz), 2.90 (1H, dd, J=13.1, 3.0 Hz), 3.15 (1H, d, J=13.4 Hz), 4.08 (1H, d, J=13.4 Hz), 7.20-7.33 (5H, m).

Step 4: (6R)-7-benzyl-6-methyl-4-(trifluoroacetyl)-4,7-diazaspiro[2.5]octane

The compound obtained in Step 3 above was reacted in the same way as in Step 4 of Reference Example 14 to give the title compound as a colorless oil.
$^1H$-NMR (400 MHz, $CDCl_3$) δ: 0.64-0.92 (3H, m), 1.20 (3H, d, J=6.1 Hz), 1.30-1.36 (1H, m), 2.16 (1H, d, J=12.0 Hz), 2.48 (1H, d, J=12.0 Hz), 2.58 (1H, brs), 3.15 (1H, d, J=13.4 Hz), 3.31 (1H, dd, J=13.4, 9.5 Hz), 3.76 (1H, d, J=13.4 Hz), 4.02 (1H, d, J=13.4 Hz), 7.23-7.33 (5H, m).

Step 5: (6R)-6-methyl-4-(trifluoroacetyl)-4,7-diazaspiro[2.5]octane hydrochloride

The compound obtained in Step 4 above was reacted in the same way as in Step 5 of Reference Example 14 to give the title compound as a colorless solid.
$^1H$-NMR (400 MHz, DMSO-$d_6$, 70°C) δ: 0.93 (1H, brs), 1.17 (1H, brs), 1.24-1.30 (1H, m), 1.34 (3H, d, J=6.3 Hz), 1.40 (1H, brs), 2.89 (1H, d, J=12.0 Hz), 3.14 (1H, brs), 3.40-3.46 (2H, m), 4.04 (1H, brs), 9.84 (2H, brs).
MS (ESI) m/z: 223 [$(M+H)^+$].

Reference Example 21

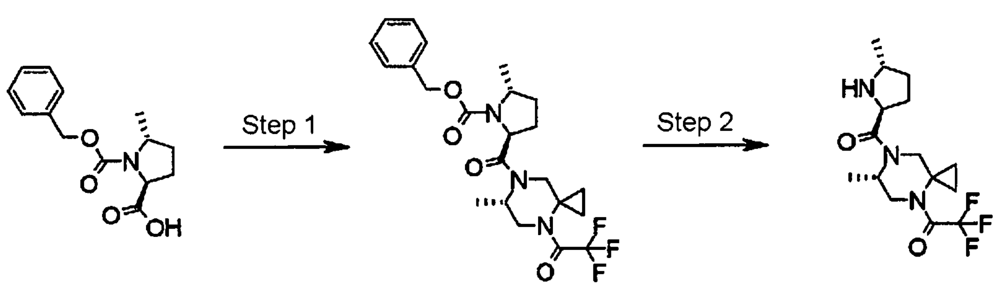


Step 1: benzyl (2R,5S)-2-methyl-5-{[(6S)-6-methyl-4-(trifluoroacetyl)-4,7-diazaspiro[2.5]oct-7-yl]carbonyl}pyrrolidine-1-carboxylate

(5R)-1-[(benzyloxy)carbonyl]-5-methyl-L-proline was reacted in the same way as in Step 7 of Example 1 using the compound obtained in Step 5 of Reference Example 14 instead of 4,7-diazaspiro[2.5]octane hydrochloride to give the title compound as a colorless solid.
$^1H$-NMR (400 MHz, DMSO-$d_6$) δ: 0.63 (2H, s), 1.02-1.08 (3H, m), 1.18-1.25 (4H, m), 1.45-1.53 (2H, m), 1.69 (1H, ddd, J=8.9, 5.4, 3.5 Hz), 2.20 (2H, dd, J=25.3, 17.7 Hz), 3.45 (3H, brs), 3.84 (1H, s), 4.05 (1H, dt, J=15.1, 4.7 Hz), 4.65 (2H,

d, J=51. 0 Hz), 5.00-5.04 (2H, m), 7.28-7.31 (5H, m).
MS (ESI) m/z: 468 [$(M+H)^+$].

Step 2: (6S)-6-methyl-7-[(5R)-5-methyl-L-prolyl]-4-(trifluoroacetyl)-4,7-diazaspiro[2.5]octane

The compound obtained in Step 1 above was reacted in the same way as in Step 2 of Reference Example 1 to give the title compound as a colorless solid.
$^1$H-NMR (400 MHz, $CDCl_3$, 70°C) δ: 0.63 (1H, brs), 0.79-0.82 (1H, m), 1.14 (3H, d, J=6.3 Hz), 1.18-1.28 (4H, m), 1.41 (1H, ddd, J=18.4, 10.4, 6.5 Hz), 1.65-1.67 (1H, m), 1.78 (1H, ddd, J=18.4, 9.9, 6.0 Hz), 1.93-2.01 (1H, m), 2.06-2.15 (1H, m), 2.36 (1H, brs), 3.40-3.48 (1H, m), 3.50-3.72 (2H, m), 3.94 (1H, brs), 4.59 (1H, brs).
MS (ESI) m/z: 334 [$(M+H)^+$].

Reference Example 22

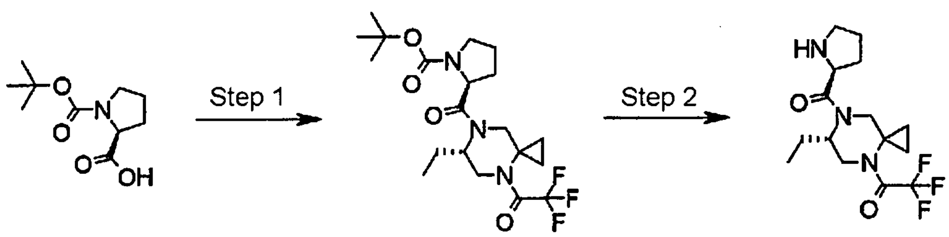


Step 1: tert-butyl (2S)-2-{[(6S)-6-ethyl-4-(trifluoroacetyl)-4,7-diazaspiro[2.5]oct-7-yl]carbonyl}pyrrolidine-1-carboxylate

1-(tert-butoxycarbonyl)-L-proline was reacted in the same way as in Step 1 of Reference Example 17 using Step 4 of Reference Example 16 instead of the compound obtained in Step 5 of Reference Example 14 to give the title compound as a colorless oil.
MS (ESI) m/z: 456 [$(M+Na)^+$]

Step 2: (6S)-6-ethyl-7-L-prolyl-4-(trifluoroacetyl)-4,7-diazaspiro[2.5]octane

The compound obtained in Step 1 above was reacted in the same way as in Step 2 of Reference Example 15 to give the title compound as a colorless solid.
$^1$H-NMR (400 MHz, DMSO-$d_6$) δ: 0.68-0.75 (2H, m), 0.79-0.86 (3H, m), 1.24-1.32 (1H, m), 1.42-1.55 (3H, m), 1.63-1.69 (2H, m), 1.69-1.75 (1H, m), 1.84-1.91 (1H, m), 2.67-2.73 (1H, m), 2.89-2.95 (1H, m), 3.29-3.94 (5H, m), 4.53-4.62 (1H, m).
MS (ESI) m/z: 334 [$(M+H)^+$].

Reference Example 23

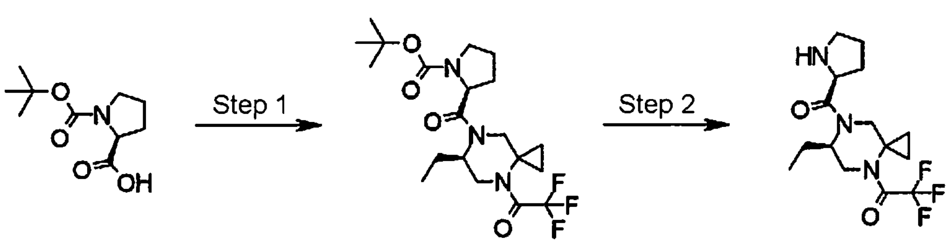

Step 1: tert-butyl (2S)-2-{[(6R)-6-ethyl-4-(trifluoroacetyl)-4,7-diazaspiro[2.5]oct-7-yl]carbonyl}pyrrolidine-1-carboxylate

1-(tert-butoxycarbonyl)-L-proline was reacted in the same way as in Step 1 of Reference Example 17 using the compound obtained in Step 4 of Reference Example 18 instead of the compound obtained in Step 5 of Reference Example 14 to give the title compound as a colorless oil. MS (ESI) m/z: 456 [(M+Na)$^+$].

Step 2: (6R)-6-ethyl-7-L-prolyl-4-(trifluoroacetyl)-4,7-diazaspiro[2.5]octane

The compound obtained in Step 1 above was reacted in the same way as in Step 2 of Reference Example 15 to give the title compound as a colorless solid.
$^1$H-NMR (400 MHz, DMSO-d$_6$) δ: 0.67-0.73 (1H, m), 0.75-0.91 (4H, m), 1.24-1.32 (1H, m), 1.39-1.71 (6H, m), 1.87-1.98 (1H, m), 2.62-2.70 (1H, m), 2.93-3.05 (2H, m), 3.24-3.34 (1H, m), 3.52-3.62 (1H, m), 3.75-3.83 (1H, m), 4.07-4.18 (1H, m), 4.58-4.68 (1H, m).
MS (ESI) m/z: 334 [(M+H)$^+$].

Reference Example 24

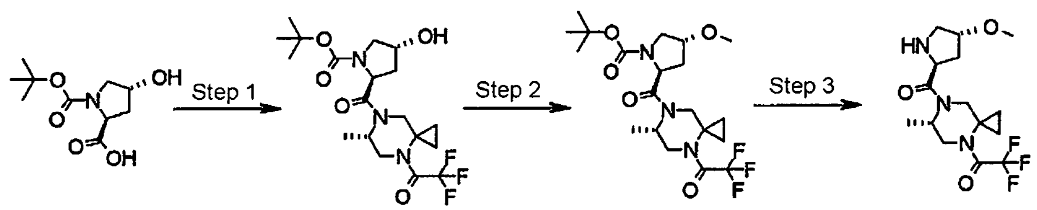


Step 1: tert-butyl (2S,4R)-4-hydroxy-2-{[(6S)-6-methyl-4-(trifluoroacetyl)-4,7-diazaspiro[2.5]oct-7-yl]carbonyl}pyrrolidine-1-carboxylate

(4R)-1-(tert-butoxycarbonyl)-4-hydroxy-L-proline was reacted in the same way as in Step 7 of Example 1 using the compound obtained in Step 5 of Reference Example 14 instead of 4,7-diazaspiro[2.5]octane hydrochloride to give the title compound as a pale yellow oil.
$^1$H-NMR (400 MHz, DMSO-d$_6$) δ: 0.67-0.72 (2H, m), 1.12 (3H, d, J=6.3 Hz), 1.27-1.31 (2H, m), 1.38 (9H, s), 1.50 (1H, dd, J=17.3, 7.1 Hz), 1.85-1.91 (1H, m), 2.11 (1H, s), 2.74 (1H, s), 2.90 (1H, s), 3.31 (1H, d, J=9.8 Hz), 3.43-3.50 (3H, m), 4.30 (1H, s), 4.66 (2H, d, J=3.9 Hz).
MS (ESI) m/z: 458 [(M+Na)$^+$].

Step 2: tert-butyl (2S,4R)-4-methoxy-2-{[(6S)-6-methyl-4-(trifluoroacetyl)-4,7-diazaspiro[2.5]oct-7-yl]carbonyl}pyrrolidine-1-carboxylate

Methyl iodide (0.30 ml, 4.80 mmol) and silver oxide (556 mg, 2.40 mmol) were added to an acetone (5 ml) solution of the compound (210 mg, 0.48 mmol) obtained in Step 1 above and the resulting mixture was stirred at 60˚C for 19 hours. The reaction mixture was left standing to cool, then insoluble matter was removed by filtration through celite and the filtrate was concentrated under reduced pressure. The residue was purified by silica gel column chromatography [chloroform:methanol = 30:1 (v/v)] to give the title compound (158 mg, 73%) as a yellow oil.
MS (ESI) m/z: 450 [(M+H)$^+$].

Step 3: (6S)-7-[(4R)-4-methoxy-L-prolyl]-6-methyl-4-(trifluoroacetyl)-4,7-diazaspiro[2.5]octane

The compound obtained in Step 2 above was reacted in the same way as in Step 2 of Reference Example 15 to give the title compound as a yellow oil.
MS (ESI) m/z: 350 [(M+H)$^+$].

Reference Example 25

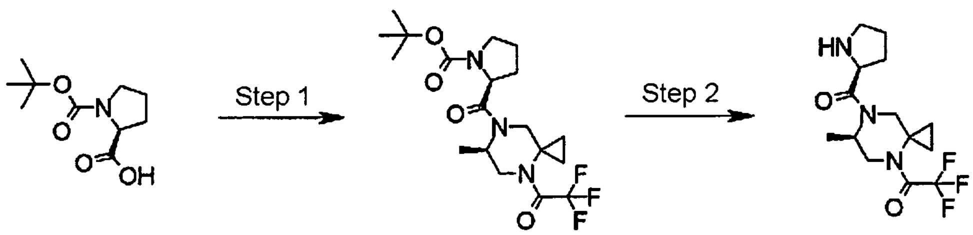


Step 1: tert-butyl (2S)-2-{[(6R)-6-methyl-4-(trifluoroacetyl)-4,7-diazaspiro[2.5]oct-7-yl]carbonyl}pyrrolidine-1-carboxylate

1-(tert-butoxycarbonyl)-L-proline was reacted in the same way as in Step 7 of Example 1 using the compound obtained in Step 5 of Reference Example 20 instead of 4,7-diazaspiro[2.5]octane hydrochloride to give the title compound as a colorless solid.
$^1$H-NMR (400 MHz, DMSO-$d_6$) δ: 0.73 (2H, dd, J=10.1, 5.2 Hz), 1.12 (3H, s), 1.29 (1H, dd, J=14.9, 7.1 Hz), 1.37 (9H, s), 1.49 (1H, dd, J=17.2, 7.0 Hz), 1.66 (1H, s), 1.79 (2H, s), 2.19 (1H, s), 3.36 (3H, t, J=6.8 Hz), 3.56-3.68 (2H, m), 3.93 (1H, s), 4.54 (2H, d, J=5.1 Hz).
MS (ESI) m/z: 420 [(M+H)$^+$].

Step 2: (6R)-6-methyl-7-L-prolyl-4-(trifluoroacetyl)-4,7-diazaspiro[2.5]octane

The compound obtained in Step 1 above was reacted in the same way as in Step 2 of Reference Example 15 to give the title compound as a pale yellow oil.
$^1$H-NMR (400 MHz, DMSO-$d_6$) δ: 0.68-0.73 (1H, m), 0.80 (1H, s), 1.10 (3H, d, J=6.3 Hz), 1.27 (1H, dt, J=12.5, 4.9 Hz), 1.48 (1H, dt, J=12.5, 5.2 Hz), 1.58-1.72 (4H, m), 1.96 (1H, s), 2.68 (1H, dt, J=12.5, 5.2 Hz), 3.53 (4H, s), 3.76 (1H, dd, J=8.2, 6.0 Hz), 3.92 (1H, s), 4.63 (1H, s). MS (ESI) m/z: 320 [(M+H)$^+$].

Reference Example 26

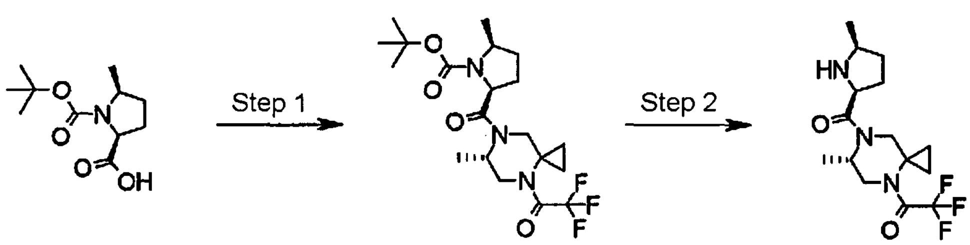


Step 1: tert-butyl (2S,5S)-2-methyl-5-{[(6S)-6-methyl-4-(trifluoroacetyl)-4,7-diazaspiro[2.5]oct-7-yl]carbonyl}pyrrolidine-1-carboxylate

(5S)-1-(tert-butoxycarbonyl)-5-methyl-L-proline was reacted in the same way as in Step 7 of Example 1 using the compound obtained in Step 5 of Reference Example 14 instead of 4,7-diazaspiro[2.5]octane hydrochloride to give the title compound as a colorless solid.
$^1$H-NMR (400 MHz, DMSO-$d_6$) δ: 0.67-0.76 (2H, m), 1.11 (3H, d, J=6.6 Hz), 1.22 (3H, d, J=6.1 Hz), 1.26-1.33 (1H, m),

1.37 (9H, s), 1.45-1.53 (1H, m), 1.54-1.60 (1H, m), 1.72-1.79 (1H, m), 1.91-2.00 (1H, m), 2.06-2.16 (1H, m), 3.44-3.60 (3H, m), 3.82-3.93 (2H, m), 4.55 (1H, brs), 4.67 (1H, brs).
MS (ESI) m/z: 434 [$(M+H)^+$].

Step 2: (6S)-6-methyl-7-[(5S)-5-methyl-L-prolyl]-4-(trifluoroacetyl)-4,7-diazaspiro[2.5]octane

The compound obtained in Step 1 above was reacted in the same way as in Step 2 of Reference Example 15 to give the title compound as a colorless solid.
$^1$H-NMR (400 MHz, DMSO-$d_6$) δ: 0.66-0.75 (2H, m), 1.10 (6H, d, J=5.6 Hz), 1.14-1.20 (1H, m), 1.24-1.30 (1H, m), 1.43-1.52 (1H, m), 1.70-1.78 (1H, m), 1.79-1.96 (2H, m), 3.40-3.60 (4H, m), 3.73-3.95 (2H, m), 4.61-4.67 (1H, m).
MS (ESI) m/z: 334 [$(M+H)^+$].

Reference Example 27

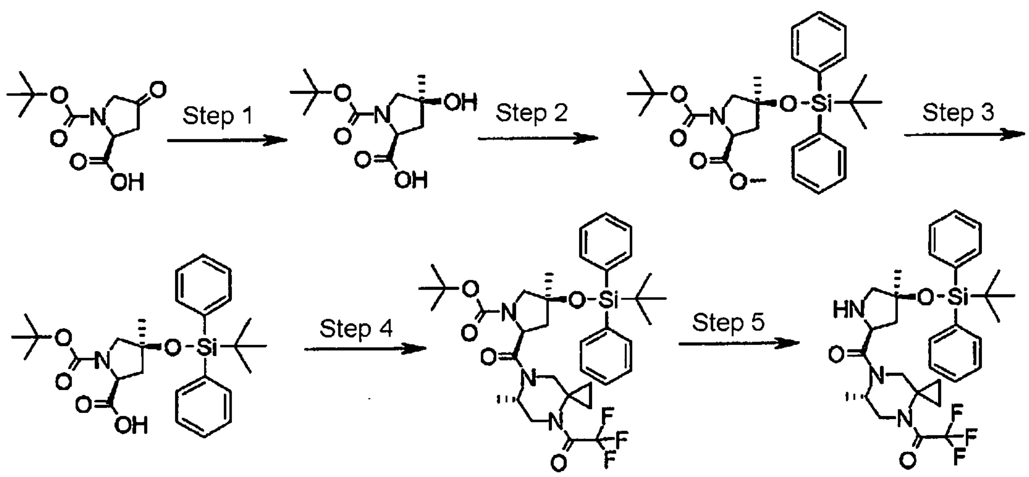


Step 1: (4S)-1-(tert-butoxycarbonyl)-4-hydroxy-4-methyl-L-proline

Methylmagnesium bromide (3.0 M diethyl ether solution) (24.5 ml, 73.1 mmol) was added dropwise to a tetrahydrofuran (250 ml) solution of 1-(tert-butoxycarbonyl)-4-oxo-L-proline (6.70 g, 29.2 mmol) at - 20˚C in a nitrogen atmosphere. The resulting mixture was stirred at the same temperature for 1 hour and then further stirred at room temperature for 10 hours. The reaction mixture was added into 1 N aqueous hydrochloric acid solution (100 ml) under ice cooling, followed by extraction with ethyl acetate. The organic layer was washed with brine and dried over anhydrous sodium sulfate. The solvent was evaporated under reduced pressure to give the title compound (4.44 g, 62%) as a pale yellow solid.
$^1$H-NMR (400 MHz, $CDCl_3$) δ: 1.39 (3H, s), 1.47 (9H, s), 2.15-2.18 (1H, m), 2.31-2.33 (1H, m), 3.28 (1H, d, J=11.2 Hz), 3.56 (1H, d, J=11.2 Hz), 4.40 (1H, d, J=9.5 Hz), 5.23 (1H, brs).

Step 2: 1-tert-butyl 2-methyl(2S,4S)-4-{[tert-butyl(diphenyl)silyl]oxy}-4-methylpyrrolidine-1,2-dicarboxylate

Trimethylsilyldiazomethane (2.0 M hexane solution) (3.00 ml, 6.00 mmol) was added dropwise to a suspension of the compound (1.20 g, 4.89 mmol) obtained in Step 1 above in benzene (50 ml) and methanol (5 ml). The resulting mixture was stirred for 1 hour, then the reaction mixture was concentrated under reduced pressure and the residue obtained was diluted with dimethylformamide (3 ml). Imidazole (0.90 g, 13.2 mmol) and tert-butyldiphenylchlorosilane (1.50 ml, 5.77 mmol) were added and the resulting mixture was heated to 60˚C and stirred for 24 hours. The reaction mixture was diluted with ethyl acetate, washed with an aqueous solution of saturated ammonium chloride and brine, and then dried over anhydrous sodium sulfate. The solvent was evaporated under reduced pressure and the residue was purified by silica gel column chromatography [n-hexane:ethyl acetate = 1:1 (v/v)] to give the title compound (0.95 g, 39%) as a colorless oil.
$^1$H-NMR (400 MHz, $CDCl_3$, rotameric mixture) δ: 0.99 and 1.01 (total 9H, each s), 1.13 and 1.14 (total 3H, each s), 1.39

and 1.42 (total 9H, each s), 2.04-2.25 (2H, m), 3.19 and 3.39 (total 1H, each d, J=10.7 Hz), 3.45 and 3.54 (total 1H, each d, J=10.7 Hz), 3.70 and 3.71 (total 3H, each s), 4.07-4.18 (1H, m), 7.37-7.45 (6H, m), 7.69-7.75 (4H, m).

Step 3: (4S)-1-(tert-butoxycarbonyl)-4-{[tert-butyl(diphenyl)silyl]oxy}-4-methyl-L-proline

0.5 N aqueous sodium hydroxide solution (6 ml) was added to a dioxane (10 ml) solution of the compound (0.95 g, 1.91 mmol) obtained in Step 2 above. The resulting mixture was stirred at room temperature for 18 hours and then the reaction mixture was concentrated under reduced pressure. The residue was diluted with water and made acidic with 0.5 N aqueous hydrochloric acid solution under ice cooling and the deposited solid was collected by filtration and dried to give the title compound (0.92 g, 99%) as a colorless solid.
$^1$H-NMR (400 MHz, $CDCl_3$, 70°C) δ: 1.05 (9H, s), 1.18 (3H, s), 1.49 (9H, s), 2.06-2.09 (1H, m), 2.66 (1H, brs), 3.25-3.28 (1H, m), 3.46-3.48 (1H, m), 4.27-4.29 (1H, m), 7.42-7.46 (6H, m), 7.75-7.76 (4H, m).
MS (ESI) m/z: 506 [$(M+Na)^+$].

Step 4: tert-butyl (2S,4S)-4-{[tert-butyl(diphenyl)silyl]oxy}-4-methyl-2-{[(6S)-6-methyl-4-(trifluoroacetyl)-4,7-diazaspiro[2.5]oct-7-yl]carbonyl}pyrrolidine-1-carboxylate

The compound obtained in Step 3 above was reacted in the same way as in Step 7 of Example 1 using the compound obtained in Step 5 of Reference Example 14 instead of 4,7-diazaspiro[2.5]octane hydrochloride to give the title compound as a colorless solid.
$^1$H-NMR (400 MHz, DMSO-$d_6$, 100°C) δ: 0.56 (1H, brs), 0.69 (1H, brs), 1.00 (9H, s), 1.05 (3H, d, J=6.6 Hz), 1.20 (3H, s), 1.24-1.26 (1H, m), 1.33 (9H, s), 1.47-1.49 (1H, m), 1.92 (1H, brs), 2.09 (1H, brs), 3.23-3.56 (4H, m), 3.87 (1H, brs), 4.43 (1H, brs), 4.59 (1H, brs), 7.41-7.49 (6H, m), 7.64-7.67 (4H, m).

Step 5: (6S)-7-[(4S)-4-{[tert-butyl(diphenyl)silyl]oxy}-4-methyl-L-prolyl]-6-methyl-4-(trifluoroacetyl)-4,7-diazaspiro[2.5]octane

The compound obtained in Step 4 above was reacted in the same way as in Step 2 of Reference Example 15 to give the title compound as a pale yellow oil.
$^1$H-NMR (400 MHz, DMSO-$d_6$, 100°C) δ: 0.62-0.64 (1H, m), 0.68-0.71 (1H, m), 0.98 (9H, s), 1.07 (3H, d, J=6.8 Hz), 1.21 (3H, s), 1.25 (1H, dt, J=12.6, 4.9 Hz), 1.48 (1H, dt, J=12.6, 5.2 Hz), 1.90 (1H, dd, J=12.6, 8.7 Hz), 2.01 (1H, dd, J=12.6, 7.7 Hz), 2.46 (1H, brs), 2.97-3.00 (1H, m), 3.20-3.98 (5H, m), 4.55 (1H, brs), 7.41-7.44 (6H, m), 7.65-7.68 (4H, m).
MS (ESI) m/z: 588 [$(M+H)^+$].

Reference Example 28

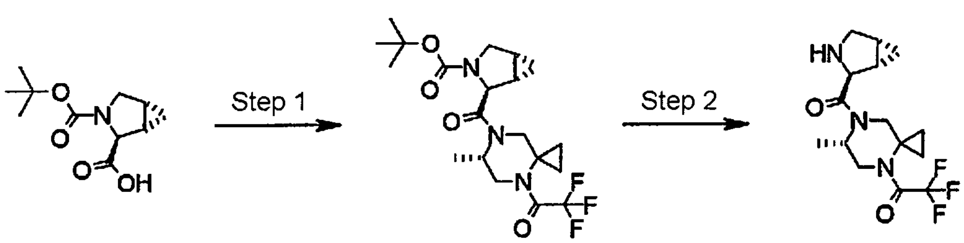


Step 1: tert-butyl (1S,2S,5R)-2-{[(6S)-6-methyl-4-(trifluoroacetyl)-4,7-diazaspiro[2.5]oct-7-yl]-carbonyl}-3-azabicyclo[3.1.0]hexane-3-carboxylate

(1S,2S,5R)-3-(tert-butoxycarbonyl)-3-azabicyclo[3.1.0]hexane-2-carboxylic acid was reacted in the same way as in Step 7 of Example 1 using the compound obtained in Step 5 of Reference Example 14 instead of 4,7-diazaspiro[2.5]octane hydrochloride to give the title compound as a colorless solid.
MS (ESI) m/z: 432 [$(M+H)^+$].

Step 2: (6S)-7-[(1S,2S,5R)-3-azabicyclo[3.1.0]hex-2-ylcarbonyl]-6-methyl-4-(trifluoroacetyl)-4,7-diazaspiro[2.5]octane

The compound obtained in Step 1 above was reacted in the same way as in Step 2 of Reference Example 15 to give the title compound as a colorless solid.
$^1$H-NMR (400 MHz, DMSO-$d_6$, 90˚C) δ: 0.26-0.29 (1H, m), 0.47-0.52 (1H, m), 0.72 (2H, brs), 1.09 (3H, d, J=6.1 Hz), 1.16-1.52 (5H, m), 2.64-2.66 (1H, m), 2.77-2.80 (2H, m), 3.49 (3H, brs), 3.86 (1H, brs), 4.67 (1H, brs).
MS (ESI) m/z: 332 [(M+H)$^+$].

Reference Example 29

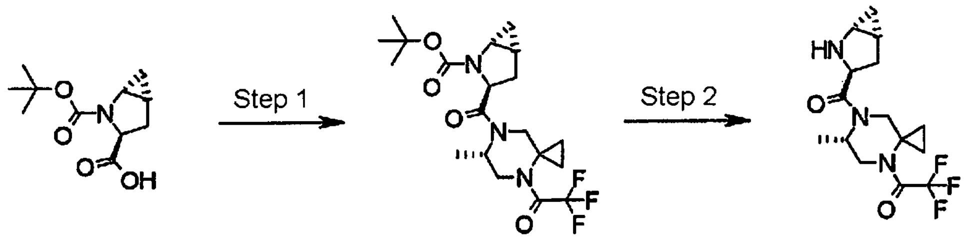


Step 1: tert-butyl (1R,3S,5R)-3-{[(6S)-6-methyl-4-(trifluoroacetyl)-4,7-diazaspiro[2.5]oct-7-yl]carbonyl}-2-azabicyclo [3.1.0]hexane-2-carboxylate

(1R,3S,5R)-2-(tert-butoxycarbonyl)-2-azabicyclo[3.1.0]hexane-3-carboxylic acid was reacted in the same way as in Step 7 of Example 1 using the compound obtained in Step 5 of Reference Example 14 instead of 4,7-diazaspiro [2.5]octane hydrochloride to give the title compound as a colorless solid.
$^1$H-NMR (400 MHz, DMSO-$d_6$, 100˚C) δ: 0.41 (1H, td, J=4.9, 2.5 Hz), 0.67-0.78 (3H, m), 1.11 (3H, d, J=6.8 Hz), 1.23-1.30 (1H, m), 1.39 (9H, s), 1.50 (1H, dt, J=12.7, 5.0 Hz), 1.55-1.62 (1H, m), 1.99-2.06 (1H, m), 2.26 (1H, dd, J=12.6, 9.6 Hz), 3.33 (1H, dq, J=7.3, 2.2 Hz), 3.48-3.58 (3H, m), 3.90 (1H, brs), 4.37 (1H, brs), 4.60 (1H, brs).

Step 2: (6S)-7-[(1R,3S,5R)-2-azabicyclo[3.1.0]hex-3-ylcarbonyl]-6-methyl-4-(trifluoroacetyl)-4,7-diazaspiro[2.5]octane

The compound obtained in Step 1 above was reacted in the same way as in Step 2 of Reference Example 15 to give the title compound as a colorless oil.
$^1$H-NMR (400 MHz, DMSO-$d_6$: 100˚C) δ: 0.33-0.39 (2H, m), 0.71 (2H, t, J=8.7 Hz), 1.10 (3H, d, J=6.8 Hz), 1.27 (1H, dd, J=15.1, 8.5 Hz), 1.31-1.37 (1H, m), 1.49 (1H, dd, J=15.1, 8.3 Hz), 1.75-1.82 (1H, m), 1.97 (1H, dd, J=12.0, 7.6 Hz), 2.73 (1H, brs), 2.83 (1H, td, J=5.9, 3.4 Hz), 3.38-3.62 (4H, m), 3.88 (1H, brs), 4.62 (1H, brs).

Reference Example 30

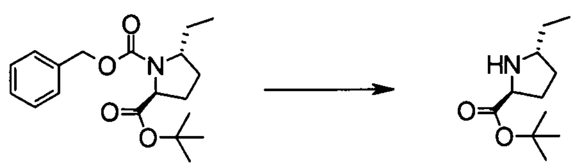

The compound obtained in Step 1 of Reference Example 9 was reacted in the same way as in Step 2 of Reference Example 1 to give the title compound as a colorless solid. $^1$H-NMR (400 MHz, $CDCl_3$) δ: 1.06 (3H, t, J=7.6 Hz), 1.49 (9H, s), 1.85-2.03 (3H, m), 2.12-2.24 (2H, m), 2.50-2.54 (1H, m), 3.50-3.54 (1H, m), 4.49 (1H, t, J=7.9 Hz).

Reference Example 31

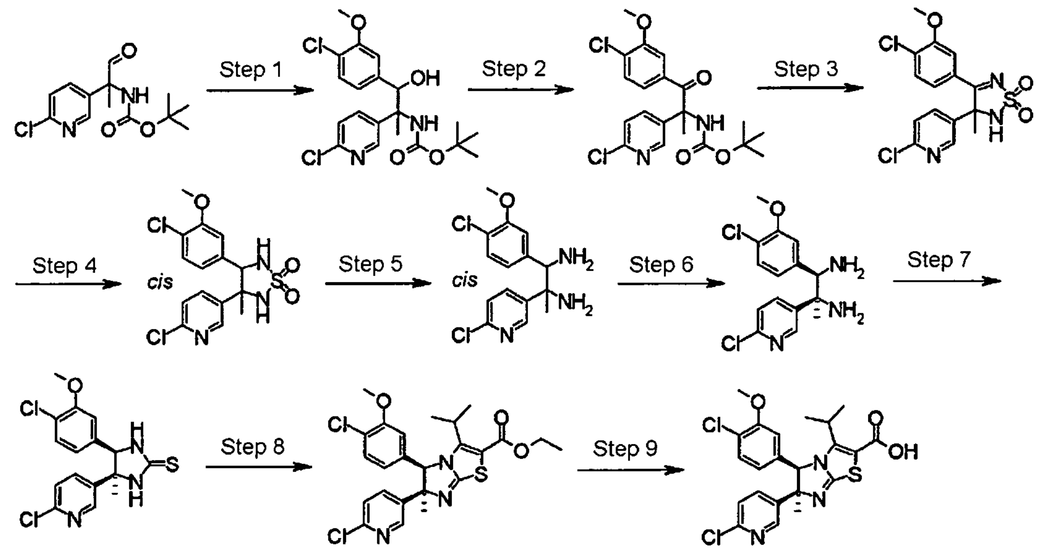


Step 1: tert-butyl [2-(4-chloro-3-methoxyphenyl)-1-(6-chloropyridin-3-yl)-2-hydroxy-1-methylethyl]carbamate

5-bromo-2-chloroanisole (11.7 g, 52.7 mmol) was gradually added to a tetrahydrofuran (50 ml) suspension of magnesium (flake) (1.07 g, 43.9 mmol) and iodine (10 mg). The resulting mixture was stirred for 1 hour and then cooled on ice and a tetrahydrofuran (50 ml) solution of tert-butyl [1-(6-chloropyridin-3-yl)-1-methyl-2-oxoethyl]carbamate (5.00 g, 17.6 mmol) was added dropwise. The resulting mixture was stirred at the same temperature for 2 hours and then 1 N aqueous hydrochloric acid solution was added, followed by extraction with ethyl acetate. The organic layer was washed with saturated aqueous sodium bicarbonate solution and brine and then dried over anhydrous sodium sulfate and the solvent was evaporated under reduced pressure. The residue was purified by silica gel column chromatography [n-hexane:ethyl acetate = 1:1 (v/v)] to give the title compound (6.89 g, 91.8%) as a colorless solid.
$^1$H-NMR (400 MHz, $CDCl_3$) δ: 1.38 (9H, s), 1.54 (3H, s), 3.63 (3H, s), 4.88 (1H, s), 5.58-5.63 (1H, m), 6.19-6.22 (1H, m), 6.55 (1H, dd, J=8.3, 1.7 Hz), 7.18-7.25 (2H, m), 7.37-7.43 (1H, m), 8.21 (1H, d, J=2.4 Hz).
MS (ESI) m/z: 427 [$(M+H)^+$].

Step 2: tert-butyl [2-(4-chloro-3-methoxyphenyl)-1-(6-chloropyridin-3-yl)-1-methyl-2-oxoethyl]carbamate

The compound obtained in Step 1 above was reacted in the same way as in Step 5 of Reference Example 13 to give the title compound as a colorless solid.
$^1$H-NMR (400 MHz, $CDCl_3$) δ: 1.32 (9H, brs), 2.07 (3H, s), 3.83 (3H, s), 6.46 (1H, brs), 6.98-7.04 (1H, m), 7.23-7.28 (2H, m), 7.34 (1H, dd, J=8.4, 0.6 Hz), 7.65-7.76 (1H, m), 8.45-8.50 (1H, m).

Step 3: 2-chloro-5-[4-(4-chloro-3-methoxyphenyl)-3-methyl-1,1-dioxido-2,3-dihydro-1,2,5-thiadiazol-3-yl]pyridine

The compound obtained in Step 2 above was reacted in the same way as in Step 6 of Reference Example 13 and then reacted in the same way as in Step 7 of Reference Example 13 to give the title compound as a colorless solid.
$^1$H-NMR (400 MHz, $CDCl_3$) δ: 2.12 (3H, s), 3.82 (1H, brs), 3.90 (3H, s), 6.92 (1H, dd, J=8.3, 2.1 Hz), 7.32 (1H, d, J=8.4 Hz), 7.42 (1H, dd, J=8.5, 0.7 Hz), 7.57 (1H, d, J=2.1 Hz), 7.72 (1H, dd, J=8.4, 2.7 Hz), 8.54 (1H, d, J=2.7 Hz).

Step 4: 2-chloro-5-[(3S*,4R*)-4-(4-chloro-3-methoxyphenyl)-3-methyl-1,1-dioxido-1,2,5-thiadiazolidin-3-yl]pyridine

The compound obtained in Step 3 above was reacted in the same way as in Step 8 of Reference Example 13 to give the title compound as a colorless solid.
$^1$H-NMR (400 MHz, $CDCl_3$) δ: 1.89 (3H, s), 3.62 (3H, s), 4.65 (2H, brs), 4.97 (1H, d, J=5.4 Hz), 6.20-6.22 (1H, m), 6.57 (1H, dd, J=8.0, 2.0 Hz), 7.22 (1H, d, J=8.5 Hz), 7.25-7.29 (1H, m), 7.57 (1H, dd, J=8.5, 2.7 Hz), 7.97 (1H, d, J=2.7 Hz).

Step 5: (1R*,2S*)-1-(4-chloro-3-methoxyphenyl)-2-(6-chloropyridin-3-yl)propane-1,2-diamine

The compound obtained in Step 4 above was reacted in the same way as in Step 9 of Reference Example 13 to give the title compound as an orange oil.
$^1$H-NMR (400 MHz, $CDCl_3$) δ: 1.54 (3H, s), 1.59 (4H, brs), 3.70 (3H, s), 4.07 (1H, s), 6.46-6.48 (1H, m), 6.64 (1H, dd, J=8.3, 2.1 Hz), 7.21 (2H, dd, J=8.2, 3.5 Hz), 7.59 (1H, dd, J=8.4, 2.5 Hz), 8.35 (1H, d, J=2.5 Hz).

Step 6: (1R,2S)-1-(4-chloro-3-methoxyphenyl)-2-(6-chloropyridin-3-yl)propane-1,2-diamine

The same procedures as in Step 1 of Example 1 were performed using the compound obtained in Step 5 above to give the title compound as a colorless solid.
$^1$H-NMR (400 MHz, $CDCl_3$) δ: 1.54 (3H, s), 1.62 (4H, brs), 3.71 (3H, s), 4.08 (1H, s), 6.46-6.48 (1H, m), 6.64 (1H, dd, J=8.1, 1.7 Hz), 7.21 (2H, dd, J=8.2, 2.8 Hz), 7.59 (1H, dd, J=8.3, 2.6 Hz), 8.36 (1H, d, J=2.6 Hz).

Step 7: (4S,5R)-5-(4-chloro-3-methoxyphenyl)-4-(6-chloropyridin-3-yl)-4-methylimidazolidine-2-thione

The compound obtained in Step 6 above was reacted in the same way as in Step 2 of Example 1 to give the title compound as a pale yellow solid.
$^1$H-NMR (400 MHz, $CDCl_3$) δ: 1.91 (3H, s), 3.66 (3H, s), 5.00 (1H, s), 6.29 (1H, d, J=1.8 Hz), 6.56 (1H, dd, J=8.0, 1.8 Hz), 6.73 (1H, brs), 7.11 (1H, d, J=8.4 Hz), 7.19 (1H, d, J=8.0 Hz), 7.23 (1H, dd, J=8.4, 2.6 Hz), 7.32 (1H, brs), 8.02 (1H, d, J=2.6 Hz).

Step 8: ethyl (5R,6S)-5-(4-chloro-3-methoxyphenyl)-6-(6-chloropyridin-3-yl)-3-isopropyl-6-methyl-5,6-dihydroimidazo [2,1-b][1,3]thiazole-2-carboxylate

The compound obtained in Step 7 above was reacted in the same way as in Step 3 of Example 1 to give the title compound as a pale yellow oil.
$^1$H-NMR (400 MHz, $CDCl_3$) δ: 0.92 (3H, d, J=7.3 Hz), 1.04 (3H, d, J=7.1 Hz), 1.34 (3H, t, J=7.2 Hz), 1.84 (3H, s), 3.31-3.46 (1H, m), 3.71 (3H, s), 4.21-4.31 (2H, m), 5.10 (1H, s), 6.06-6.66 (2H, m), 7.00 (1H, d, J=8.3 Hz), 7.12 (1H, d, J=8.0 Hz), 7.49 (1H, dd, J=8.3, 2.4 Hz), 8.25 (1H, d, J=2.4 Hz).

Step 9: (5R,6S)-5-(4-chloro-3-methoxyphenyl)-6-(6-chloropyridin-3-yl)-3-isopropyl-6-methyl-5,6-dihydroimidazo[2,1-b] [1,3]thiazole-2-carboxylic acid

The compound obtained in Step 8 above was reacted in the same way as in Step 4 of Example 1 to give the title compound as a colorless solid.
$^1$H-NMR (400 MHz, $CDCl_3$) δ: 0.93 (3H, d, J=7.0 Hz), 1.07 (3H, d, J=7.0 Hz), 1.95 (3H, s), 3.37-3.50 (1H, m), 3.72 (3H, brs), 5.27 (1H, s), 6.03-6.60 (1H, m), 7.03 (1H, d, J=8.5 Hz), 7.14 (1H, d, J=8.1 Hz), 7.25-7.27 (1H, m), 7.53 (1H, dd, J=8.3, 2.1 Hz), 8.26 (1H, d, J=2.1 Hz).

Reference Example 32

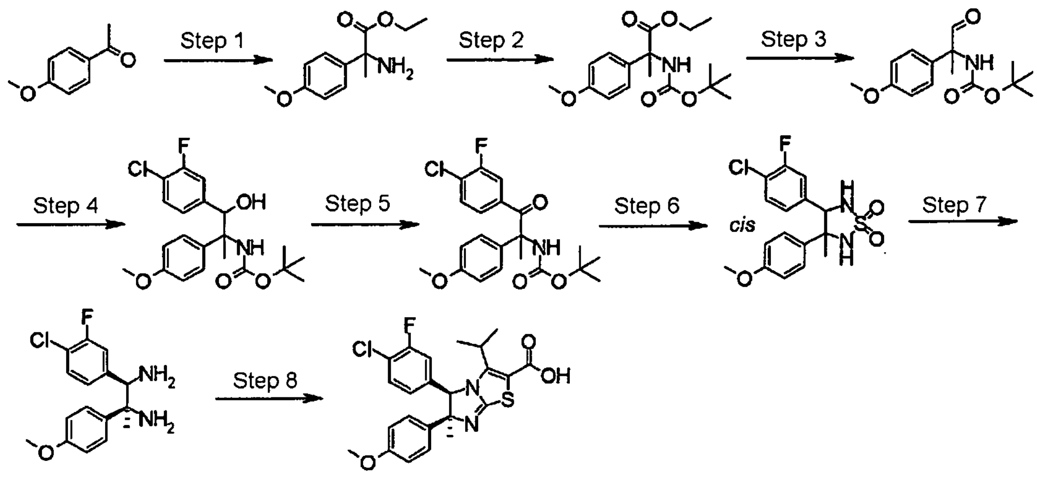


Step 1: ethyl 2-amino-2-(4-methoxyphenyl)propanoate

4-methoxyacetophenone instead of 1-(6-chloropyridin-3-yl)ethanone was reacted in the same way as in Step 1 of Reference Example 13 to give the title compound as a yellow oil.
$^1$H-NMR (400 MHz, $CDCl_3$) δ: 1.22 (3H, t, J=7.3 Hz), 1.68 (3H, s), 1.92 (2H, brs), 3.80 (3H, s), 4.08-4.23 (2H, m), 6.87 (2H, dt, J=9.5, 2.6 Hz), 7.41 (2H, dt, J=9.4, 2.6 Hz).

Step 2: ethyl 2-[(tert-butoxycarbonyl)amino]-2-(4-methoxyphenyl)propanoate

The compound obtained in Step 1 above was reacted in the same way as in Step 2 of Reference Example 13 to give the title compound as a pale yellow oil.
$^1$H-NMR (400 MHz, $CDCl_3$) δ: 1.19 (3H, t, J=7.1 Hz), 1.39 (9H, s), 1.96 (3H, s), 3.80 (3H, s), 4.11-4.21 (2H, m), 5.75 (1H, s), 6.86 (2H, d, J=8.8 Hz), 7.36 (2H, d, J=8.8 Hz).

Step 3: tert-butyl [1-(4-methoxyphenyl)-1-methyl-2-oxoethyl]carbamate

The compound obtained in Step 2 above was reacted in the same way as in Step 3 of Reference Example 13 to give the title compound as an orange oil.
$^1$H-NMR (400 MHz, $CDCl_3$) δ: 1.43 (9H, s), 1.82 (3H, s), 3.80 (3H, s), 5.64 (1H, brs), 6.91 (2H, dt, J=9.7, 2.7 Hz), 7.30 (2H, dt, J=9.7, 2.7 Hz), 9.26 (1H, s).

Step 4: tert-butyl [2-(4-chloro-3-fluorophenyl)-2-hydroxy-1-(4-methoxyphenyl)-1-methylethyl]carbamate

The compound obtained in Step 3 above was reacted in the same way as in Step 4 of Reference Example 13 to give the title compound as a colorless solid.
$^1$H-NMR (400 MHz, DMSO-$d_6$, 100°C) δ: 1.31 (9H, s), 1.47 (3H, s), 3.75 (3H, s), 4.79 (1H, d, J=4.6 Hz), 5.80 (1H, d, J=4.6 Hz), 6.25 (1H, s), 6.71-6.77 (1H, m), 6.78-6.82 (3H, m), 7.09-7.12 (2H, m), 7.31 (1H, t, J=7.9 Hz).

Step 5: tert-butyl [2-(4-chloro-3-fluorophenyl)-1-(4-methoxyphenyl)-1-methyl-2-oxoethyl]carbamate

The compound obtained in Step 4 above was reacted in the same way as in Step 5 of Reference Example 13 to give the title compound as a colorless solid.
$^1$H-NMR (400 MHz, $CDCl_3$) δ: 1.28 (9H, s), 1.93 (3H, s), 3.81 (3H, s), 6.09 (1H, s), 6.88-7.01 (3H, m), 7.22-7.50 (4H, m).

Step 6: (3R*,4S*)-4-(4-chloro-3-fluorophenyl)-3-(4-methoxyphenyl)-3-methyl-1,2,5-thiazolidine 1,1-dioxide

The compound obtained in Step 5 above was reacted in the same way as in Step 6 to Step 8 of Reference Example 13 in order to give the title compound as a colorless solid.
$^1$H-NMR (400 MHz, $CDCl_3$) δ: 1.87 (3H, s), 3.77 (3H, s), 4.58 (1H, brs), 4.67 (1H, s), 4.88 (1H, s), 6.60 (1H, dd, J=8.2, 2.1 Hz), 6.65 (1H, dd, J=9.6, 2.1 Hz), 6.74 (2H, dt, J=9.7, 2.7 Hz), 7.03 (2H, dt, J=9.7, 2.7 Hz), 7.19 (1H, t, J=8.2 Hz).

Step 7: (1R,2S)-1-(4-chloro-3-fluorophenyl)-2-(4-methoxyphenyl)propane-1,2-diamine

The compound obtained in Step 6 above was reacted in the same way as in Step 9 of Reference Example 13 and then the same procedures as in Step 1 of Example 1 were performed to give the title compound as a colorless solid. $^1$H-NMR (400 MHz, $CDCl_3$) δ: 1.46 (3H, s), 3.79 (3H, s), 4.05 (1H, s), 6.76 (1H, dd, J=8.3, 2.0 Hz), 6.81 (2H, dt, J=9.5, 2.6 Hz), 6.90 (1H, dd, J=10.5, 2.0 Hz), 7.17 (1H, t, J=7.9 Hz), 7.20-7.30 (2H, m).

Step 8: (5R,6S)-5-(4-chloro-3-fluorophenyl)-3-isopropyl-6-(4-methoxyphenyl)-6-methyl-5,6-dihydroimidazo[2,1-b][1,3]thiazole-2-carboxylic acid

The compound obtained in Step 7 above was reacted in the same way as in Step 2 to Step 4 of Example 1 in order to give the title compound as a pale yellow solid.
$^1$H-NMR (400 MHz, DMSO-$d_6$, 100˚C) δ: 0.92 (3H, d, J=7.1 Hz), 1.01 (3H, d, J=7.1 Hz), 1.79 (3H, s), 3.29-3.45 (1H, m), 3.64 (3H, s), 5.65 (1H, s), 6.64 (2H, dt, J=9.5, 2.6 Hz), 6.72 (1H, d, J=8.8 Hz), 6.86-6.94 (1H, m), 7.12 (2H, dt, J=9.5, 2.6 Hz), 7.27 (1H, t, J=8.1 Hz).
MS (ESI) m/z: 461 [$(M+H)^+$].

Reference Example 33

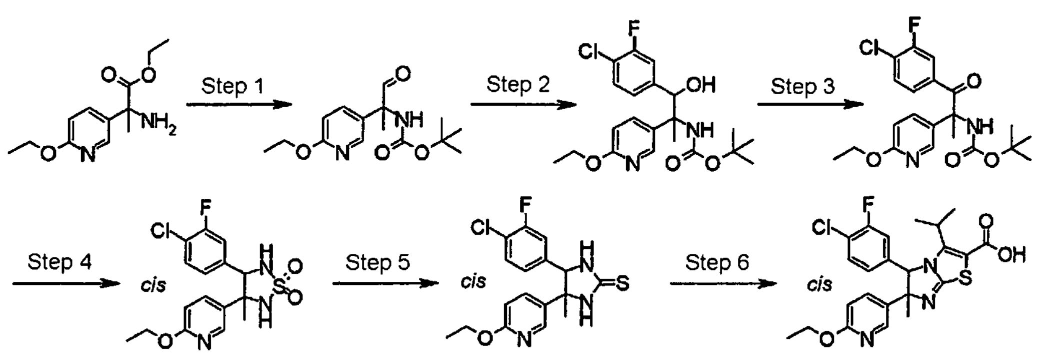


Step 1: tert-butyl [1-(6-ethoxypyridin-3-yl)-1-methyl-2-oxoethyl]carbamate

Ethyl 2-amino-2-(6-ethoxypyridin-3-yl)propanoate (Bioorg. Med. Chem. Lett., 2004, 14, 3525) was reacted in the same way as in Step 2 of Reference Example 13 and then reacted in the same way as in Step 3 of Reference Example 13 to give the title compound as a yellow oil. $^1$H-NMR (400 MHz, $CDCl_3$) δ: 1.39 (3H, t, J=7.1 Hz), 1.42 (9H, s), 1.81 (3H, s), 4.35 (2H, q, J=7.1 Hz), 5.69 (1H, s), 6.73 (1H, d, J=8.8 Hz), 7.56 (1H, dd, J=8.8, 2.7 Hz), 8.17 (1H, d, J=2.7 Hz), 9.28 (1H, s).

Step 2: tert-butyl [2-(4-chloro-3-fluorophenyl)-1-(6-ethoxypyridin-3-yl)-2-hydroxy-1-methylethyl]carbamate

The compound obtained in Step 1 above was reacted in the same way as in Step 4 of Reference Example 13 to give the title compound as a colorless solid.
$^1$H-NMR (400 MHz, $CDCl_3$, 70˚C) δ: 1.38 (3H, t, J=6.8 Hz), 1.43 (9H, s), 1.47 (3H, s), 4.37 (2H, q, J=6.8 Hz), 4.56-4.78 (1H, m), 4.98-5.10 (2H, m), 6.67 (1H, d, J=8.8 Hz), 6.77-6.85 (1H, m), 7.00 (1H, dd, J=10.1, 2.1 Hz), 7.17-7.30 (1H, m), 7.51 (1H, dd, J=8.8, 2.7 Hz), 8.10 (1H, d, J=2.7 Hz).

Step 3: tert-butyl [2-(4-chloro-3-fluorophenyl)-1-(6-ethoxypyridin-3-yl)-1-methyl-2-oxoethyl]carbamate

The compound obtained in Step 2 above was reacted in the same way as in Step 5 of Reference Example 13 to give the title compound as a colorless solid.
$^1$H-NMR (400 MHz, $CDCl_3$) δ: 1.30 (9H, s), 1.39 (3H, t, J=7.1 Hz), 1.98 (3H, s), 4.36 (2H, q, J=7.1 Hz), 6.18 (1H, s), 6.73 (1H, d, J=8.8 Hz), 7.28-7.40 (2H, m), 7.47 (1H, d, J=9.8 Hz), 7.57 (1H, d, J=8.0 Hz), 8.20 (1H, d, J=2.4 Hz).

Step 4: 5-[(3R*,4S*)-4-(4-chloro-3-fluorophenyl)-3-methyl-1,1-dioxido-1,2,5-thiadiazolidin-3-yl]-2-ethoxypyridine

The compound obtained in Step 3 above was reacted in the same way as in Step 6 to Step 8 of Reference Example 13 in order to give the title compound as a colorless solid.
$^1$H-NMR (400 MHz, $CDCl_3$) δ: 1.35 (3H, t, J=7.1 Hz), 1.87 (3H, s), 4.28 (2H, q, J=7.1 Hz), 4.79 (1H, s), 4.84 (1H, d, J=5.9 Hz), 4.93 (1H, d, J=5.9 Hz), 6.58 (1H, d, J=8.8 Hz), 6.67 (1H, d, J=8.3 Hz), 6.73 (1H, dd, J=9.5, 2.0 Hz), 7.20-7.30 (1H, m), 7.36 (1H, dd, J=8.8, 2.7 Hz), 7.83 (1H, d, J=2.7 Hz).

Step 5: (4R*,5S*)-5-(4-chloro-3-fluorophenyl)-4-(6-ethoxypyridin-3-yl)-4-methylimidazolidine-2-thione

The compound obtained in Step 4 above was reacted in the same way as in Step 9 of Reference Example 13 and then reacted in the same way as in Step 2 of Example 1 to give the title compound as a colorless solid.
$^1$H-NMR (400 MHz, $CDCl_3$) δ: 1.33 (3H, t, J=7.1 Hz), 1.90 (3H, s), 4.26 (2H, q, J=7.1 Hz), 4.94 (1H, s), 6.23 (1H, brs), 6.38 (1H, brs), 6.50 (1H, d, J=8.8 Hz), 6.68 (1H, dd, J=8.3, 2.0 Hz), 6.73 (1H, dd, J=9.5, 2.0 Hz), 7.15 (1H, dd, J=8.7, 2.4 Hz), 7.19 (1H, t, J=7.9 Hz), 7.76 (1H, d, J=2.4 Hz).

Step 6: (5R*,6S*)-5-(4-chloro-3-fluorophenyl)-6-(6-ethoxypyridin-3-yl)-3-isopropyl-6-methyl-5,6-dihydroimidazo[2,1-b][1,3]thiazole-2-carboxylic acid

The compound obtained in Step 5 above was reacted in the same way as in Step 3 of Example 1 and then reacted in the same way as in Step 4 of Example 1 to give the title compound as a colorless solid.
$^1$H-NMR (400 MHz, DMSO-$d_6$) δ: 0.74 (3H, d, J=7.1 Hz), 1.01 (3H, d, J=7.1 Hz), 1.19 (3H, t, J=6.8 Hz), 1.68 (3H, s), 3.52-3.67 (1H, m), 4.14 (2H, q, J=6.8 Hz), 5.57 (1H, s), 6.18-6.68 (2H, m), 6.46 (1H, d, J=8.8 Hz), 7.35 (1H, t, J=8.0 Hz), 7.47 (1H, dd, J=8.8, 2.4 Hz), 7.98 (1H, d, J=2.4 Hz).
MS (ESI) m/z: 476 [$(M+H)^+$].

Reference Example 34

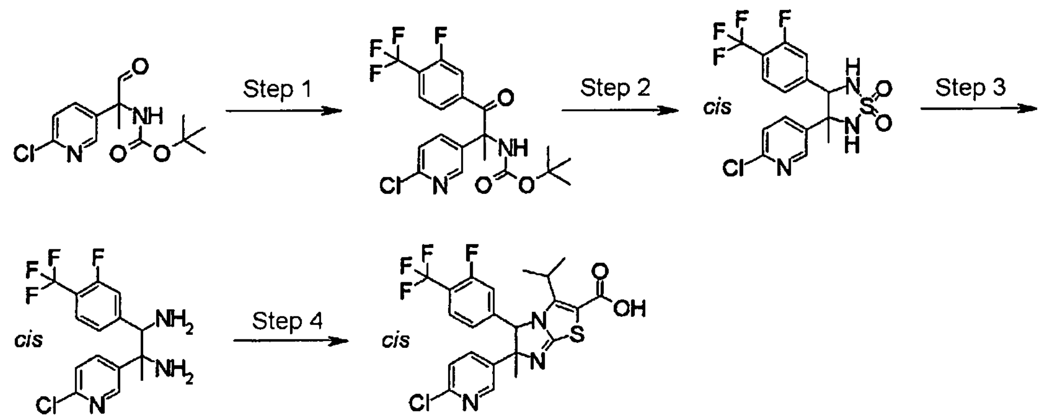


Step 1: tert-butyl {1-(6-chloropyridin-3-yl)-2-[3-fluoro-4-(trifluoromethyl)phenyl]-1-methyl-2-oxoethyl}carbamate

4-bromo-2-fluoro-1-(trifluoromethyl)benzene instead of 5-bromo-2-chloroanisole was reacted in the same way as in Step 1 of Reference Example 31 and then reacted in the same way as in Step 5 of Reference Example 13 to give the title compound as a colorless solid.
$^1$H-NMR (400 MHz, $CDCl_3$) δ: 1.30 (9H, s), 2.01 (3H, s), 6.13 (1H, s), 7.37 (1H, dd, J=8.5, 0.5 Hz), 7.44-7.46 (1H, m), 7.51 (1H, d, J=11.2 Hz), 7.57 (1H, t, J=7.6 Hz), 7.72 (1H, d, J=7.6 Hz), 8.46 (1H, d, J=2.4 Hz).

Step 2: 2-chloro-5-{4-[3-fluoro-4-(trifluoromethyl)phenyl]-3-methyl-1,1-dioxido-1,2,5-thiadiazolidin-3-yl}pyridine

The compound obtained in Step 1 above was reacted in the same way as in Step 6 to Step 8 of Reference Example 13 in order to give the title compound as a colorless solid.
$^1$H-NMR (400 MHz, DMSO-$d_6$) δ: 1.83 (3H, s), 5.08 (1H, d, J=4.4 Hz), 7.13 (1H, d, J=8.1 Hz), 7.24 (1H, d, J=12.0 Hz), 7.31 (1H, d, J=8.5 Hz), 7.54 (1H, dd, J=8.5, 2.7 Hz), 7.60 (1H, t, J=7.9 Hz), 8.10 (1H, d, J=2.4 Hz), 8.15 (2H, d, J=7.3 Hz).

Step 3: 2-(6-chloropyridin-3-yl)-1-[3-fluoro-4-(trifluoromethyl)phenyl]propane-1,2-diamine

The compound obtained in Step 2 above was reacted in the same way as in Step 9 of Reference Example 13 to give the title compound as a yellow oil.
$^1$H-NMR (400 MHz, $CDCl_3$) δ: 1.56 (3H, s), 1.62 (4H, s), 4.15 (1H, s), 6.88 (1H, d, J=8.3 Hz), 7.04 (1H, d, J=11.7 Hz), 7.24 (1H, d, J=8.5 Hz), 7.42 (1H, t, J=7.7 Hz), 7.62 (1H, dd, J=8.5, 2.7 Hz), 8.37 (1H, d, J=2.0 Hz).

Step 4: 6-(6-chloropyridin-3-yl)-5-[3-fluoro-4-(trifluoromethyl)phenyl]-3-isopropyl-6-methyl-5,6-dihydroimidazo[2,1-b][1,3]thiazole-2-carboxylic acid

The compound obtained in Step 3 above was reacted in the same way as in Step 2 to Step 4 of Example 1 in order to give the title compound as a pale yellow solid.
$^1$H-NMR (400 MHz, DMSO-$d_6$) δ: 0.93 (3H, d, J=7.1 Hz), 1.02 (3H, d, J=7.3 Hz), 1.92 (3H, s), 3.32-3.39 (1H, m), 5.96 (1H, s), 6.98 (1H, s), 7.15 (1H, s), 7.19 (1H, d, J=8.3 Hz), 7.55 (1H, t, J=7.8 Hz), 7.65 (1H, dd, J=8.4, 2.6 Hz), 8.26 (1H, d, J=2.7 Hz).
MS (ESI) m/z: 500 [$(M+H)^+$].

Reference Example 35

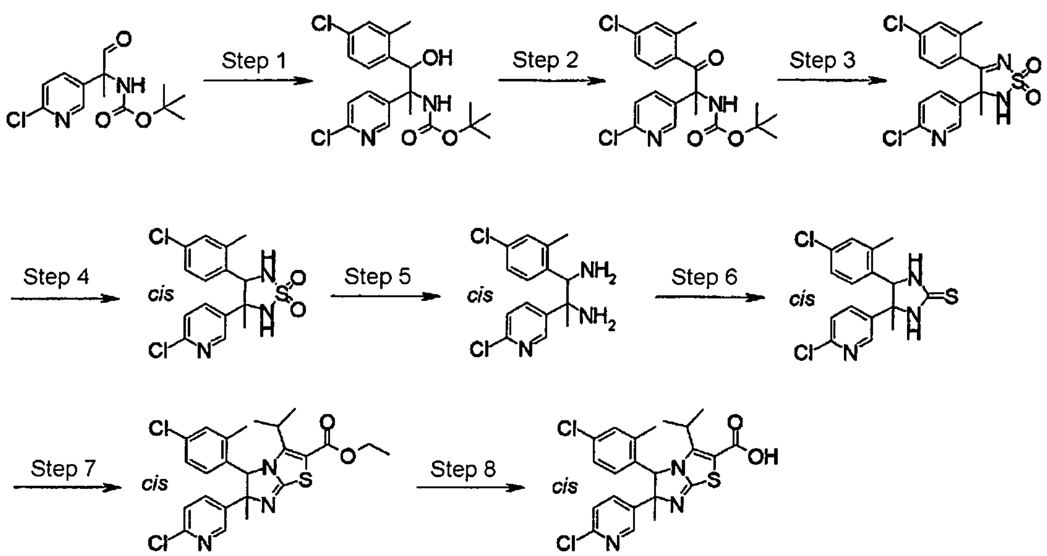


Step 1: tert-butyl [2-(4-chloro-2-methylphenyl)-1-(6-chloropyridin-3-yl)-2-hydroxy-1-methylethyl]carbamate

2-bromo-5-chlorotoluene instead of 5-bromo-2-chloroanisole was reacted in the same way as in Step 1 of Reference Example 31 to give the title compound as a colorless solid.
$^1$H-NMR (400 MHz, $CDCl_3$) δ: 1.45 (9H, brs), 1.55 (3H, s), 1.94 (3H, s), 4.27 (1H, brs), 5.18-5.47 (2H, m), 7.04-7.08 (1H, m), 7.20-7.27 (2H, m), 7.44 (1H, d, J=8.3 Hz), 7.51-7.60 (1H, m), 8.38 (1H, s).

Step 2: tert-butyl [2-(4-chloro-2-methylphenyl)-1-(6-chloropyridin-3-yl)-1-methyl-2-oxoethyl]carbamate

The compound obtained in Step 1 above was reacted in the same way as in Step 5 of Reference Example 13 to give the title compound as a colorless solid.
$^1$H-NMR (400 MHz, $CDCl_3$) δ: 1.33 (9H, s), 1.96 (3H, s), 2.19 (3H, s), 6.33-6.52 (2H, m), 6.97 (1H, dd, J=8.4, 2.1 Hz), 7.19-7.22 (1H, m), 7.34 (1H, d, J=8.4 Hz), 7.66 (1H, dd, J=8.4, 2.6 Hz), 8.41 (1H, d, J=2.6 Hz).

Step 3: 2-chloro-5-[4-(4-chloro-2-methylphenyl)-3-methyl-1,1-dioxido-2,3-dihydro-1,2,5-thiadiazol-3-yl]pyridine

The compound obtained in Step 2 above was reacted in the same way as in Step 6 of Reference Example 13 and then reacted in the same way as in Step 7 of Reference Example 13 to give the title compound as a yellow oil. $^1$H-NMR (400 MHz, $CDCl_3$) δ: 2.02 (3H, s), 2.16 (3H, s), 4.89 (1H, s), 6.73 (1H, d, J=8.5 Hz), 7.14 (1H, dd, J=8.5, 2.0 Hz), 7.30 (1H, d, J=2.0 Hz), 7.39 (1H, d, J=8.5 Hz), 7.80 (1H, dd, J=8.4, 2.7 Hz), 8.28 (1H, d, J=2.7 Hz).

Step 4: 2-chloro-5-[(3R*,4S*)-4-(4-chloro-2-methylphenyl)-3-methyl-1,1-dioxido-1,2,5-thiadiazolidin-3-yl] pyridine

The compound obtained in Step 3 above was reacted in the same way as in Step 8 of Reference Example 13 to give the title compound as a colorless solid.
$^1$H-NMR (400 MHz, $CDCl_3$) δ: 1.87 (3H, s), 2.43 (3H, s), 4.52 (1H, d, J=5.9 Hz), 4.63 (1H, s), 5.32 (1H, d, J=6.1 Hz), 6.34 (1H, d, J=8.5 Hz), 6.93 (1H, dd, J=8.5, 2.1 Hz), 7.19-7.24 (2H, m), 7.61 (1H, dd, J=8.5, 2.7 Hz), 7.95 (1H, d, J=2.7 Hz).

Step 5: (1R*,2S*)-1-(4-chloro-2-methylphenyl)-2-(6-chloropyridin-3-yl)propane-1,2-diamine

The compound obtained in Step 4 above was reacted in the same way as in Step 9 of Reference Example 13 to give the title compound as a pale yellow oil.
$^1$H-NMR (400 MHz, $CDCl_3$) δ: 1.53 (4H, brs), 1.58 (3H, s), 2.13 (3H, s), 4.28 (1H, s), 7.04-7.12 (3H, m), 7.19 (1H, d, J=8.4 Hz), 7.57 (1H, dd, J=8.4, 2.6 Hz), 8.38 (1H, d, J=2.6 Hz) .

Step 6: (4R*,5S*)-5-(4-chloro-2-methylphenyl)-4-(6-chloropyridin-3-yl)-4-methylimidazolidine-2-thione

The compound obtained in Step 5 above was reacted in the same way as in Step 2 of Example 1 to give the title compound as a pale yellow solid.
$^1$H-NMR (400 MHz, $CDCl_3$) δ: 1.99 (3H, s), 2.17 (3H, s), 5.25 (1H, s), 6.24 (1H, brs), 6.44 (1H, brs), 6.88 (1H, d, J=8.3 Hz), 6.95-7.04 (2H, m), 7.07 (1H, dd, J=8.4, 0.6 Hz), 7.32 (1H, dd, J=8.3, 2.7 Hz), 8.11 (1H, d, J=2.0 Hz).

Step 7: ethyl (5R*,6S*)-5-(4-chloro-2-methylphenyl)-6-(6-chloropyridin-3-yl)-3-isopropyl-6-methyl-5,6-dihydroimidazo [2,1-b][1,3]thiazole-2-carboxylate

The compound obtained in Step 6 above was reacted in the same way as in Step 3 of Example 1 to give the title compound as a colorless solid.
$^1$H-NMR (400 MHz, $CDCl_3$) δ: 0.89 (3H, d, J=7.2 Hz), 0.98 (3H, d, J=7.2 Hz), 1.34 (3H, t, J=7.1 Hz), 1.88 (3H, s), 2.23 (3H, s), 3.19-3.30 (1H, m), 4.26 (2H, q, J=7.1 Hz), 5.34 (1H, s), 6.55 (1H, d, J=8.5 Hz), 6.92 (1H, d, J=2.2 Hz), 6.95-7.01 (2H, m), 7.47 (1H, dd, J=8.3, 2.4 Hz), 8.23 (1H, d, J=2.4 Hz).
MS (ESI) m/z: 490 [$(M+H)^+$] .

Step 8: (5R*,6S*)-5-(4-chloro-2-methylphenyl)-6-(6-chloropyridin-3-yl)-3-isopropyl-6-methyl-5,6-dihydroimidazo[2,1-b] [1,3]thiazole-2-carboxylic acid

The compound obtained in Step 7 above was reacted in the same way as in Step 4 of Example 1 to give the title compound as a colorless solid.
$^1$H-NMR (400 MHz, $CDCl_3$) δ: 0. 88 (3H, d, J=7.2 Hz), 1.00 (3H, d, J=7.2 Hz), 2.02 (3H, s), 2.23 (3H, s), 3.25-3.39 (1H, m), 5.49 (1H, s), 6.51 (1H, d, J=8.3 Hz), 6.95 (1H, d, J=2.0 Hz), 6.98-7.06 (2H, m), 7.52 (1H, dd, J=8.4, 2.5 Hz), 8.24 (1H, d, J=2.5 Hz).
MS (ESI) m/z: 462 [$(M+H)^+$] .

Reference Example 36

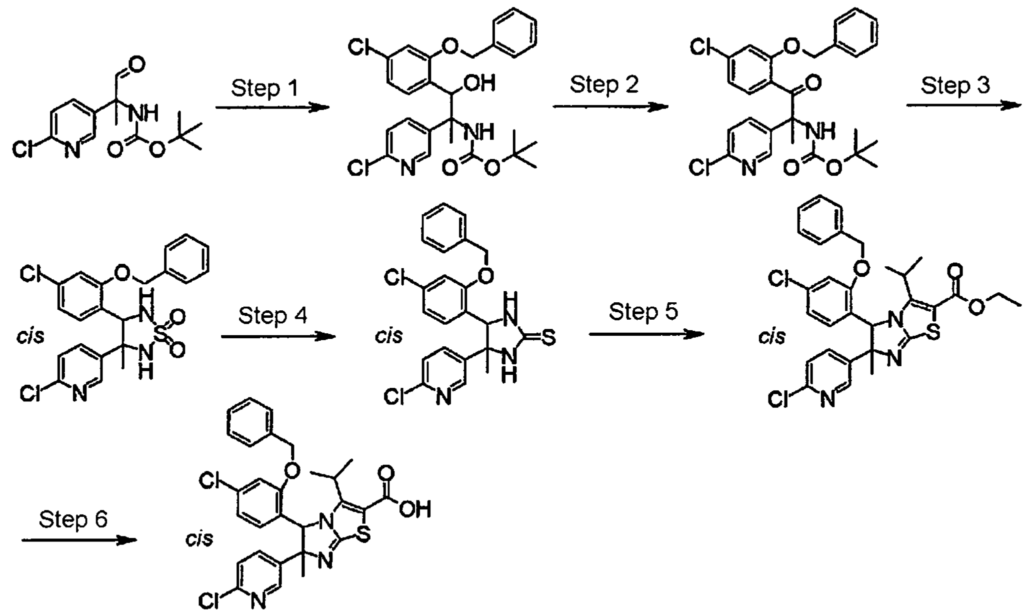


Step 1: tert-butyl {2-[2-(benzyloxy)-4-chlorophenyl]-1-(6-chloropyridin-3-yl)-2-hydroxy-1-methylethyl}carbamate

2-(benzyloxy)-1-bromo-4-chlorobenzene (W02004/039753) instead of 5-bromo-2-chloroanisole was reacted in the same way as in Step 1 of Reference Example 31 to give the title compound as a colorless solid. $^1$H-NMR (400 MHz, $CDCl_3$, 70°C) δ: 1.37 (9H, s), 1.60 (3H, s), 3.54 (1H, brs), 4.95 (2H, dd, J=13.9, 11.2 Hz), 5.28 (1H, d, J=6.3 Hz), 5.59 (1H, s), 6.89-6.97 (3H, m), 7.10 (1H, d, J=8.3 Hz), 7.32-7.47 (6H, m), 8.22 (1H, d, J=2.4 Hz) .

Step 2: tert-butyl {2-[2-(benzyloxy)-4-chlorophenyl]-1-(6-chloropyridin-3-yl)-1-methyl-2-oxoethyl}carbamate

The compound obtained in Step 1 above was reacted in the same way as in Step 5 of Reference Example 13 to give the title compound as a colorless solid.
MS (ESI) m/z: 501 [$(M+H)^+$] .

Step 3: 5-{(3R*,4S*)-4-[2-(benzyloxy)-4-chlorophenyl]-3-methyl-1,1-dioxido-1,2,5-thiadiazolidin-3-yl}-2-chloropyridine

The compound obtained in Step 2 above was reacted in the same way as in Step 6 to Step 8 of Reference Example 13 in order to give the title compound as a colorless solid.
$^1$H-NMR (400 MHz, $CDCl_3$) δ: 1.77 (3H, s), 4.43 (1H, d, J=10.7 Hz), 4.57 (1H, s), 4.77 (1H, d, J=10.7 Hz), 4.95 (1H, d, J=10.5 Hz), 5.92 (1H, d, J=10.5 Hz), 6.84 (1H, d, J=2.0 Hz), 6.94 (1H, dd, J=8.4, 0.6 Hz), 7.00 (1H, dd, J=8.4, 2.0 Hz), 7.09 (1H, d, J=8.4 Hz), 7.18-7.23 (2H, m), 7.38-7.46 (3H, m), 7.54 (1H, dd, J=8.4, 2.4 Hz), 7.71 (1H, d, J=2.4 Hz).

Step 4: (4R*, 5S*) -5- [2- (benzyloxy) -4- chlorophenyl] -4- (6-chloropyridin-3-yl)-4-methylimidazolidine-2-thione

The compound obtained in Step 3 above was reacted in the same way as in Step 9 of Reference Example 13 and then reacted in the same way as in Step 2 of Example 1 to give the title compound as a colorless solid.
$^1$H-NMR (400 MHz, DMSO-$d_6$) δ: 1.65 (3H, s), 4.93 (1H, d, J=11.7 Hz), 5.02 (1H, d, J=11.7 Hz), 5.17 (1H, s), 6.80 (1H, d, J=8.3 Hz), 6.87 (1H, d, J=8.3 Hz), 6.93 (1H, d, J=1.7 Hz), 7.24 (1H, d, J=8.4 Hz), 7.29 (1H, dd, J=8.4, 2.6 Hz), 7.38 (1H, t, J=7.2 Hz), 7.45 (2H, t, J=7.4 Hz), 7.51 (2H, d, J=7.1 Hz), 7.91 (1H, d, J=2.6 Hz), 8.73 (1H, s), 8.87 (1H, s).

Step 5: ethyl (5R*,6S*)-5-[2-(benzyloxy)-4-chlorophenyl]-6-(6-chloropyridin-3-yl)-3-isopropyl-6-methyl-5,6-dihydroimidazo[2,1-b][1,3]thiazole-2-carboxylate

The compound obtained in Step 4 above was reacted in the same way as in Step 3 of Example 1 to give the

title compound as a colorless solid.
$^1$H-NMR (400 MHz, DMSO-$d_6$) δ: 0.87 (3H, d, J=6.8 Hz), 0.97 (3H, d, J=6.8 Hz), 1.26 (3H, t, J=7.2 Hz), 1.69 (3H, s), 3.14-3.30 (1H, m), 4.21 (2H, q, J=7.2 Hz), 5.10 (1H, d, J=12.2 Hz), 5.16 (1H, d, J=12.2 Hz), 5.72 (1H, s), 6.54 (1H, d, J=8.3 Hz), 6.82 (1H, dd, J=8.3, 2.2 Hz), 6.98 (1H, d, J=2.2 Hz), 7.11 (1H, d, J=8.3 Hz), 7.29-7.57 (6H, m), 8.13 (1H, d, J=2.2 Hz).

Step 6: (5R*,6S*)-5-[2-(benzyloxy)-4-chlorophenyl]-6-(6-chloropyridin-3-yl)-3-isopropyl-6-methyl-5,6-dihydroimidazo [2,1-b][1,3]thiazole-2-carboxylic acid

The compound obtained in Step 5 above was reacted in the same way as in Step 4 of Example 1 to give the title compound as a colorless solid.
$^1$H-NMR (400 MHz, DMSO-$d_6$) δ: 0.81 (3H, d, J=7.1 Hz), 0.94 (3H, d, J=7.1 Hz), 1.67 (3H, s), 5.03 (1H, d, J=12.0 Hz), 5.16 (1H, d, J=12.0 Hz), 5.71 (1H, s), 6.51 (1H, d, J=8.3 Hz), 6.85 (1H, dd, J=8.3, 1.7 Hz), 6.99 (1H, d, J=1.7 Hz), 7.18-7.55 (8H, m), 8.11 (1H, d, J=2.4 Hz).

Reference Example 37

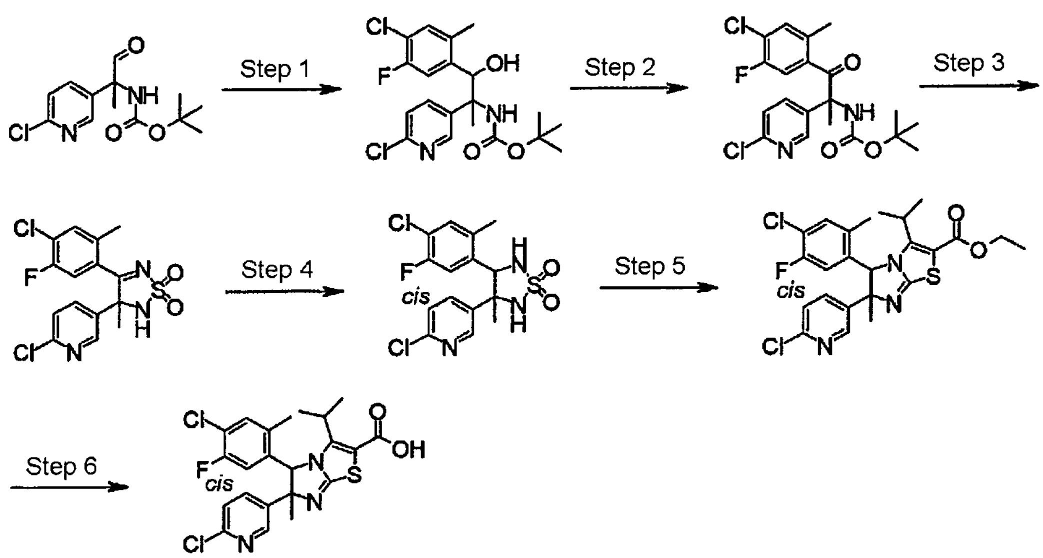


Step 1: tert-butyl [2-(4-chloro-5-fluoro-2-methylphenyl)-1-(6-chloropyridin-3-yl)-2-hydroxy-1-methylethyl]carbamate

1-bromo-4-chloro-5-fluoro-2-methylbenzene (WO2008/119744) instead of 5-bromo-2-chloroanisole was reacted in the same way as in Step 1 of Reference Example 31 to give the title compound as a pale yellow solid. $^1$H-NMR (400 MHz, $CDCl_3$) δ: 1.47 (9H, brs), 1.56 (3H, s), 2.00 (3H, s), 4.95 (1H, br), 5.30 (1H, br), 7.12 (1H, d, J=7.4 Hz), 7.27-7.36 (2H, m), 7.63-7.68 (1H, m), 8.41-8.43 (1H, m).
MS (ESI) m/z: 429 [$(M+H)^+$] .

Step 2: tert-butyl [2-(4-chloro-5-fluoro-2-methylphenyl)-1-(6-chloropyridin-3-yl)-1-methyl-2-oxoethyl]carbamate

The compound obtained in Step 1 above was reacted in the same way as in Step 5 of Reference Example 13 to give the title compound as a pale yellow oil.
$^1$H-NMR (400 MHz, $CDCl_3$) δ: 1.32 (9H, s), 1.96 (3H, s), 2.13 (3H, s), 6.20 (1H, brs), 6.45 (1H, brs), 7.24 (1H, d, J=7.4 Hz), 7.35 (1H, d, J=8.5 Hz), 7.67 (1H, dd, J=8.5, 2.5 Hz), 8.41 (1H, d, J=2.5 Hz).
MS (ESI) m/z: 427 [$(M+H)^+$].

Step 3: 2-chloro-5-[4-(4-chloro-5-fluoro-2-methylphenyl)-3-methyl-1,1-dioxido-2,3-dihydro-1,2,5-thiadiazol-3-yl]pyridine

The compound obtained in Step 2 above was reacted in the same way as in Step 6 of Reference Example 13

and then reacted in the same way as in Step 7 of Reference Example 13 to give the title compound as a pale yellow oil. $^1$H-NMR (400 MHz, $CDCl_3$) δ: 2.04 (3H, s), 2.06 (3H, s), 5.05 (1H, s), 6.65 (1H, d, J=9.0 Hz), 7.34 (1H, d, J=7.3 Hz), 7.40 (1H, d, J=8.5 Hz), 7.81 (1H, dd, J=8.5, 2.5 Hz), 8.28 (1H, d, J=2.5 Hz).

Step 4: 2-chloro-5-[(3R*,4S*)4-(4-chloro-5-fluoro-2-methylphenyl)-3-methyl-1,1-dioxido-1,2,5-thiadiazolidin-3-yl] pyridine

The compound obtained in Step 3 above was reacted in the same way as in Step 8 of Reference Example 13 to give the title compound as a colorless solid.
$^1$H-NMR (400 MHz, DMSO-$d_6$) δ: 1.85 (3H, s), 2.21 (3H, s), 5.02 (1H, s), 6.84 (1H, d, J=11.4 Hz), 7.31-7.36 (2H, m), 7.60 (1H, dd, J=8.5, 2.6 Hz), 8.09 (2H, br), 8.15 (1H, d, J=2.6 Hz).
MS (ESI) m/z: 390 [$(M+H)^+$] .

Step 5: ethyl (5R*,6S*)-5-(4-chloro-5-fluoro-2-methylphenyl)-6-(6-chloropyridin-3-yl)-3-isopropyl-6-methyl-5,6-dihydroimidazo[2,1-b][1,3]thiazole-2-carboxylate

The compound obtained in Step 4 above was reacted in the same way as in Step 9 of Reference Example 13, reacted in the same way as in Step 2 of Example 1, and then further reacted in the same way as in Step 3 of Example 1 to give the title compound as a colorless solid. $^1$H-NMR (400 MHz, $CDCl_3$) δ: 0.89 (3H, d, J=7.1 Hz), 1.02 (3H, d, J=7.1 Hz), 1.35 (3H, t, J=7.3 Hz), 1.88 (3H, s), 2.21 (3H, s), 3.28-3.36 (1H, m), 4.27 (2H, q, J=7.3 Hz), 5.30 (1H, s), 6.42 (1H, d, J=7.4 Hz), 6.98 (1H, d, J=7.4 Hz), 7.02 (1H, d, J=8.3 Hz), 7.53 (1H, dd, J=8.3, 2.4 Hz), 8.23 (1H, d, J=2.4 Hz).
MS (ESI) m/z: 508 [$(M+H)^+$].

Step 6: (5R*,6S*)-5-(4-chloro-5-fluoro-2-methylphenyl)-6-(6-chloropyridin-3-yl)-3-isopropyl-6-methyl-5,6-dihydroimidazo[2,1-b][1,3]thiazole-2-carboxylic acid

The compound obtained in Step 5 above was reacted in the same way as in Step 4 of Example 1 to give the title compound as a colorless solid.
$^1$H-NMR (400 MHz, DMSO-$d_6$) δ: 0.85 (3H, d, J=7.1 Hz), 1.12 (3H, d, J=7.1 Hz), 2.08 (3H, s), 2.27 (3H, s), 2.80-2.84 (1H, m), 6.08 (1H, s), 6.83 (1H, d, J=10.0 Hz), 7.31-7.35 (2H, m), 7.78 (1H, dd, J=8.3, 2.7 Hz), 8.36 (1H, d, J=2.7 Hz) .

Reference Example 38

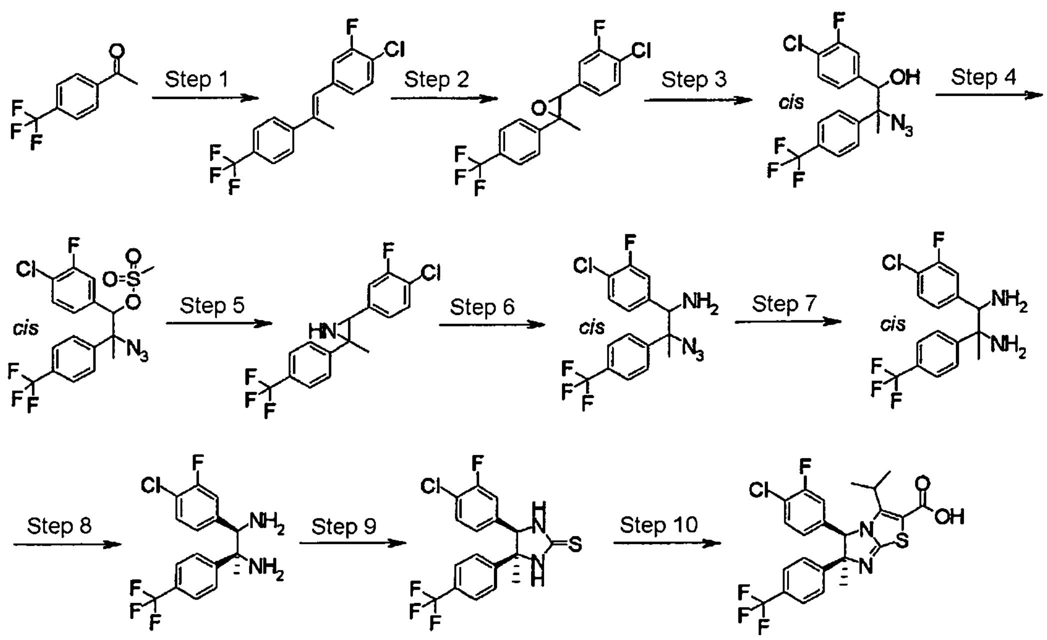

Step 1: 1-chloro-2-fluoro-4-{(1E)-2-[4-(trifluoromethyl)phenyl]prop-1-en-1-yl}benzene

Sodium hydride (55% containing powder) (1.20 g, 72.4 mmol) was gradually added to a dimethylformamide (20 ml) solution of diethyl (4-chloro-3-fluorobenzyl)phosphonate (7.07 g, 25.2 mmol) under ice cooling, the resulting mixture was stirred for 50 minutes and then a dimethylformamide (5 ml) solution of 1-[4-(trifluoromethyl)phenyl]ethanone (4.31 g, 22.9 mmol) was added. The resulting mixture was stirred at room temperature for 18 hours and then the reaction mixture was added into ice-cold 1 N aqueous hydrochloric acid solution (50 ml). After extraction with ethyl acetate, the organic layer was washed with brine and then dried over anhydrous sodium sulfate. The solvent was evaporated under reduced pressure, then methanol was added to the residue obtained and the precipitated solid was collected by filtration and dried to give the title compound (4.15 g, 58%) as a pale yellow solid.
$^{1}$H-NMR (400 MHz, $CDCl_3$) δ: 2.28 (3H, d, J=1.2 Hz), 6.76 (1H, s), 7.08 (1H, dd, J=8.3, 1.7 Hz), 7.15 (1H, dd, J=8.3, 1.7 Hz), 7.40 (1H, t, J=8.3 Hz), 7.59 (2H, d, J=8.8 Hz), 7.63 (2H, d, J=8.8 Hz).

Step 2: (2R*,3R*)-3-(4-chloro-3-fluorophenyl)-2-methyl-2-[4-(trifluoromethyl)phenyl]oxirane

Disodium hydrogen phosphate (5.62 g, 39.6 mmol) and m-chloroperbenzoic acid (70% containing) (3.90 g, 15.8 mmol) were added to a dichloromethane (60 ml) solution of the compound (4.15 g, 13.2 mmol) obtained in Step 1 above and the resulting mixture was stirred at room temperature for 20 hours. The reaction mixture was diluted with dichloromethane and then stirred after addition of 10% aqueous sodium thiosulfate solution. The organic layer was washed with 1 N aqueous sodium hydroxide solution and brine and dried over anhydrous sodium sulfate. The solvent was evaporated under reduced pressure to give the title compound (4.87 g, 99%) as a colorless solid.
$^{1}$H-NMR (400 MHz, $CDCl_3$) δ: 1.49 (3H, s), 3.89 (1H, s), 7.11 (1H, dd, J=8.2, 1.8 Hz), 7.18 (1H, dd, J=9.6, 1.8 Hz), 7.44 (1H, t, J=7.8 Hz), 7.55 (2H, d, J=8.3 Hz), 7.66 (2H, d, J=8.3 Hz).

Step 3: (1R*,2S*)-2-azido-1-(4-chloro-3-fluorophenyl)-2-[4-(trifluoromethyl)phenyl]propan-1-ol

Sodium azide (4.90 g, 75.4 mmol), ammonium chloride (2.10 g, 39.3 mmol), and water (40 ml) were added to an ethanol (120 ml) solution of the compound (4.97 g, 15.0 mmol) obtained in Step 2 above and the resulting mixture was heated to reflux for 72 hours. The reaction mixture was left standing to cool and then concentrated under reduced pressure. The residue was diluted with water, followed by extraction with ethyl acetate. The organic layer was washed with brine and then dried over anhydrous sodium sulfate. The solvent was evaporated under reduced pressure and the residue was purified by silica gel
column chromatography [n-hexane:ethyl acetate = 5:1
(v/v)] to give the title compound (4.20 g, 75%) as a pale yellow oil.
$^{1}$H-NMR (400 MHz, $CDCl_3$) δ: 1.79 (3H, s), 2.30 (1H, d, J=3.9 Hz), 4.73 (1H, d, J=3.9 Hz), 6.78 (1H, d, J=8.3 Hz), 6.95 (1H, dd, J=10.1, 1.8 Hz), 7.23 (1H, d, J=7.8 Hz), 7.42 (2H, d, J=8.3 Hz), 7.60 (2H, d, J=8.3 Hz).

Step 4: (1R*,2S*)-2-azido-1-(4-chloro-3-fluorophenyl)-2-[4-(trifluoromethyl)phenyl]propylmethanesulfonate

The compound obtained in Step 3 above was reacted in the same way as in Step 1 of Reference Example 3 to give the title compound as a colorless oil.
$^{1}$H-NMR (400 MHz, $CDCl_3$) δ: 1.92 (3H, s), 2.71 (3H, s), 5.47 (1H, s), 6.74 (1H, dd, J=8.2, 1.8 Hz), 6.99 (1H, dd, J=9.5, 2.0 Hz), 7.26 (1H, t, J=7.8 Hz), 7.42 (2H, d, J=8.3 Hz), 7.61 (2H, d, J=8.3 Hz).

Step 5: (2R*,3R*)-3-(4-chloro-3-fluorophenyl)-2-methyl-2-[4-(trifluoromethyl)phenyl]aziridine

Lithium aluminum hydride (0.61 g, 16.2 mmol) was gradually added to a tetrahydrofuran solution (80 ml) of the compound (3.65 g, 8.08 mmol) obtained in Step 4 above under ice cooling. The resulting mixture was stirred for 1 hour and then sodium sulfate decahydrate (5.25 g) was gradually added. Insoluble matter was removed by filtration and then the filtrate was concentrated under reduced pressure to give the title compound (2.71 g, 99%) as a yellow oil.
$^{1}$H-NMR (400 MHz, $CDCl_3$) δ: 1.29 (3H, brs), 1.44 (1H, brs), 3.32 (1H, brs), 7.13-7.14 (1H, m), 7.19-7.24 (1H, m), 7.39 (1H, t, J=7.8 Hz), 7.51 (2H, d, J=8.3 Hz), 7.64 (2H, d, J=8.3 Hz).

Step 6: (1R*,2S*)-2-azido-1-(4-chloro-3-fluorophenyl)-2-[4-(trifluoromethyl)phenyl]propane-1-amine

Sodium azide (2.67 g, 41.0 mmol), ammonium chloride (1.11 g, 21.0 mmol), and water (20 ml) were added to an ethanol (60 ml) solution of the compound (2.71 g, 8.21 mmol) obtained in Step 5 above and the resulting mixture was heated to reflux for 21 hours. The reaction mixture was left standing to cool and then concentrated under reduced

pressure. The residue was diluted with water, followed by extraction with ethyl acetate. The organic layer was washed with brine and then dried over anhydrous sodium sulfate. The solvent was evaporated under reduced pressure and the residue was purified by silica gel column chromatography [n-hexane:ethyl acetate = 2:1 (v/v)] to give the title compound (1.64 g, 54%) as a colorless oil.
$^1$H-NMR (400 MHz, $CDCl_3$) δ: 1.77 (3H, s), 4.11 (1H, s), 6.84 (1H, dd, J=8.3, 1.7 Hz), 7.00 (1H, dd, J=10.3, 2.0 Hz), 7.24 (1H, t, J=7.9 Hz), 7.43 (2H, d, J=8.3 Hz), 7.61 (2H, d, J=8.3 Hz).

Step 7: (1R*,2S*)-1-(4-chloro-3-fluorophenyl)-2-[4-(trifluoromethyl)phenyl]propane-1,2-diamine

Lithium aluminum hydride (0.34 g, 8.80 mmol) was gradually added to a tetrahydrofuran solution (30 ml) of the compound (1.64 g, 4.40 mmol) obtained in Step 6 above under ice cooling. The resulting mixture was stirred for 30 minutes and then stirred after addition of sodium sulfate decahydrate. Insoluble matter was removed by filtration and then the filtrate was concentrated under reduced pressure to give the title compound (1.47 g, 96%) as a pale yellow oil.
$^1$H-NMR (400 MHz, $CDCl_3$) δ: 1.54 (3H, s), 4.12 (1H, s), 6.73 (1H, dd, J=8.2, 1.8 Hz), 6.93 (1H, dd, J=10.2, 2.0 Hz), 7.19 (1H, t, J=7.9 Hz), 7.47 (2H, d, J=8.3 Hz), 7.55 (2H, d, J=8.3 Hz).

Step 8: (1R,2S)-1-(4-chloro-3-fluorophenyl)-2-[4-(trifluoromethyl)phenyl]propane-1,2-diamine

The same procedures as in Step 1 of Example 1 were performed using the compound obtained in Step 7 above to give the title compound as a colorless oil.

Step 9: (4S,5R)-5-(4-chloro-3-fluorophenyl)-4-methyl-4-[4-(trifluoromethyl)phenyl]imidazolidine-2-thione

The compound obtained in Step 8 above was reacted in the same way as in Step 2 of Example 1 to give the title compound as a colorless solid.
$^1$H-NMR (400 MHz, $CDCl_3$) δ: 1.94 (3H, s), 4.98 (1H, s), 6.33 (1H, brs), 6.59 (1H, brs), 6.62 (1H, dd, J=8.3, 1.5 Hz), 6.69 (1H, dd, J=9.5, 2.0 Hz), 7.10 (2H, d, J=8.5 Hz), 7.14 (1H, t, J=8.0 Hz), 7.43 (2H, d, J=8.5 Hz).

Step 10: (5R,6S)-5-(4-chloro-3-fluorophenyl)-3-isopropyl-6-methyl-6-[4-(trifluoromethyl)phenyl]-5,6-dihydroimidazo [2,1-b][1,3]thiazole-2-carboxylic acid

The compound obtained in Step 9 above was reacted in the same way as in Step 3 of Example 1 and then reacted in the same way as in Step 4 of Example 1 to give the title compound as a colorless solid.
$^1$H-NMR (400 MHz, DMSO-$d_6$, 70°C) δ: 0.96 (3H, d, J=7.1 Hz), 1.01 (3H, d, J=7.3 Hz), 1.97 (3H, s), 3.32-3.34 (1H, m), 6.03 (1H, s), 6.81 (1H, brs), 7.03 (1H, brs), 7.31 (1H, t, J=8.3 Hz), 7.46 (2H, d, J=9.0 Hz), 7.49 (2H, d, J=9.0 Hz).

Reference Example 39

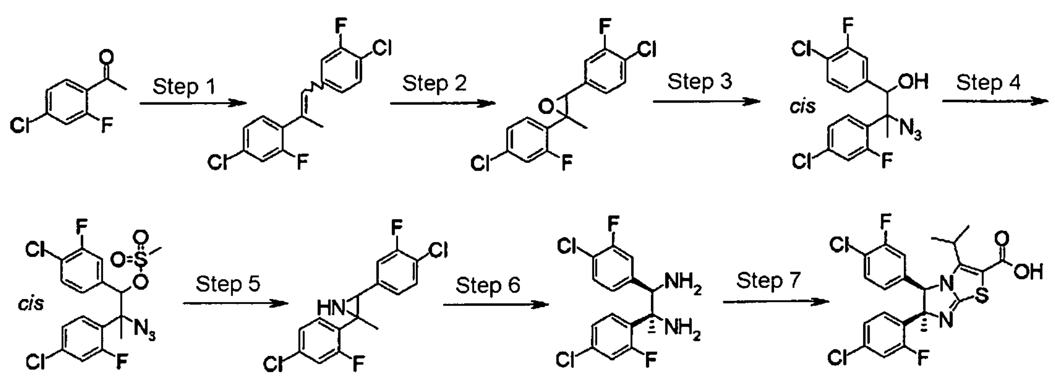

Step 1: 4-chloro-1-[(E)-2-(4-chloro-3-fluorophenyl)-1-methylvinyl]-2-fluorobenzene and 4-chloro-1-[(Z)-2-(4-chloro-3-fluorophenyl)-1-methylvinyl]-2-fluorobenzene

1-(4-chloro-2-fluorophenyl)ethanone instead of 1-(4-chlorophenyl)ethanone was reacted in the same way as in Step 1 of Example 5 to give the title mixture as a colorless oil.
$^1$H-NMR (400 MHz, $CDCl_3$) δ: 2.17 and 2.22 (total 3H, each s), 6.50 and 6.54 (total 1H, each s), 6.64 (0.5H, dd, J=8.3, 1.5 Hz), 6.69 (0.5H, dd, J=10.6, 1.8 Hz), 7.00 (0.5H, t, J=8.0 Hz), 7.07-7.17 (total 4H, m), 7.27-7.38 (1H, m).

Step 2: (2R*,3R*)-2-(4-chloro-2-fluorophenyl)-3-(4-chloro-3-fluorophenyl)-2-methyloxirane

The mixture of stereoisomers obtained in Step 1 above was reacted in the same way as in Step 2 of Reference Example 38 and then the mixture of stereoisomers obtained was resolved by silica gel column chromatography [n-hexane:chloroform = 10:1 (v/v)] to give the title compound as a colorless oil.
$^1$H-NMR (400 MHz, $CDCl_3$) δ: 1.35 (3H, s), 3.93 (1H, s), 7.10-7.19 (4H, m), 7.42 (2H, t, J=8.2 Hz).

Step 3: (1R*,2S*)-2-azido-2-(4-chloro-2-fluorophenyl)-1-(4-chloro-3-fluorophenyl)propan-1-ol

The compound obtained in Step 2 above was reacted in the same way as in Step 3 of Reference Example 38 to give the title compound as a colorless oil.
$^1$H-NMR (400 MHz, $CDCl_3$) δ: 1.94 (3H, d, J=1.2 Hz), 2.40 (1H, d, J=4.6 Hz), 5.11 (1H, d, J=4.6 Hz), 6.77 (1H, d, J=8.3 Hz), 6.99-7.02 (2H, m), 7.08 (1H, dd, J=12.0, 2.0 Hz), 7.12-7.19 (2H, m).

Step 4: (1R*,2S*)-2-azido-2-(4-chloro-2-fluorophenyl)-1-(4-chloro-3-fluorophenyl)propylmethanesulfonate

The compound obtained in Step 3 above was reacted in the same way as in Step 1 of Reference Example 3 to give the title compound as a pale yellow oil.
$^1$H-NMR (400 MHz, $CDCl_3$) δ: 2.02 (3H, d, J=1.5 Hz), 2.84 (3H, s), 5.93 (1H, s), 6.87 (1H, dd, J=8.3, 1.7 Hz), 7.02 (1H, dd, J=8.3, 2.2 Hz), 7.12 (1H, dd, J=4.4, 2.0 Hz), 7.14 (1H, t, J=2.0 Hz), 7.19 (1H, t, J=8.3 Hz), 7.24 (1H, dd, J=8.3, 7.6 Hz).

Step 5: (2R*, 3R*)-2-(4-chloro-2-fluorophenyl)-3-(4-chloro-3-fluorophenyl)-2-methylaziridine

The compound obtained in Step 4 above was reacted in the same way as in Step 5 of Reference Example 38 to give the title compound as a colorless solid.
$^1$H-NMR (400 MHz, $CDCl_3$) δ: 1.14 (3H, brs), 1.43 (1H, brs), 3.32 (1H, brs), 7.12-7.40 (6H, m).

Step 6: (1R,2S)-2-(4-chloro-2-fluorophenyl)-1-(4-chloro-3-fluorophenyl)propane-1,2-diamine

The compound obtained in Step 5 above was reacted in the same way as in Step 6 to Step 8 of Reference Example 38 in order to give the title compound as a colorless oil. $^1$H-NMR (400 MHz, $CDCl_3$) δ: 1.57 (3H, d, J=1.7 Hz), 4.46 (1H, s), 6.74 (1H, dd, J=8.4, 1.8 Hz), 6.97 (1H, d, J=2.2 Hz), 6.99 (1H, t, J=2.2 Hz), 7.05-7.16 (3H, m).

Step 7: (5R,6S)-6-(4-chloro-2-fluorophenyl)-5-(4-chloro-3-fluorophenyl)-3-isopropyl-6-methyl-5,6-dihydroimidazo [2,1-b][1,3]thiazole-2-carboxylic acid

The compound obtained in Step 6 above was reacted in the same way as in Step 2 to Step 4 of Example 1 in order to give the title compound as a pale yellow solid.
$^1$H-NMR (400 MHz, $CDCl_3$) δ: 0.99 (3H, d, J=7.3 Hz), 1.05 (3H, d, J=7.3 Hz), 1.92 (3H, s), 3.43-3.50 (1H, m), 5.45 (1H, d, J=3.2 Hz), 6.71 (1H, brs), 6.79 (1H, brs), 6.82 (1H, dd, J=11.2, 2.0 Hz), 6.99 (1H, dd, J=8.3, 2.0 Hz), 7.17 (1H, t, J=7.9 Hz), 7.58 (1H, t, J=8.3 Hz).

Reference Example 40

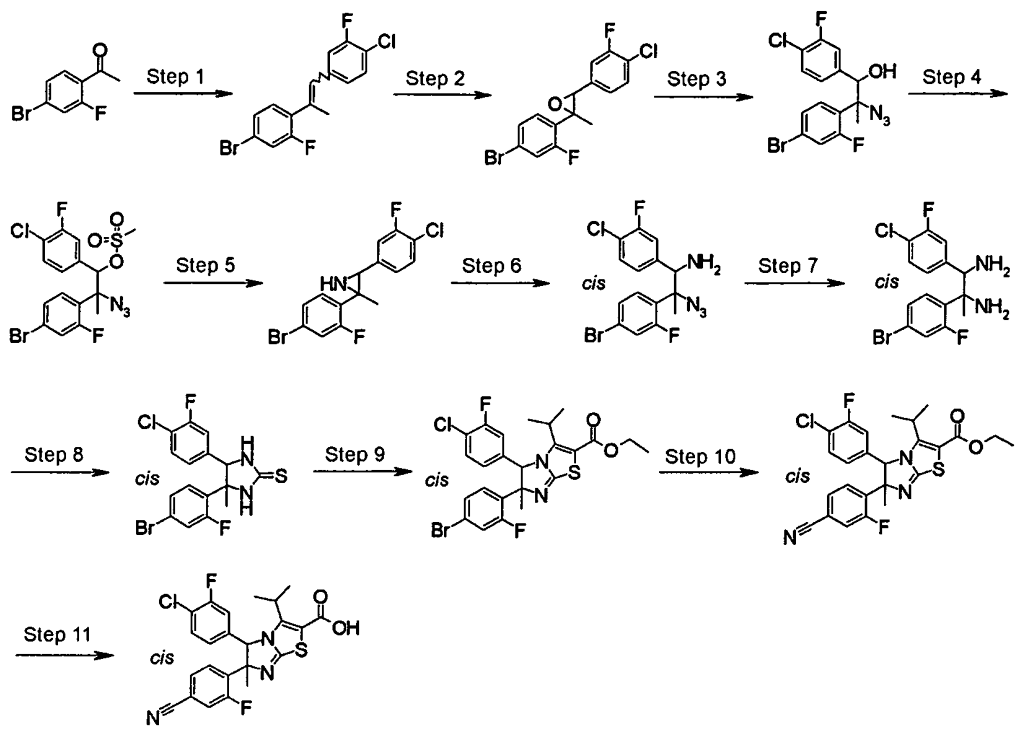


Step 1: 4-bromo-1-[(E)-2-(4-chloro-3-fluorophenyl)-1-methylvinyl]-2-fluorobenzene and 4-bromo-1-[(Z)-2-(4-chloro-3-fluorophenyl)-1-methylvinyl]-2-fluorobenzene

1-(4-bromo-2-fluorophenyl)ethanone instead of 1-[4-(trifluoromethyl)phenyl]ethanone was reacted in the same way as in Step 1 of Reference Example 38 to give the title mixture as a yellow oil.
$^1$H-NMR (400 MHz, $CDCl_3$) δ: 2.15 and 2.13 (total 3H, each d, J=1.5 Hz), 6.49 and 6.55 (total 1H, each d, J=1.2 Hz), 6.63 (1H, dd, J=8.3, 2.0 Hz), 6.68 (1H, dd, J=10.5, 2.0 Hz), 6.93 (1H, t, J=8.1 Hz), 7.07-7.417 (total 3H, m).

Step 2: (2R*,3R*)-2-(4-bromo-2-fluorophenyl)-3-(4-chloro-3-fluorophenyl)-2-methyloxirane

The mixture of stereoisomers obtained in Step 1 above was reacted in the same way as in Step 2 of Reference Example 38 and then the mixture of stereoisomers obtained was resolved by silica gel column chromatography [n-hexane:chloroform = 10:1 (v/v)] to give the title compound as a colorless oil.
$^1$H-NMR (400 MHz, $CDCl_3$) δ: 1.37 (3H, d, J=1.0 Hz), 3.95 (1H, s), 7.14 (1H, dd, J=8.3, 2.0 Hz), 7.19 (1H, dd, J=9.6, 1.8 Hz), 7.27-7.40 (3H, m), 7.44 (1H, t, J=7.8 Hz).

Step 3: (1R*,2S*)-2-azido-2-(4-bromo-2-fluorophenyl)-1-(4-chloro-3-fluorophenyl)propan-1-ol

The compound obtained in Step 2 above was reacted in the same way as in Step 3 of Reference Example 38 to give the title compound as a yellow oil.
$^1$H-NMR (400 MHz, $CDCl_3$) δ: 1.95 (3H, d, J=1.5 Hz), 2.41 (1H, d, J=4.6 Hz), 5.12 (1H, d, J=4.6 Hz), 6.78 (1H, d, J=8.3 Hz), 7.02-7.26 (5H, m).

Step 4: (1R*,2S*)-2-azido-2-(4-bromo-2-fluorophenyl)-1-(4-chloro-3-fluorophenyl)propylmethanesulfonate

The compound obtained in Step 3 above was reacted in the same way as in Step 1 of Reference Example 3 to give the title compound as a colorless oil.
$^1$H-NMR (400 MHz, $CDCl_3$) δ: 2.00 (3H, d, J=1.0 Hz), 2.82 (3H, s), 5.92 (1H, s), 6.86 (1H, dd, J=8.3, 1.7 Hz), 7.09-7.17 (3H, m), 7.21-7.28 (2H, m).

Step 5: (2R*,3R*)-2-(4-bromo-2-fluorophenyl)-3-(4-chloro-3-fluorophenyl)-2-methylaziridine

The compound obtained in Step 4 above was reacted in the same way as in Step 5 of Reference Example 38 to give the title compound as a pale yellow oil.
MS (ESI) m/z: 358, 360 [$(M+H)^+$] .

Step 6: (1R*,2S*)-2-azido-2-(4-bromo-2-fluorophenyl)-1-(4-chloro-3-fluorophenyl)propane-1-amine

The compound obtained in Step 5 above was reacted in the same way as in Step 6 of Reference Example 38 to give the title compound as a colorless solid.
$^1$H-NMR (400 MHz, $CDCl_3$) δ: 1.91 (3H, d, J=1.7 Hz), 4.36 (1H, s), 6.78 (1H, dd, J=7.9, 1.8 Hz), 6.96-7.07 (2H, m), 7.09-7.16 (2H, m), 7.21 (1H, dd, J=11.5, 2.0 Hz).

Step 7: (1R*,2S*)-2-(4-bromo-2-fluorophenyl)-1-(4-chloro-3-fluorophenyl)propane-1,2-diamine

The compound obtained in Step 6 above was reacted in the same way as in Step 7 of Reference Example 38 to give the title compound as a yellow oil.
$^1$H-NMR (400 MHz, $CDCl_3$) δ: 1.58 (3H, d, J=1.7 Hz), 4.47 (1H, s), 6.74 (1H, dd, J=8.3, 2.0 Hz), 6.94-7.08 (2H, m), 7.13-7.16 (2H, m), 7.22 (1H, dd, J=11.7, 2.0 Hz).
MS (ESI) m/z: 375, 377 [$(M+H)^+$] .

Step 8: (4R*,5S*)-4-(4-bromo-2-fluorophenyl)-5-(4-chloro-3-fluorophenyl)-4-methylimidazolidine-2-thione

The compound obtained in Step 7 above was reacted in the same way as in Step 2 of Example 1 to give the title compound as a pale yellow solid.
$^1$H-NMR (400 MHz, $CDCl_3$) δ: 1.93 (3H, d, J=1.5 Hz), 4.98 (1H, d, J=2.7 Hz), 6.52 (1H, brs), 6.62 (1H, brs), 6.77-6.90 (3H, m), 6.99-7.03 (1H, m), 7.09-7.26 (2H, m).

Step 9: ethyl (5R*,6S*)-6-(4-bromo-2-fluorophenyl)-5-(4-chloro-3-fluorophenyl)-3-isopropyl-6-methyl-5,6-dihydroimidazo[2,1-b] [1,3]thiazole-2-carboxylate

The compound obtained in Step 8 above was reacted in the same way as in Step 3 of Example 1 to give the title compound as a pale yellow solid.
$^1$H-NMR (400 MHz, $CDCl_3$) δ: 0.91 (3H, d, J=7.1 Hz), 1.00 (3H, d, J=7.1 Hz), 1.33 (3H, t, J=7.1 Hz), 1.75 (4H, s), 3.38-3.41 (1H, m), 4.25 (2H, q, J=7.1 Hz), 5.23 (1H, d, J=3.4 Hz), 6.72-6.75 (2H, m), 6.94-6.96 (1H, m), 7.03-7.13 (2H, m), 7.58 (1H, t, J=8.3 Hz).

Step 10: ethyl (5R*,6S*)-5-(4-chloro-3-fluorophenyl)-6-(4-cyano-2-fluorophenyl)-3-isopropyl-6-methyl-5,6-dihydroimidazo[2,1-b][1,3]thiazole-2-carboxylate

Copper cyanide (4.25 g, 47.5 mmol) was added to a 1-methyl-2-pyrrolidone(100 ml) solution of the compound (13.2 g, 23.8 mmol) obtained in Step 9 above and the resulting mixture was heated at 140˚C for 30 hours in a nitrogen atmosphere. The reaction mixture was left standing to cool and then the reaction mixture was added to ammonia water, followed by extraction with ethyl acetate. The organic layer was washed with brine and then dried over anhydrous sodium sulfate. The solvent was evaporated under reduced pressure and the residue was purified by silica gel column chromatography [n-hexane:ethyl acetate = 5:1 (v/v)] to give the title compound (8.68 g, 73%) as a pale yellow solid.
$^1$H-NMR (400 MHz, $CDCl_3$) δ: 0.91 (3H, d, J=7.1 Hz), 1.00 (3H, d, J=7.1 Hz), 1.33 (3H, t, J=7.2 Hz), 1.78 (3H, d, J=1.2 Hz), 3.38-3.40 (1H, m), 4.25 (2H, q, J=7.2 Hz), 5.26 (1H, d, J=3.2 Hz), 6.74 (2H, brs), 7.06 (1H, d, J=10.5 Hz), 7.13 (1H, brs), 7.30 (1H, dd, J=8.1, 1.5 Hz), 7.87 (1H, t, J=7.8 Hz).

Step 11: (5R*,6S*)-5-(4-chloro-3-fluorophenyl)-6-(4-cyano-2-fluorophenyl)-3-isopropyl-6-methyl-5,6-dihydroimidazo [2,1-b][1,3]thiazole-2-carboxylic acid

The compound obtained in Step 10 above was reacted in the same way as in Step 4 of Example 1 to give the title compound as a colorless solid.
$^1$H-NMR (400 MHz, DMSO-$d_6$) δ: 0.86 (3H, d, J=7.1 Hz), 0.98 (3H, d, J=7.1 Hz), 1.72 (3H, s), 3.26-3.30 (1H, m), 5.72 (1H, d, J=2.9 Hz), 6.83 (1H, brs), 7.09 (1H, brs), 7.33-7.35 (1H, m), 7.48-7.50 (2H, m), 7.74-7.82 (1H, m).

(Test Example 1 Mdm2/p53 binding assay)

A protein dilution containing 6.25 nM each of His-p53 (fusion protein of a p53 partial protein having p53 amino acids at positions 1 to 132, with a histidine protein) and GST-Mdm2 (fusion protein of a Mdm2 partial protein, having Mdm2 amino acids at positions 25 to 108 with leucine residue 33 substituted by glutamic acid, with glutathione transferase) proteins was prepared using a protein buffer solution (20 mM HEPES pH 7.4, 150 mM NaCl, 0.1% BSA). This protein dilution was added in an amount of 8 μL/well to a 384-well plate (384-well low volume NBC, Corning Inc., catalog No: 3676).
Next, a test compound was diluted with protein buffer solution containing 10% DMSO, and this test compound dilution was added in an amount of 4 μL/well to the plate.
Subsequently, a dilution containing an XL665-labeled anti-His antibody (HTRF monoclonal anti-6HIS antibody labeled with XL665 (catalog No: 61HISXL), Schering-Plough Corp.) and a europium (Eu)-labeled anti-GST antibody (HTRF monoclonal anti-GST antibody labeled with europium cryptate, Schering-Plough Corp., catalog No: 61GSTKL) at concentrations of 2.5 μg/mL and 0.325 μg/mL, respectively, was prepared using an antibody diluting buffer solution (20 mM HEPES pH 7.4, 150 mM NaCl, 0.1% BSA, 0.5 M KF). These dilutions were added in an amount of 8 μL/well (total reaction solution volume: 20 μl/well). Then, the plate was left at 25°C for 1 hour.
Time-resolved fluorescence at 620 and 665 nm was measured at an excitation wavelength of 320 nm using a plate reader (ARVOsx, PerkinElmer Co., Ltd.). Ratio (R) was calculated using the measured values (cps 620 nm and cps 665 nm) according to the following formula:

$$R = (\text{cps } 665 \text{ nm} - BI - C \times \text{cps } 620 \text{ nm}) / \text{cps } 620 \text{ nm}$$

BI: measured value at 665 nm of reaction solution (only each buffer solution) nonsupplemented with each protein, the compound, and the antibodies

$$C \text{ (correction factor)} = (A - BI)/D$$

A and D: each measured value at 665 nm and 620 nm of reaction solution supplemented with only Eu-labeled anti-GST antibody solution.
The R value calculated from the well supplemented with His-p53, GST-Mdm2, the test compound, and each antibody was defined as R (sample). The R value calculated from the well supplemented with His-p53, GST-Mdm2, and each antibody but without the test compound was defined as R (control). The R value calculated from the well supplemented with GST-Mdm2, the test compound, and each antibody but without His-p53 was defined as R (background). T/C was calculated from the formula shown below. An $IC_{50}$ value for Mdm2/p53 binding was calculated by sigmoid fitting using Prism (GraphPad Software). The results are shown in Table 1.
$$T/C = (R \text{ (sample)} - R \text{ (background)}) / (R \text{ (control)} - R \text{ (background)})$$

The compounds of Examples 1 to 80 exhibited Mdm2/p53 binding inhibiting activity of $IC_{50}$ (μM)<0.2.

(Test Example 2 Cell growth inhibition assay)

A cell growth inhibition assay was conducted using human lung cancer-derived cell line NCI-H460 having wild-type p53. The test was conducted on the compounds of Examples 1, 5, 8, 11 to 14, 16 to 26, 30 to 34, 38 to 41, 53 to 66, 69, 70, 72 to 74, and 77 to 79.
NCI-H460 cells were suspended in a medium (RPMI1640 medium containing 10% fetal bovine serum) and the suspension was inoculated in an amount of 500 cells/150 μL/well to a 96-well multiwell plate. A test compound was dissolved in DMSO and this solution was diluted with medium to prepare a sample solution (DMSO concentration: 1% or lower). On the next day of inoculation, medium containing DMSO nonsupplemented with the test compound (hereinafter, referred to as a DMSO dilution, DMSO concentration: 1% or lower) or the sample solution was added in an

amount of 50 μL/well. MTT assay was conducted immediately after addition of the sample solution or the DMSO dilution to the cells and after culture of the cells (after addition of the sample solution or the DMSO dilution) at 37˚C for 72 hours in a 5% $CO_2$ atmosphere. The MTT assay was conducted as shown below.

A 5 mg/mL MTT (3-(4,5-dimethylthiazol-2-yl)-2,5-diphenyltetrazolium bromide, Sigma-Aldrich Co., M-2128) solution was prepared using a phosphate buffer solution (Dulbecco's Phosphate-buffered Saline). This MTT solution was added in an amount of 20 μL/well. Then, the plate was cultured at 37˚C for 4 hours in a 5% $CO_2$ atmosphere. The plate was centrifuged at 1200 rpm for 5 minutes and then the culture supernatant was removed by aspiration using a dispenser. DMSO was added in an amount of 150 μL/well to dissolve generated formazan.

The plate was stirred using a plate mixer for uniform color development from each well. The absorbance of each well was measured under conditions of OD 540 nm and reference 660 nm using a plate reader (SpectraMax PLUS384, Molecular Devices, CA, USA).

The OD value measured immediately after addition of the sample solution was defined as S. The OD value measured 72 hours after addition of the sample solution was defined as T. The OD value measured 72 hours after addition of the DMSO dilution was defined as C. T/C (%) was determined at each concentration according to the calculation formula shown below to prepare a dose response curve, from which 50% growth inhibition concentration ($GI_{50}$ value) was calculated.

$$T/C\ (\%) = (T-S)/(C-S)\times 100$$

The compounds subjected to the test exhibited cell growth inhibiting effect of $GI_{50}$ (μM)<4.

(Test Example 3 Metabolic stability test)

100 μL of 100 mM phosphate buffer solution (pH 7.4) containing 3 μM test compound was added to 100 μL of reaction solution containing 100 mM phosphate buffer solution (pH 7.4), 30 mM glucose 6-phosphate, 10 mM $MgCl_2 \cdot 6H_2O$, 3 units/mL glucose 6-phosphate 1-dehydrogenase, and 0.3 to 1.5 mgP/mL human liver microsomes and the mixture was incubated at 37˚C for 20 minutes. Then, 70 μL of 100 mM phosphate buffer solution (pH 7.4) containing 3 mM $NADP^+$ was added and the mixture was incubated at 37˚C for 30 minutes to conduct a microsomal metabolism test. The metabolic stability was calculated according to the following formula:

$$T/C\ (\%) = (\text{concentration of test compound after addition of } NADP^+ \text{ and incubation for 30 minutes})/(\text{concentration of test compound before addition of } NADP^+)\times 100.$$

The compounds of Examples 1 to 3, 5 to 14, 16 to 28, 30 to 33, 38 to 42, 44, 45, 50, 52 to 65, and 68 to 74 exhibited metabolic stability of T/C%≥50 in the test using human-derived liver microsomes.

## Claims

**1.** A compound represented by general formula (1) or a salt thereof:

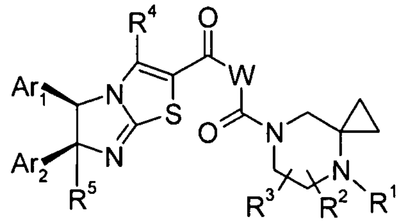

(1)

wherein

$Ar_1$ and $Ar_2$ each independently represent a phenyl group which may have a substituent(s) or a 5- or 6-membered aromatic heterocyclic group which may have a substituent(s);

$R^1$ represents a $C_1$-$C_6$ alkyl group which may have a substituent(s), a $C_2$-$C_6$ alkenyl group which may have a substituent(s), a $C_2$-$C_6$ alkynyl group which may have a substituent(s), an amino group which may have a substituent (s), a $C_1$-$C_6$ alkanoyl group which may have a substituent(s), a $C_2$-$C_6$ alkoxycarbonyl group which may have a substituent(s), a $C_2$-$C_6$ alkylaminocarbonyl group which may have a substituent(s), a $C_1$-$C_6$ alkylsulfonyl group which may have a substituent(s), a phenyl group which may have a substituent(s), an aralkyl group which may have a substituent(s), a 5- or 6-membered aromatic heterocyclic group which may have a substituent(s), a hydrogen atom, or a hydroxy group;

$R^2$ and $R^3$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), a phenyl group which may have a substituent(s), an aralkyl group which may have a substituent(s), a 5- or 6-membered aromatic heterocyclic group which may have a substituent(s), or a hydrogen atom, or $R^2$ and $R^3$ may together form an oxo group, or $R^2$ and $R^3$ together with the carbon atoms to which $R^2$ and $R^3$ are respectively bonded may form a 3- to 5-membered saturated hydrocarbon ring in a spiro or condensed form;

$R^4$ represents a $C_1$-$C_6$ alkyl group which may have a substituent(s);

$R^5$ represents a $C_1$-$C_6$ alkyl group which may have a substituent(s); and

W represents a group represented by the following general formula (2a) or (2b):

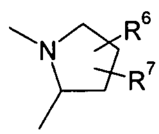

(2a)

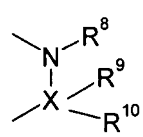

(2b)

wherein

in general formula (2a),

$R^6$ and $R^7$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), or a hydrogen atom, or $R^6$ and $R^7$ may together form an oxo group, or $R^6$ and $R^7$ together with the carbon atoms to which $R^6$ and $R^7$ are respectively bonded may form a 3- to 5-membered saturated hydrocarbon ring in a condensed form; and

in general formula (2b),

$R^8$ represents a $C_1$-$C_6$ alkyl group which may have a substituent(s), a $C_2$-$C_6$ alkenyl group which may have a substituent(s), or a $C_2$-$C_6$ alkynyl group which may have a substituent(s); X represents a $C_1$-$C_3$ alkylene group; and $R^9$ and $R^{10}$ each independently represent a $C_1$-$C_6$ alkyl group which may have a substituent(s), a $C_2$-$C_6$ alkenyl group which may have a substituent(s), a $C_2$-$C_6$ alkynyl group which may have a substituent(s), a phenyl group which may have a substituent(s), an aralkyl group which may have a substituent(s), a 5- or 6-membered aromatic heterocyclic group which may have a substituent(s), a $C_1$-$C_6$ alkoxy group which may have a substituent(s), an amino group which may have a substituent(s), a $C_1$-$C_6$ alkanoyl group which may have a substituent(s), a carbamoyl group which may have a substituent(s), a carbamoyloxy group which may have a substituent(s), a hydrogen atom, a halogen atom, a hydroxy group, or a cyano group, or $R^9$ and $R^{10}$ together with the carbon atoms to which $R^9$ and $R^{10}$ are respectively bonded may form a 3- to 5-membered saturated hydrocarbon ring in a spiro or condensed form.

**2.** A compound according to claim 1 or a salt thereof, wherein

$Ar_1$ and $Ar_2$ are each independently a phenyl group which may have one or more substituents selected from the following Group A, or a 5- or 6-membered aromatic heterocyclic group which may have one or more substituents selected from the following Group B;

$R^1$ is a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, an amino group which may have one or more substituents selected from the following Group D, a $C_1$-$C_6$ alkanoyl group which may have one or more substituents selected from the following Group E, a $C_1$-$C_6$ alkoxycarbonyl group which may have one or more substituents selected from the following Group E, a $C_1$-$C_6$ alkylaminocarbonyl group which may have one or more substituents selected from the following Group E, a $C_1$-$C_6$ alkylsulfonyl group which may have one or more substituents selected from the following Group E, a phenyl group which may have one or more substituents selected from the following Group A, an aralkyl group which may have one or more substituents selected from the following Group F, a 5- or 6-membered aromatic heterocyclic group which may have one or more substituents selected from the following Group B, a hydrogen atom, or a hydroxy group;

$R^2$ and $R^3$ are each independently a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, a phenyl group which may have one or more substituents selected from the following Group A, an aralkyl group which may have one or more substituents selected from the following Group F, a 5- or 6-membered aromatic heterocyclic group which may have one or more substituents selected from the following Group B, or a hydrogen atom, or $R^2$ and $R^3$ together form an oxo group, or $R^2$ and $R^3$ together with the carbon atoms to which $R^2$ and $R^3$ are respectively bonded form a 3- to 5-membered saturated hydrocarbon ring in a spiro or condensed form;

$R^4$ is a $C_1$-$C_6$ alkyl group which may have one or more substituents selected from the following Group C;

$R^5$ is a $C_1$-$C_6$ alkyl group which may have one or more substituents selected from the following Group C; and

W represents a group represented by the following general formula (2a) or (2b):

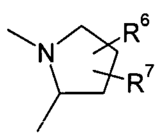

(2a)

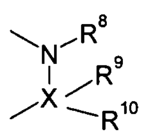

(2b)

wherein

in general formula (2a),

$R^6$ and $R^7$ are each independently a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, or a hydrogen atom, or $R^6$ and $R^7$ together form an oxo group, or $R^6$ and $R^7$ together with the carbon atoms to which $R^6$ and $R^7$ are respectively bonded form a 3- to 5-membered saturated hydrocarbon ring in a condensed form; and

in general formula (2b),

$R^8$ represents a $C_1$-$C_6$ alkyl group which may have one or more substituents selected from the following Group C, a $C_2$-$C_6$ alkenyl group which may have one or more substituents selected from the following Group C, or a $C_2$-$C_6$ alkynyl group which may have one or more substituents selected from the following Group C; X represents a $C_1$-$C_3$ alkylene group; $R^9$ and $R^{10}$ are each independently a $C_1$-$C_6$ alkyl group which may have one or more substituents selected from the following Group C, a $C_2$-$C_6$ alkenyl group which may have one or more substituents selected from the following Group C, a $C_2$-$C_6$ alkynyl group which may have one or more substituents selected from the following Group C, a phenyl group which may have one or more substituents selected from the following Group A, an aralkyl group which may have one or more substituents selected from the following Group F, a 5- or 6-membered aromatic heterocyclic group which may have one or more substituents selected from the following Group B, a $C_1$-$C_6$ alkoxy group which may have one or more substituents selected from the following Group G, an amino group which may have one or more substituents selected from the following Group D, a $C_1$-$C_6$ alkanoyl group which may have one or more substituents selected from the following Group E, a carbamoyl group which may have one or more substituents selected from the following Group H, a carbamoyloxy group which may have one or more substituents selected from the following Group H, a hydrogen atom, a halogen atom, a hydroxy group, or a cyano group, or $R^9$ and $R^{10}$

together with the carbon atoms to which $R^9$ and $R^{10}$ are respectively bonded form a 3- to 5-membered saturated hydrocarbon ring in a spiro or condensed form:
Group A: a halogen atom, a hydroxy group, a $C_1$-$C_6$ alkyl group, a $C_1$-$C_6$ alkoxy group, a carbamoyl group, an amino group, a $C_1$-$C_6$ alkanoyl group, and a cyano group;
Group B: a halogen atom, a $C_1$-$C_6$ alkyl group, an amino group, and a $C_1$-$C_6$ alkoxy group;
Group C: a halogen atom, a hydroxy group, a $C_1$-$C_6$ alkoxy group, a carbamoyl group, an amino group, a $C_1$-$C_6$ alkanoyl group, and a cyano group;
Group D: a $C_1$-$C_6$ alkyl group, a $C_1$-$C_6$ alkanoyl group, a $C_1$-$_C6$ alkylsulfonyl group, a $C_2$-$C_6$ alkoxycarbonyl group, a carbamoyl group, a $C_1$-$C_6$ alkylcarbamoyl group, and a di-$C_1$-$C_6$ alkylcarbamoyl group;
Group E: a halogen atom and a hydroxy group;
Group F: a halogen atom, a hydroxy group, a $C_1$-$C_6$ alkoxy group, an amino group, and a $C_1$-$C_6$ alkyl group;
Group G: a halogen atom; and
Group H: a $C_1$-$C_6$ alkyl group.

**3.** A compound according to claim 1 or 2 or a salt thereof, wherein $R^1$ is a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, a hydrogen atom, or a hydroxy group.

**4.** A compound according to any one of claims 1 to 3 or a salt thereof, wherein $R^2$ and $R^3$ are each independently a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, or a hydrogen atom.

**5.** A compound according to any one of claims 1 to 4 or a salt thereof, wherein $R^5$ is a $C_1$-$C_6$ alkyl group.

**6.** A compound according to any one of claims 1 to 5 or a salt thereof, wherein W is represented by the following general formula (2a) :

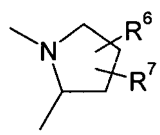

(2a)

wherein $R^6$ and $R^7$ are each independently a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, or a hydrogen atom.

**7.** A compound according to any one of claims 1 to 5 or a salt thereof, wherein W is represented by the following general formula (2b):

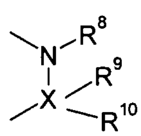

(2b)

wherein $R^8$ is a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group.

**8.** A compound according to any one of claims 1 to 5 and 7 or a salt thereof, wherein W is represented by the following

general formula (2b):

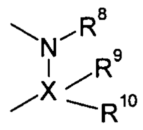

(2b)

wherein $R^9$ and $R^{10}$ are each independently a $C_1$-$C_6$ alkyl group which may have one or more substituents selected from a halogen atom, a hydroxy group, a $C_1$-$C_6$ alkoxy group, a carbamoyl group, an amino group, a $C_1$-$C_6$ alkanoyl group, and a cyano group, or a hydrogen atom, or $R^9$ and $R^{10}$ together with the carbon atoms to which $R^9$ and $R^{10}$ are respectively bonded form a cyclopropane ring bonded in a spiro or condensed form.

**9.** A compound according to any one of claims 1 to 8 or a salt thereof, wherein $Ar_1$ is a phenyl group which may have one or more substituents selected from a halogen atom, a hydroxy group, a $C_1$-$C_6$ alkyl group, a $C_1$-$C_6$ alkoxy group, a carbamoyl group, an amino group, a $C_1$-$C_6$ alkanoyl group, and a cyano group.

**10.** A compound according to any one of claims 1 to 9 or a salt thereof, wherein $Ar_2$ is a phenyl group which may have one or more substituents selected from a halogen atom, a hydroxy group, a $C_1$-$C_6$ alkyl group, a $C_1$-$C_6$ alkoxy group, a carbamoyl group, an amino group, a $C_1$-$C_6$ alkanoyl group, and a cyano group, or a 6-membered aromatic heterocyclic group which may have one or more substituents selected from a halogen atom, a $C_1$-$C_6$ alkyl group, an amino group, and a $C_1$-$C_6$ alkoxy group.

**11.** A compound according to any one of claims 1 to 10 or a salt thereof, wherein $R^4$ is a $C_1$-$C_6$ alkyl group.

**12.** An inhibitor of Mdm2 comprising a compound according to any one of claims 1 to 11 or a salt thereof.

**13.** An inhibitor of Mdm2 ubiquitin ligase comprising a compound according to any one of claims 1 to 11 or a salt thereof.

**14.** An inhibitor of p53-Mdm2 binding comprising a compound according to any one of claims 1 to 11 or a salt thereof.

**15.** An inhibitor of suppression of p53 transcription activity comprising a compound according to any one of claims 1 to 11 or a salt thereof.

**16.** An inhibitor of p53 degradation comprising a compound according to any one of claims 1 to 11 or a salt thereof.

**17.** A medicament comprising a compound according to any one of claims 1 to 11 or a salt thereof as an active ingredient.

**18.** An anti-tumor agent comprising a compound according to any one of claims 1 to 11 or a salt thereof as an active ingredient.

**19.** A pharmaceutical composition comprising a compound according to any one of claims 1 to 11 or a salt thereof and a pharmaceutically acceptable carrier.

**20.** A method for treating cancer, comprising administering a compound according to any one of claims 1 to 11 or a salt thereof.

**21.** Use of a compound according to any one of claims 1 to 11 or a salt thereof for the manufacture of a medicament.

**22.** Use of a compound according to any one of claims 1 to 11 or a salt thereof for the manufacture of an anti-tumor agent.

**INTERNATIONAL SEARCH REPORT**

International application No.
PCT/JP2009/060575

A. CLASSIFICATION OF SUBJECT MATTER
*C07D513/04*(2006.01)i, *A61K31/496*(2006.01)i, *A61P35/00*(2006.01)i, *A61P43/00*(2006.01)i

According to International Patent Classification (IPC) or to both national classification and IPC

B. FIELDS SEARCHED

Minimum documentation searched (classification system followed by classification symbols)
C07D513/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2006-508906 A (Cyclacel Ltd.), 16 March, 2006 (16.03.06), & WO 2004/005278 A1 & EP 1519932 A1 & US 2005/0215548 A1 & CA 2488816 A & CN 1665802 A & AT 374763 T | 1-19,21,22 |
| P,A | WO 2008/072655 A1 (Daiichi Sankyo Co., Ltd.), 19 June, 2008 (19.06.08) | 1-19,21,22 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 July, 2009 (16.07.09) | 28 July, 2009 (28.07.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/060575

**Box No. II Observations where certain claims were found unsearchable (Continuation of item 2 of first sheet)**

This international search report has not been established in respect of certain claims under Article 17(2)(a) for the following reasons:

1. [x] Claims Nos.: 20
because they relate to subject matter not required to be searched by this Authority, namely:
Claim 20 includes the methods for treatment of the human body or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
- [ ] The additional search fees were accompanied by the applicant's protest and, where applicable, payment of a protest fee.
- [ ] The additional search fees were accompanied by the applicant's protest but the applicable protest fee was not paid within the time limit specified in the invitation.
- [ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199949065 A
- WO 200015657 A
- WO 200624837 A
- WO 200480460 A
- WO 200351359 A
- WO 200351360 A
- WO 20053097 A
- WO 20052575 A
- WO 200511099 A
- WO 2005123691 A
- WO 200697261 A
- WO 200341715 A
- WO 200395625 A
- US 2004197893 A
- US 2004220179 A
- WO 200496134 A
- WO 200691646 A
- WO 200763013 A
- WO 200836168 A
- US 200526916 B
- JP 2000072743 A
- JP 2005075754 A
- US 20050026916 A
- US 2004259867 A
- US 2004259884 A
- WO 2005110996 A
- WO 200175145 A
- WO 200376608 A
- US 2001827292 A
- WO 9748706 A
- WO 200726920 A
- WO 2004039753 A
- WO 2008119744 A


### Non-patent literature cited in the description


- *Science,* 2004, vol. 303, 844-848
- *Proceedings of the National Academy of Sciences of the United States of America,* vol. 103, 1888-1893
- *Analytical Biochemistry,* 2004, vol. 331, 138-146
- *Bioorganic & Medicinal Chemistry Letters,* 2005, vol. 15, 765-770
- *Journal of Medicinal Chemistry,* 2005, vol. 48, 909-912
- *Chemical Biology & Drug Design,* 2005, vol. 67, 201-205
- *Bioorganic & Medicinal Chemistry Letters,* 2005, vol. 15, 1857-1861
- *Molecular Cancer Therapeutics,* 2006, vol. 5 (1), 160-169
- *Bioorganic & Medicinal Chemistry Letters,* 2006, vol. 16, 3115-3120
- *Bioorganic & Medicinal Chemistry Letters,* 2006, vol. 16, 3310-3314
- *Bioorganic & Medicinal Chemistry Letters,* 2006, vol. 16, 3463-3468
- *Journal of the American Chemical Society,* 2005, vol. 127, 10130-10131
- *Tetrahedron Letters,* 2005, vol. 46, 5949-5951
- *Journal of Medicinal Chemistry,* 2006, vol. 49, 3432-3435
- Development of Pharmaceutical Products. Molecule Design. Hirokawa-Shoten Ltd, 1990, vol. 7, 163-198
- *Synlett,* 1998, 623
- *Tetrahedron Lett.,* 2005, vol. 46, 4031
- *J. Am. Chem. Soc.,* 2002, vol. 124, 136672
- *J. Am. Chem. Soc.,* 2001, vol. 123, 7707
- *Synlett,* 2004, 525
- *Tetrahedron Asymmetry,* 1995, vol. 6, 3
- *Synlett,* 1998, 623-624
- *Tetrahedron Asymmetry,* 1995, vol. 6, 2199
- *J. Med. Chem.,* 2000, vol. 43, 4781
- *J. Med. Chem.,* 1998, vol. 31, 2004
- *Bioorg. Med. Chem. Lett.,* 2004, vol. 14, 3525